(19) Europäisches Patentamt
European Patent Office
Office européen des brevets

(11) EP 1 682 668 B1

(12) **EUROPEAN PATENT SPECIFICATION**

(45) Date of publication and mention of the grant of the patent:
**16.03.2011 Bulletin 2011/11**

(21) Application number: **04816948.6**

(22) Date of filing: **12.11.2004**

(51) Int Cl.:
*C12N 15/82* (2006.01)

(86) International application number:
**PCT/US2004/037584**

(87) International publication number:
**WO 2005/047516 (26.05.2005 Gazette 2005/21)**

(54) **PLANT TRANSCRIPTIONAL REGULATORS**

PFLANZLICHE TRANSKRIPTIONSREGULATOREN

REGULATEURS TRANSCRIPTIONNELS DE PLANTES

(84) Designated Contracting States:
**AT BE BG CH CY CZ DE DK EE ES FI FR GB GR HU IE IS IT LI LU MC NL PL PT RO SE SI SK TR**

(30) Priority: **13.11.2003 US 714887**
**05.12.2003 US 527658 P**
**05.02.2004 US 542928 P**

(43) Date of publication of application:
**26.07.2006 Bulletin 2006/30**

(73) Proprietor: **Mendel Biotechnology, Inc.**
**Hayward, CA 94545 (US)**

(72) Inventors:
- **HEARD, Jacqueline, E.**
  **Stonington, Connecticut 06378 (US)**
- **RIECHMANN, Jose Luis**
  **Pasadena, California 91101 (US)**
- **CREELMAN, Robert, A.**
  **Castro Valley, California 94546 (US)**
- **RATCLIFFE, Oliver, J.**
  **Oakland, California 94606 (US)**
- **CANALES, Roger, D.**
  **San Francisco, California 94112 (US)**
- **REPETTI, Peter**
  **Emeryville, California 94608 (US)**
- **KUMIMOTO, Roderick, W.**
  **San Bruno, California 94066 (US)**
- **GUTTERSON, Neal, I.**
  **Oakland, California 94618 (US)**
- **REUBER, T., Lynne**
  **San Mateo, California 94402 (US)**
- **PINEDA, Omaira**
  **Vero Beach, Florida 32960 (US)**
- **SHERMAN, Bradley, K.**
  **Berkeley, California 94708 (US)**
- **MORRISON, Tracy, A.**
  **San Leandro, CA 94579 (US)**
- **KEDDIE, James, S.**
  **San Mateo, California 94402 (US)**
- **JIANG, Cai-Zhong**
  **Fremont, California 94555 (US)**
- **CENTURY, Karen, S.**
  **Albany, California 94706 (US)**
- **ADAM, Luc**
  **Hayward, California 94545 (US)**
- **ZHANG, James, Z.**
  **Palo Alto, California 94303 (US)**
- **HEMPEL, Frederick, D.**
  **Hayward, California 94544 (US)**
- **LIBBY, Jeffrey, M.**
  **Cupertino, California 95014 (US)**

(74) Representative: **Brasnett, Adrian Hugh et al**
**Mewburn Ellis LLP**
**33 Gutter Lane**
**London**
**EC2V 8AS (GB)**

(56) References cited:
**WO-A-01/36598**
**WO-A-03/014327**
**WO-A-2004/076638**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Mendel Biotechnology, Inc.

<120> PLANT TRANSCRIPTIONAL REGULATORS

<130> AHB/FP6379341

<160> 64

<170> PatentIn version 3.2

<210> 1
<211> 785
<212> DNA
<213> Arabidopsis thaliana

<220>
<223> G47 reference sequence; clade identifier

<400> 1

```
cttcttcttc acatcgatca tcatacaaca acaaaaaatg gattacagag aatccaccgg      60
tgaaagtcag tcaaagtaca aaggaatccg tcgtcggaaa tggggcaaat gggtatcaga     120
gattagagtt ccgggaactc gtgaccgtct ctggttaggt tcattctcaa cagcagaagg     180
tgccgccgta gcacacgacg ttgctttctt ctgtttacac caacctgatt ctttagaatc     240
tctcaatttc cctcatttgc ttaatccttc actcgtttcc agaacttctc cgagatctat     300
ccagcaagct gcttctaacg ccggcatggc cattgacgcc ggaatcgtcc acagtaccag     360
cgtgaactct ggatgcggag atacgacgac gtattacgag aatggagctg atcaagtgga     420
gccgttgaat atttcagtgt atgattatct gggcggccac gatcacgttt gatttatctc     480
gacggtcatg atcacgtttg atcttctttt gagtaagatt ttgtaccata atcaaaacag     540
gtgtggtgct aaaatcttac tcaaaacaag attaggtacc acagagaaac aatcaaatgg     600
ttgtgaatat acattataag gttttgatta atgtttgttt cactgattta gtgaagtttg     660
gtccattgta tacaaatcta ttcaagaaac ctagcgcgag atcatgtttc gtgattgaag     720
attgagattt ttaagtattc gtaatatttt tgtaaaatac aaataaaaaa aaaaaaaaaa     780
aaaaa                                                                 785
```

<210> 2
<211> 144
<212> PRT
<213> Arabidopsis thaliana

<220>
<223> G47 polypeptide reference sequence; clade identifier

<400> 2

```
Met Asp Tyr Arg Glu Ser Thr Gly Glu Ser Gln Ser Lys Tyr Lys Gly
1               5                   10                  15
```

Ile Arg Arg Arg Lys Trp Gly Lys Trp Val Ser Glu Ile Arg Val Pro
20 25 30

Gly Thr Arg Asp Arg Leu Trp Leu Gly Ser Phe Ser Thr Ala Glu Gly
35 40 45

Ala Ala Val Ala His Asp Val Ala Phe Phe Cys Leu His Gln Pro Asp
50 55 60

Ser Leu Glu Ser Leu Asn Phe Pro His Leu Leu Asn Pro Ser Leu Val
65 70 75 80

Ser Arg Thr Ser Pro Arg Ser Ile Gln Gln Ala Ala Ser Asn Ala Gly
85 90 95

Met Ala Ile Asp Ala Gly Ile Val His Ser Thr Ser Val Asn Ser Gly
100 105 110

Cys Gly Asp Thr Thr Thr Tyr Tyr Glu Asn Gly Ala Asp Gln Val Glu
115 120 125

Pro Leu Asn Ile Ser Val Tyr Asp Tyr Leu Gly Gly His Asp His Val
130 135 140

<210> 3
<211> 1449
<212> DNA
<213> Arabidopsis thaliana

<220>
<223> G922 reference sequence; clade identifier

<400> 3

atggtggcta tgtttcaaga agataatgga acatcttctg tagcttcatc accacttcaa 60
gtcttctcaa ctatgtcact caacagaccg actctcctcg cttcttcatc tccgtttcat 120
tgtctcaaag atctcaaacc agaggagcgt ggtctctact taatccacct cttgctaact 180
tgtgccaacc acgtggcttc aggtagcctc caaaacgcta acgcagcgct cgagcagctc 240
tctcacctcg cttctcctga cggcgacacg atgcagcgaa tcgctgctta cttcaccgaa 300
gcgcttgcta acagaatcct taagtcctgg cctggtcttt acaaggctct taacgcaact 360
cagacaagaa ctaacaatgt ctctgaggag attcatgtta gaagactctt ctttgagatg 420
ttcccgatac tcaaagtctc ttacttgctc actaatcgag ctatactcga ggctatggaa 480
ggagagaaga tggttcatgt gattgatctc gatgcttctg agccagctca atggcttgct 540
ttgcttcaag cttttaactc taggcctgaa ggtccacctc atttgagaat cactggtgtt 600
catcaccaga aggaagtgct tgaacaaatg gctcatagac tcattgagga agcagagaaa 660
ctcgatatcc cgtttcagtt taatcccgtt gtgagtaggt tagactgttt aaatgtagaa 720

```
cagttgcggg ttaaaacagg agaggcctta gccgttagct cggttcttca attgcatacc    780
ttcttggcct ctgatgatga tctcatgaga aagaactgcg ctttacggtt tcagaacaac    840
cctagtggag ttgacttgca gagagttcta atgatgagcc atggctctgc agctgaggca    900
cgtgagaatg atatgagtaa caacaatggg tatagcccta gcggtgactc ggcctcatct    960
ttgcctttac caagttcagg aaggactgat agcttcctca atgctatttg gggtttgtct   1020
ccaaaggtca tggtggtcac tgagcaagac tcagaccaca acggctccac actaatggag   1080
aggctattag aatcacttta cacctacgca gcattgtttg attgcttgga aacaaaagtt   1140
ccaagaacgt ctcaagatag gatcaaagtg gagaagatgc tcttcgggga ggagatcaag   1200
aacatcatat cctgcgaggg atttgagaga agagaaagac acgagaagct tgagaaatgg   1260
agccagagga tcgatttggc tggttttggg aatgttcctc ttagctatta tgcgatgttg   1320
caggctagga gattgcttca agggtgcggt tttgatgggt atagaatcaa ggaagagagc   1380
gggtgcgcag taatttgctg gcaagatcga cctctatact cggtatcagc ttggagatgc   1440
aggaagtga                                                           1449
```

<210> 4
<211> 482
<212> PRT
<213> Arabidopsis thaliana

<220>
<223> G922 polypeptide reference sequence; clade identifier

<400> 4

```
Met Val Ala Met Phe Gln Glu Asp Asn Gly Thr Ser Ser Val Ala Ser
1               5                   10                  15

Ser Pro Leu Gln Val Phe Ser Thr Met Ser Leu Asn Arg Pro Thr Leu
            20                  25                  30

Leu Ala Ser Ser Ser Pro Phe His Cys Leu Lys Asp Leu Lys Pro Glu
        35                  40                  45

Glu Arg Gly Leu Tyr Leu Ile His Leu Leu Leu Thr Cys Ala Asn His
    50                  55                  60

Val Ala Ser Gly Ser Leu Gln Asn Ala Asn Ala Ala Leu Glu Gln Leu
65                  70                  75                  80

Ser His Leu Ala Ser Pro Asp Gly Asp Thr Met Gln Arg Ile Ala Ala
                85                  90                  95

Tyr Phe Thr Glu Ala Leu Ala Asn Arg Ile Leu Lys Ser Trp Pro Gly
            100                 105                 110
```

Leu Tyr Lys Ala Leu Asn Ala Thr Gln Thr Arg Thr Asn Asn Val Ser
115 120 125

Glu Glu Ile His Val Arg Arg Leu Phe Phe Glu Met Phe Pro Ile Leu
130 135 140

Lys Val Ser Tyr Leu Leu Thr Asn Arg Ala Ile Leu Glu Ala Met Glu
145 150 155 160

Gly Glu Lys Met Val His Val Ile Asp Leu Asp Ala Ser Glu Pro Ala
165 170 175

Gln Trp Leu Ala Leu Leu Gln Ala Phe Asn Ser Arg Pro Glu Gly Pro
180 185 190

Pro His Leu Arg Ile Thr Gly Val His His Gln Lys Glu Val Leu Glu
195 200 205

Gln Met Ala His Arg Leu Ile Glu Glu Ala Glu Lys Leu Asp Ile Pro
210 215 220

Phe Gln Phe Asn Pro Val Val Ser Arg Leu Asp Cys Leu Asn Val Glu
225 230 235 240

Gln Leu Arg Val Lys Thr Gly Glu Ala Leu Ala Val Ser Ser Val Leu
245 250 255

Gln Leu His Thr Phe Leu Ala Ser Asp Asp Asp Leu Met Arg Lys Asn
260 265 270

Cys Ala Leu Arg Phe Gln Asn Asn Pro Ser Gly Val Asp Leu Gln Arg
275 280 285

Val Leu Met Met Ser His Gly Ser Ala Ala Glu Ala Arg Glu Asn Asp
290 295 300

Met Ser Asn Asn Asn Gly Tyr Ser Pro Ser Gly Asp Ser Ala Ser Ser
305 310 315 320

Leu Pro Leu Pro Ser Ser Gly Arg Thr Asp Ser Phe Leu Asn Ala Ile
325 330 335

Trp Gly Leu Ser Pro Lys Val Met Val Val Thr Glu Gln Asp Ser Asp
340 345 350

His Asn Gly Ser Thr Leu Met Glu Arg Leu Leu Glu Ser Leu Tyr Thr

355 360 365

Tyr Ala Ala Leu Phe Asp Cys Leu Glu Thr Lys Val Pro Arg Thr Ser
370 375 380

Gln Asp Arg Ile Lys Val Glu Lys Met Leu Phe Gly Glu Glu Ile Lys
385 390 395 400

Asn Ile Ile Ser Cys Glu Gly Phe Glu Arg Arg Glu Arg His Glu Lys
405 410 415

Leu Glu Lys Trp Ser Gln Arg Ile Asp Leu Ala Gly Phe Gly Asn Val
420 425 430

Pro Leu Ser Tyr Tyr Ala Met Leu Gln Ala Arg Arg Leu Leu Gln Gly
435 440 445

Cys Gly Phe Asp Gly Tyr Arg Ile Lys Glu Glu Ser Gly Cys Ala Val
450 455 460

Ile Cys Trp Gln Asp Arg Pro Leu Tyr Ser Val Ser Ala Trp Arg Cys
465 470 475 480

Arg Lys

<210> 5
<211> 585
<212> DNA
<213> Arabidopsis thaliana

<220>
<223> G1274 reference sequence; clade identifier

<400> 5

atgaatatct ctcaaaaccc tagccctaat tttacgtact tctccgatga aaactttatt 60

aatccgttta tggataacaa cgatttctca aatttgatgt tctttgacat agatgaagga 120

ggtaacaatg gattaatcga ggaagagatc tcatctccga caagcatcgt ttcgtcggag 180

acatttaccg gggaaagcgg cggatccggc agcgcaacaa cgttgagtaa aaaggaatca 240

actaatagag gaagtaaaga gagtgatcag acgaaggaga cgggtcatcg agttgcattt 300

agaacgagat cgaagattga tgtgatggat gatggtttta aatggaggaa gtatggcaag 360

aaatctgtca aaaacaacat taacaagagg aattactaca aatgctcaag tgaaggttgc 420

tcggtgaaga agagggtaga gagagatggt gacgatgcag cttatgtaat tacaacatat 480

gaaggagtcc ataaccatga gagtctctct aatgtctatt acaatgaaat ggttttatct 540

tatgatcatg ataactggaa ccaacactct cttcttcgat cttaa 585

<210> 6
<211> 194
<212> PRT
<213> Arabidopsis thaliana

<220>
<223> G1274 polypeptide reference sequence; clade identifier

<400> 6

```
Met Asn Ile Ser Gln Asn Pro Ser Pro Asn Phe Thr Tyr Phe Ser Asp
1               5                   10                  15

Glu Asn Phe Ile Asn Pro Phe Met Asp Asn Asn Asp Phe Ser Asn Leu
            20                  25                  30

Met Phe Phe Asp Ile Asp Glu Gly Gly Asn Asn Gly Leu Ile Glu Glu
        35                  40                  45

Glu Ile Ser Ser Pro Thr Ser Ile Val Ser Ser Glu Thr Phe Thr Gly
    50                  55                  60

Glu Ser Gly Gly Ser Gly Ser Ala Thr Thr Leu Ser Lys Lys Glu Ser
65                  70                  75                  80

Thr Asn Arg Gly Ser Lys Glu Ser Asp Gln Thr Lys Glu Thr Gly His
                85                  90                  95

Arg Val Ala Phe Arg Thr Arg Ser Lys Ile Asp Val Met Asp Asp Gly
            100                 105                 110

Phe Lys Trp Arg Lys Tyr Gly Lys Lys Ser Val Lys Asn Asn Ile Asn
        115                 120                 125

Lys Arg Asn Tyr Tyr Lys Cys Ser Ser Glu Gly Cys Ser Val Lys Lys
    130                 135                 140

Arg Val Glu Arg Asp Gly Asp Asp Ala Ala Tyr Val Ile Thr Thr Tyr
145                 150                 155                 160

Glu Gly Val His Asn His Glu Ser Leu Ser Asn Val Tyr Tyr Asn Glu
                165                 170                 175

Met Val Leu Ser Tyr Asp His Asp Asn Trp Asn Gln His Ser Leu Leu
            180                 185                 190

Arg Ser
```

<210> 7
<211> 696
<212> DNA
<213> Arabidopsis thaliana

<220>
<223> G1792 reference sequence; clade identifier

<400> 7

```
aatccataga tctcttatta aataacagtg ctgaccaagc tcttacaaag caaaccaatc      60
tagaacacca aagttaatgg agagctcaaa caggagcagc aacaaccaat cacaagatga     120
caagcaagct cgtttccggg gagttcgaag aaggccttgg ggaaagtttg cagcagagat     180
tcgagacccg tcgagaaacg gtgcccgtct ttggctcggg acatttgaga ccgctgagga     240
ggcagcaagg gcttatgacc gagcagcctt taaccttagg ggtcatctcg ctatactcaa     300
cttccctaat gagtattatc cacgtatgga cgactactcg cttcgccctc cttatgcttc     360
ttcttcttcg tcgtcgtcat cgggttcaac ttctactaat gtgagtcgac aaaaccaaag     420
agaagttttc gagtttgagt atttggacga taaggttctt gaagaacttc ttgattcaga     480
agaaaggaag agataatcac gattagtttt gttttgatat tttatgtggc actgttgtgg     540
ctacctacgt gcattatgtg catgtatagg tcgcttgatt agtactttat aacatgcatg     600
ccacgaccat aaattgtaag agaagacgta ctttgcgttt tcatgaaata tgaatgttag     660
atggtttgag tacaaaaaaa aaaaaaaaaa aaaaaa                               696
```

<210> 8
<211> 139
<212> PRT
<213> Arabidopsis thaliana

<220>
<223> G1792 polypeptide reference sequence; clade identifier

<400> 8

```
Met Glu Ser Ser Asn Arg Ser Ser Asn Asn Gln Ser Gln Asp Asp Lys
1               5                   10                  15

Gln Ala Arg Phe Arg Gly Val Arg Arg Arg Pro Trp Gly Lys Phe Ala
            20                  25                  30

Ala Glu Ile Arg Asp Pro Ser Arg Asn Gly Ala Arg Leu Trp Leu Gly
        35                  40                  45

Thr Phe Glu Thr Ala Glu Glu Ala Ala Arg Ala Tyr Asp Arg Ala Ala
    50                  55                  60

Phe Asn Leu Arg Gly His Leu Ala Ile Leu Asn Phe Pro Asn Glu Tyr
65                  70                  75                  80
```

Tyr Pro Arg Met Asp Asp Tyr Ser Leu Arg Pro Pro Tyr Ala Ser Ser
85 90 95

Ser Ser Ser Ser Ser Ser Gly Ser Thr Ser Thr Asn Val Ser Arg Gln
100 105 110

Asn Gln Arg Glu Val Phe Glu Phe Glu Tyr Leu Asp Asp Lys Val Leu
115 120 125

Glu Glu Leu Leu Asp Ser Glu Glu Arg Lys Arg
130 135

<210> 9
<211> 1029
<212> DNA
<213> Arabidopsis thaliana

<220>
<223> G2053 reference sequence; clade identifier

<400> 9

```
atggagaatc cggtgggttt aagattccgt ccgaccgaca aggagatcgt cgtcgattac     60
ctccgaccaa aaaactccga tagggacacg agtcatgttg atcgagtcat tagcacagtc    120
actatccgta gcttcgaccc ttgggagtta ccttgccagt ctaggatcaa actgaaagat    180
gagtcttggt gtttcttcag ccctaaggag aacaaatatg gcagaggtga tcaacaaatt    240
agaaaaacga aatctggtta ctggaagatt actggcaaac caaagcctat cttgcgtaac    300
cgccaagaga tcggtgagaa aaaggttttg atgttttaca tgagtaagga acttggtgga    360
tccaaatccg actgggttat gcacgagtac catgctttct ctcctactca gatgatgatg    420
acatatacaa tatgtaaagt tatgtttaag ggtgacgtga gagagatttc ttcttcttct    480
gcttcttatg gtagtgaaat tgagcagagt cgtgactctt taatccctct tcttgtgaac    540
gattctgagg aagaagctca aatcgaggat gctataccaa tagaggaatg ggaaacatgg    600
ttgactgatg atggtgttga tgagcaggtg aatcatatta tgaatatgaa agatgatcgc    660
aacaaccaca ggcctcaaaa gccattgact ggtgtcttga ttgacgatag tagtgatgat    720
gatgatgatt ctgatttgct atctccaaca acaaattcta ttgaaaattc gagcacttgt    780
gatagttttg gtagctcaga ccaaatcaac ttagtgtcac taactcaaga ggtgagccaa    840
gctctgataa ccagtattga tacacccgag aagattaaga gtccttatga tgatgcacaa    900
gggactgggg ctggagggca aaaattgggt caagagactc gagagaagaa acgagctggt    960
ttctttcaca ggatgataca aatattcgtc aagaaaattc accaatgttc ttctatctca   1020
agaacataa                                                           1029
```

<210> 10
<211> 342
<212> PRT
<213> Arabidopsis thaliana

<220>
<223> G2053 polypeptide reference sequence; clade identifier

<400> 10

```
Met Glu Asn Pro Val Gly Leu Arg Phe Arg Pro Thr Asp Lys Glu Ile
1               5                   10                  15

Val Val Asp Tyr Leu Arg Pro Lys Asn Ser Asp Arg Asp Thr Ser His
            20                  25                  30

Val Asp Arg Val Ile Ser Thr Val Thr Ile Arg Ser Phe Asp Pro Trp
        35                  40                  45

Glu Leu Pro Cys Gln Ser Arg Ile Lys Leu Lys Asp Glu Ser Trp Cys
    50                  55                  60

Phe Phe Ser Pro Lys Glu Asn Lys Tyr Gly Arg Gly Asp Gln Gln Ile
65                  70                  75                  80

Arg Lys Thr Lys Ser Gly Tyr Trp Lys Ile Thr Gly Lys Pro Lys Pro
                85                  90                  95

Ile Leu Arg Asn Arg Gln Glu Ile Gly Glu Lys Lys Val Leu Met Phe
            100                 105                 110

Tyr Met Ser Lys Glu Leu Gly Gly Ser Lys Ser Asp Trp Val Met His
        115                 120                 125

Glu Tyr His Ala Phe Ser Pro Thr Gln Met Met Met Thr Tyr Thr Ile
    130                 135                 140

Cys Lys Val Met Phe Lys Gly Asp Val Arg Glu Ile Ser Ser Ser Ser
145                 150                 155                 160

Ala Ser Tyr Gly Ser Glu Ile Glu Gln Ser Arg Asp Ser Leu Ile Pro
                165                 170                 175

Leu Leu Val Asn Asp Ser Glu Glu Glu Ala Gln Ile Glu Asp Ala Ile
            180                 185                 190

Pro Ile Glu Glu Trp Glu Thr Trp Leu Thr Asp Asp Gly Val Asp Glu
        195                 200                 205

Gln Val Asn His Ile Met Asn Met Lys Asp Asp Arg Asn Asn His Arg
    210                 215                 220
```

Pro Gln Lys Pro Leu Thr Gly Val Leu Ile Asp Asp Ser Ser Asp Asp
225 230 235 240

Asp Asp Asp Ser Asp Leu Leu Ser Pro Thr Thr Asn Ser Ile Glu Asn
245 250 255

Ser Ser Thr Cys Asp Ser Phe Gly Ser Ser Asp Gln Ile Asn Leu Val
260 265 270

Ser Leu Thr Gln Glu Val Ser Gln Ala Leu Ile Thr Ser Ile Asp Thr
275 280 285

Pro Glu Lys Ile Lys Ser Pro Tyr Asp Asp Ala Gln Gly Thr Gly Ala
290 295 300

Gly Gly Gln Lys Leu Gly Gln Glu Thr Arg Glu Lys Lys Arg Ala Gly
305 310 315 320

Phe Phe His Arg Met Ile Gln Ile Phe Val Lys Lys Ile His Gln Cys
325 330 335

Ser Ser Ile Ser Arg Thr
340

<210> 11
<211> 485
<212> DNA
<213> Arabidopsis thaliana

<220>
<223> G2133 Predicted polypeptide sequence is paralogous to G47

<400> 11

atctcatctt catccaccca aaaacatgga ttcaagagac accggagaaa ctgaccagag 60
caagtacaaa ggtatccgtc gtcggaaatg gggaaaatgg gtatcagaga ttcgtgtccc 120
gggaactcgt caacgtctct ggttaggctc tttctccacc gcagaaggcg ctgccgtagc 180
ccacgacgtc gctttttact gcttgcaccg accatcttcc ctcgacgacg aatcttttaa 240
cttccctcac ttacttacaa cctccctcgc ctccaatata tctcctaagt ccatccaaaa 300
agctgcttcc gacgccggca tggccgtgga cgccggattc catggtgctg tgtctgggag 360
tggtggttgt gaagagagat cttccatggc gaatatggag gaggaggaca aacttagtat 420
ctccgtgtat gattatcttg aagacgatct cgtttgatct atacgagtac gtttttagca 480
gttaa 485

<210> 12
<211> 143
<212> PRT
<213> Arabidopsis thaliana

<220>
<223> G2133 polypeptide Paralogous to G47

<400> 12

```
Met Asp Ser Arg Asp Thr Gly Glu Thr Asp Gln Ser Lys Tyr Lys Gly
1               5                   10                  15

Ile Arg Arg Arg Lys Trp Gly Lys Trp Val Ser Glu Ile Arg Val Pro
            20                  25                  30

Gly Thr Arg Gln Arg Leu Trp Leu Gly Ser Phe Ser Thr Ala Glu Gly
        35                  40                  45

Ala Ala Val Ala His Asp Val Ala Phe Tyr Cys Leu His Arg Pro Ser
    50                  55                  60

Ser Leu Asp Asp Glu Ser Phe Asn Phe Pro His Leu Leu Thr Thr Ser
65                  70                  75                  80

Leu Ala Ser Asn Ile Ser Pro Lys Ser Ile Gln Lys Ala Ala Ser Asp
                85                  90                  95

Ala Gly Met Ala Val Asp Ala Gly Phe His Gly Ala Val Ser Gly Ser
            100                 105                 110

Gly Gly Cys Glu Glu Arg Ser Ser Met Ala Asn Met Glu Glu Glu Asp
        115                 120                 125

Lys Leu Ser Ile Ser Val Tyr Asp Tyr Leu Glu Asp Asp Leu Val
    130                 135                 140
```

<210> 13
<211> 891
<212> DNA
<213> Arabidopsis thaliana

<220>
<223> G2999 reference sequence: clade identifier

<400> 13

```
tattagggtt ttctttattc agaagcttca agatcagaaa agatggaagt tagagagaag      60
aaagatgaga aaatggagat gacaagaaga aaatcatcag ctctagatca tcatcgtctt     120
cctccgtaca cttactccca aaccgccaac aaagagaaac ccaccaccaa aagaaacggg     180
tcggacccgg atccggatcc ggatcttgat actaacccaa tttctatatc tcatgctcca     240
agatcatacg ctaggcctca aactacttct ccggggaagg caaggtatag agaatgccaa     300
```

```
aagaatcacg cggcgagctc cggtggacac gttgtcgacg gttgcggcga gtttatgtct    360
tccggggaag aaggcacggt ggagtctctt ctttgtgcag cttgtgattg ccaccggagc    420
ttccatagaa aagagatcga tggtttgttt gttgtcaatt tcaatagctt tggccattca    480
cagagaccac tcggaagccg ccacgtgtct ccgataatga tgagttttgg tggtggtggt    540
ggttgtgcgg cggagtcatc gacggaggat ctaaacaagt ttcatcaatc ttttagtggt    600
tatggagtgg atcagtttca tcattatcaa cccaagaaac ggtttaggac aaagtttaat    660
gaagaacaga aggagaagat gatggagttt gctgagaaga ttggttggag aatgacaaaa    720
ctagaagatg atgaagtgaa tcggttttgt cgtgagatta aagttaaaag acaagtgttt    780
aaagtttgga tgcacaacaa taaacaagca gcaaagaaga aggatttgta agagatttct    840
atatatatat atatactatt tttacttctc tttgttcaac attgctttaa c             891
```

<210> 14
<211> 262
<212> PRT
<213> Arabidopsis thaliana

<220>
<223> G2999 polypeptide reference sequence; clade identifier

<400> 14

```
Met Glu Val Arg Glu Lys Lys Asp Glu Lys Met Glu Met Thr Arg Arg
1               5                   10                  15

Lys Ser Ser Ala Leu Asp His His Arg Leu Pro Pro Tyr Thr Tyr Ser
            20                  25                  30

Gln Thr Ala Asn Lys Glu Lys Pro Thr Thr Lys Arg Asn Gly Ser Asp
        35                  40                  45

Pro Asp Pro Asp Pro Asp Leu Asp Thr Asn Pro Ile Ser Ile Ser His
    50                  55                  60

Ala Pro Arg Ser Tyr Ala Arg Pro Gln Thr Thr Ser Pro Gly Lys Ala
65                  70                  75                  80

Arg Tyr Arg Glu Cys Gln Lys Asn His Ala Ala Ser Ser Gly Gly His
                85                  90                  95

Val Val Asp Gly Cys Gly Glu Phe Met Ser Ser Gly Glu Glu Gly Thr
            100                 105                 110

Val Glu Ser Leu Leu Cys Ala Ala Cys Asp Cys His Arg Ser Phe His
        115                 120                 125
```

```
Arg Lys Glu Ile Asp Gly Leu Phe Val Val Asn Phe Asn Ser Phe Gly
    130                 135                 140

His Ser Gln Arg Pro Leu Gly Ser Arg His Val Ser Pro Ile Met Met
145                 150                 155                 160

Ser Phe Gly Gly Gly Gly Gly Cys Ala Ala Glu Ser Ser Thr Glu Asp
                165                 170                 175

Leu Asn Lys Phe His Gln Ser Phe Ser Gly Tyr Gly Val Asp Gln Phe
            180                 185                 190

His His Tyr Gln Pro Lys Lys Arg Phe Arg Thr Lys Phe Asn Glu Glu
        195                 200                 205

Gln Lys Glu Lys Met Met Glu Phe Ala Glu Lys Ile Gly Trp Arg Met
    210                 215                 220

Thr Lys Leu Glu Asp Asp Glu Val Asn Arg Phe Cys Arg Glu Ile Lys
225                 230                 235                 240

Val Lys Arg Gln Val Phe Lys Val Trp Met His Asn Asn Lys Gln Ala
                245                 250                 255

Ala Lys Lys Lys Asp Leu
            260
```

<210> 15
<211> 1621
<212> DNA
<213> Arabidopsis thaliana

<220>
<223> G3086 reference sequence; clade identifier

<400> 15

```
aagtttctct cacgttctct tttttaattt taatttctcg ccggaaacaa tctcatctcc      60
cggcgaacga aacttccggt gtggtactgc aaacggagaa aaaaataacc aaagaagaga     120
gaaactcaaa agctactaag atggaatcag aattccagca acatcacttc cttctccacg     180
atcatcaaca ccagagacca agaaactcag gattgattcg ttaccaatca gcaccaagtt     240
cgtacttttc gagtttcggt gaatcaatcg aagagttttt agatcgaccc acaagtcctg     300
aaactgagcg aatcttatct ggctttttac aaaccaccga cacaagcgac aacgttgata     360
gtttccttca ccatactttt aacagtgatg gaactgagaa gaaacctccg gaagttaaaa     420
cagaggacga agatgctgaa attccggtga ctgcgacggc gacggcgatg gaggttgttg     480
tttccggtga tggtgaaatc tcagtgaatc ctgaagtatc gattgggtat gtggcttcgg     540
```

```
tttcgaggaa taagagacca agagagaaag atgatcggac tccggtgaat aatctagctc     600
gtcataatag ttcaccggcc ggattatttt catccattga tgttgaaaca gcttatgcag     660
ctgtaatgaa aagtatggga ggttttggag gaagtaatgt gatgagtaca agcaatactg     720
aagcttcgtc tcttactcct agaagcaagt tacttcctcc tacttctaga gcgatgagtc     780
cgatctctga ggttgatgtt aaacccggtt tctcgtctag attgcctcct cggacgcttt     840
ccggtgggtt taatcgttct tttgggaatg aagggtctgc ttcttccaag cttacagctc     900
ttgctaggac ccaatctgga ggtctagatc aatacaaaac caaggatgag gattcagcaa     960
gtagacgtcc tcctttggca catcacatga gtttgcccaa gtctttatca gatattgaac    1020
agttactgtc agattctatc ccatgtaaga tcagagccaa gcggggttgt gcaactcatc    1080
ctcgaagcat agccgagagg gtgagaagaa ccaagatcag tgaaagaatg aggaagctgc    1140
aagaccttgt tccaaacatg gacacgcaaa caaacacagc agacatgttg gatcttgcgg    1200
ttcaatacat caaggacctg caagaacaag tgaaggcgct cgaagagagt cgggcaagat    1260
gtagatgctc tagtgcgtga aactagaagt gggagtatgc gcgagtgcta gccagggagg    1320
gagttgtgca tagaagtatc ggttcggcct ttggagaaaa gtcgaagata gcaaagtaga    1380
gaagagatca tgaacaaagc taaatttggt ggtggtggtg aaaagggttt ttgtaaagtt    1440
ggaacctttt tttggtaggg aagaaagtag caaggttgtg taatggtccg aactccaatg    1500
ctattgtatg ttcttacatc caaaaaaaag aaaagcagaa gagaagtgat gtacaatgag    1560
aagcttattt tattgtaact caagaataat tcaggtaatt agtttaaaaa aaaaaaaaaa    1620
a                                                                    1621
```

<210> 16
<211> 379
<212> PRT
<213> Arabidopsis thaliana

<220>
<223> G3086 polypeptide reference sequence; clade identifier

<400> 16

```
Met Glu Ser Glu Phe Gln Gln His His Phe Leu Leu His Asp His Gln
1               5                   10                  15

His Gln Arg Pro Arg Asn Ser Gly Leu Ile Arg Tyr Gln Ser Ala Pro
            20                  25                  30

Ser Ser Tyr Phe Ser Ser Phe Gly Glu Ser Ile Glu Glu Phe Leu Asp
        35                  40                  45

Arg Pro Thr Ser Pro Glu Thr Glu Arg Ile Leu Ser Gly Phe Leu Gln
```

50 55 60

Thr Thr Asp Thr Ser Asp Asn Val Asp Ser Phe Leu His His Thr Phe
65 70 75 80

Asn Ser Asp Gly Thr Glu Lys Lys Pro Pro Glu Val Lys Thr Glu Asp
85 90 95

Glu Asp Ala Glu Ile Pro Val Thr Ala Thr Ala Thr Ala Met Glu Val
100 105 110

Val Val Ser Gly Asp Gly Glu Ile Ser Val Asn Pro Glu Val Ser Ile
115 120 125

Gly Tyr Val Ala Ser Val Ser Arg Asn Lys Arg Pro Arg Glu Lys Asp
130 135 140

Asp Arg Thr Pro Val Asn Asn Leu Ala Arg His Asn Ser Ser Pro Ala
145 150 155 160

Gly Leu Phe Ser Ser Ile Asp Val Glu Thr Ala Tyr Ala Ala Val Met
165 170 175

Lys Ser Met Gly Gly Phe Gly Gly Ser Asn Val Met Ser Thr Ser Asn
180 185 190

Thr Glu Ala Ser Ser Leu Thr Pro Arg Ser Lys Leu Leu Pro Pro Thr
195 200 205

Ser Arg Ala Met Ser Pro Ile Ser Glu Val Asp Val Lys Pro Gly Phe
210 215 220

Ser Ser Arg Leu Pro Pro Arg Thr Leu Ser Gly Gly Phe Asn Arg Ser
225 230 235 240

Phe Gly Asn Glu Gly Ser Ala Ser Ser Lys Leu Thr Ala Leu Ala Arg
245 250 255

Thr Gln Ser Gly Gly Leu Asp Gln Tyr Lys Thr Lys Asp Glu Asp Ser
260 265 270

Ala Ser Arg Arg Pro Pro Leu Ala His His Met Ser Leu Pro Lys Ser
275 280 285

Leu Ser Asp Ile Glu Gln Leu Leu Ser Asp Ser Ile Pro Cys Lys Ile
290 295 300

Arg Ala Lys Arg Gly Cys Ala Thr His Pro Arg Ser Ile Ala Glu Arg
305 310 315 320

Val Arg Arg Thr Lys Ile Ser Glu Arg Met Arg Lys Leu Gln Asp Leu
325 330 335

Val Pro Asn Met Asp Thr Gln Thr Asn Thr Ala Asp Met Leu Asp Leu
340 345 350

Ala Val Gln Tyr Ile Lys Asp Leu Gln Glu Gln Val Lys Ala Leu Glu
355 360 365

Glu Ser Arg Ala Arg Cys Arg Cys Ser Ser Ala
370 375

<210> 49
<211> 729
<212> DNA
<213> Arabidopsis thaliana

<220>
<223> G2992 Predicted polypeptide sequence is paralogous to G2999

<400> 49

atggatttgt cttccaaacc acaacaacaa cttctaaact ctcttcccat cgccggagaa 60
ctaactgtca ccggagaaat gggtgtttgt tacaaagagt gtttgaaaaa ccacgcggct 120
aatctcggcg gccatgctct cgacggttgc ggcgagttta tgccgagtcc cacggctact 180
tccaccgatc cttcttctct ccgttgcgct gcttgtggct gccaccgtaa cttccaccgc 240
cgtgacccctt ccgagaatct caacttcctc accgcgcctc cgatttcttc tccctccggc 300
actgagtcgc cgccgtctcg tcacgtgtct tctcctgttc cttgctctta ctacacctca 360
gctcctccac atcacgtgat tctctctctc agctccggtt tccctggacc gtcagatcaa 420
gatccaacgg tggttaggtc agagaacagt tcaagaggag caatgaggaa acgaacaaga 480
accaaattca cgccggagca gaagatcaag atgagagcct tcgcagagaa agctggttgg 540
aaaatcaacg gatgtgatga aaagtcggtg agagagtttt gtaacgaagt tgggattgag 600
agaggagttc ttaaagtgtg gatgcataac aacaagtact cacttctcaa tggcaagatc 660
agagagatcg aacatggcct gtgtctgaac actcacagca atgatggtga tgggtcttca 720
tcttcttga 729

<210> 50
<211> 242
<212> PRT
<213> Arabidopsis thaliana

<220>
<223> G2992 polypeptide Paralogous to G2999

<400> 50

```
Met Asp Leu Ser Ser Lys Pro Gln Gln Gln Leu Leu Asn Ser Leu Pro
1               5                   10                  15

Ile Ala Gly Glu Leu Thr Val Thr Gly Glu Met Gly Val Cys Tyr Lys
            20                  25                  30

Glu Cys Leu Lys Asn His Ala Ala Asn Leu Gly Gly His Ala Leu Asp
        35                  40                  45

Gly Cys Gly Glu Phe Met Pro Ser Pro Thr Ala Thr Ser Thr Asp Pro
    50                  55                  60

Ser Ser Leu Arg Cys Ala Ala Cys Gly Cys His Arg Asn Phe His Arg
65                  70                  75                  80

Arg Asp Pro Ser Glu Asn Leu Asn Phe Leu Thr Ala Pro Pro Ile Ser
                85                  90                  95

Ser Pro Ser Gly Thr Glu Ser Pro Pro Ser Arg His Val Ser Ser Pro
            100                 105                 110

Val Pro Cys Ser Tyr Tyr Thr Ser Ala Pro Pro His His Val Ile Leu
        115                 120                 125

Ser Leu Ser Ser Gly Phe Pro Gly Pro Ser Asp Gln Asp Pro Thr Val
    130                 135                 140

Val Arg Ser Glu Asn Ser Ser Arg Gly Ala Met Arg Lys Arg Thr Arg
145                 150                 155                 160

Thr Lys Phe Thr Pro Glu Gln Lys Ile Lys Met Arg Ala Phe Ala Glu
                165                 170                 175

Lys Ala Gly Trp Lys Ile Asn Gly Cys Asp Glu Lys Ser Val Arg Glu
            180                 185                 190

Phe Cys Asn Glu Val Gly Ile Glu Arg Gly Val Leu Lys Val Trp Met
        195                 200                 205

His Asn Asn Lys Tyr Ser Leu Leu Asn Gly Lys Ile Arg Glu Ile Glu
    210                 215                 220

His Gly Leu Cys Leu Asn Thr His Ser Asn Asp Gly Asp Gly Ser Ser
225                 230                 235                 240

Ser Ser
```

<210> 87
<211> 1082
<212> DNA
<213> Glycine max

<220>
<223> G3643 Predicted polypeptide sequence is orthologous to G47

<400> 87

```
ccacgcgtcc gcttcttctc tcagaataca caacacaaag tcaatataat tatagtatat      60
ccctatgagt aggagttcgg cgatgcatgg aattacaagc acaaacaaca agttgaaggg     120
agttcggcgt cgaaaatggg gcaaatgggt gtcggagatt cgtgttccgg gcacgcaaga     180
gcgtttgtgg ttgggaacct acgccacgcc ggaggctgcc gcggtggctc acgacgttgc     240
cgtctactgt ctaagtaggc cttcttcgtt ggacaaactt aacttccccg aaaccttgtc     300
ttcgtacagt gttcagctca gggacatgtc tccgaggtct gtgcagaagg tggcttccga     360
tgttggcatg gatgttgatg caagaaacat tgttgcgggc aaaacttcaa cggtgggggc     420
agaaactaat tgcgagagtg atgagaggac tagtactgcg tctgtgtgta atgttgttgg     480
agaaggtggt gctgatcatt cggatgtgtt ttggtgggat gatgatggtg ggtcttggca     540
tggaagtggt ggagattcta cggaaaggga tgccttgagc atttccattg aagattatct     600
ttagctgttc taggtttcaa ctttagttat cttttttttt ttttttttga gttatgatcg     660
atgcgcatag ttagtagtta cagataatta ctgctagtgt tgggtgttta aacggtcaga     720
gatgatgata tataaatttg atgtgcgcta gctgcctttt tgaagaaact aaaaaaaggt     780
aaaacaagaa agattgtacc cccaaaatga acttggtcaa tttcacactt tcacgccatt     840
tgcattttgt gcacgttgtt agttttcaaa ttgttaattc ccttgctaaa cccactcaaa     900
cttgagtgcg ttcacctttt attaccacgt agtaactgag agttaacgaa aatatttctt     960
tataaaaatt atttaccatt tagtgtcttt cgtttacttg taattattca acttgtcaat    1020
aagatgaaag ttctaatttt agatataata aaattcagct agtatttgtg aattactcac    1080
tt                                                                   1082
```

<210> 88
<211> 179
<212> PRT
<213> Glycine max

<220>
<223> G3643 polypeptide Orthologous to G47

<400> 88

```
Met Ser Arg Ser Ser Ala Met His Gly Ile Thr Ser Thr Asn Asn Lys
```

```
1               5                   10                  15
Leu Lys Gly Val Arg Arg Arg Lys Trp Gly Lys Trp Val Ser Glu Ile
            20                  25                  30
Arg Val Pro Gly Thr Gln Glu Arg Leu Trp Leu Gly Thr Tyr Ala Thr
        35                  40                  45
Pro Glu Ala Ala Ala Val Ala His Asp Val Ala Val Tyr Cys Leu Ser
    50                  55                  60
Arg Pro Ser Ser Leu Asp Lys Leu Asn Phe Pro Glu Thr Leu Ser Ser
65                  70                  75                  80
Tyr Ser Val Gln Leu Arg Asp Met Ser Pro Arg Ser Val Gln Lys Val
                85                  90                  95
Ala Ser Asp Val Gly Met Asp Val Asp Ala Arg Asn Ile Val Ala Gly
            100                 105                 110
Lys Thr Ser Thr Val Gly Ala Glu Thr Asn Cys Glu Ser Asp Glu Arg
        115                 120                 125
Thr Ser Thr Ala Ser Val Cys Asn Val Val Gly Glu Gly Gly Ala Asp
    130                 135                 140
His Ser Asp Val Phe Trp Trp Asp Asp Asp Gly Gly Ser Trp His Gly
145                 150                 155                 160
Ser Gly Gly Asp Ser Thr Glu Arg Asp Ala Leu Ser Ile Ser Ile Glu
                165                 170                 175
Asp Tyr Leu
```

<210> 89
<211> 687
<212> DNA
<213> Oryza sativa (japonica cultivar-group)

<220>
<223> G3644 Predicted polypeptide sequence is orthologous to G47

<400> 89

```
atgccttttg tacgtacact tgctttccca acgctcgcga atcaaatcga gggtgaaatt     60
aagtcaagaa cggagagaga tcacggtgag gttgatctca gctcgccgga ggaggcaatg    120
agccgggcgg agtgcggcgg cggcgaggag gaggagcggt gcaggtacag ggcgtgcggg    180
cggcggcggt gggggaagtg ggtgtcggag atccgggtgc ccggcacgcg ggagcggctg    240
```

```
tggctggggt cctacgccac gccggaggcc gccgccgtcg cgcacgacac ggccgtctac      300
ttcctccgcg gaggcgcggg cgacggcggt ggcggcggcg cgacgctcaa cttcccggag      360
cgcgcggcgg ccacgtacgg cggcggcgcc gccgtggcgc gcctgtcgcc gcggtccgtg      420
cagcgcgtgg cgtccgacgc cggcatggcc gccgacgcgc agctcgtggc ggcgcgggac      480
gccgcgcccg cgcccgcgcc ggcgacggcg tacgcgcgcc cggatcactg cgccggcgcg      540
acgacggcgc ggcacgacga gctggcgcgc cgcgggatgt acggcgctca cgcgcatgcc      600
gccggcgcga acgccaggac gagcggcgag cggcagctcg tctgtgccga ggagattagc      660
gtggatgaca tggagatcct gatgtaa                                           687
```

<210> 90
<211> 228
<212> PRT
<213> Oryza sativa (japonica cultivar-group)

<220>
<223> G3644 polypeptide Orthologous to G47

<400> 90

```
Met Pro Phe Val Arg Thr Leu Ala Phe Pro Thr Leu Ala Asn Gln Ile
1               5                   10                  15

Glu Gly Glu Ile Lys Ser Arg Thr Glu Arg Asp His Gly Glu Val Asp
            20                  25                  30

Leu Ser Ser Pro Glu Glu Ala Met Ser Arg Ala Glu Cys Gly Gly Gly
        35                  40                  45

Glu Glu Glu Glu Arg Cys Arg Tyr Arg Gly Val Arg Arg Arg Arg Trp
    50                  55                  60

Gly Lys Trp Val Ser Glu Ile Arg Val Pro Gly Thr Arg Glu Arg Leu
65                  70                  75                  80

Trp Leu Gly Ser Tyr Ala Thr Pro Glu Ala Ala Ala Val Ala His Asp
                85                  90                  95

Thr Ala Val Tyr Phe Leu Arg Gly Gly Ala Gly Asp Gly Gly Gly Gly
            100                 105                 110

Gly Ala Thr Leu Asn Phe Pro Glu Arg Ala Ala Ala Thr Tyr Gly Gly
        115                 120                 125

Gly Ala Ala Val Ala Arg Leu Ser Pro Arg Ser Val Gln Arg Val Ala
    130                 135                 140
```

Ser Asp Ala Gly Met Ala Ala Asp Ala Gln Leu Val Ala Ala Arg Asp
145 150 155 160

Ala Ala Pro Ala Pro Ala Pro Ala Thr Ala Tyr Ala Arg Pro Asp His
165 170 175

Cys Ala Gly Ala Thr Thr Ala Arg His Asp Glu Leu Ala Arg Arg Gly
180 185 190

Met Tyr Gly Ala His Ala His Ala Ala Gly Ala Asn Ala Arg Thr Ser
195 200 205

Gly Glu Arg Gln Leu Val Cys Ala Glu Glu Ile Ser Val Asp Asp Met
210 215 220

Glu Ile Leu Met
225

<210> 91
<211> 656
<212> DNA
<213> Brassica rapa subsp. Pekinensis

<220>
<223> G3645 Predicted polypeptide sequence is orthologous to G47

<400> 91

cccacgcgtc cgatggatta catcgacaac accgtcgaaa ctcaatcaaa gtacaaaggc 60
atccgtcgcc ggaaatgggg gaaatgggta tcggagattc gagttccggg aactcgcgac 120
cgtctctggt taggctcatt ctccacggcg gaaggcgcag ccgtggcgca cgacgtggct 180
ttctactgtt tacaccaacc aaactcgctc gaatctctca acttccctca cttgcttcct 240
ccttccattg tttccaagac ttcgccgagg tctatccagc aagctgcttc taatgccgga 300
atggccgttg acgccggaat cgttaacagc tgtgatcacg cgtcagggaa ctctgggaat 360
ggagatacaa cgacggcgta ttgtgagaat ggaggtgcgt tgaatatatc agtgtatgat 420
tatttggacg gtcacgatca cgtttgaact tcatcttctt gttttttcgt ttaaagatac 480
agctactcaa aagaagcagt gatggagcct ggtgtgtaag caagcaaaac gttgtgaata 540
tatataccgg tatgtttcgc tgttggccca atgcaagaaa cttttgtagt acgaatatat 600
atattttatt ttgaatgcct tcaagaggat tataatgcga gtgaagcttt gtttca 656

<210> 92
<211> 144
<212> PRT
<213> Brassica rapa subsp. Pekinensis

<220>
<223> G3645 polypeptide Orthologous to G47

<400> 92

Met Asp Tyr Ile Asp Asn Thr Val Glu Thr Gln Ser Lys Tyr Lys Gly
1 5 10 15

Ile Arg Arg Arg Lys Trp Gly Lys Trp Val Ser Glu Ile Arg Val Pro
20 25 30

Gly Thr Arg Asp Arg Leu Trp Leu Gly Ser Phe Ser Thr Ala Glu Gly
35 40 45

Ala Ala Val Ala His Asp Val Ala Phe Tyr Cys Leu His Gln Pro Asn
50 55 60

Ser Leu Glu Ser Leu Asn Phe Pro His Leu Leu Pro Pro Ser Ile Val
65 70 75 80

Ser Lys Thr Ser Pro Arg Ser Ile Gln Gln Ala Ala Ser Asn Ala Gly
85 90 95

Met Ala Val Asp Ala Gly Ile Val Asn Ser Cys Asp His Ala Ser Gly
100 105 110

Asn Ser Gly Asn Gly Asp Thr Thr Thr Ala Tyr Cys Glu Asn Gly Gly
115 120 125

Ala Leu Asn Ile Ser Val Tyr Asp Tyr Leu Asp Gly His Asp His Val
130 135 140

<210> 93
<211> 649
<212> DNA
<213> Brassica oleracea

<220>
<223> G3646 Predicted polypeptide sequence is orthologous to G47

<400> 93

cagccgcata acatattact attccccctg gtcatatctt tgacttttat ttttatttgc 60
tctaacctaa aagtattata taagtattgc acaattcata caatcggaat tgactttctt 120
ctcctccaat cgtattttta ttcaacagtt cctctcaaga tcatcaactc aaaaaatgga 180
tcctagagac ggcggagaaa cccatcaggc caagtacaaa ggcatccgtc gccggaaatg 240
gggaaaatgg gtatcggaga ttagggttcc agcaactcgt gaacgactct ggttaggctc 300
tttctccacc gccgaaggag ctgcggtagc ccacgacgtc gctttttact gcttgcaccg 360
accatcttct ctcgacaacg aagcttttaa cttccctcac ttgctgcaac cttcccttgc 420

ctccaacaca tctcctaagt ccatacaaaa agctgcttcg gacgcaggca tgggcgtaga 480
cgcaggattc gccctaaaca acgacagcgc gagtggtggc gtggaggaag gcaccgaacg 540
ggaaacgttg aacatctccg tgtacgatta tctagacgac ggtcgcattt gatatattgg 600
tttatatcta cgagcacctt atattagtaa ttaatatagg atgtgaata 649

<210> 94
<211> 138
<212> PRT
<213> Brassica oleracea

<220>
<223> G3646 polypeptide Orthologous to G47

<400> 94

Met Asp Pro Arg Asp Gly Gly Glu Thr His Gln Ala Lys Tyr Lys Gly
1 5 10 15

Ile Arg Arg Arg Lys Trp Gly Lys Trp Val Ser Glu Ile Arg Val Pro
20 25 30

Ala Thr Arg Glu Arg Leu Trp Leu Gly Ser Phe Ser Thr Ala Glu Gly
35 40 45

Ala Ala Val Ala His Asp Val Ala Phe Tyr Cys Leu His Arg Pro Ser
50 55 60

Ser Leu Asp Asn Glu Ala Phe Asn Phe Pro His Leu Leu Gln Pro Ser
65 70 75 80

Leu Ala Ser Asn Thr Ser Pro Lys Ser Ile Gln Lys Ala Ala Ser Asp
85 90 95

Ala Gly Met Gly Val Asp Ala Gly Phe Ala Leu Asn Asn Asp Ser Ala
100 105 110

Ser Gly Gly Val Glu Glu Gly Thr Glu Arg Glu Thr Leu Asn Ile Ser
115 120 125

Val Tyr Asp Tyr Leu Asp Asp Gly Arg Ile
130 135

<210> 95
<211> 495
<212> DNA
<213> Zinnia elegans

<220>
<223> G3647 Predicted polypeptide sequence is orthologous to G47

<400> 95

```
attcgcggcc gcgaatatga gtaccagctc agatgaaggt aacaactgtt taagccaaaa      60
gacttacaaa ggcgttaggt gccgacgatg gggcaaatgg gtgtcagaga ttcgagttcc     120
aggaagtcga gaacggctct ggctaggcac gtactctacg cctgagggtg cagctgtggc     180
tcatgatgta gcctcgtact gtttaaaagg gaatacgtct tttcataaac ttaatattcc     240
gtctatgtta cctccgacag cacggacaga cctatctcct aggtccatcc aaaaggctgc     300
gtctgatgct ggtatggcca tagacgcacg gtttatcgcg tctagagata ccacaccgac     360
taatgaggcg ttgaacattt ctgtagatga ttatctttaa attttgagaa ctaatattgt     420
gtcaccaata ttgtaagtcg atctacattg gcaaacacaa tgtacgtgtt tggtggcact     480
tccagattat tgttt                                                      495
```

<210> 96
<211> 127
<212> PRT
<213> Zinnia elegans

<220>
<223> G3647 polypeptide Orthologous to G47

<400> 96

```
Met Ser Thr Ser Ser Asp Glu Gly Asn Asn Cys Leu Ser Gln Lys Thr
1               5                   10                  15

Tyr Lys Gly Val Arg Cys Arg Arg Trp Gly Lys Trp Val Ser Glu Ile
            20                  25                  30

Arg Val Pro Gly Ser Arg Glu Arg Leu Trp Leu Gly Thr Tyr Ser Thr
        35                  40                  45

Pro Glu Gly Ala Ala Val Ala His Asp Val Ala Ser Tyr Cys Leu Lys
    50                  55                  60

Gly Asn Thr Ser Phe His Lys Leu Asn Ile Pro Ser Met Leu Pro Pro
65                  70                  75                  80

Thr Ala Arg Thr Asp Leu Ser Pro Arg Ser Ile Gln Lys Ala Ala Ser
                85                  90                  95

Asp Ala Gly Met Ala Ile Asp Ala Arg Phe Ile Ala Ser Arg Asp Thr
            100                 105                 110

Thr Pro Thr Asn Glu Ala Leu Asn Ile Ser Val Asp Asp Tyr Leu
        115                 120                 125
```

<210> 97
<211> 594
<212> DNA
<213> Oryza saliva (japonica cultivar-group)

<220>
<223> G3649 Predicted polypeptide sequence is orthologous to G47

<400> 97

```
atgggccggg tggcggcgag cggcggcggc ggcggcggag gggagatgat gaggtacagg     60
ggcgtgcggc ggcggcggtg ggggaagtgg gtgtcggaga tccgggtgcc cgggacgcgg    120
gagcgcctgt ggctcggctc ctacgccacc gccgaggccg ccgccgtcgc gcacgacgcc    180
gccgtctgcc tcctccggct cggcggcggc cgccgcgccg ccgcaggcgg aggcggcggg    240
ctcaacttcc ccgcccgcgc gctcgccgcg gcggcggccg cctcctccta cggcggcgcc    300
ggcggtctcc tgtccccgcg ctccgtgcag cgcgtggcgt ccgacgccgg catggccgcc    360
gacgcgcagc tcgtggacct gcgccgcgac cacccgcccg ccgccgccgc cgcctcatcc    420
tccggcagcg gcgtggcggg agacggtgca agaaagcaag ggacacgtgg cgaggttagc    480
gacacgtatt ggtgtaggaa tggagaggat gggagcagaa gccggagctc cgggagtgag    540
gagctcattg tttacgaggg cttaagtgta gatgacatgg aaattttgat gtaa          594
```

<210> 98
<211> 197
<212> PRT
<213> Oryza sativa (japonica cultivar-group)

<220>
<223> G3649 polypeptide Orthologous to G47

<400> 98

```
Met Gly Arg Val Ala Ala Ser Gly Gly Gly Gly Gly Gly Gly Glu Met
1               5                   10                  15

Met Arg Tyr Arg Gly Val Arg Arg Arg Arg Trp Gly Lys Trp Val Ser
            20                  25                  30

Glu Ile Arg Val Pro Gly Thr Arg Glu Arg Leu Trp Leu Gly Ser Tyr
        35                  40                  45

Ala Thr Ala Glu Ala Ala Ala Val Ala His Asp Ala Ala Val Cys Leu
    50                  55                  60

Leu Arg Leu Gly Gly Gly Arg Arg Ala Ala Ala Gly Gly Gly Gly Gly
65                  70                  75                  80

Leu Asn Phe Pro Ala Arg Ala Leu Ala Ala Ala Ala Ala Ala Ser Ser
                85                  90                  95
```

Tyr Gly Gly Ala Gly Gly Leu Leu Ser Pro Arg Ser Val Gln Arg Val
100 105 110

Ala Ser Asp Ala Gly Met Ala Ala Asp Ala Gln Leu Val Asp Leu Arg
115 120 125

Arg Asp His Pro Pro Ala Ala Ala Ala Ala Ser Ser Ser Gly Ser Gly
130 135 140

Val Ala Gly Asp Gly Ala Arg Lys Gln Gly Thr Arg Gly Glu Val Ser
145 150 155 160

Asp Thr Tyr Trp Cys Arg Asn Gly Glu Asp Gly Ser Arg Ser Arg Ser
165 170 175

Ser Gly Ser Glu Glu Leu Ile Val Tyr Glu Gly Leu Ser Val Asp Asp
180 185 190

Met Glu Ile Leu Met
195

<210> 99
<211> 702
<212> DNA
<213> Oryza sativa (japonica cultivar-group)

<220>
<223> G3651 Predicted polypeptide sequence is orthologous to G47

<400> 99

atggctgacc tcccatgcat atatatacgc gtagtacgta cacttgcttt cccaacgctc 60
gcgaatcaaa tcgagggtga aattaagtca agaacggaga gagatcacgg tgaggttgat 120
ctcagctcgc cggaggaggc aatgagccgg gcggagtgcg gcggcggcga ggaggaggag 180
cggtgcaggt acaggggcgt gcggcggcgg cggtggggga agtgggtgtc ggagatccgg 240
gtgcccggca cgcgggagcg gctgtggctg gggtcctacg ccacgccgga ggccgccgcc 300
gtcgcgcacg acacggccgt ctacttcctc cgcggaggcg cgggcgacgg cggtggcggc 360
ggcgcgaccg ctcaacttcc cggagcgcgc ggcggccacc gtacggcggc cgccgtggcg 420
cgcctgtcgc cgcggtccgt gcagcgcgtg gcgtccgacg cggcatggcc gccgacgcgc 480
agctcggcgt gccgggaccc ggcccggccc gcgccggcga cggcgtacgc gcgcccggat 540
cactgcgccg gcgcgacgac ggcgcggcac gacgagctgg cgcgccgcgg gatgtacggc 600
gctcacgcgc atgccgccgg cgcgaacgcc aggacgagcg gcgagcggca gctcgtctgt 660
gccgaggaga ttagcgtgga tgacatggag atcctgatgt aa 702

<210> 100
<211> 233
<212> PRT
<213> Oryza sativa (japonica cultivar-group)

<220>

<223> G3651 polypeptide Orthologous to G47

<400> 100

```
Met Ala Asp Leu Pro Cys Ile Tyr Ile Arg Val Val Arg Thr Leu Ala
1               5                   10                  15

Phe Pro Thr Leu Ala Asn Gln Ile Glu Gly Glu Ile Lys Ser Arg Thr
            20                  25                  30

Glu Arg Asp His Gly Glu Val Asp Leu Ser Ser Pro Glu Glu Ala Met
        35                  40                  45

Ser Arg Ala Glu Cys Gly Gly Gly Glu Glu Glu Glu Arg Cys Arg Tyr
    50                  55                  60

Arg Gly Val Arg Arg Arg Arg Trp Gly Lys Trp Val Ser Glu Ile Arg
65                  70                  75                  80

Val Pro Gly Thr Arg Glu Arg Leu Trp Leu Gly Ser Tyr Ala Thr Pro
                85                  90                  95

Glu Ala Ala Ala Val Ala His Asp Thr Ala Val Tyr Phe Leu Arg Gly
            100                 105                 110

Gly Ala Gly Asp Gly Gly Gly Gly Gly Ala Thr Ala Gln Leu Pro Gly
        115                 120                 125

Ala Arg Gly Gly His Arg Thr Ala Ala Ala Val Ala Arg Leu Ser Pro
    130                 135                 140

Arg Ser Val Gln Arg Val Ala Ser Asp Ala Ala Trp Pro Pro Thr Arg
145                 150                 155                 160

Ser Ser Ala Cys Arg Asp Pro Ala Arg Pro Ala Pro Ala Thr Ala Tyr
                165                 170                 175

Ala Arg Pro Asp His Cys Ala Gly Ala Thr Thr Ala Arg His Asp Glu
            180                 185                 190

Leu Ala Arg Arg Gly Met Tyr Gly Ala His Ala His Ala Ala Gly Ala
        195                 200                 205

Asn Ala Arg Thr Ser Gly Glu Arg Gln Leu Val Cys Ala Glu Glu Ile
    210                 215                 220

Ser Val Asp Asp Met Glu Ile Leu Met
225                 230
```

<210> 223
<211> 2217
<212> DNA
<213> Arabidopsis thaliana

<220>
<221> misc_feature
<222> (2192)..(2192)
<223> n is a, c, g, or t

<220>
<223> G175 reference sequence; predicted polypeptide sequence is paralogous to G1274

<400> 223

```
atttctaaaa atagttaaac tgttggtgat ttttgtggaa ctgattaatt aacacaattg     60
gagaaaacaa attgaaactt ttttgtttgt tagagagtta aggagaaatt ttatctaacc    120
aagctgtgta aatctcttgt agtttttctg ccgatataca ttttcattgt gttgagggta    180
aacgataatc aagaacgaga gagagagaga gcaagagcaa gagatttcta ctacagaaga    240
tttattatat tgatcatttt gtgtgatcaa cccataaaaa cagagagaca tagacaagtc    300
catgtttcga tgtttcgatc tctcttactg tctaaacggc gaaataaaaa gtctgatggg    360
tgtcacttat tgcatgtata ttagtaaatc agcttgagcc caagttaaag ctgaaacttg    420
ggtttgcaat ggctggtatt gataataaag ctgctgtaat gggagaatgg ttcgactgta    480
gtactactaa ccacaggaag agatcgaaag cggaacttgg tagagagttt tctttaaatt    540
acatcaagaa tgaggattct ttgcaaacca cctttcaaga aagttcacga ggagctcttc    600
gtgaaaggat tgctgcgaga tccgggttta atgcaccgtg gttaaacact gaggatattc    660
ttcagtcgaa atctttaacc atctcttctc ctggtcttag tcctgcaact ctgttagagt    720
ctcctgtttt cctctcaaac cctttgctat ctccaacaac cgggaagctc tcatcagtac    780
cttctgataa ggctaaagct gagttatttg acgacattac cacatcctta gccttccaaa    840
ccatttcagg aagtggcctt gatcctacta acatcgcttt agaacccgat gattcccaag    900
actatgaaga aagacagctc ggcggtttag gagactcgat ggcttgttgt gcacctgcag    960
atgatggata caactggaga aaatatggac aaaagctagt taaaggaagt gagtatccgc   1020
ggagctatta caagtgcacg cacccgaatt gtgaggccaa gaagaaggtt gaacggtctc   1080
gggaaggtca tattatagag atcatataca caggagatca tatacacagc aaacctccac   1140
ctaaccgccg gtcagggatt ggatcatccg gtactggcca agacatgcaa atagatgcaa   1200
ccgaatacga aggttttgct ggaaccaatg agaacataga atggacatca cctgtatctg   1260
```

```
cagagctcga atacggaagc cattcaggat caatgcaggt tcaaaacggg actcatcagt   1320
tcgggtatgg tgatgcagca gctgatgcct tatatagaga tgaaaacgaa gatgatcgca   1380
cgtcccacat gagtgtttcc ctgacttacg atggagaggt agaagagtcc gaatcaaaga   1440
gaaggaaact agaagcttat gcaacagaaa cgagtggatc aaccagagcc agccgtgagc   1500
caagagttgt ggtgcagacc acaagtgaca ttgacatcct cgatgatggt tatcgctggc   1560
gcaagtatgg gcaaaaagtc gttaaaggaa acccgaatcc aaggagctac tataaatgca   1620
cagctaatgg atgtaccgta acgaagcatg tagagagagc ctctgatgac ttcaagagcg   1680
tactaacaac ttatataggc aagcacaccc acgttgtacc agcagcacgc aacagcagcc   1740
acgtcggtgc aggcagttca gggactctcc aaggcagttt agcgactcag acccacaacc   1800
acaatgtgca ctatccaatg ccacacagta gatctgaggg actggccaca gccaactcat   1860
ctctatttga cttccagtca cacctgaggc atcctacagg tttctccgtt tacataggcc   1920
aatctgagct ttctgatctt tcaatgcctg gtctaactat tgggcaagag aagcttacca   1980
gcctgcaggc gcctgacatt ggggatccaa ctggcctaat gttgcagtta gcagcacagc   2040
cgaaggtgga accagtgtca ccacaacagg gacttgattt gtcagcgagc tcattgatat   2100
gcagagagat gttgagtaga ttacgacaga tatgaaacaa atctctttgt tcactgattg   2160
ctcaaatttt tgaataaatg aaaaattgag anaaaaaaaa aaaaaaaaaa aaaaaaa      2217
```

<210> 224
<211> 568
<212> PRT
<213> Arabidopsis thaliana

<220>
<223> G175 polypeptide reference sequence; paralogous to G1274

<400> 224

```
Met Ala Gly Ile Asp Asn Lys Ala Ala Val Met Gly Glu Trp Phe Asp
1               5                   10                  15

Cys Ser Thr Thr Asn His Arg Lys Arg Ser Lys Ala Glu Leu Gly Arg
            20                  25                  30

Glu Phe Ser Leu Asn Tyr Ile Lys Asn Glu Asp Ser Leu Gln Thr Thr
        35                  40                  45

Phe Gln Glu Ser Ser Arg Gly Ala Leu Arg Glu Arg Ile Ala Ala Arg
    50                  55                  60

Ser Gly Phe Asn Ala Pro Trp Leu Asn Thr Glu Asp Ile Leu Gln Ser
65                  70                  75                  80
```

```
Lys Ser Leu Thr Ile Ser Ser Pro Gly Leu Ser Pro Ala Thr Leu Leu
                85                  90                  95

Glu Ser Pro Val Phe Leu Ser Asn Pro Leu Leu Ser Pro Thr Thr Gly
            100                 105                 110

Lys Leu Ser Ser Val Pro Ser Asp Lys Ala Lys Ala Glu Leu Phe Asp
        115                 120                 125

Asp Ile Thr Thr Ser Leu Ala Phe Gln Thr Ile Ser Gly Ser Gly Leu
    130                 135                 140

Asp Pro Thr Asn Ile Ala Leu Glu Pro Asp Asp Ser Gln Asp Tyr Glu
145                 150                 155                 160

Glu Arg Gln Leu Gly Gly Leu Gly Asp Ser Met Ala Cys Cys Ala Pro
                165                 170                 175

Ala Asp Asp Gly Tyr Asn Trp Arg Lys Tyr Gly Gln Lys Leu Val Lys
            180                 185                 190

Gly Ser Glu Tyr Pro Arg Ser Tyr Tyr Lys Cys Thr His Pro Asn Cys
        195                 200                 205

Glu Ala Lys Lys Lys Val Glu Arg Ser Arg Glu Gly His Ile Ile Glu
    210                 215                 220

Ile Ile Tyr Thr Gly Asp His Ile His Ser Lys Pro Pro Pro Asn Arg
225                 230                 235                 240

Arg Ser Gly Ile Gly Ser Ser Gly Thr Gly Gln Asp Met Gln Ile Asp
                245                 250                 255

Ala Thr Glu Tyr Glu Gly Phe Ala Gly Thr Asn Glu Asn Ile Glu Trp
            260                 265                 270

Thr Ser Pro Val Ser Ala Glu Leu Glu Tyr Gly Ser His Ser Gly Ser
        275                 280                 285

Met Gln Val Gln Asn Gly Thr His Gln Phe Gly Tyr Gly Asp Ala Ala
    290                 295                 300

Ala Asp Ala Leu Tyr Arg Asp Glu Asn Glu Asp Asp Arg Thr Ser His
305                 310                 315                 320

Met Ser Val Ser Leu Thr Tyr Asp Gly Glu Val Glu Glu Ser Glu Ser
                325                 330                 335
```

Lys Arg Arg Lys Leu Glu Ala Tyr Ala Thr Glu Thr Ser Gly Ser Thr
340 345 350

Arg Ala Ser Arg Glu Pro Arg Val Val Val Gln Thr Thr Ser Asp Ile
355 360 365

Asp Ile Leu Asp Asp Gly Tyr Arg Trp Arg Lys Tyr Gly Gln Lys Val
370 375 380

Val Lys Gly Asn Pro Asn Pro Arg Ser Tyr Tyr Lys Cys Thr Ala Asn
385 390 395 400

Gly Cys Thr Val Thr Lys His Val Glu Arg Ala Ser Asp Asp Phe Lys
405 410 415

Ser Val Leu Thr Thr Tyr Ile Gly Lys His Thr His Val Val Pro Ala
420 425 430

Ala Arg Asn Ser Ser His Val Gly Ala Gly Ser Ser Gly Thr Leu Gln
435 440 445

Gly Ser Leu Ala Thr Gln Thr His Asn His Asn Val His Tyr Pro Met
450 455 460

Pro His Ser Arg Ser Glu Gly Leu Ala Thr Ala Asn Ser Ser Leu Phe
465 470 475 480

Asp Phe Gln Ser His Leu Arg His Pro Thr Gly Phe Ser Val Tyr Ile
485 490 495

Gly Gln Ser Glu Leu Ser Asp Leu Ser Met Pro Gly Leu Thr Ile Gly
500 505 510

Gln Glu Lys Leu Thr Ser Leu Gln Ala Pro Asp Ile Gly Asp Pro Thr
515 520 525

Gly Leu Met Leu Gln Leu Ala Ala Gln Pro Lys Val Glu Pro Val Ser
530 535 540

Pro Gln Gln Gly Leu Asp Leu Ser Ala Ser Ser Leu Ile Cys Arg Glu
545 550 555 560

Met Leu Ser Arg Leu Arg Gln Ile
565

<210> 225
<211> 1072
<212> DNA
<213> Arabidopsis thaliana

<220>
<223> G303 reference sequence

<400> 225

```
aaaagataac gtagccatta acttttatgc attaactcct tcatttcttt ttgtgctcgt      60
ttggttgaga gagagagagg ctatatatga tgttccaaca agattaccct catggctttt     120
cactcgtgga aacatcctta agttacgaaa tgttggatta ctttcaaaac atcgtcgttt     180
cgaactctga agacgtggcg tcacagcaaa attccatttc gtcctcttct tattcatcag     240
cgacactctc ctgctccata acagagcaaa aatctcactt aactgaaaag ttatctcctc     300
tacgagaaag atatggttgc ggtgactttc tgtcgcggaa gaggagaagg agaagtgaaa     360
aaacgattgt agataaagag aatcaaagga tgaatcacat tgccgtcgag cgtaaccgga     420
gaaaacagat gaatcatttt ctgtctatcc tcaagtctat gatgcctctc tcttattctc     480
aacctaatga ccaagcatca atcatagaag ggaccattag ctatctgaag aagctagaac     540
aacgtctcca atctctcgaa gcccaattaa aagctactaa actcaatcaa tcaccaaata     600
tattttccga cttcttcatg ttccctcaat actccaccgc cactgccact gccaccgcca     660
ctgcctcctc atcctcctcg agccaccacc atcacaagcg actagaggtg gttgctgacg     720
tggaggttac aatggtagaa agacatgcca acattaaagt gttaacgaag acacagccaa     780
gattgctctt caagattatc aatgagttta actctttagg tttaagtact cttcatctca     840
acctcacaac ttccaaagac atgtctctct tcacttttag cgtcaaggta gaggcagatt     900
gtcaattgac gccttctggt aatgaggtcg caaatacggt gcatgaagtc gttagaagag     960
ttcacaagga acgttgaatt ttgtttacat actagctaac tttgaaattc tattttattg    1020
tataaacaat ctcttatgtg tgtaatttac atatatacaa ttaattaaca tt            1072
```

<210> 226
<211> 296
<212> PRT
<213> Arabidopsis thaliana

<220>
<223> G303 polypeptide reference sequence

<400> 226

```
Met Met Phe Gln Gln Asp Tyr Pro His Gly Phe Ser Leu Val Glu Thr
1               5                   10                  15

Ser Leu Ser Tyr Glu Met Leu Asp Tyr Phe Gln Asn Ile Val Val Ser
            20                  25                  30
```

Asn Ser Glu Asp Val Ala Ser Gln Gln Asn Ser Ile Ser Ser Ser Ser
35 40 45

Tyr Ser Ser Ala Thr Leu Ser Cys Ser Ile Thr Glu Gln Lys Ser His
50 55 60

Leu Thr Glu Lys Leu Ser Pro Leu Arg Glu Arg Tyr Gly Cys Gly Asp
65 70 75 80

Phe Leu Ser Arg Lys Arg Arg Arg Arg Ser Glu Lys Thr Ile Val Asp
85 90 95

Lys Glu Asn Gln Arg Met Asn His Ile Ala Val Glu Arg Asn Arg Arg
100 105 110

Lys Gln Met Asn His Phe Leu Ser Ile Leu Lys Ser Met Met Pro Leu
115 120 125

Ser Tyr Ser Gln Pro Asn Asp Gln Ala Ser Ile Ile Glu Gly Thr Ile
130 135 140

Ser Tyr Leu Lys Lys Leu Glu Gln Arg Leu Gln Ser Leu Glu Ala Gln
145 150 155 160

Leu Lys Ala Thr Lys Leu Asn Gln Ser Pro Asn Ile Phe Ser Asp Phe
165 170 175

Phe Met Phe Pro Gln Tyr Ser Thr Ala Thr Ala Thr Ala Thr Ala Thr
180 185 190

Ala Ser Ser Ser Ser Ser Ser His His His His Lys Arg Leu Glu Val
195 200 205

Val Ala Asp Val Glu Val Thr Met Val Glu Arg His Ala Asn Ile Lys
210 215 220

Val Leu Thr Lys Thr Gln Pro Arg Leu Leu Phe Lys Ile Ile Asn Glu
225 230 235 240

Phe Asn Ser Leu Gly Leu Ser Thr Leu His Leu Asn Leu Thr Thr Ser
245 250 255

Lys Asp Met Ser Leu Phe Thr Phe Ser Val Lys Val Glu Ala Asp Cys
260 265 270

Gln Leu Thr Pro Ser Gly Asn Glu Val Ala Asn Thr Val His Glu Val
275 280 285

Val Arg Arg Val His Lys Glu Arg
290 295

<210> 227

<211> 628
<212> DNA
<213> Arabidopsis thaliana

<220>
<223> G354 reference sequence

<400> 227

```
cctagaagtc actaagtcga ttcaaaatgg ttgcgagaag tgaggaaatt gtgatagtgg     60
aagaagatac gactgcgaaa tgtttgatgt tgttatcaag agtcggagaa tgcggcggcg    120
gctgcggggg agatgaacgt gttttccgat gcaagacttg tcttaaagag ttctcatcgt    180
ttcaagcttt gggaggtcat cgtgcaagcc acaagaaact tatcaacagt gacaatccat    240
cacttcttgg atccttgtcc aacaagaaaa ctaaaacgtc tcatccttgt ccgatatgtg    300
gagtgaagtt tccgatggga caagctcttg gtggtcacat gaggagacat aggaacgaga    360
aagtctcagg ctcgttggtt acacgttctt ttctaccgga gacgacgacg gtgacggctt    420
tgaagaaatt tagtagtggg aagagagtgg cttgtttgga tttggactta gattcgatgg    480
agagtttggt caattggaag ttggagttgg gaagaacgat ttcttggagt taagtttttg    540
ggttgtatac agtttcacat gattttgtaa tctttgttga tccaattatc gtaccgatcg    600
atgtgaatat tattttgata caataaaa                                       628
```

<210> 228
<211> 168
<212> PRT
<213> Arabidopsis thaliana

<220>
<223> G354 polypeptide reference sequence

<400> 228

```
Met Val Ala Arg Ser Glu Glu Ile Val Ile Val Glu Glu Asp Thr Thr
1               5                   10                  15

Ala Lys Cys Leu Met Leu Leu Ser Arg Val Gly Glu Cys Gly Gly Gly
            20                  25                  30

Cys Gly Gly Asp Glu Arg Val Phe Arg Cys Lys Thr Cys Leu Lys Glu
        35                  40                  45

Phe Ser Ser Phe Gln Ala Leu Gly Gly His Arg Ala Ser His Lys Lys
    50                  55                  60
```

```
Leu Ile Asn Ser Asp Asn Pro Ser Leu Leu Gly Ser Leu Ser Asn Lys
65                  70                  75                  80

Lys Thr Lys Thr Ser His Pro Cys Pro Ile Cys Gly Val Lys Phe Pro
                85                  90                  95

Met Gly Gln Ala Leu Gly Gly His Met Arg Arg His Arg Asn Glu Lys
            100                 105                 110

Val Ser Gly Ser Leu Val Thr Arg Ser Phe Leu Pro Glu Thr Thr Thr
        115                 120                 125

Val Thr Ala Leu Lys Lys Phe Ser Ser Gly Lys Arg Val Ala Cys Leu
    130                 135                 140

Asp Leu Asp Leu Asp Ser Met Glu Ser Leu Val Asn Trp Lys Leu Glu
145                 150                 155                 160

Leu Gly Arg Thr Ile Ser Trp Ser
                165
```

<210> 229
<211> 869
<212> DNA
<213> Arabidopsis thaliana

<220>
<223> G489 reference sequence

<400> 229

```
tggtagtgaa tggatcaaca agaccatgga cagtctggag ctatgaacta tggcacaaac      60
ccataccaaa ccaacccgat gagcaccact gctgctactg tagcaggagg tgcggcacaa     120
ccaggccagc tggcgttcca ccagatccat cagcagcagc agcagcaaca gctggcacag     180
cagcttcaag cattttggga gaaccaattc aaagagattg agaagactac cgatttcaag     240
aaccacagcc ttccccttgc gagaatcaag aaaatcatga aagcggatga agatgtccgt     300
atgatctcgg ctgaggcgcc ggtcgtgttt gcaagggcct gtgagatgtt catcctggag     360
ctgacactca ggtcgtggaa ccacactgag gagaataaga ggcggacgtt gcagaagaac     420
gatattgctg ctgctgtgac tagaaccgat atttttgatt tccttgtgga tattgttccc     480
cgggaggatc tccgagatga agtcttggga agtattccga ggggcactgt cccggaagct     540
gctgctgctg gttacccgta tggatacttg cctgcaggaa ctgctccaat aggaaatccg     600
ggaatggtta tgggtaatcc cggtggtgcg tatccaccta atccttatat gggtcaacca     660
atgtggcaac aacaggcacc tgaccaacct gaccaggaaa attagcaaga aactgtgagt     720
cttccagctt cttttaggcc taccttgtag tcttggggtt ttgtttctgt tttcgaataa     780
```

tggtaacctt tgtataactt atttcagtat cgtctcagtt tggtactatg tcagttttgg 840

tattcttatc ttattgaagt aaaaatgtg 869

<210> 230
<211> 220
<212> PRT
<213> Arabidopsis thaliana

<220>
<223> G489 polypeptide reference sequence

<400> 230

```
Met Asn Tyr Gly Thr Asn Pro Tyr Gln Thr Asn Pro Met Ser Thr Thr
1               5                   10                  15

Ala Ala Thr Val Ala Gly Gly Ala Ala Gln Pro Gly Gln Leu Ala Phe
            20                  25                  30

His Gln Ile His Gln Gln Gln Gln Gln Gln Gln Leu Ala Gln Gln Leu
        35                  40                  45

Gln Ala Phe Trp Glu Asn Gln Phe Lys Glu Ile Glu Lys Thr Thr Asp
    50                  55                  60

Phe Lys Asn His Ser Leu Pro Leu Ala Arg Ile Lys Lys Ile Met Lys
65                  70                  75                  80

Ala Asp Glu Asp Val Arg Met Ile Ser Ala Glu Ala Pro Val Val Phe
                85                  90                  95

Ala Arg Ala Cys Glu Met Phe Ile Leu Glu Leu Thr Leu Arg Ser Trp
            100                 105                 110

Asn His Thr Glu Glu Asn Lys Arg Arg Thr Leu Gln Lys Asn Asp Ile
        115                 120                 125

Ala Ala Ala Val Thr Arg Thr Asp Ile Phe Asp Phe Leu Val Asp Ile
    130                 135                 140

Val Pro Arg Glu Asp Leu Arg Asp Glu Val Leu Gly Ser Ile Pro Arg
145                 150                 155                 160

Gly Thr Val Pro Glu Ala Ala Ala Ala Gly Tyr Pro Tyr Gly Tyr Leu
                165                 170                 175

Pro Ala Gly Thr Ala Pro Ile Gly Asn Pro Gly Met Val Met Gly Asn
            180                 185                 190
```

Pro Gly Gly Ala Tyr Pro Pro Asn Pro Tyr Met Gly Gln Pro Met Trp
195 200 205

Gln Gln Gln Ala Pro Asp Gln Pro Asp Gln Glu Asn
210 215 220

<210> 231
<211> 798
<212> DNA
<213> Arabidopsis thaliana

<220>
<223> G634 reference sequence

<400> 231

atggagcaag gaggaggtgg tggtggtaat gaagttgtgg aggaagcttc acctattagt 60
tcaagacctc ctgctaacaa cttagaagag cttatgagat tctcagccgc cgcggatgac 120
ggtggattag gaggtggagg tggaggagga ggaggaggaa gtgcttcttc ttcatcggga 180
aatcgatggc cgagagaaga aactttagct cttcttcgga tccgatccga tatggattct 240
acttttcgtg atgctactct caaagctcct ctttgggaac atgtttccag gaagctattg 300
gagttaggtt acaaacgaag ttcaaagaaa tgcaaagaga aattcgaaaa cgttcagaaa 360
tattacaaac gtactaaaga aactcgcggt ggtcgtcatg atggtaaagc ttacaagttc 420
ttctctcagc ttgaagctct caacactact cctcctcctc ctccttctca tcctcacgct 480
catcaaccag aacagaaaca acaacaacaa ccacaacaag agatggtcat gagctcggaa 540
caatcatcat taccatcatc atcaagatgg ccaaaggcag agattctagc gcttataaac 600
ctgagaagtg gaatggaacc aaggtaccaa gataatgtac ctaaaggact tctatgggaa 660
gagatctcaa cttcaatgaa gagaatggga tacaacagaa acgctaagag atgtaaagag 720
aaatgggaaa acataaacaa atactacaag aaagttaaag aaagcaacaa cagcaactac 780
aacaacaaga atcaatga 798

<210> 232
<211> 265
<212> PRT
<213> Arabidopsis thaliana

<220>
<223> G634 polypeptide reference sequence

<400> 232

Met Glu Gln Gly Gly Gly Gly Gly Gly Asn Glu Val Val Glu Glu Ala
1 5 10 15

Ser Pro Ile Ser Ser Arg Pro Pro Ala Asn Asn Leu Glu Glu Leu Met
20 25 30

```
Arg Phe Ser Ala Ala Ala Asp Asp Gly Gly Leu Gly Gly Gly Gly Gly
        35                  40                  45

Gly Gly Gly Gly Gly Ser Ala Ser Ser Ser Ser Gly Asn Arg Trp Pro
    50                  55                  60

Arg Glu Glu Thr Leu Ala Leu Leu Arg Ile Arg Ser Asp Met Asp Ser
65                  70                  75                  80

Thr Phe Arg Asp Ala Thr Leu Lys Ala Pro Leu Trp Glu His Val Ser
                85                  90                  95

Arg Lys Leu Leu Glu Leu Gly Tyr Lys Arg Ser Ser Lys Lys Cys Lys
            100                 105                 110

Glu Lys Phe Glu Asn Val Gln Lys Tyr Tyr Lys Arg Thr Lys Glu Thr
        115                 120                 125

Arg Gly Gly Arg His Asp Gly Lys Ala Tyr Lys Phe Phe Ser Gln Leu
    130                 135                 140

Glu Ala Leu Asn Thr Thr Pro Pro Pro Pro Pro Ser His Pro His Ala
145                 150                 155                 160

His Gln Pro Glu Gln Lys Gln Gln Gln Gln Pro Gln Gln Glu Met Val
                165                 170                 175

Met Ser Ser Glu Gln Ser Ser Leu Pro Ser Ser Ser Arg Trp Pro Lys
            180                 185                 190

Ala Glu Ile Leu Ala Leu Ile Asn Leu Arg Ser Gly Met Glu Pro Arg
        195                 200                 205

Tyr Gln Asp Asn Val Pro Lys Gly Leu Leu Trp Glu Glu Ile Ser Thr
    210                 215                 220

Ser Met Lys Arg Met Gly Tyr Asn Arg Asn Ala Lys Arg Cys Lys Glu
225                 230                 235                 240

Lys Trp Glu Asn Ile Asn Lys Tyr Tyr Lys Lys Val Lys Glu Ser Asn
                245                 250                 255

Asn Ser Asn Tyr Asn Asn Lys Asn Gln
            260                 265
```

<210> 233
<211> 341
<212> DNA
<213> Arabidopsis thaliana

<220>
<223> G682 reference sequence

<400> 233

acatggataa ccatcgcagg actaagcaac ccaagaccaa ctccatcgtt acttcttctt 60

ctgaaggaac agaagtgagt agtcttgagt gggaagttgt gaacatgagt caagaagaag 120

aagatttggt ctctcgaatg cataagcttg tcggtgacag gtgggaactg atagctggga 180

ggatcccagg aagaaccgct ggagaaattg agaggttttg ggtcatgaaa aattgaaaag 240

tgatcaatat atctctatag tttcactgat tgtttcgatt atattccgtt gtattttaaa 300

tttaccaact ctgttataag aagaaaactt tcttaaatcc c 341

<210> 234
<211> 77
<212> PRT
<213> Arabidopsis thaliana

<220>
<223> G682 polypeptide reference sequence

<400> 234

Met Asp Asn His Arg Arg Thr Lys Gln Pro Lys Thr Asn Ser Ile Val
1 5 10 15

Thr Ser Ser Ser Glu Gly Thr Glu Val Ser Ser Leu Glu Trp Glu Val
20 25 30

Val Asn Met Ser Gln Glu Glu Glu Asp Leu Val Ser Arg Met His Lys
35 40 45

Leu Val Gly Asp Arg Trp Glu Leu Ile Ala Gly Arg Ile Pro Gly Arg
50 55 60

Thr Ala Gly Glu Ile Glu Arg Phe Trp Val Met Lys Asn
65 70 75

<210> 235
<211> 1797
<212> DNA
<213> Arabidopsis thaliana

<220>
<223> G916 reference sequence

<400> 235

aaaaaaaaaa acaatctaag atatctatca agatcttaag atatctacat ttatagagat 60

gtttcgtttt ccggtaagtc ttggaggagg tccacgtgag aatctgaagc catcagatga 120

```
gcagcatcaa cgtgcggtgg tgaatgaggt tgactttttc cgatcagcgg agaagagaga    180
tagggtttca cgtgaagaac aaaacattat cgccgatgag actcataggg ttcatgtcaa    240
aagggagaat tcacgtgttg atgatcatga cgatcgttct actgatcaca tcaatattgg    300
acttaatctt ctcactgcga atacgggaag cgacgagtca atggtggatg atggattgtc    360
tgtggatatg gaagagaaac gtacaaagtg tgagaatgca caacttcgcg aagagctaaa    420
gaaggcgagt gaagataatc aaagactaaa gcaaatgcta agtcaaacaa ccaacaactt    480
caattccttg cagatgcaac ttgttgctgt catgaggcaa caagaagatc atcatcacct    540
agctacgacc gagaacaatg acaatgtaaa gaaccgacat gaagtgcctg aaatggttcc    600
aagacagttc atcgatttgg gaccgcattc tgacgaagtg tcgtccgagg agaggacgac    660
ggttcggtcg ggatctcctc cctcgcttct agagaaatct agctcacgtc aaaacggaaa    720
gagagtgctt gtaagagaag aaagcccgga aaccgaatcc aacggctgga gaaaccctaa    780
caaagttcct aaacaccatg catcatccag catttgcggt ggcaatggca gtgaaaatgc    840
aagtagcaag gtcattgagc aagcggccgc cgaagccacc atgcgtaaag cccgtgtctc    900
ggttcgtgct cgatccgaag ctcccatgtt aagcgatgga tgtcaatgga gaaaatacgg    960
acaaaaaatg gcgaaaggaa acccgtgccc tcgagcttat taccgttgca caatggctgt   1020
tggatgtcct gttcgcaagc aagtgcaacg ttgcgcggaa gatagaacca ttctcataac   1080
aacctacgaa ggaaaccata accatccatt acctcctgcg gctatgaaca tggcttcaac   1140
tacaacagca gccgcaagca tgcttctctc aggctccacc atgtcgaacc aagacggttt   1200
aatgaaccca acaaatctct tggctcgaac catattaccg tgttcctcaa gcatggctac   1260
tatctcagcc tctgcaccat tcccaaccat tacattagac ctcacagagt cacccaacgg   1320
gaacaatcca accaataacc cgctgatgca attctctcaa cggtctggtt tggtggagtt   1380
gaaccaatcg gttttgcctc atatgatggg tcaggctttg tactacaacc aacagtctaa   1440
gttttcgggt ttacatatgc cgtctcagcc gctaaacgct ggtgagagtg ttagcgccgc   1500
tactgccgca atcgcctcca atcccaactt tgccgcggct ctagctgcag ccataacttc   1560
gattatcaac ggttcgaaca atcagcagaa tgggaacaac aataacagta atgttacaac   1620
gagcaacgtt gacaataggc aataacattt ttttataagt tttagttagg gactttttat   1680
cggtcgattt tgttttgttt ttcttttatt acattatttt ttagttacgg cttttttttg   1740
tttttctttt tctttcccaa acaacaagta ttgagagcaa ttcccccccc cccccct      1797
```

<210> 236
<211> 528
<212> PRT
<213> Arabidopsis thaliana

<220>
<223> G916 polypeptide reference sequence

<400> 236

```
Met Phe Arg Phe Pro Val Ser Leu Gly Gly Gly Pro Arg Glu Asn Leu
1               5                   10                  15

Lys Pro Ser Asp Glu Gln His Gln Arg Ala Val Val Asn Glu Val Asp
            20                  25                  30

Phe Phe Arg Ser Ala Glu Lys Arg Asp Arg Val Ser Arg Glu Glu Gln
        35                  40                  45

Asn Ile Ile Ala Asp Glu Thr His Arg Val His Val Lys Arg Glu Asn
    50                  55                  60

Ser Arg Val Asp Asp His Asp Asp Arg Ser Thr Asp His Ile Asn Ile
65                  70                  75                  80

Gly Leu Asn Leu Leu Thr Ala Asn Thr Gly Ser Asp Glu Ser Met Val
                85                  90                  95

Asp Asp Gly Leu Ser Val Asp Met Glu Glu Lys Arg Thr Lys Cys Glu
            100                 105                 110

Asn Ala Gln Leu Arg Glu Glu Leu Lys Lys Ala Ser Glu Asp Asn Gln
        115                 120                 125

Arg Leu Lys Gln Met Leu Ser Gln Thr Thr Asn Asn Phe Asn Ser Leu
    130                 135                 140

Gln Met Gln Leu Val Ala Val Met Arg Gln Gln Glu Asp His His His
145                 150                 155                 160

Leu Ala Thr Thr Glu Asn Asn Asp Asn Val Lys Asn Arg His Glu Val
                165                 170                 175

Pro Glu Met Val Pro Arg Gln Phe Ile Asp Leu Gly Pro His Ser Asp
            180                 185                 190

Glu Val Ser Ser Glu Glu Arg Thr Thr Val Arg Ser Gly Ser Pro Pro
        195                 200                 205

Ser Leu Leu Glu Lys Ser Ser Ser Arg Gln Asn Gly Lys Arg Val Leu
    210                 215                 220

Val Arg Glu Glu Ser Pro Glu Thr Glu Ser Asn Gly Trp Arg Asn Pro
225                 230                 235                 240
```

```
Asn Lys Val Pro Lys His His Ala Ser Ser Ser Ile Cys Gly Gly Asn
                245                 250                 255

Gly Ser Glu Asn Ala Ser Ser Lys Val Ile Glu Gln Ala Ala Ala Glu
            260                 265                 270

Ala Thr Met Arg Lys Ala Arg Val Ser Val Arg Ala Arg Ser Glu Ala
        275                 280                 285

Pro Met Leu Ser Asp Gly Cys Gln Trp Arg Lys Tyr Gly Gln Lys Met
    290                 295                 300

Ala Lys Gly Asn Pro Cys Pro Arg Ala Tyr Tyr Arg Cys Thr Met Ala
305                 310                 315                 320

Val Gly Cys Pro Val Arg Lys Gln Val Gln Arg Cys Ala Glu Asp Arg
                325                 330                 335

Thr Ile Leu Ile Thr Thr Tyr Glu Gly Asn His Asn His Pro Leu Pro
            340                 345                 350

Pro Ala Ala Met Asn Met Ala Ser Thr Thr Thr Ala Ala Ala Ser Met
        355                 360                 365

Leu Leu Ser Gly Ser Thr Met Ser Asn Gln Asp Gly Leu Met Asn Pro
    370                 375                 380

Thr Asn Leu Leu Ala Arg Thr Ile Leu Pro Cys Ser Ser Ser Met Ala
385                 390                 395                 400

Thr Ile Ser Ala Ser Ala Pro Phe Pro Thr Ile Thr Leu Asp Leu Thr
                405                 410                 415

Glu Ser Pro Asn Gly Asn Asn Pro Thr Asn Asn Pro Leu Met Gln Phe
            420                 425                 430

Ser Gln Arg Ser Gly Leu Val Glu Leu Asn Gln Ser Val Leu Pro His
        435                 440                 445

Met Met Gly Gln Ala Leu Tyr Tyr Asn Gln Gln Ser Lys Phe Ser Gly
    450                 455                 460

Leu His Met Pro Ser Gln Pro Leu Asn Ala Gly Glu Ser Val Ser Ala
465                 470                 475                 480

Ala Thr Ala Ala Ile Ala Ser Asn Pro Asn Phe Ala Ala Ala Leu Ala
                485                 490                 495
```

Ala Ala Ile Thr Ser Ile Ile Asn Gly Ser Asn Asn Gln Gln Asn Gly
500 505 510

Asn Asn Asn Asn Ser Asn Val Thr Thr Ser Asn Val Asp Asn Arg Gln
515 520 525

<210> 237
<211> 768
<212> DNA
<213> Arabidopsis thaliana

<220>
<223> G975 reference sequence

<400> 237

attactcatc atcaagttcc tactttctct ctgacaaaca tcacagagta agtaagaatg 60
gtacagacga agaagttcag aggtgtcagg caacgccatt ggggttcttg ggtcgctgag 120
attcgtcatc ctctcttgaa acggaggatt tggctaggga cgttcgagac cgcagaggag 180
gcagcaagag catacgacga ggccgccgtt ttaatgagcg gccgcaacgc caaaaccaac 240
tttcccctca acaacaacaa caccggagaa acttccgagg gcaaaaccga tatttcagct 300
tcgtccacaa tgtcatcctc aacatcatct tcatcgctct cttccatcct cagcgccaaa 360
ctgaggaaat gctgcaagtc tccttcccca tccctcacct gcctccgtct tgacacagcc 420
agctcccata tcggcgtctg gcagaaacgg gccggttcaa agtctgactc cagctgggtc 480
atgacggtgg agctaggtcc cgcaagctcc tcccaagaga ctactagtaa agcttcacaa 540
gacgctattc ttgctccgac cactgaagtt gaaattggtg gcagcagaga agaagtattg 600
gatgaggaag aaaaggttgc tttgcaaatg atagaggagc ttctcaatac aaactaaatc 660
ttatttgctt atatatatgt acctattttc attgctgatt tacagccaaa ataatcaatt 720
ataccgtgta ttttatagat gttttatatt aaaaggttgt tagatata 768

<210> 238
<211> 199
<212> PRT
<213> Arabidopsis thaliana

<220>
<223> G975 polypeptide reference sequence

<400> 238

Met Val Gln Thr Lys Lys Phe Arg Gly Val Arg Gln Arg His Trp Gly
1 5 10 15

Ser Trp Val Ala Glu Ile Arg His Pro Leu Leu Lys Arg Arg Ile Trp
20 25 30

```
Leu Gly Thr Phe Glu Thr Ala Glu Glu Ala Ala Arg Ala Tyr Asp Glu
        35                  40                  45

Ala Ala Val Leu Met Ser Gly Arg Asn Ala Lys Thr Asn Phe Pro Leu
    50                  55                  60

Asn Asn Asn Asn Thr Gly Glu Thr Ser Glu Gly Lys Thr Asp Ile Ser
65                  70                  75                  80

Ala Ser Ser Thr Met Ser Ser Ser Thr Ser Ser Ser Ser Leu Ser Ser
                85                  90                  95

Ile Leu Ser Ala Lys Leu Arg Lys Cys Cys Lys Ser Pro Ser Pro Ser
            100                 105                 110

Leu Thr Cys Leu Arg Leu Asp Thr Ala Ser Ser His Ile Gly Val Trp
        115                 120                 125

Gln Lys Arg Ala Gly Ser Lys Ser Asp Ser Ser Trp Val Met Thr Val
    130                 135                 140

Glu Leu Gly Pro Ala Ser Ser Ser Gln Glu Thr Thr Ser Lys Ala Ser
145                 150                 155                 160

Gln Asp Ala Ile Leu Ala Pro Thr Thr Glu Val Glu Ile Gly Gly Ser
                165                 170                 175

Arg Glu Glu Val Leu Asp Glu Glu Glu Lys Val Ala Leu Gln Met Ile
            180                 185                 190

Glu Glu Leu Leu Asn Thr Asn
        195
```

<210> 239
<211> 1116
<212> DNA
<213> Arabidopsis thaliana

<220>
<223> G1069 Reference sequence; functionally related, homologous to G1073

<400> 239

```
ttggaaccct agaggccttt caagcaaatc atcagggtaa caatttcttg atctttcttt      60
ttagcgaatt tccagttttt ggtcaatcat ggcaaaccct tggtggacga accagagtgg     120
tttagcgggc atggtggacc attcggtctc ctcaggccat caccaaaacc atcaccacca     180
aagtcttctt accaaaggag atcttggaat agccatgaat cagagccaag acaacgacca     240
agacgaagaa gatgatccta gagaaggagc cgttgaggtg gtcaaccgta gaccaagagg     300
```

```
tagaccacca ggatccaaaa acaaacccaa agctccaatc tttgtgacaa gagacagccc    360
caacgcactc cgtagccatg tcttggagat ctccgacggc agtgacgtcg ccgacacaat    420
cgctcacttc tcaagacgca ggcaacgcgg cgtttgcgtt ctcagcggga caggctcagt    480
cgctaacgtc accctccgcc aagccgccgc accaggaggt gtggtctctc tccaaggcag    540
gtttgaaatc ttatctttaa ccggtgcttt cctccctgga ccttccccac ccgggtcaac    600
cggtttaacg gtttacttag ccggggtcca gggtcaggtc gttggaggta gcgttgtagg    660
cccactctta gccatagggt cggtcatggt gattgctgct actttctcta acgctactta    720
tgagagattg cccatggaag aagaggaaga cggtggcggc tcaagacaga ttcacggagg    780
cggtgactca ccgcccagaa tcggtagtaa cctgcctgat ctatcaggga tggccgggcc    840
aggctacaat atgccgccgc atctgattcc aaatggggct ggtcagctag ggcacgaacc    900
atatacatgg gtccacgcaa gaccacctta ctgactcagt gagccatttc tatatataat    960
ggtctatata aataaatata tagatgaata taagcaagca atttgaggta gtctattaca   1020
aagcttttgc tctggttgga aaaataaata agtatcaaag ctttgtttgt tcttaatgga   1080
aatatagagc ttgggaaggt agaaagagac gacatt                             1116
```

<210> 240
<211> 281
<212> PRT
<213> Arabidopsis thaliana

<220>
<223> G1069 Reference sequence; functionally related, homologous to G1073

<400> 240

```
Met Ala Asn Pro Trp Trp Thr Asn Gln Ser Gly Leu Ala Gly Met Val
1               5                   10                  15

Asp His Ser Val Ser Ser Gly His His Gln Asn His His His Gln Ser
            20                  25                  30

Leu Leu Thr Lys Gly Asp Leu Gly Ile Ala Met Asn Gln Ser Gln Asp
        35                  40                  45

Asn Asp Gln Asp Glu Glu Asp Asp Pro Arg Glu Gly Ala Val Glu Val
    50                  55                  60

Val Asn Arg Arg Pro Arg Gly Arg Pro Pro Gly Ser Lys Asn Lys Pro
65                  70                  75                  80

Lys Ala Pro Ile Phe Val Thr Arg Asp Ser Pro Asn Ala Leu Arg Ser
                85                  90                  95
```

His Val Leu Glu Ile Ser Asp Gly Ser Asp Val Ala Asp Thr Ile Ala
100 105 110

His Phe Ser Arg Arg Arg Gln Arg Gly Val Cys Val Leu Ser Gly Thr
115 120 125

Gly Ser Val Ala Asn Val Thr Leu Arg Gln Ala Ala Ala Pro Gly Gly
130 135 140

Val Val Ser Leu Gln Gly Arg Phe Glu Ile Leu Ser Leu Thr Gly Ala
145 150 155 160

Phe Leu Pro Gly Pro Ser Pro Pro Gly Ser Thr Gly Leu Thr Val Tyr
165 170 175

Leu Ala Gly Val Gln Gly Gln Val Val Gly Gly Ser Val Val Gly Pro
180 185 190

Leu Leu Ala Ile Gly Ser Val Met Val Ile Ala Ala Thr Phe Ser Asn
195 200 205

Ala Thr Tyr Glu Arg Leu Pro Met Glu Glu Glu Glu Asp Gly Gly Gly
210 215 220

Ser Arg Gln Ile His Gly Gly Gly Asp Ser Pro Pro Arg Ile Gly Ser
225 230 235 240

Asn Leu Pro Asp Leu Ser Gly Met Ala Gly Pro Gly Tyr Asn Met Pro
245 250 255

Pro His Leu Ile Pro Asn Gly Ala Gly Gln Leu Gly His Glu Pro Tyr
260 265 270

Thr Trp Val His Ala Arg Pro Pro Tyr
275 280

<210> 241
<211> 1371
<212> DNA
<213> Arabidopsis thaliana

<220>
<223> G1452 Reference sequence; functionally related, homologous to G512

<400> 241

atttattaag catcaatgag agaacttcag agctgggttt gagttctgtc caataataca 60

taaccacgtt atcatttttg tcctttacta tctcattaca ctcttctgtt attcgcccaa 120

ttcttacagt cattactctc tatagggctc gagcggccgc ccgggcaggt ttctatgcag 180

```
atggttcaca cttcccgctc cattgcccag attgggttcg gtgttaagtc gcaattagta      240
ctcactatag ggctcgagcg gccgcccggg caggtaaaag atcaaacaat gtctaaagaa      300
gctgagatgt cgatcgcggt gtcggctttg ttccctggtt ttagattctc tcctactgat      360
gttgaactta tctcgtacta tcttcgtcgt aaaatcgatg gtgatgagaa ctctgttgct      420
gtgattgctg aggtcgagat ttacaagttc gagccgtggg acttgccaga ggaatcgaaa      480
ctgaaatcgg agaacgagtg gttttacttc tgcgcgaggg ggaggaagta cccgcacggg      540
tcacaaagcc ggcgagccac acagctagga tattggaaag cgaccggtaa agagcggagt      600
gttaaatccg ggaaccaagt tgttggaacc aagagaacgc ttgtatttca tatcggtcgg      660
gctcctcgtg gcgagagaac ggagtggatt atgcatgaat actgcatcca tggagcccca      720
caggatgcat tagtggtgtg ccggttaaga aaaaatgctg attttcgggc tagttcgacc      780
caaaaaattg aggatggtgt tgtgcaagac gatggctacg ttggccaaag aggtggtttg      840
gacaaggagg acaaatccta ctatgaatct gagcatcaga taccaaatgg tgacatcgca      900
gaatcatcaa atgttgttga ggatcaggcc gataccgatg atgattgtta cgccgagatt      960
ctgaacgatg atataataaa gctcgacgaa gaagcgttga aagctagcca agcgtttcga     1020
ccaactaatc caactcatca agaaacaata tcaagcgagt catcgagtaa gaggtcaaaa     1080
tgtggtataa aaaaagaatc aacggaaaca atgaattgtt acgctttgtt caggatcaag     1140
aacgttgccg gaaccgactc cagctggaga ttcccgaacc cgttcaaaat caagaaagat     1200
gatagccaga gattgatgaa gaatgttctg gccactactg ttttcttggc tatcttattt     1260
tctttctttt ggactgtatt aatagctagg aactaaagct agttacgaca tacatattat     1320
ttatacataa ataaatatag tattttgtct atggcaaaaa aaaaaaaaaa a              1371
```

<210> 242
<211> 373
<212> PRT
<213> Arabidopsis thaliana

<220>
<223> G1452 polypeptide Reference sequence; functionally related, homologous to G512

<400> 242

```
Met Gln Met Val His Thr Ser Arg Ser Ile Ala Gln Ile Gly Phe Gly
1               5                   10                  15

Val Lys Ser Gln Leu Val Leu Thr Ile Gly Leu Glu Arg Pro Pro Gly
            20                  25                  30

Gln Val Lys Asp Gln Thr Met Ser Lys Glu Ala Glu Met Ser Ile Ala
        35                  40                  45
```

Val Ser Ala Leu Phe Pro Gly Phe Arg Phe Ser Pro Thr Asp Val Glu
50 55 60

Leu Ile Ser Tyr Tyr Leu Arg Arg Lys Ile Asp Gly Asp Glu Asn Ser
65 70 75 80

Val Ala Val Ile Ala Glu Val Glu Ile Tyr Lys Phe Glu Pro Trp Asp
85 90 95

Leu Pro Glu Glu Ser Lys Leu Lys Ser Glu Asn Glu Trp Phe Tyr Phe
100 105 110

Cys Ala Arg Gly Arg Lys Tyr Pro His Gly Ser Gln Ser Arg Arg Ala
115 120 125

Thr Gln Leu Gly Tyr Trp Lys Ala Thr Gly Lys Glu Arg Ser Val Lys
130 135 140

Ser Gly Asn Gln Val Val Gly Thr Lys Arg Thr Leu Val Phe His Ile
145 150 155 160

Gly Arg Ala Pro Arg Gly Glu Arg Thr Glu Trp Ile Met His Glu Tyr
165 170 175

Cys Ile His Gly Ala Pro Gln Asp Ala Leu Val Val Cys Arg Leu Arg
180 185 190

Lys Asn Ala Asp Phe Arg Ala Ser Ser Thr Gln Lys Ile Glu Asp Gly
195 200 205

Val Val Gln Asp Asp Gly Tyr Val Gly Gln Arg Gly Gly Leu Asp Lys
210 215 220

Glu Asp Lys Ser Tyr Tyr Glu Ser Glu His Gln Ile Pro Asn Gly Asp
225 230 235 240

Ile Ala Glu Ser Ser Asn Val Val Glu Asp Gln Ala Asp Thr Asp Asp
245 250 255

Asp Cys Tyr Ala Glu Ile Leu Asn Asp Asp Ile Ile Lys Leu Asp Glu
260 265 270

Glu Ala Leu Lys Ala Ser Gln Ala Phe Arg Pro Thr Asn Pro Thr His
275 280 285

Gln Glu Thr Ile Ser Ser Glu Ser Ser Ser Lys Arg Ser Lys Cys Gly

290 295 300

Ile Lys Lys Glu Ser Thr Glu Thr Met Asn Cys Tyr Ala Leu Phe Arg
305 310 315 320

Ile Lys Asn Val Ala Gly Thr Asp Ser Ser Trp Arg Phe Pro Asn Pro
325 330 335

Phe Lys Ile Lys Lys Asp Asp Ser Gln Arg Leu Met Lys Asn Val Leu
340 345 350

Ala Thr Thr Val Phe Leu Ala Ile Leu Phe Ser Phe Phe Trp Thr Val
355 360 365

Leu Ile Ala Arg Asn
370

<210> 243
<211> 732
<212> DNA
<213> Arabidopsis thaliana

<220>
<223> G1820 Reference sequence

<400> 243

```
ctcacttcca acatccaaat ccctagaaat tgtaaatggc tgagaacaac aacaacaacg     60
gcgacaacat gaacaacgac aaccaccagc aaccaccgtc gtactcgcag ctgccgccga    120
tggcatcatc caaccctcag ttacgtaatt actggattga gcagatggaa accgtctcgg    180
atttcaaaaa ccgtcagctt ccattggctc gaattaagaa gatcatgaag gctgatccag    240
atgtgcacat ggtctccgca gaggctccga tcatcttcgc aaaggcttgc gaaatgttca    300
tcgttgatct cacgatgcgg tcgtggctca aagccgagga gaacaaacgc cacacgcttc    360
agaaatcgga tatctccaac gcagtggcta gctctttcac ctacgatttc cttcttgatg    420
ttgtccctaa ggacgagtct atcgccaccg ctgatcctgg ctttgtggct atgccacatc    480
ctgacggtgg aggagtaccg caatattatt atccaccggg agtggtgatg ggaactccta    540
tggttggtag tggaatgtac gcgccatcgc aggcgtggcc agcagcggct ggtgacgggg    600
aggatgatgc tgaggataat ggaggaaacg gcggcggaaa ttgaagtgta gatttagggt    660
ttgtaaccgc ctatgtggga aatttgaaat ttggtggtgt ttattagggt tcttcaattc    720
gtcggatttg ct                                                        732
```

<210> 244
<211> 202
<212> PRT
<213> Arabidopsis thaliana

<220>
<223> G1820 polypeptide Reference sequence

<400> 244

Met Ala Glu Asn Asn Asn Asn Asn Gly Asp Asn Met Asn Asn Asp Asn
1               5                   10                  15

His Gln Gln Pro Pro Ser Tyr Ser Gln Leu Pro Pro Met Ala Ser Ser
            20                  25                  30

Asn Pro Gln Leu Arg Asn Tyr Trp Ile Glu Gln Met Glu Thr Val Ser
        35                  40                  45

Asp Phe Lys Asn Arg Gln Leu Pro Leu Ala Arg Ile Lys Lys Ile Met
    50                  55                  60

Lys Ala Asp Pro Asp Val His Met Val Ser Ala Glu Ala Pro Ile Ile
65                  70                  75                  80

Phe Ala Lys Ala Cys Glu Met Phe Ile Val Asp Leu Thr Met Arg Ser
                85                  90                  95

Trp Leu Lys Ala Glu Glu Asn Lys Arg His Thr Leu Gln Lys Ser Asp
            100                 105                 110

Ile Ser Asn Ala Val Ala Ser Ser Phe Thr Tyr Asp Phe Leu Leu Asp
        115                 120                 125

Val Val Pro Lys Asp Glu Ser Ile Ala Thr Ala Asp Pro Gly Phe Val
    130                 135                 140

Ala Met Pro His Pro Asp Gly Gly Gly Val Pro Gln Tyr Tyr Tyr Pro
145                 150                 155                 160

Pro Gly Val Val Met Gly Thr Pro Met Val Gly Ser Gly Met Tyr Ala
                165                 170                 175

Pro Ser Gln Ala Trp Pro Ala Ala Ala Gly Asp Gly Glu Asp Asp Ala
            180                 185                 190

Glu Asp Asn Gly Gly Asn Gly Gly Gly Asn
        195                 200

<210> 245
<211> 866
<212> DNA
<213> Arabidopsis thaliana

<220>
<223> G2701 reference sequence; predicted polypeptide sequence is paralogous to G1634

<400> 245

gtgtttgtag ttgaaactta ttcttccctt tttttgtttt taggtatgga gactctgcat 60
ccattctctc acctacctat ctctgaccac cggttcgttg ttcaagagat ggtgagctta 120
cacagctcga gtagcggtag ctggactaaa gaagagaaca agatgttcga acgagctctt 180
gcgatatacg ctgaagactc gcctgatcgc tggtttaaag ttgcttccat gatccctgga 240
aagactgttt ttgatgttat gaagcaatat agtaagcttg aagaagacgt tttcgatatt 300
gaagcaggac gtgttcccat tcctggttat cctgcagctt cttctccctt ggggtttgac 360
acggacatgt gtcgtaaacg gcctagtgga gctagaggat ctgatcaaga tcgaaagaaa 420
ggagtccctt ggacagagga agaacacagg agattcttgt taggccttct caagtacggt 480
aaaggagatt ggagaaacat atcgagaaac ttcgtggtgt caaagacgcc aacgcaagtg 540
gcgagccacg cccaaaagta ttaccagaga cagctctccg gagccaagga caaacgcagg 600
ccaagtatcc atgacatcac aaccggcaat cttctcaatg ccaatctcaa ccgttccttt 660
tccgatcata gagatattct ccctgattta gggtttatcg ataaggatga tacggaggag 720
ggagtaatat ttatgggtca gaatctctct tcagaaaatc tgttttctcc atcaccaact 780
tcattcgaag ctgccattaa cttcgccgga gaaaatgtct tcagtgccgg agcttaaggc 840
aacatagaat ccccaaactc agcggc 866

<210> 246
<211> 263
<212> PRT
<213> Arabidopsis thaliana

<220>
<223> G2701 polypeptide reference sequence; paralogous to G1634

<400> 246

Met Glu Thr Leu His Pro Phe Ser His Leu Pro Ile Ser Asp His Arg
1 5 10 15

Phe Val Val Gln Glu Met Val Ser Leu His Ser Ser Ser Ser Gly Ser
20 25 30

Trp Thr Lys Glu Glu Asn Lys Met Phe Glu Arg Ala Leu Ala Ile Tyr
35 40 45

Ala Glu Asp Ser Pro Asp Arg Trp Phe Lys Val Ala Ser Met Ile Pro
50 55 60

```
Gly Lys Thr Val Phe Asp Val Met Lys Gln Tyr Ser Lys Leu Glu Glu
65                  70                  75                  80

Asp Val Phe Asp Ile Glu Ala Gly Arg Val Pro Ile Pro Gly Tyr Pro
                85                  90                  95

Ala Ala Ser Ser Pro Leu Gly Phe Asp Thr Asp Met Cys Arg Lys Arg
            100                 105                 110

Pro Ser Gly Ala Arg Gly Ser Asp Gln Asp Arg Lys Lys Gly Val Pro
        115                 120                 125

Trp Thr Glu Glu Glu His Arg Arg Phe Leu Leu Gly Leu Leu Lys Tyr
    130                 135                 140

Gly Lys Gly Asp Trp Arg Asn Ile Ser Arg Asn Phe Val Val Ser Lys
145                 150                 155                 160

Thr Pro Thr Gln Val Ala Ser His Ala Gln Lys Tyr Tyr Gln Arg Gln
                165                 170                 175

Leu Ser Gly Ala Lys Asp Lys Arg Arg Pro Ser Ile His Asp Ile Thr
            180                 185                 190

Thr Gly Asn Leu Leu Asn Ala Asn Leu Asn Arg Ser Phe Ser Asp His
        195                 200                 205

Arg Asp Ile Leu Pro Asp Leu Gly Phe Ile Asp Lys Asp Asp Thr Glu
    210                 215                 220

Glu Gly Val Ile Phe Met Gly Gln Asn Leu Ser Ser Glu Asn Leu Phe
225                 230                 235                 240

Ser Pro Ser Pro Thr Ser Phe Glu Ala Ala Ile Asn Phe Ala Gly Glu
                245                 250                 255

Asn Val Phe Ser Ala Gly Ala
            260
```

<210> 247
<211> 1040
<212> DNA
<213> Arabidopsis thaliana

<220>
<223> G2789 Reference sequence; predicted polypeptide sequence is paralogous to G596

<400> 247

```
ctttagggac accaaatcta ttcaacctaa aagccttctt ttcccctata ttgaccaact      60
```

ttttagcgaa tcagaagagg aatggatgag gtatctcgtt ctcatacacc gcaatttcta 120
tcaagtgatc atcagcacta tcaccatcaa aacgctggac gacaaaaacg cggcagagaa 180
gaagaaggag ttgaacccaa caatataggg gaagacctag ccacctttcc ttccggagaa 240
gagaatatca agaagagaag gccacgtggc agacctgctg gttccaagaa caaacccaaa 300
gcaccaatca tagtcactcg cgactccgcg aacgccttca gatgtcacgt catggagata 360
accaacgcct gcgatgtaat ggaaagccta gccgtcttcg ctagacgccg tcagcgtggc 420
gtttgcgtct tgaccggaaa cggggccgtt acaaacgtca ccgttagaca acctggcgga 480
ggcgtcgtca gtttacacgg acggtttgag attctttctc tctcgggttc gtttcttcct 540
ccaccggcac caccagctgc gtctggttta aaggtttact tagccggtgg tcaaggtcaa 600
gtgatcggag gcagtgtggt gggaccgctt acggcatcaa gtccggtggt cgttatggca 660
gcttcatttg gaaacgcatc ttacgagagg ctgccactag aggaggagga ggaaactgaa 720
agagaaatag atggaaacgc ggctagggcg attggaacgc aaacgcagaa acagttaatg 780
caagatgcga catcgtttat tgggtcgccg tcgaatttaa ttaactctgt ttcgttgcca 840
ggtgaagctt attggggaac gcaacgaccg tctttctaag ataatatcat tgataatata 900
agtttcgtct tcttattctt tttcactttt tacctttttc actttcttag gttttgtttt 960
aacgtttgat taatacctga aggtttttgg aaaattttcg atcggataaa aggatttatg 1020
ttgcgagccg aaacgcggcc 1040

<210> 248
<211> 265
<212> PRT
<213> Arabidopsis thaliana

<220>
<223> G2789 polypeptide reference sequence; paralogous to G596

<400> 248

Met Asp Glu Val Ser Arg Ser His Thr Pro Gln Phe Leu Ser Ser Asp
1 5 10 15

His Gln His Tyr His His Gln Asn Ala Gly Arg Gln Lys Arg Gly Arg
20 25 30

Glu Glu Glu Gly Val Glu Pro Asn Asn Ile Gly Glu Asp Leu Ala Thr
35 40 45

Phe Pro Ser Gly Glu Glu Asn Ile Lys Lys Arg Arg Pro Arg Gly Arg
50 55 60

Pro Ala Gly Ser Lys Asn Lys Pro Lys Ala Pro Ile Ile Val Thr Arg

65 70 75 80

Asp Ser Ala Asn Ala Phe Arg Cys His Val Met Glu Ile Thr Asn Ala
85 90 95

Cys Asp Val Met Glu Ser Leu Ala Val Phe Ala Arg Arg Arg Gln Arg
100 105 110

Gly Val Cys Val Leu Thr Gly Asn Gly Ala Val Thr Asn Val Thr Val
115 120 125

Arg Gln Pro Gly Gly Gly Val Val Ser Leu His Gly Arg Phe Glu Ile
130 135 140

Leu Ser Leu Ser Gly Ser Phe Leu Pro Pro Pro Ala Pro Pro Ala Ala
145 150 155 160

Ser Gly Leu Lys Val Tyr Leu Ala Gly Gly Gln Gly Gln Val Ile Gly
165 170 175

Gly Ser Val Val Gly Pro Leu Thr Ala Ser Ser Pro Val Val Val Met
180 185 190

Ala Ala Ser Phe Gly Asn Ala Ser Tyr Glu Arg Leu Pro Leu Glu Glu
195 200 205

Glu Glu Glu Thr Glu Arg Glu Ile Asp Gly Asn Ala Ala Arg Ala Ile
210 215 220

Gly Thr Gln Thr Gln Lys Gln Leu Met Gln Asp Ala Thr Ser Phe Ile
225 230 235 240

Gly Ser Pro Ser Asn Leu Ile Asn Ser Val Ser Leu Pro Gly Glu Ala
245 250 255

Tyr Trp Gly Thr Gln Arg Pro Ser Phe
260 265

<210> 249
<211> 495
<212> DNA
<213> Arabidopsis thaliana

<220>
<223> G2839 Reference sequence; predicted polypeptide sequence is paralogous to G354

<400> 249

atggttgcga gaagtgagga agttgagata gtggaagata cggcggcgaa atgtttgatg 60

```
ttgttatcaa gagttggaga atgcggcgga ggaggagaga aacgagtttt ccgatgcaag    120
acttgtctta aagagttttc gtcgtttcaa gctttgggag gtcatcgtgc aagccacaag    180
aaactcatta acagtagcga tccatcactt cttggatcct tgtctaacaa gaaaactaaa    240
acggcgacgt ctcatccttg tccgatatgt ggcgtggagt ttccgatggg gcaagctctt    300
ggtggtcaca tgaggagaca taggagtgag aaagcctcac caggcacgtt ggttacacgt    360
tctttttac cggagacgac gacggtgacg actttgaaaa aatcgagtag tgggaagaga     420
gtggcttgtt tggacttaga ttcgatggag agtttagtca attggaagtt ggagttggga    480
agaacgattt cttga                                                     495
```

<210> 250
<211> 164
<212> PRT
<213> Arabidopsis thaliana

<220>
<223> G2839 polypeptide reference sequence; paralogous to G354

<400> 250

```
Met Val Ala Arg Ser Glu Glu Val Glu Ile Val Glu Asp Thr Ala Ala
1               5                   10                  15

Lys Cys Leu Met Leu Leu Ser Arg Val Gly Glu Cys Gly Gly Gly Gly
            20                  25                  30

Glu Lys Arg Val Phe Arg Cys Lys Thr Cys Leu Lys Glu Phe Ser Ser
        35                  40                  45

Phe Gln Ala Leu Gly Gly His Arg Ala Ser His Lys Lys Leu Ile Asn
    50                  55                  60

Ser Ser Asp Pro Ser Leu Leu Gly Ser Leu Ser Asn Lys Lys Thr Lys
65                  70                  75                  80

Thr Ala Thr Ser His Pro Cys Pro Ile Cys Gly Val Glu Phe Pro Met
                85                  90                  95

Gly Gln Ala Leu Gly Gly His Met Arg Arg His Arg Ser Glu Lys Ala
            100                 105                 110

Ser Pro Gly Thr Leu Val Thr Arg Ser Phe Leu Pro Glu Thr Thr Thr
        115                 120                 125

Val Thr Thr Leu Lys Lys Ser Ser Ser Gly Lys Arg Val Ala Cys Leu
    130                 135                 140
```

Asp Leu Asp Ser Met Glu Ser Leu Val Asn Trp Lys Leu Glu Leu Gly
145 150 155 160

Arg Thr Ile Ser

<210> 251
<211> 1569
<212> DNA
<213> Arabidopsis thaliana

<220>
<223> G2854 Reference sequence; predicted polypeptide sequence is paralogous to G1940

<400> 251

```
tctctctttt gcccttacgt cctcgagctc aaccctttaa ttgcatctac gacagtcgta      60
ccttttgcta ggttatcgtc gtcatgatgc agcagccacc tccagcttcc aacggtgctg     120
caacagggcc agggcagatt ccttccgacc aacaagctta cctccagcag cagcagtcgt     180
ggatgatgca gcaccagcag caacaacaag gtcagccgcc tgcaggatgg aatcagcagt     240
ctgcaccgtc ttctggtcaa ccacagcagc agcagtatgg tggtggtgga tctcagaatc     300
caggatcagc tggtgagatc cggtccctgt ggatcggtga cttgcagcca tggatggatg     360
agaactatct catgaacgtc tttggtctta ctggcgaggc tacagcagct aaagttattc     420
gcaataaaca gaacggatat tcagaaggtt atggctttat tgagtttgtg aaccatgcta     480
cagctgagag gaatttacag acttacaatg gtgctccgat gccgagcagt gagcaggcct     540
tcaggttgaa ctgggctcag cttggagctg gagagagacg ccaggctgaa gggcctgagc     600
acacagtttt tgttggagac ttggcacctg atgttaccga ccacatgctt actgaaacgt     660
ttaaagctgt gtattcctct gtcaagggag ctaaagttgt gaatgatagg actactggac     720
ggtccaaggg ttatggattt gtcaggtttg cggatgaaag tgagcagatt cgtgccatga     780
ctgaaatgaa tggtcaatac tgctcatcaa ggcctatgcg tactggtcct gctgccaaca     840
agaagcctct tacaatgcaa ccagcttcat atcagaacac tcaaggaaat tcaggagaaa     900
gtgatccaac taacacaaca atttttgttg gagctgtgga tcaaagtgta acagaagatg     960
atttgaagtc agtttttggt caatttggtg aactagttca tgtgaaaata cccgcaggaa    1020
aacgttgcgg atttgttcaa tacgccaata gggcatgtgc tgagcaagca ctttctgtgt    1080
tgaacggaac acaacttggg ggacaaagca ttcgtctttc atggggtcgc agtccttcca    1140
acaaacagac tcaacctgat caagcccagt atggtggtgg tggaggatac tatgggtatc    1200
ctcctcaagg atatgaagca tacggatatg cacctcctcc tcaggaccct aacgcctact    1260
acggtggtta tgctgggggc ggctatggaa actaccagca gcctggtgga taccagcagc    1320
aacagcagtg aagcatgatg tgtaagtcgt gatcgagctg aagttgtaga aagctgtaac    1380
```

cgtttgacaa tggtgaatga attattgaag tggatgtgta ttagctcttc ttgtctcaga 1440

ttctctcttc aactcttttt tttttctttt ggattatttt tctttcgcgt ggctattgtt 1500

gagtgtcctt gcaagtcata acaaaaagct taaacccaag tgtttttgt catgactaat 1560

atgaccatt 1569

<210> 252
<211> 415
<212> PRT
<213> Arabidopsis thaliana

<220>
<223> G2854 polypeptide reference sequence; paralogous to G1940

<400> 252

Met Met Gln Gln Pro Pro Pro Ala Ser Asn Gly Ala Ala Thr Gly Pro
1 5 10 15

Gly Gln Ile Pro Ser Asp Gln Gln Ala Tyr Leu Gln Gln Gln Gln Ser
20 25 30

Trp Met Met Gln His Gln Gln Gln Gln Gln Gly Gln Pro Pro Ala Gly
35 40 45

Trp Asn Gln Gln Ser Ala Pro Ser Ser Gly Gln Pro Gln Gln Gln Gln
50 55 60

Tyr Gly Gly Gly Gly Ser Gln Asn Pro Gly Ser Ala Gly Glu Ile Arg
65 70 75 80

Ser Leu Trp Ile Gly Asp Leu Gln Pro Trp Met Asp Glu Asn Tyr Leu
85 90 95

Met Asn Val Phe Gly Leu Thr Gly Glu Ala Thr Ala Ala Lys Val Ile
100 105 110

Arg Asn Lys Gln Asn Gly Tyr Ser Glu Gly Tyr Gly Phe Ile Glu Phe
115 120 125

Val Asn His Ala Thr Ala Glu Arg Asn Leu Gln Thr Tyr Asn Gly Ala
130 135 140

Pro Met Pro Ser Ser Glu Gln Ala Phe Arg Leu Asn Trp Ala Gln Leu
145 150 155 160

Gly Ala Gly Glu Arg Arg Gln Ala Glu Gly Pro Glu His Thr Val Phe
165 170 175

```
Val Gly Asp Leu Ala Pro Asp Val Thr Asp His Met Leu Thr Glu Thr
            180                 185                 190

Phe Lys Ala Val Tyr Ser Ser Val Lys Gly Ala Lys Val Val Asn Asp
        195                 200                 205

Arg Thr Thr Gly Arg Ser Lys Gly Tyr Gly Phe Val Arg Phe Ala Asp
    210                 215                 220

Glu Ser Glu Gln Ile Arg Ala Met Thr Glu Met Asn Gly Gln Tyr Cys
225                 230                 235                 240

Ser Ser Arg Pro Met Arg Thr Gly Pro Ala Ala Asn Lys Lys Pro Leu
                245                 250                 255

Thr Met Gln Pro Ala Ser Tyr Gln Asn Thr Gln Gly Asn Ser Gly Glu
            260                 265                 270

Ser Asp Pro Thr Asn Thr Thr Ile Phe Val Gly Ala Val Asp Gln Ser
        275                 280                 285

Val Thr Glu Asp Asp Leu Lys Ser Val Phe Gly Gln Phe Gly Glu Leu
    290                 295                 300

Val His Val Lys Ile Pro Ala Gly Lys Arg Cys Gly Phe Val Gln Tyr
305                 310                 315                 320

Ala Asn Arg Ala Cys Ala Glu Gln Ala Leu Ser Val Leu Asn Gly Thr
                325                 330                 335

Gln Leu Gly Gly Gln Ser Ile Arg Leu Ser Trp Gly Arg Ser Pro Ser
            340                 345                 350

Asn Lys Gln Thr Gln Pro Asp Gln Ala Gln Tyr Gly Gly Gly Gly Gly
        355                 360                 365

Tyr Tyr Gly Tyr Pro Pro Gln Gly Tyr Glu Ala Tyr Gly Tyr Ala Pro
    370                 375                 380

Pro Pro Gln Asp Pro Asn Ala Tyr Tyr Gly Gly Tyr Ala Gly Gly Gly
385                 390                 395                 400

Tyr Gly Asn Tyr Gln Gln Pro Gly Gly Tyr Gln Gln Gln Gln Gln
                405                 410                 415
```

<210> 253
<211> 914
<212> DNA
<213> Arabidopsis thaliana

<220>
<223> G3083 reference sequence

<400> 253

```
caaaatatga gccaagagac agcgatagca agcttcaaga aattccaaca gtcatggatc     60
gagcagctac gaaaccacct gaaccacctc cgctccgccc aaaaccatca ccggaactct    120
gctaccggtg atgaggaacg gttaagggag gctgtggaca gagtgatgga acactttaga    180
gaatatcaca gggccaagtg ggctgcgacg gataaagacg tcatcgaagt tatggcttct    240
ccttgggctt cagctcttga acggtcgctt cagtgggtcg gtggttggcg accaaccacc    300
ttgttccatc tggtttacac tgagtcgagt attttatttg agtctcgtat cgttgatata    360
cttcgtggct ttcgcaccgg tgatctcagt gatctctccc cttctcagtt caggacggta    420
agtgagctac aatgtgagac cgtgaaggaa gagaatgcga taacggaaga gttgtcggag    480
tggcaagacg atgcgagtga ccttgtcatg gggacatcgt cagatcccga ccagaggatc    540
cgacggctag cagaaatcgt ccaccgaact gatgatctgc gactgagaac gatcacacgt    600
gtggtggagg tcttgagtcc gctccaacaa gcggagtttc ttgtcgctgc ggctgagctt    660
cgtacaggcg ttgctggttg ggggactagc cacgaccgtc gtcgaagttc cgaagtttaa    720
agctaactaa aaccggtcta aaggtaatga gaatcgagga agaaaaatag tttttggata    780
ttaattatat gtttttacca tgtttttggt tatccagact gagtttggtt ttacatggtt    840
atgaaaacaa aaaaaactca tatgttatgt ctctacggtt gcaataaaga tcaacccacg    900
tgtggtgctt gtta                                                       914
```

<210> 254
<211> 237
<212> PRT
<213> Arabidopsis thaliana

<220>
<223> G3083 polypeptide: reference sequence

<400> 254

```
Met Ser Gln Glu Thr Ala Ile Ala Ser Phe Lys Lys Phe Gln Gln Ser
1               5                   10                  15

Trp Ile Glu Gln Leu Arg Asn His Leu Asn His Leu Arg Ser Ala Gln
            20                  25                  30

Asn His His Arg Asn Ser Ala Thr Gly Asp Glu Glu Arg Leu Arg Glu
        35                  40                  45

Ala Val Asp Arg Val Met Glu His Phe Arg Glu Tyr His Arg Ala Lys
```

50 55 60

Trp Ala Ala Thr Asp Lys Asp Val Ile Glu Val Met Ala Ser Pro Trp
65 70 75 80

Ala Ser Ala Leu Glu Arg Ser Leu Gln Trp Val Gly Gly Trp Arg Pro
85 90 95

Thr Thr Leu Phe His Leu Val Tyr Thr Glu Ser Ser Ile Leu Phe Glu
100 105 110

Ser Arg Ile Val Asp Ile Leu Arg Gly Phe Arg Thr Gly Asp Leu Ser
115 120 125

Asp Leu Ser Pro Ser Gln Phe Arg Thr Val Ser Glu Leu Gln Cys Glu
130 135 140

Thr Val Lys Glu Glu Asn Ala Ile Thr Glu Glu Leu Ser Glu Trp Gln
145 150 155 160

Asp Asp Ala Ser Asp Leu Val Met Gly Thr Ser Ser Asp Pro Asp Gln
165 170 175

Arg Ile Arg Arg Leu Ala Glu Ile Val His Arg Thr Asp Asp Leu Arg
180 185 190

Leu Arg Thr Ile Thr Arg Val Val Glu Val Leu Ser Pro Leu Gln Gln
195 200 205

Ala Glu Phe Leu Val Ala Ala Ala Glu Leu Arg Thr Gly Val Ala Gly
210 215 220

Trp Gly Thr Ser His Asp Arg Arg Arg Ser Ser Glu Val
225 230 235

## Description

### FIELD OF THE INVENTI0N

The present invention relates to compositions and methods for modifying the phenotype of a plant, to provide increased tolerance to drought.

### BACKGROUND OF THE INVENTION

Control of Cellular Processes by Transcription Factors. Studies from a diversity of prokaryotic and eukaryotic organisms suggest a gradual evolution of biochemical and physiological mechanisms and metabolic pathways. Despite different evolutionary pressures, proteins that regulate the cell cycle in yeast, plant, nematode, fly, rat, and man have common chemical or structural features and modulate the same general cellular activity. A comparison of gene sequences with known structure and/or function from one plant species, for example, *Arabidopsis thaliana*, with those from other plants, allows researchers to develop models for manipulating a plant's traits and developing varieties with valuable properties.

A plant's traits may be controlled through a number of cellular processes. One important way to manipulate that control is through transcription factors - proteins that influence the expression of a particular, gene or sets of genes. Because transcription factors are key controlling elements of biological pathways, altering the expression levels of one or more transcription factors can change entire biological pathways in an organism. Strategies for manipulating a plant's biochemical, developmental or phenotypic characteristics by altering a transcription factor expression can result in plants and crops with new and/or improved commercially valuable properties, including traits that improve yield or survival and yield during periods of abiotic stress, improve shade tolerance, or alter a plant's sensing of its carbon/nitrogen balance.

Problems associated with water deprivation. In the natural environment, plants often grow under unfavorable conditions, such as drought (low water availability), high salinity, chilling, freezing, or high temperature. Any of these abiotic stresses can delay growth and development, reduce productivity, and in extreme cases, cause the plant to die. Of these stresses, low water availability, which in a severe form is referred to as a drought, is a major factor in crop yield reduction worldwide. Problems for plants caused by low water availability include mechanical stresses caused by the withdrawal of cellular water. Drought also causes plants to become more susceptible to various diseases (Simpson, ed. (1981) "The Value of Physiological Knowledge of Water Stress in Plants", in Water Stress on Plants, Praeger, NY, pp. 235-265).

In addition to the many land regions of the world that are too arid for most if not all crop plants, overuse and over-utilization of available water is resulting in an increasing loss of agriculturally-usable land, a process which, in the extreme, results in desertification. The problem is further compounded by increasing salt accumulation in soils, which adds to the loss of available water in soils.

Water deficit is a common component of many plant stresses. Water deficit occurs in plant cells when the whole plant transpiration rate exceeds the water uptake. In addition to drought, other stresses, such as salinity and low temperature, produce cellular dehydration (McCue and Hanson (1990) Trends Biotechnol. 8: 358-362).

Salt and drought stress signal transduction include ionic and osmotic homeostasis signaling pathways. The ionic aspect of salt stress is signaled via the SOS pathway where a calcium-responsive SOS3-SOS2 protein kinase complex controls the expression and activity of ion transporters such as SOS1. The pathway regulating ion homeostasis in response to salt stress has been described recently by Xiong and Zhu (2002) Plant Cell Environ. 25: 131-139 and Ohta et al. (2003) Proc Natl Acad Sci USA 100:11771-11776.

The osmotic component of salt stress involves complex plant reactions that overlap with drought and/or low temperature stress responses.

Common aspects of drought, cold and salt stress response have been reviewed recently by Xiong and Zhu (2002) *supra*. Those included.

(a) transient changes in the cytoplasmic calcium levels very early in the signaling event (Knight, (2000) Int. Rev. Cytol. 195: 269-324; Sanders et al. (1999) Plant. Cell 11: 691-706);

(b) signal transduction via mitogen-activated and/or calcium dependent protein kinases (CDPKs; Xiong and Zhu (2002) *supra*) and protein phosphatases (Merlot et al. (2001) Plant J. 25: 295-303; Tahtiharju and Palva (2001) Plant J. 26: 461-470);

(c) increases in abscisic acid (ABA) levels in response to stress triggering a subset of responses (Xiong and Zhu (2002) *supra,* and references therein);

(d) inositol phosphates as signal molecules (at least for a subset of the stress responsive transcriptional changes (Xiong et al. (2001) Genes Dev. 15: 1971-1984);

(e) activation of phospholipases which in turn generate a diverse array of second messenger molecules, some of which might regulate the activity of stress responsive kinases (phospholipase D functions in an ABA independent pathway, Frank et al. (2000) Plant Cell 12:111-124);
(f) induction of late embryogenesis abundant (LEA) type genes including the CRT/DRE responsive COR/RD genes (Xiong and Zhu (2002) *supra*);
(g) increased levels of antioxidants and compatible osmolytes such as proline and soluble sugars (Hasegawa et al. (2000) Annu. Rev Plant Mol. Plant Physiol. 51: 463-499); and
(h) accumulation of reactive oxygen species such as superoxide, hydrogen peroxide, and hydroxyl radicals (Hasegawa et al. (2000) *supra).*

Abscisic acid biosynthesis is regulated by osmotic stress at multiple points. Both ABA-dependent and -independent osmotic stress signaling first modify constitutively expressed transcription factors, leading to the expression of early response transcriptional activators, which then activate downstream transcriptional activators and stress tolerance effector genes.

Based on the commonality of many aspects of low-temperature, drought and salt stress responses, it can be concluded that genes that increase tolerance to low temperature or salt stress can also improve drought stress protection. In fact, this has already been demonstrated for transcription factors, as in the case of AtCBF/DREB 1, and for other genes such as OsCDPK7 (Saijo et al. (2000) Plant J. 23: 319-327) or AVP1 (a vacuolar pyrophosphatase-proton-pump, Gaxiola et al. (2001) Proc. Natl. Acad. Sci. USA 98: 11444-11449).

## SUMMARY OF THE INVENTION

The present method is directed to recombinant polynucleotides that confer abiotic stress tolerance in plants when the expression of any of these recombinant polynucleotides is altered (e.g., by overexpression). Related sequences that are described herein include nucleotide sequences that hybridize to the complement of the sequences of the invention under stringent conditions. One Example of a stringent condition that defines the invention includes a hybridization procedure that incorporates two wash steps of 6x SSC and 65˚C, each step being 10-30 minutes in duration. For example, G2133 (polynucleotide SEQ ID NO:11 and polypeptide SEQ ID NO:12) confers tolerance to a drought, when this polypeptide is overexpressed in plants. The invention thus includes the use of the G2133 polynucleotide and polypeptide, as well as nucleotide sequences that are structurally similar in that they or their complement hybridize to SEQ 101 NO: 11 under stringent hybridization conditions.

In particular, the invention provides a method for producing a transgenic plant having greater tolerance to water deprivation than a control plant, the method steps comprising (a) producing an expression vector comprising a nucleotide sequence encoding a polypeptide comprising a conserved domain with at least 70% amino acid sequence identity to amino acid coordinates 10-77 of SEQ ID NO: 12; wherein the polypeptide has a property of SEQ ID NO: 12 of increasing tolerance to water deprivation in a plant relative to the control plant; and (b) introducing the expression vector into a target plant to produce a transgenic plant; wherein the polypeptide is overexpressed in the transgenic plant and said overexpression results in the transgenic plant having greater tolerance to water deprivation than the control plant.

The invention further provides a transgenic seed which comprises an expression vector comprising a nucleotide sequence encoding a polypeptide comprising a conserved domain with at least 70% amino acid sequence identity to amino acid coordinates 10-77 of SEQ ID NO:12 produced by a transgenic plant produced by any of the methods of claims 1 to 3, wherein a progeny plant grown from the transgenic seed has greater tolerance to drought relative to a control plant; and wherein said sequence is operably linked to a promoter which is inducible in response to heat, drought or osmotic stress, as well as a seed, fruit, leaf, root, or progeny of a plant comprising an expression vector comprising a nucleotide sequence encoding a polypeptide comprising a conserved domain with at least 70% amino acid sequence identity to amino acid coordinates 10-77 of SEQ ID NO: 12; wherein the polypeptide has a property of SEQ ID NO: 12 of increasing tolerance to water deprivation in a plant relative to the control plant obtainable by the method of any one of claims 1 to 3, wherein said plant, seed, fruit, leaf, root, plant cell or progeny has greater tolerance to water deprivation than the control plant, and wherein said sequence is operably linked to a promoter which is inducible in response to heat, or drought stress.

The invention further provides the use of an expression vector to produce a transgenic plant having greater tolerance to water deprivation relative to a control plant, wherein the expression vector encodes a polypeptide comprising a conserved domain with at least 70% amino acid sequence identity to amino acid coordinates 10-77 of SEQ ID NO:12.

The invention also relates to a method for producing a transgenic plant having increased tolerance to drought. These method steps include first providing an expression vector that contains a nucleotide sequence that hybridizes to the complement of a polynucleotide of the invention under stringent hybridization conditions. The expression vector is then introduced into a plant cell, the plant cell is cultured, from which a plant is generated. Due to the presence of the expression vector in the plant, the polypeptide encoded by the nucleotide sequence is overexpressed. This polypeptide has the property of regulating drought in a plant, compared to a control plant that does not overexpress the polypeptide.

After the drought tolerant transgenic plant is produced, it may be identified by comparing it with one or more non-transformed plants that do not overexpress the polypeptide. These method steps may further include selfing or crossing the abiotic stress-tolerant plant with itself or another plant, respectively, to produce seed. "Selfing" refers to self-pollinating, or using pollen from one plant to fertilize the same plant or another plant in the same line, whereas "crossing" generally refers to cross pollination with plant from a different line, such as a non-transformed or wild-type plant, or another transformed plant from a different transgenic line of plants. Crossing provides the advantage of being able to produce new varieties. The resulting seed may then be used to grow a progeny plant that is transgenic and has increased tolerance to abiotic stress.

Thus described herein is a method for increasing a plant's tolerance to drought. This method includes first providing a vector that comprises (i) regulatory elements effective in controlling expression of a polynucleotide sequence in a target plant, where the regulatory elements flank the polynucleotide sequence; and (ii) the polynucleotide sequences itself which encodes a polypeptide that has the ability to regulate drought tolerance in a plant, as compared to a control plant of the same species that does not overexpress the polypeptide. The plant is transformed with the vector in order to generate a transformed plant with increased tolerance to drought.

## BRIEF DESCRIPTION OF THE SEQUENCE LISTING AND FIGURES

The Sequence Listing provides exemplary polynucleotide and polypeptide sequences of the invention. The traits associated with the use of the sequences are included in the Examples.

CD-ROM1 and CD-ROM2 are read-only memory computer-readable compact discs. The content of each CD is identical, and each contains a copy of the Sequence Listing in ASCII text format. The Sequence Listing is named "MBI0058PCT.ST25.txt" and is 2004 kilobytes in size. The copies of the Sequence Listing on the CD-ROM discs are hereby incorporated by reference in their entirety.

Figure 1 shows a conservative estimate of phylogenetic relationships among the orders of flowering plants (modified from Angiosperm Phylogeny Group (1998) Ann. Missouri Bot. Gard. 84: 1-49). Those plants with a single cotyledon (monocots) are a monophyletic clade nested within at least two major lineages of dicots; the eudicots are further divided into rosids and asterids. *Arabidopsis* is a rosid eudicot classified within the order Brassicales; rice is a member of the monocot order Poales. Figure 1 was adapted from Daly et al. (2001) Plant Physiol 127: 1328-1333.

Figure 2 shows a phylogenic dendogram depicting phylogenetic relationships of higher plant taxa, including clades containing tomato and *Arabidopsis,* adapted from Ku et al. (2000) Proc. Natl. Acad. Sci. 97: 9121-9126; and Chase et al. (1993) Ann. Missouri Bot. Gard. 80: 528-580.

Figures 3 is a multiple amino acid sequence alignment of subsequences within the AP2 domain of G47, G2133 and their orthologs. Clade orthologs and paralogs are indicated by the black bar on the left side of the figure. Of the sequences examined to date, two valine residues were found that are present in members of the G47 clade but not outside of the clade (arrows). Residues that may be used to identify a G47 clade member are indicated by the residues shown in the boxes in Figure 3

Figure 4 illustrates the relationship of G47 and related sequences in this phylogenetic tree of the G47 clade and similar sequences. The tree building method used was "Neighbor Joining" with "Systematic Tie-Breaking" and Bootstrapping with 1000 replicates (Uncorrected ("p"), with gaps distributed proportionally). Full-length polypeptides were used to build the phylogeny as defined in Figure 4. The members of the clade shown within the box are predicted to contain functional homologs of G47. Abbreviations: At *Arabidopsis thaliana;* Os *Oryza sativa;* Zm *Zea mays;* Gm *Glycine max;* Mt *Medicago trincatula, Br Brassica rapa; Bo Brassica oleracea;* Ze; *Zinnia elegans.*

Figures 5A and 5B compare the recovery from a drought treatment of wild-type controls and two lines of *Arabidopsis Plants* overexpressing G2133, a paralog of G47. Figures 5A and 5B show two 35S::G2133 lines of plants (one line in each figure) in the pot on the left of each figure and control plants on the right of each figure. Each pot contained several plants grown under 24 hours light. All were deprived of water for eight days, and are shown after re-watering. All of the plants of the G2133 overexpressor lines recovered, and all of the control plants were either dead or severely and adversely affected by the drought treatment.

## DETAILED DESCRIPTION OF THE SPECIFIC EMBODIMENTS

The data presented herein represent the results of a screen of a transcription factor collection to identify genes that can be applied to reduce yield losses that arise from low nutrient, drought-related stress, and/or shade avoidance responses.

We have identified numerous transcription factor genes that confer improved drought-tolerance relative to wild type plants when their expression is altered, such as by overexpression or knocking-out of the gene in transgenic plants. Thus, the present invention is directed in part to recombinant polynucleotides that confer drought-related stress tolerance in plants when the expression of recombinant polynucleotides of the invention is altered (e.g., by overexpression). In the present studies, soil-based assays were performed in which transgenic plants are first deprived of water, evaluated by comparison to control plants, rewatered, and their recovery also evaluated by comparison to control plants similarly treated.

A secondary screen was then conducted in which either two or three individual overexpression lines (or a different homozygous seed lot, in the case of knockout lines) were retested in the assay. The individual transgenic lines that showed prominent phenotypes in the second round assay were given an "A" priority ranking. The set of sequences assigned a "B" priority ranking in the results table have yet to be confirmed in the secondary screen or did not show a prominent phenotype.

The present invention relates in part to polynucleotides and polypeptides, for example, for modifying phenotypes of plants, particularly those associated with improved drought stress. Throughout this disclosure, various information sources are referred to. The information sources include scientific journal articles, patent documents, textbooks, and World Wide Web browser-inactive page addresses. The reference to these information sources clearly indicates that they can be used by one of skill in the art. The contents and teachings of each and every one of the information sources can be relied on and used to make and use embodiments of the invention.

As used herein and in the appended claims, the singular forms "a," "an," and "the" include plural reference unless the context clearly dictates otherwise. Thus, for example, a reference to "a plant" includes a plurality of such plants, and a reference to "a stress" is a reference to one or more stresses and equivalents thereof known to those skilled in the art, and so forth.

## DEFINITIONS

"Nucleic acid molecule" refers to an oligonucleotide, polynucleotide or any fragment thereof. It may be DNA or RNA of genomic or synthetic origin, double-stranded or single-stranded, and combined with carbohydrate, lipids, protein, or other materials to perform a particular activity such as transformation or form a useful composition such as a peptide nucleic acid (PNA).

"Polynucleotide" is a nucleic acid molecule comprising a plurality of polymerized nucleotides, for example, at least about 15 or more consecutive polymerized nucleotides. A polynucleotide may be a nucleic acid, oligonucleotide, nucleotide, or any fragment thereof. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, or the like. The polynucleotide can be single-stranded or double-stranded DNA or RNA. The polynucleotide optionally comprises modified bases or a modified backbone. The polynucleotide can be, for example, genomic DNA or RNA, a transcript (such as an mRNA), a cDNA, a PCR product, a cloned DNA, a synthetic DNA or RNA, or the like. The polynucleotide can be combined with carbohydrate, lipids, protein, or other materials to perform a particular activity such as transformation or form a useful composition such as a peptide nucleic acid (PNA). The polynucleotide can comprise a sequence in either sense or antisense orientations. "Oligonucleotide" is substantially equivalent to the terms amplimer, primer, oligomer, element, target, and probe and is preferably single-stranded.

"Gene" or "gene sequence" refers to the partial or complete coding sequence of a gene, its complement, and its 5' or 3' untranslated regions. A gene is also a functional unit of inheritance, and in physical terms is a particular segment or sequence of nucleotides along a molecule of DNA (or RNA, in the case of RNA viruses) involved in producing a polypeptide chain. The latter may be subjected to subsequent processing such as chemical modification, splicing and folding to obtain a functional protein or polypeptide. A gene may be isolated partially isolated, or be found with an organism's genome. By way of example, a transcription factor gene encodes a transcription factor polypeptide, which may be functional or require processing to function as an initiator of transcription.

Operationally, genes may be defined by the cis-trans test, a genetic test that determines whether two mutations occur in the same gene and that may be used to determine the limits of the genetically active unit (Rieger et al. (1976) Glossary of Genetics and Cytogenetics: Classical and Molecular, 4th ed., Springer Verlag. Berlin). A gene generally includes regions preceding ("leaders"; upstream) and following ("trailers"; downstream) the coding region. A gene may also include intervening, non-coding sequences, referred to as "introns", located between individual coding segments, referred to as "exons". Most genes have an associated promoter region, a regulatory sequence 5' of the transcription initiation codon (there are some genes that do not have an identifiable promoter). The function of a gene may also be regulated by enhancers, operators, and other regulatory elements.

A "recombinant polynucleotide" is a polynucleotide that is not in its native state, for example, the polynucleotide comprises a nucleotide sequence not found in nature, or the polynucleotide is in a context other than that in which it is naturally found, for example, separated from nucleotide sequences with which it typically is in proximity in nature, or adjacent (or contiguous with) nucleotide sequences with which it typically is not in proximity. For example, the sequence at issue can be cloned into a vector, or otherwise recombined with one or more additional nucleic acid.

An "isolated polynucleotide" is a polynucleotide, whether naturally occurring or recombinant, that is present outside the cell in which it is typically found in nature, whether purified or not. Optionally, an isolated polynucleotide is subject to one or more enrichment or purification procedures, for example, cell lysis, extraction, centrifugation, precipitation, or the like.

A "polypeptide" is an amino acid sequence comprising a plurality of consecutive polymerized amino acid residues for example, at least about 15 consecutive polymerized amino acid residues. In many instances, a polypeptide comprises a polymerized amino acid residue sequence that is a transcription factor or a domain or portion or fragment thereof. Additionally, the polypeptide may comprise: (i) a localization domain; (ii) an activation domain; (iii) a repression domain; (iv) an oligomerization domain; or (v) a DNA-binding domain, or the like. The polypeptide optionally comprises modified amino acid residues, naturally occurring amino acid residues not encoded by a codon, or non-naturally occurring amino acid residues.

"Protein" refers to an amino acid sequence, oligopeptide, peptide, polypeptide or portions thereof whether naturally occurring or synthetic.

"Portion", as used herein, refers to any part of a protein used for any purpose, but especially for the screening of a library of molecules that specifically bind to that portion or for the production of antibodies.

A "recombinant polypeptide" is a polypeptide produced by translation of a recombinant polynucleotide. A "synthetic polypeptide" is a polypeptide created by consecutive polymerization of isolated amino acid residues using methods well known in the art. An "isolated polypeptide," whether a naturally occurring or a recombinant polypeptide, is more enriched in (or out of) a cell than the polypeptide in its natural state in a wild-type cell, for example, more than about 5% enriched, or at least 105% relative to wild type standardized at 100%. Such an enrichment is not the result of a natural response of a wild-type plant. Alternatively, or additionally, the isolated polypeptide is separated from other cellular components with which it is typically associated, for example, by any of the various protein purification methods herein.

"Homology" refers to sequence similarity between a reference sequence and at least a fragment of a newly sequenced clone insert or its encoded amino acid sequence. Additionally, the terms "homology" and "homologous sequence(s)" may refer to one or more polypeptide sequences that are modified by chemical or enzymatic means. The homologous sequence may be a sequence modified by lipids, sugars, peptides, organic or inorganic compounds, by the use of modified amino acids or the like. Protein modification techniques are illustrated in Ausubel et al. (eds) Current Protocols in Molecular Biology, John Wiley & Sons (1998).

"identity" or "similarity" refers to sequence similarity between two polynucleotide sequences or between two polypeptide sequences, with identity being a more strict comparison. The phrases "percent identity" and "% identity" refer to the percentage of sequence similarity found in a comparison of two or more polynucleotide sequences or two or more polypeptide sequences. "Sequence similarity" refers to the percent similarity in base pair sequence (as determined by any suitable method) between two or more polynucleotide sequences. Two or more sequences can be anywhere from 0-100% similar, or any integer value therebetween Identity or similarity can be determined by comparing a position in each sequence that may be aligned for purposes of comparison. When a position in the compared sequence is occupied by the same nucleotide base or amino acid, then the molecules are identical at that position. A degree of similarity or identity between polynucleotide sequences is a function of the number of identical, matching of corresponding nucleotides at positions shared by the polynucleotide sequences. A degree of identity of polypeptide sequences is a function of the number of identical amino acids at corresponding positions shared by the polypeptide sequences. A degree of homology or similarity of polypeptide sequences is a function of the number of amino acids at corresponding positions shared by the polypeptide sequences.

With regard to polypeptides, the terms "substantial identity" or "substantially identical" may refer to sequences of sufficient similarity and structure to the transcription factors in the Sequence Listing to produce similar function when expressed or overexpressed in a plant; in the present invention, this function is altered C/N sensing or increased tolerance to drought or shade. Sequences that are at least about 50% identical, and preferably at least 82% identical, to the instant polypeptide sequences are considered to have "substantial identity" with the latter. Sequences having lesser degrees of identity but comparable biological activity are considered to be equivalents. The structure required to maintain proper functionality is related to the tertiary structure of the polypeptide. There are discreet domains and motifs within a transcription factor that must be present within the polypeptide to confer function and , specificity. These specific structures are required so that interactive sequences will be properly oriented to retain the desired activity. "Substantial identity" may thus also be used with regard to subsequences, for example, motifs, that are of sufficient structure and similarity, being at least about 50% identical, and preferably at least 82% identical, to similar motifs in other related sequences so that each confers or is required for altered C/N sensing or increased tolerance to drought or shade.

The term "amino acid consensus motif" refers to the portion or subsequence of a polypeptide sequence that is substantially conserved among the polypeptide transcription factors listed in the Sequence Listing.

"Alignment" refers to a number of nucleotide or amino acid residue sequences aligned by lengthwise comparison so that components in common (i.e., nucleotide bases or amino acid residues) may be visually and readily identified. The fraction or percentage of components in common is related to the homology or identity between the sequences.

Alignments such as those found the Figures may be used to identify conserved domains and relatedness within these domains . An alignment may suitably be determined by means of computer programs known in the art, such as MacVector (1999) (Accelrys, Inc., San Diego, CA).

A "conserved domain" or "conserved region" as used herein refers to a region in heterologous polynucleotide or polypeptide sequences where there is a relatively high degree of sequence identity between the distinct sequences. AP2 domains are examples of conserved domains.

With respect to polynucleotides encoding presently disclosed transcription factors, a conserved domain is preferably at least 10 base pairs (bp) in length.

A "conserved domain", with respect to presently disclosed polypeptides refers to a domain within a transcription factor family that exhibits a higher degree of sequence homology, such as at least 70% sequence similarity, including conservative substitutions, and more preferably at least 79% sequence identity, and even more preferably at least 81 %, or at least about 86%, or at least about 87%, or at least about 89%, or at least about 91 %, or at least about 95%, or at least about 98% amino acid residue sequence identity to the conserved domain. Sequences are also encompassed by the invention that possess or encode conserved domains that recognizable fall within a given clade of transcription factor polypeptides and that have comparable biological activity to the sequences of this invention. A fragment or domain can be referred to as outside a conserved domain, outside a consensus sequence, or outside a consensus DNA-binding site that is known to exist or that exists for a particular transcription factor class, family, or sub-family. In this case, the fragment or domain will not include the exact amino acids of a consensus sequence or consensus DNA-binding site of a transcription factor class, family or sub-family, or the exact amino acids of a particular transcription factor consensus sequence or consensus DNA-binding site. Furthermore, a particular fragment, region, or domain of a polypeptide, or a polynucleotide encoding a polypeptide, can be "outside a conserved domain" if all the amino acids of the fragment, region, or domain fall outside of a defined conserved domain(s) for a polypeptide or protein. Sequences having lesser degrees of identity but comparable biological activity are considered to be equivalents.

As one of ordinary skill in the art recognizes, conserved domains may be identified as regions or domains of identity to a specific consensus sequence (for example, Riechmann et al. (2000) *supra).* Thus, by using alignment methods well known in the art, the conserved domains of the AP2 plant transcription factors may be determined.

The conserved domains for a number of the sequences that confer drought tolerance and altered C/N sensing are found in Tables 1 and 3, respectively. A comparison of the regions of the polypeptides in Table 1 or 3 allows one of skill in the art to identify conserved domains for any of the polypeptides listed or referred to in this disclosure.

"Complementary" refers to the natural hydrogen bonding by base pairing between purines and pyrimidines. For example, the sequence A-C-G-T (5' -> 3') forms hydrogen bonds with its complements A-C-G-T (5' -> 3') or A-C-G-U (5' → 3'). Two single-stranded molecules may be considered partially complementary, if only some of the nucleotides bond, or "completely complementary" if all of the nucleotides bond. The degree of complementarity between nucleic acid strands affects the efficiency and strength of hybridization and amplification reactions. "Fully complementary" refers to the case where bonding occurs between every base pair and its complement in a pair of sequences, and the two sequences have the same number of nucleotides.

The terms "highly stringent" or "highly stringent condition" refer to conditions that permit hybridization of DNA strands whose sequences are highly complementary, wherein these same conditions exclude hybridization of significantly mismatched DNAs. Polynucleotide sequences capable of hybridizing under stringent conditions with the polynucleotides of the present invention may be, for example, variants of the disclosed polynucleotide sequences, including allelic or splice variants, or sequences that encode orthologs or paralogs of presently disclosed polypeptides. Nucleic acid hybridization methods are disclosed in detail by Kashima et al. (1985) Nature 313:402-404, Sambrook et al. (1989) Molecular Cloning: A Laboratory Manual. 2nd Ed., Cold Spring Harbor Laboratory, Cold Spring Harbor, N.Y. ("Sambrook"), and by Hames and Higgins, "Nucleic Acid Hybridisation: A Practical Approach", IRL Press, Washington, D.C. (1985), which references are incorporated herein by reference.

In general, stringency is determined by the temperature, ionic strength, and concentration of denaturing agents (for example, formamide) used in a hybridization and washing procedure (for a more detailed description of establishing and determining stringency, see below). The degree to which two nucleic acids hybridize under various conditions of stringency is correlated with the extent of their similarity. Thus, similar nucleic acid sequences from a variety of sources, such as within a plant's genome (as in the case of paralogs) or from another plant (as in the case of orthologs) that may perform similar functions can be isolated on the basis of their ability to hybridize with known transcription factor sequences. Numerous variations are possible in the conditions and means by which nucleic acid hybridization can be performed to isolate transcription factor sequences having similarity to transcription factor sequences known in the art and are not limited to those explicitly disclosed herein. Such an approach may be used to isolate polynucleotide sequences having various degrees of similarity with disclosed transcription factor sequences, such as, for example, transcription factors having 60% identity, or more preferably greater than about 70% identity, most preferably 72% or greater identity with disclosed transcription factors.

Regarding the terms "paralog" and "ortholog", homologous polynucleotide sequences and homologous polypeptide sequences may be paralogs or orthologs of the claimed polynucleotide or polypeptides sequence. Orthologs and paralogs are evolutionarily-related genes that have similar sequence and similar functions. Orthologs are structurally related genes in different species that are derived by a speciation event. Paralogs are structurally related genes within a single species that are derived by a duplication event. Sequences that are sufficiently similar to one another will be appreciated by those of skill in the art and may be based upon percentage identity of the complete sequences, percentage identity of a conserved domain or sequence within the complete sequence, percentage similarity to the complete sequence, percentage similarity to a conserved domain or sequence within the complete sequence, and/or an arrangement of contiguous nucleotides or peptides particular to a conserved domain or complete sequence. Sequences that are sufficiently similar to one another will also bind in a similar manner to the same DNA binding sites of transcriptional regulatory elements using methods well known to those of skill in the art.

The term "equivalog" describes members of a set of homologous proteins that are conserved with respect to function since their last common ancestor. Related proteins are grouped into equivalog families, and otherwise into protein families with other hierarchically defined homology types. This definition is provided at the Institute for Genomic Research (TIGR) world wide web (www) website, " tigr.org " under the heading "Terms associated with TIGRFAMs".

The term "variant", as used herein, may refer to polynucleotides or polypeptides that differ from the presently disclosed polynucleotides or polypeptides, respectively, in sequence from each other, and as set forth below.

With regard to polynucleotide variants, differences between presently disclosed polynucleotides and polynucleotide variants are limited so that the nucleotide sequences of the former and the latter are closely similar overall and, in many regions, identical. Due to the degeneracy of the genetic code, difference between the former and latter nucleotide sequences may be silent (i.e., the amino acids encoded by the polynucleotide are the same, and the variant polynucleotide sequence encodes the same amino acid sequence as the presently disclosed polynucleotide. Variant nucleotide sequences may encode different amino acid sequences, in which case such nucleotide differences will result in amino acid substitutions, additions, deletions, insertions, truncations or fusions with respect to the similar disclosed polynucleotide sequences. These variations may result in polynucleotide variants encoding polypeptides that share at least one functional characteristic. The degeneracy of the genetic code also dictates that many different variant polynucleotides can encode identical and/or substantially similar polypeptides in addition to those sequences illustrated in the Sequence Listing.

Presently disclosed polypeptide sequences and similar polypeptide variants may differ in amino acid sequence by one or more substitutions, additions, deletions, fusions and truncations, which may be present in any combination. These differences may produce silent changes and result in a functionally equivalent transcription factor. Thus, it will be readily appreciated by those of skill in the art, that any of a variety of polynucleotide sequences is capable of encoding the transcription factors and transcription factor homolog polypeptides of the invention. A polypeptide sequence variant may have "conservative" changes, wherein a substituted amino acid has similar structural or chemical properties. Deliberate amino acid substitutions may thus be made on the basis of similarity in polarity, charge, solubility, hydrophobicity, hydrophilicity, and/or the amphipathic nature of the residues, as long as a substantial amount of the functional or biological activity of the transcription factor is retained. For example, negatively charged amino acids may include aspartic acid and glutamic acid, positively charged amino acids may include lysine and arginine, and amino acids with uncharged polar head groups having similar hydrophilicity values may include leucine, isoleucine, and valine; glycine and alanine; asparagine and glutamine; serine and threonine; and phenylalanine and tyrosine (for more detail on conservative substitutions, see Table 6). More rarely, a variant may have "non-conservative" changes, for example, replacement of a glycine with a tryptophan. Similar minor variations may also include amino acid deletions or insertions, or both. Related polypeptides may comprise, for example, additions and/or deletions of one or more N-linked or O-linked glycosylation sites, or an addition and/or a deletion of one or more cysteine residues. Guidance in determining which and how many amino acid residues may be substituted, inserted or deleted without abolishing functional or biological activity may be found using computer programs well known in the art, for example, DNASTAR software (USPN 5,840,544).

Also within the scope of the invention is a variant of a transcription factor nucleic acid listed in the Sequence Listing, that is, one having a sequence that differs from the one of the polynucleotide sequences in the Sequence Listing, or a complementary sequence, that encodes a functionally equivalent polypeptide (i.e., a polypeptide having some degree of equivalent or similar biological activity) but differs in sequence from the sequence in the Sequence Listing, due to degeneracy in the genetic code. Included within this definition are polymorphisms that may or may not be readily detectable using a particular oligonucleotide probe of the polynucleotide encoding polypeptide, and improper or unexpected hybridization to allelic variants, with a locus other than the normal chromosomal locus for the polynucleotide sequence encoding polypeptide.

"Allelic variant" or polynucleotide allelic variant" refers to any of two or more alternative forms of a gene occupying the same chromosomal locus. Allelic variation arises naturally through mutation, and may result in phenotypic polymorphism within populations. Gene mutations may be "silent" or may encode polypeptides having altered amino acid sequence. "Allelic variant" and "polypeptide allelic variant" may also be used with respect to polypeptides, and in this case the term refer to a polypeptide encoded by an allelic variant of a gene.

"Splice variant" or "polynucleotide splice variant" as used herein refers to alternative forms of RNA transcribed from a gene. Splice variation naturally occurs as a result of alternative sites being spliced within a single transcribed RNA molecule or between separately transcribed RNA molecules, and may result in several different forms of mRNA transcribed from the same gene. Thus, splice variants may encode polypeptides having different amino acid sequences, which may or may not have similar functions in the organism. "Splice variant" or "polypeptide splice variant" may also refer to a polypeptide encoded by a splice variant of a transcribed mRNA.

As used herein, "polynucleotide variants" may also refer to polynucleotide sequences that encode paralogs and orthologs of the presently disclosed polypeptide sequences. "Polypeptide variants" may refer to polypeptide sequences that are paralogs and orthologs of the presently disclosed polypeptide sequences.

"Ligand" refers to any molecule, agent, or compound that will bind specifically to a complementary site on a nucleic acid molecule or protein. Such ligands stabilize or modulate the activity of nucleic acid molecules or proteins of the invention and may be composed of at least one of the following: inorganic and organic substances including nucleic acids, proteins, carbohydrates, fats, and lipids.

"Modulates" refers to a change in activity (biological, chemical, or immunological) or lifespan resulting from specific binding between a molecule and either a nucleic acid molecule or a protein.

The term "plant" includes whole plants, shoot vegetative organs/structures (for example, leaves, stems and tubers), roots, flowers and floral organs/structures (for example, bracts, sepals, petals, stamens, carpels, anthers and ovules), seed (including embryo, endosperm, and seed coat) and fruit (the mature ovary), plant tissue (for example, vascular tissue, ground tissue, and the like) and cells (for example, guard cells, egg cells, and the like), and progeny of same. The class of plants that can be used in the method of the invention is generally as broad as the class of higher and lower plants amenable to transformation techniques, including angiosperms (monocotyledonous and dicotyledonous plants), gymnosperms, ferns, horsetails, psilophytes, lycophytes, bryophytes, and multicellular algae (as shown, for example, in Figure 1, adapted from Daly et al. (2001) Plant Physiol. 127: 1328-1333, and in Figure 2, adapted from Ku et al. (2000) Proc. Natl. Acad. Sci. 97: 9121-9126; and in Tudge (2000) in The Variety of Life, Oxford University Press, New York, NY, pp. 547-606).

A "transgenic plant" refers to a plant that contains genetic material not found in a wild-type plant of the same species, variety or cultivar. The genetic material may include a transgene, an insertional mutagenesis event (such as by transposon or T-DNA insertional mutagenesis), an activation tagging sequence, a mutated sequence, a homologous recombination event or a sequence modified by chimeraplasty. Typically, the foreign genetic material has been introduced into the plant by human manipulation, but any method can be used as one of skill in the art recognizes.

A transgenic plant may contain an expression vector or cassette. The expression cassette typically comprises a polypeptide-encoding sequence operably linked (i.e., under regulatory control of) to appropriate inducible or constitutive regulatory sequences that allow for the expression of polypeptide. The expression cassette can be introduced into a plant by transformation or by breeding after transformation of a parent plant. A plant refers to a whole plant as well as to a plant part, such as seed, fruit, leaf, or root, plant tissue, plant cells or any other plant material, for example, a plant explant, as well as to progeny thereof, and to *in vitro* systems that mimic biochemical or cellular components or processes in a cell.

"Wild type" or "wild-type", as used herein, refers to a plant cell, seed, plant component, plant tissue, plant organ or whole plant that has not been genetically modified or treated in an experimental sense. Wild-type cells, seed, components, tissue, organs or whole plants may be used as controls to compare levels of expression and the extent and nature of trait modification with cells, tissue or plants of the same species in which a transcription factor expression is altered, for example, in that it has been knocked out, overexpressed, or ectopically expressed.

A "control plant" as used in the present invention refers to a plant cell, seed, plant component, plant tissue, plant organ or whole plant used to compare against transgenic or genetically modified plant for the purpose of identifying an enhanced phenotype in the transgenic or genetically modified plant. A control plant may in some cases be a transgenic plant line that comprises an empty vector or marker gene, but does not contain the recombinant polynucleotide of the present invention that is expressed in the transgenic or genetically modified plant being evaluated. In general, a control plant is a plant of the same line or variety as the transgenic or genetically modified plant being tested. A suitable control plant would include a genetically unaltered or non-transgenic plant of the parental line used to generate a transgenic plant herein.

"Fragment", with respect to a polynucleotide, refers to a clone or any part of a polynucleotide molecule that retains a usable, functional characteristic. Useful fragments include oligonucleotides and polynucleotides that may be used in hybridization or amplification technologies or in the regulation of replication, transcription or translation. A "polynucleotide fragment" refers to any subsequence of a polynucleotide, typically, of at least about nine consecutive nucleotides, preferably at least about 30 nucleotides, more preferably at least about 50 nucleotides, of any of the sequences provided herein. Exemplary polynucleotide fragments are the first sixty consecutive nucleotides of the transcription factor polynucleotides listed in the Sequence Listing. Exemplary fragments include fragments comprising a region that encodes a conserved domain (for example, an AP2 domain) of a transcription factor.

Fragments may also include subsequences of polypeptides and protein molecules, or a subsequence of the polypeptide. Fragments may have uses in that they may have antigenic potential. In some cases, the fragment or domain is a subsequence of the polypeptide which performs at least one biological function of the intact polypeptide in substantially the same manner, or to a similar extent, as does the intact polypeptide. For example, a polypeptide fragment can comprise a recognizable structural motif or functional domain such as a DNA-binding site or domain that binds to a DNA promoter region, an activation domain, or a domain for protein-protein interactions, and may initiate transcription. Fragments can vary in size from as few as 3 amino acid residues to the full length of the intact polypeptide, but are preferably at least about 30 amino acid residues in length and more preferably at least about 60 amino acid residues in length. Exemplary polypeptide fragments are the first twenty consecutive amino acids of the transcription factor polypeptides listed in the Sequence Listing. Exemplary fragments also include fragments that comprise an AP2 domain of a transcription factor, for example, amino acid residues 10-77 of G2133 (SEQ ID NO: 12), as noted in Table 1.

The invention also encompasses production of DNA sequences that encode transcription factors and transcription factor derivatives, or fragments thereof, entirely by synthetic chemistry. After production, the synthetic sequence may be inserted into any of the many available expression vectors and cell systems using reagents well known in the art. Moreover, synthetic chemistry may be used to introduce mutations into a sequence encoding transcription factors or any fragment thereof.

"Derivative" refers to the chemical modification of a nucleic acid molecule or amino acid sequence. Chemical modifications can include replacement of hydrogen by an alkyl, acyl, or amino group or glycosylation, pegylation, or any similar process that retains or enhances biological activity or lifespan of the molecule or sequence.

A "trait" refers to a physiological, morphological, biochemical, or physical characteristic of a plant or particular plant material or cell. In some instances, this characteristic is visible to the human eye, such as seed or plant size, or can be measured by biochemical techniques, such as detecting the protein, starch, or oil content of seed or leaves, or by observation of a metabolic or physiological process, for example, by measuring tolerance to water deprivation or particular salt or sugar concentrations, or by the observation of the expression level of a gene or genes, for example, by employing Northern analysis, RT-PCR, microarray gene expression assays, or reporter gene expression systems, or by agricultural observations such as drought stress tolerance or yield. Any technique can be used to measure the amount of, comparative level of, or difference in any selected chemical compound or macromolecule in the transgenic plants, however.

"Trait modification" refers to a detectable difference in a characteristic in a plant ectopically expressing a polynucleotide or polypeptide of the present invention relative to a plant not doing so, such as a wild-type plant. In some cases, the trait modification can be evaluated quantitatively. For example, the trait modification can entail at least about a 2% increase or decrease in an observed trait, or an even greater difference, as compared with a wild-type or control plant. It is known that there can be a natural variation in the modified trait. Therefore, the trait modification observed entails a change of the normal distribution and magnitude of the trait in the plants compared with the distribution and magnitude observed in wild-type plants.

The term "transcript profile" refers to the expression levels of a set of genes in a cell in a particular state, particularly by comparison with the expression levels of that same set of genes in a cell of the same type in a reference state. For example, the transcript profile of a particular transcription factor in a suspension cell is the expression levels of a set of genes in a cell repressing or overexpressing that transcription factor compared with the expression levels of that same set of genes in a suspension cell that has normal levels of that transcription factor. The transcript profile can be presented as a list of those genes whose expression level is significantly different between the two treatments, and the difference ratios. Differences and similarities between expression levels may also be evaluated and calculated using statistical and clustering methods.

"Ectopic expression or altered expression" in reference to a polynucleotide indicates that the pattern of expression in, for example, a transgenic plant or plant tissue, is different from the expression pattern in a wild-type plant or a reference plant of the same species. The pattern of expression may also be compared with a reference expression pattern in a wild-type plant of the same species. For example, the polynucleotide or polypeptide is expressed in a cell or tissue type other than a cell or tissue type in which the sequence is expressed in the wild-type plant, or by expression at a time other than at the time the sequence is expressed in the wild-type plant, or by a response to different inducible agents, such as hormones or environmental signals, or at different expression levels (either higher or lower) compared with those found in a wild-type plant. The term also refers to altered expression patterns that are produced by lowering the levels of expression to below the detection level or completely abolishing expression. The resulting expression pattern can be transient or stable, constitutive or inducible. In reference to a polypeptide, the term "ectopic expression or altered expression" further may relate to altered activity levels resulting from the interactions of the polypeptides with exogenous or endogenous modulators or from interactions with factors or as a result of the chemical modification of the polypeptides.

The term "overexpression" as used herein refers to a greater expression level of a gene in a plant, plant cell or plant tissue, compared to expression in a wild-type plant, cell or tissue, at any developmental or temporal stage for the gene. Overexpression can occur when, for example, the genes encoding one or more transcription factors are under the control of a strong promoter described herein (for example, the cauliflower mosaic virus 35S transcription initiation region), or overexpression can be induced when an appropriate environmental signal is present. Overexpression may occur throughout a plant or in specific tissues of the plant, depending on the promoter used, as described below.

Overexpression may take place in plant cells normally lacking expression of polypeptides functionally equivalent or identical to the present transcription factors. Overexpression may also occur in plant cells where endogenous expression of the present transcription factors or functionally equivalent molecules normally occurs, but such normal expression is at a lower level. Overexpression thus results in a greater than normal production, or "overproduction" of the transcription factor in the plant, cell or tissue.

The term "transcription regulating region" refers to a DNA regulatory sequence that regulates expression of one or more genes in a plant when a transcription factor having one or more specific binding domains binds to the DNA regulatory sequence. Transcription factors of the present invention may possess, for example, an AP2 domain, in which case the AP2 domain of the transcription factor binds to a transcription regulating region, such as AtERF1, which binds to the motif AGCCGCC (the "GCC box") that are present in promoters of genes such as PDF1.2. The transcription factors of the invention also comprise an amino acid subsequence that forms a transcription activation domain that regulates expression of one or more abiotic stress tolerance genes in a plant when the transcription factor binds to the regulating region.

A "sample" with respect to a material containing nucleic acid molecules may comprise a bodily fluid; an extract from a cell, chromosome, organelle, or membrane isolated from a cell; genomic DNA, RNA, or cDNA in solution or bound to a substrate; a cell; a tissue; a tissue print; a forensic sample; and the like. In this context "substrate" refers to any rigid or semi-rigid support to which nucleic acid molecules or proteins are bound and includes membranes, filters, chips, slides, wafers, fibers, magnetic or nonmagnetic beads, gels, capillaries or other tubing, plates, polymers, and microparticles with a variety of surface forms including wells, trenches, pins, channels and pores. A substrate may also refer to a reactant in a chemical or biological reaction, or a substance acted upon (for example, by an enzyme).

<u>Transcription Factors Modify expression of Endogenous Genes</u>

A transcription factor may include, but is not limited to, any polypeptide that can activate or repress transcription of a single gene or a number of genes. As one of ordinary skill in the art recognizes, transcription factors can be identified by the presence of a region or domain of structural similarity or identity to a specific consensus sequence or the presence of a specific consensus DNA-binding site or DNA-binding site motif (for example, in Riechmann et al. (2000) Science 290: 2105-2110).

Generally, the transcription factors encoded by the present sequences are involved in cell differentiation and proliferation and the regulation of growth. Accordingly, one skilled in the art would recognize that by expressing the present sequences in a plant, one may change the expression of autologous genes or induce the expression of introduced genes. By affecting the expression of similar autologous sequences in a plant that have the biological activity of the present sequences, or by introducing the present sequences into a plant, one may alter a plant's phenotype to one with improved traits related to drought stress, shade tolerance or C/N sensing. The sequences of the invention may also be used to transform a plant and introduce desirable traits not found in the wild-type cultivar or strain. Plants may then be selected for those that produce the most desirable degree of over- or under-expression of target genes of interest and coincident trait improvement.

The sequences of the present invention may be from any species, particularly plant species, in a naturally-occurring form or from any source whether natural, synthetic, semi-synthetic or recombinant. The sequences of the invention may also include fragments of the present amino acid sequences. Where "amino acid sequence" is recited to refer to an amino acid sequence of a naturally occurring protein molecule, "amino acid sequence" and like terms are not meant to limit the amino acid sequence to the complete native amino acid sequence associated with the recited protein molecule.

In addition to methods for modifying a plant phenotype by employing one or more polynucleotides and polypeptides of the invention described herein, the polynucleotides and polypeptides of the invention have a variety of additional uses. These uses include their use in the recombinant production (i.e., expression) of proteins; as regulators of plant gene expression, as diagnostic probes for the presence of complementary or partially complementary nucleic acids (including for detection of natural coding nucleic acids); as substrates for further reactions, for example, mutation reactions, PCR reactions, or the like; as substrates for cloning for example, including digestion or ligation reactions; and for identifying exogenous or endogenous modulators of the transcription factors. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, or the like. The polynucleotide can be single-stranded or double-stranded DNA or RNA. The polynucleotide optionally comprises modified bases or a modified backbone. The polynucleotide can be, for example, genomic DNA or RNA, a transcript (such as an mRNA), a cDNA, a PCR product, a cloned DNA, a synthetic DNA or RNA, or the like. The polynucleotide can comprise a sequence in either sense or antisense orientations.

Expression of genes that encode transcription factors that modify expression of endogenous genes, polynucleotides, and proteins are well known in the art. In addition, transgenic plants comprising isolated polynucleotides encoding transcription factors may also modify expression of endogenous genes, polynucleotides, and proteins. Examples include Peng et al. (1997) Genes Development 11: 3194-3205, and Peng et al. (1999) Nature, 400: 256-261). In addition, many others have demonstrated that an *Arabidopsis* transcription factor expressed in an exogenous plant species elicits the same or very similar phenotypic response (for example, in Fu et al. (2001) Plant Cell 13: 1791-1802; Nandi et al. (2000) Curr. Biol 10: 215-218; Coupland (1995) Nature 377:482483; and Weigel and Nilsson (1995) Nature 377: 482-500).

In another example, Mandel et al. (1992) Cell 71-133-143, and Suzuki et al.(2001) Plant J.28: 409-418 teach that a transcription factor expressed in another plant species elicits the same or very similar phenotypic response of the endogenous sequence, as often predicted in earlier studies of *Arabidopsis* transcription factors in *Arabidopsis* (Mandel et al. (1992) *supra;* and Suzuki et al. (2001) *supra).* Other examples include Muller et al. (2001) Plant J. 28: 169-179; Kim et al. (2001) Plant J. 25: 247-259; Kyozuka and Shimamoto (2002) Plant Cell Physiol. 43: 130-135; Boss and Thomas (2002) Nature, 416: 847-850; He et al. (2000) Transgenic Res. 9: 223-227; and Robson et al. (2001) Plant J. 28: 619-631.

In yet another example, Gilmour et al. (1998) Plant J. 16: 433-442, teach an *Arabidopsis* AP2 transcription factor, CBF1, which, when overexpressed in transgenic plants, increases plant freezing tolerance. Jaglo et al. (2001) Plant Physiol. 127: 910-917, further identified sequences in *Brassica napus* which encode CBF-like genes and that transcripts for these genes accumulated rapidly in response to low temperature. Transcripts encoding CBF-like proteins were also found to accumulate rapidly in response to low temperature in wheat, as well as in tomato. An alignment of the CBF proteins from *Arabidopsis, B. napus,* wheat, rye, and tomato revealed the presence of conserved consecutive amino acid residues, PKK/RPAGRxKFxETRHP and DSAWR, that bracket the AP2/EREBP DNA binding domains of the proteins and distinguish them from other members of the AP2/EREBP protein family (Jaglo et al. (2001) *supra*).

Transcription factors mediate cellular responses and control traits through altered expression of genes containing cis-acting nucleotide sequences that are targets of the introduced transcription factor. It is well appreciated in the art that the effect of a transcription factor on cellular responses or a cellular trait is determined by the particular genes whose expression is either directly or indirectly (for example, by a cascade of transcription factor binding events and transcriptional changes) altered by transcription factor binding. In a global analysis of transcription comparing a standard condition with one in which a transcription factor is overexpressed, the resulting transcript profile associated with transcription factor overexpression is related to the trait or cellular process controlled by that transcription factor. For example, the PAP2 gene (and other genes in the MYB family) have been shown to control anthocyanin biosynthesis through regulation of the expression of genes known to be involved in the anthocyanin biosynthetic pathway (Bruce et al. (2000) Plant Cell, 12: 65-79; Borevitz et al. (2000) Plant Cell 12: 2383-93). Further, global transcript profiles have been used successfully as diagnostic tools for specific cellular states (for example, cancerous vs. non-cancerous; Bhattacharjee et al. (2001) Proc Natl. Acad. Sci., USA, 98: 13790-13795; Xu et al. (2001) Proc. Natl Acad. Sci., USA, 98: 15089.15094). Consequently, it is evident to one skilled in the art that similarity of transcript profile upon overexpression of different transcription factors would indicate similarity of transcription factor function.

## Polypeptides and Polynucleotides of the Application

The present invention provides, among other things, transcription factors (TFs), and transcription factor homolog polypeptides, and isolated or recombinant polynucleotides encoding the polypeptides, or novel sequence variant polypeptides or polynucleotides encoding novel variants of transcription factors derived from the specific sequences provided here.

The polynucleotides of the invention can be or were ectopically expressed overexpressor plant cells and the changes in the expression levels of a number of genes, polynucleotides, and/or proteins of the plant cells observed. Therefore, the polynucleotides and polypeptides can be employed to change expression levels of a genes, polynucleotides, and/or proteins of plants or plant cells. These polypeptides and polynucleotides may be employed to modify a plants characteristics, particularly drought tolerance. The polynucleotides of the invention can be or were ectopically expressed in overexpressor or knockout plants and the changes in the characteristic(s) or trait(s) of the plants observed. Therefore, the polynucleotides and polypeptides can be employed to improve the characteristics of plants. The polypeptide sequences of the sequence listing have been shown to confer increased drought or shade tolerance or altered C/N sensing when these polypeptides are overexpressed in *Arabidopsis* plants. These polynucleotides have been shown to have a strong association with these traits, in that plants that overexpress these sequences are more tolerant to drought, shade, or have altered C/N sensing, respectively. The invention also encompasses a complement of the polynucleotides. The polynucleotides are also useful for screening libraries of molecules or compounds for specific binding and for creating transgenic plants having improved traits. Altering the expression levels of equivalogs of these sequences, including paralogs and orthologs in the Sequence Listing, and other orthologs that are structurally and sequentially similar to the former orthologs, has been shown and is expected to confer similar phenotypes, including altered C/N sensing, drought and/or shade tolerance in plants.

In some cases, exemplary polynucleotides encoding the polypeptides of the sequence listing were identified in the *Arabidopsis thaliana* GenBank database using publicly available sequenoe analysis programs and parameters. Sequences initially identified were then further characterized to identify sequences comprising specified sequence strings corresponding to sequence motifs present in families of known transcription factors. In addition, further exemplary polynucleotides encoding the polypeptides of the invention were identified in the plant GenBank database using publicly available sequence analysis programs and parameters. Sequences initially identified were then further characterized to identify sequences comprising specified sequence strings corresponding to sequence motifs present in families of know transcription factors. Polynucleotide sequences meeting such criteria were confirmed as transcription factors.

Additional polynucleotides of the invention were identified by screening *Arabidopsis thaliana* and/or other plant cDNA libraries with probes corresponding to known transcription factors under low stringency hybridization conditions. Additional sequences, including full length coding sequences were subsequently recovered by the rapid amplification of cDNA ends (RACE) procedure, using a commercially available kit according to the manufacturer's instructions. Where necessary, multiple rounds of RACE are performed to isolate 5' and 3' ends. The full-length cDNA was then recovered by a routine end-to-end polymerase chain reaction (PCR) using primers specific to the isolated 5' and 3' ends. Exemplary sequences are provided in the Sequence Listing.

The polynucleotides are particularly useful when they are hybridizable array element in a microarray. Such microarray can be employed to monitor the expression of genes that are differentially expressed in response to limited light, drought, other osmotic stresses, or low nitrogen availability. The microarray can be used in large scale genetic or gene expression analysis of a large number of polynucleotides; or in the diagnosis of, for example, drought stress before phenotypic symptoms are evident. Furthermore, the microarray can be employed to investigate cellular responses, such as cell proliferation, transformation, and the like.

When the polynucleotides of the invention may also be used as hybridizable array elements in a microarray, the array element are organized in an ordered fashion so that each element is present at a specified location on the substrate. Because the array elements are at specified locations on the substrate, the hybridization patterns and intensities (which together create a unique expression profile) can be interpreted in terms of expression levels of particular genes and can be correlated with a particular stress, pathology, or treatment.

The invention also entails an agronomic composition comprising a polynucleotide of the invention in conjunction with a suitable carrier and a method for altering a plant's unit using the composition.

Examples of specific polynucleotide and polypeptides of the invention, and equivalog sequences, along with descriptions of the gene families that comprise these polynucleotides and polypeptides, are provided below.

Examples of specific polynucleotide and polypeptides of the invention, and equivalog sequences, are provided below.

Polypeptide sequences of the sequence listing, including, for example, *Arabidopsis* sequences G2133; G1274, G922, G2999, G3086, G354, G1792, G2053, G975, G1069, G916, G1820, G2701, G47, G2854, G2789, G634, G175, G2839, G1452, G3091, G489, G303, G2992, and G682 (SEQ ID NOs: 12, 6, 4, 14, 16, 228, 8, 10, 238, 240, 236, 244, 246, 2, 252, 248, 232, 224, 250, 242, 254, 230, 226, 50 and 234, respectively) have been shown to confer increased drought tolerance when expression of these polypeptides is altered in *Arabidopsis* plants. These polynucleotides have been shown to have a strong association with drought stress tolerance, in that plants that overexpress these sequences are more tolerant to drought, Exemplary sequences of the invention include G2133.

The present disclosure also encompasses the complements of these polynucleotides. The polynucleotides are also useful for screening libraries of molecules or compounds for specific binding and for creating transgenic plants having altered drought tolerance. Equivalogs of these sequences, including paralogs and orthologs in the Sequence Listing, and other orthologs that are structurally and sequentially similar to the former orthologs, are expected to confer drought tolerance in when their expression is altered.

<u>The AP2 family, including the G47/G2133 and G1792 clades. AP2</u> (APETALA2) and EREBPs (Ethylene-Responsive Element Binding Proteins) are the prototypic members of a family of transcription factors unique to plants, whose distinguishing characteristic is that they contain the so-called AP2 DNA-binding domain (Riechmann and Meyerowitz (1998) Biol. Chem. 379: 633-646). The AP2 domain was first recognized as a repeated motif within the *Arabidopsis thaliana* AP2 protein (Jofuku et al. (1994) Plant Cell 6: 1211-122). Shortly afterwards, four DNA-binding proteins from tobacco were identified that interact with a sequence that is essential for the responsiveness of some promoters to the plant hormone ethylene, and were designated as *ethylene-responsive element binding proteins* (EREBPs; Ohme-Takagi et al. (1995) Plant Cell 7: 173-182). The DNA-binding domain of EREBP-2 was mapped to a region that was common to all four proteins (Ohme-Takagi et al (1995) *supra),* and that was found to be closely related to the AP2 domain (Weigel (1995) Plant Cell 7: 388-389) but that did not bear sequence similarity to previously known DNA-binding motifs.

AP2/BREBP genes form a large family, with many members known in several plant-species (Okamuro et al. (1997) Proc Natl. Acad. Sci. USA 94: 7076-7081; Riechmann and Meyerowitz (1998) *supra).* The number of AP2/EREBP genes in the *Arabidopsis thaliana* genome is approximately 145 (Riechmann et al. (2000) Science 290: 2105-2110). The APETALA2 class is characterized by the presence of two AP2 DNA binding domains, and contains 14 genes. The AP2/ERF is the largest subfamily, and includes 125 genes which are involved in abiotic (DREB subgroup) and biotic (ERF subgroup) stress responses and the RAV subgroup includes 6 genes which all have a B3 DNA binding domain in addition to the AP2 DNA binding domain (Kagaya et al. (1999) Nucleic Acids Res. 27: 470-478).

*Arabidopsis* AP2 is involved in the specification of sepal and petal identity through its activity as a homeotic gene that forms part of the combinatorial genetic mechanism of floral organ identity determination and it is also required for normal ovule and seed development (Bowman et al. (1991) Development 112: 1-20; Jofuku et al. (1994) *supra*). *Arabidopsis* ANT is required for ovule development and it also plays a role in floral organ growth (Elliott et al. (1996) Plant Cell 8: 155-168; Klucher et al. (1996) Plant Cell 8: 137-153). Finally, maize Gll5 regulates leaf epidermal cell identity (Moose et al. (1996) Genes Dev. 10: 3018-3027).

The attack of a plant by a pathogen may induce defense responses that lead to resistance to the invasion, and these responses are associated with transcriptional activation of defense-related genes, among them those encoding pathogenesis-related (PR) proteins. The involvement of EREBP-like genes in controlling the plant defense response is based on the observation that many PR gene promoters contain a short cis-acting element that mediates their responsiveness to ethylene (ethylene appears to be one of several signal molecules controlling the activation of defense responses). Tobacco EREBP-1, -2; - 3, and -4, and tomato Pti4, Pti5 and Pti6 proteins have been shown to recognize such cis-acting elements (Ohme-Takagi (1995) *supra*; Zhou et al. (1997) EMBO J. 16: 3207-3218). In addition, Pti4, Pti5, and Pti6 proteins have been shown to directly interact with Pto, a protein kinase that confers resistance against *Pseudomonas syringae* pv tomato (Zhou et al. (1997) *supra*). Plants are also challenged by adverse environmental conditions like cold or drought, and EREBP-like proteins appear to be involved in the responses to these abiotic stresses as well. COR (for cold-regulated) gene expression is induced during cold acclimation, the process by which plants increase their resistance to freezing in response to low unfreezing temperatures. The *Arabidopsis* EREBP-like gene CBF1 (Stockinger et al. (1997) Proc. Natl. Acad. Sci. USA 94: 1035-1040) is a regulator of the cold acclimation response, because ectopic expression of CBF1 in *Arabidopsis* transgenic plants induced COR gene expression in the absence of a cold stimulus, and the plant freezing tolerance was increased (Jaglo-Ottosen et al. (1998) Science 280: 104-106). Finally, another *Arabidopsis* EREBP-like gene, ABI4, is involved in ABA signal transduction, because abi4 mutants are insensitive to ABA (ABA is a plant hormone that regulates many agronomically important aspects of plant development; Finkelstein et al. (1998) Plant Cell 10: 1043-1054).

Of the sequences examined to date, two valine residues were found that are present in members of the G47 clade but not outside of the clade (indicated by the arrows in Figure 3). All members of the clade examined thus far have the subsequence:

V-$(X)_{17}$-A-A-V-A-H-D-X-A, where X is any amino acid and the identified residues are indicated by the residues shown in the boxes in Figure 3.

**Table 1** shows exemplary sequences of the disclosure that, in many cases, confer drought tolerance when overexpressed. The polypeptides are identified by polypeptide SEQ ID NO and Identifier (for example, Gene ID (GID) No., accession number or other name), presented in order of similarity to the first *Arabidopsis* sequence listed for each set, and includes the conserved domains of the polypeptide in amino acid coordinates, the respective domain sequences, and the extent of identity in percentage terms to the first *Arabidopsis* sequence listed for each set.

Table 1. Gene families and binding domains for exemplary sequences conferring drought tolerance, including paralogs and orthologs

| SEQ ID NO | GID | Species | Conserved Domains in Polypeptide Amino Acid Coordinates | Conserved Domains in Polynucleotide Base Coordinates | Conserved Domain Sequence | %ID in conserved domain |
|---|---|---|---|---|---|---|
| | | | | | | %ID to G2133 |
| 12 | G2133 | *Arabidopsis thaliana* | AP2:10-77 | AP2:53-256 | DQSKYKGIRRRKWGKWVSEIRVPGTRQRLWLGSFSTAEGAAVAHDVAFYCLHRPSSLDDESFNFPHLL | 100% |
| 94 | G3646 | *Brassica oleracea* | AP2:10-77 | AP2:203-406 | HQAKYKGIRRRKWGKWVSEIRVPATRERLWLGSFSTAEGAAVAHDVAFYCLHRPSSLDNEAFNFPHLL | 91% |
| 92 | G3645 | *Brassica rapa subsp. Pekinensis* | AP2: 10-75 | AP2: 40-237 | TQSKYKGIRRRKWGKWVSEIRVPGTRDRLWLGSFSTAEGAAVAHDVAFYCLHQPNSLESLNFPHLL | 89% |
| 2 | G47 | *Arabidopsis thaliana* | AP2:10-75 | AP2:65-262 | SQSKYKGIRRRKWGKWVSEIRVPGTRDRLWLGSFSTAEGAAVAHDVAFFCLHQPDSLESLNFPHLL | 88% |
| 88 | G3643 | *Glycine max* | AP2: 13-78 | AP2:101-298 | TNNKLKGVRRRKWGKWVSEIRVPGTQERLWLGTYATPEAAAVAHDVAVYCLSRPSSLDKLNFPETL | 69% |
| 96 | G3647 | *Zinnia elegans* | AP2: 13-78 | AP2: 53-250 | SQKTYKGVRCRRWGKWVSEIRVPGSRERLWLGTYSTPEGAAVAHDVASYCLKGNTSFHKLNIPSML | 63% |
| 90 | G3644 | *Oryza sativa (japonica cultivar-group)* | AP2: 52-122 | AP2: 154-366 | ERCRYRGVRRRRWGKWVSEIRVPGTRERLWLGSYATPEAAAVAHDTAVYFLRGGAGDGGGGGATLNFPERA | 54% |
| 98 | G3649 | *Oryza sativa (japonica cultivar-group)* | AP2: 15-87 | AP2: 43-261 | EMMRYRGVRRRRWGKWVSEIRVPGTRERLWLGSYATAEAAAVAHDAAVCLLRLGGGRRAAAGGGGGLNFPARA | 53% |

(continued)

| SEQ ID NO | GID | Species | Conserved Domains in Polypeptide Amino Acid Coordinates | Conserved Domains in Polynucleotide Base Coordinates | Conserved Domain Sequence | %ID in conserved domain |
|---|---|---|---|---|---|---|
| | | | | | | %ID to G2133 |
| 100 | G3651 | *Oryza sativa (japonica cultivar-group)* | AP2: 60-130 | AP2: 178-390 | ERCRYRGVRRRRWGK WVSEIRVPGTRERLWL GSYATPEAAAVAHDTA VYFLRGGAGDGGGGG ATAQLPGAR | 52% |

Table 2 shows in the first column the polypeptide SEQ ID NO; in the second column the Identifier (for example, Gene ID (GID) No); in the third column the transcription factor family to which the polynucleotide belongs; and in the fourth column the amino acid residue positions of the conserved domain in amino acid (AA) coordinates:

Table 2. Gene families and conserved domains

| Polypeptide SEQ ID NO | Identifier | Family | Conserved Domains in Amino Acid Coordinates |
|---|---|---|---|
| 12 | G2133 | AP2 | 10-77 |

Producing Polypeptides

The polynucleotides of the invention include sequences that encode transcription factors and transcription factor homolog polypeptides and sequences complementary thereto, as well as unique fragments of coding sequence, or sequence complementary thereto. Such polynucleotides can be, for example, DNA or RNA, the latter including mRNA, cRNA, synthetic RNA, genomic DNA, cDNA synthetic DNA, oligonucleotides, etc. The polynucleotides are either double-stranded or single-stranded, and include either, or both sense (i.e., coding) sequences and antisense (i.e., non-coding, complementary) sequences. The polynucleotides include the coding sequence of a transcription factor, or transcription factor homolog polypeptide, in isolation, in combination with additional coding sequences (for example, a purification tag, a localization signal, as a fusion-protein, as a pre-protein, or the like), in combination with non-coding sequences (for example, introns or inteins, regulatory elements such as promoters, enhances, terminators, and the like), and/or in a vector or host environment in which the polynucleotide encoding a transcription factor or transcription factor homolog polypeptide is an endogenous or exogenous gene.

A variety of methods exist for producing the polynucleotides of the invention. Procedures for identifying and isolating DNA clones are well known to those of skill in the art, and are described in, for example, Berger and Kimmel, Guide to Molecular Cloning Techniques, Methods in Enzymology, vol. 152 Academic Press, Inc., San Diego, CA ("Berger"); Sambrook et al. Molecula Clonining - A Laboratory Manual (2nd ed.), VoL 1-3, Cold Spring Harbor Laboratory, Cold Spring Harbor, New York, 1989 ("Sambrook") and Current Protocols in Molecular Biology, Ausubel et al. eds., Current Protocols, a joint venture between Greene Publishing Associates, Inc. and John Wiley & Sons, Inc., (supplemented through 2000) ("Ausubel").

Alternatively, polynucleotides of the invention, can be produced by a variety of in vitro amplification methods adapted to the present invention by appropriate selection of specific or degenerate primers. Examples of protocols sufficient to direct persons of skill through in vitro amplification methods, including the polymerase chain reaction (PCR) the ligase chain reaction (LCR), Qβ-replicase amplification and other RNA polymerase mediated techniques (for example, NASBA), for example, for the production of the homologous nucleic acids of the invention are found in Berger (supra), Sambrook (supra), and Ausubel (supra), as well as Mullis et al. (1987) PCR Protocols A Guide to Methods and Applications (Innis et al. eds) Academic Press Inc. San Diego, CA (1990) (Innis). Improved methods for cloning in vitro amplified nucleic acids are described in Wallace et al. US Pat. No. 5,426,039. Improved methods for amplifying large nucleic acids by PCR are summarized in Cheng et al. (1994) Nature 369: 684-685 and the references cited therein, in which PCR amplicons of up to 40 kb are generated. One of skill will appreciate that essentially any RNA can be converted into a double-stranded DNA suitable for restriction digestion, PCR expansion and sequencing using reverse transcriptase and a polymerase (for example, in Ausubel, Sambrook and Berger, all *supra*).

Alternatively, polynucleotides and oligonucleotides of the invention can be assembled from fragments produced by solid-phase synthesis methods. Typically, fragments of up to approximately 100 bases are individually synthesized and then enzymatically or chemically ligated to produce a desired sequence, for example, a polynucleotide encoding all or part of a transcription factor. For example, chemical synthesis using the phosphoramidite method is described, for example, by Beaucage et al. (1981) Tetrahedron Letters 22: 1859-1869; and Matthes et al. (1984) EMBO J. 3: 801-805. According to such methods, oligonucleotides are synthesized, purified, annealed to their complementary strand, ligated and then optionally cloned into suitable vectors. And if so desired, the polynucleotides and polypeptides of the invention can be custom ordered from any of a number of commercial suppliers.

Homologous Sequences

Sequences homologous, i.e., that share significant sequence identity or similarity, to those provided in the Sequence Listing, derived from *Arabidopsis thaliana* or from other plants of choice, are also an aspect of the invention. Homologous sequences can be derived from any plant including monocots and dicots and in particular agriculturally important plant species, including but not limited to, crops such as soybean, wheat, corn (maize), potato, cotton, rice, rape, oilseed rape (including canola), sunflower, alfalfa, clover, sugarcane, and turf; or fruits and vegetables, such as banana, blackberry, blueberry, strawberry, and raspberry, cantaloupe, carrot, cauliflower, coffee, cucumber, eggplant, grapes, honeydew, lettuce, mango, melon, onion, papaya, peas, peppers, pineapple, pumpkin, spinach, squash, sweet corn, tobacco, tomato, tomatillo, watermelon, rosaceous fruits (such as apple, peach, pear, cherry and plum) and vegetable brassicas (such as broccoli, cabbage, cauliflower, Brussels sprouts, and kohlrabi). Other crops, including fruits and vegetables, whose phenotype can be changed and which comprise homologous sequences include barley; rye; millet; sorghum; currant; avocado; citrus fruits such as oranges, lemons, grapefruit and tangerines, artichoke, cherries; nuts such as the walnut and peanut; endive; leek; roots such as arrowroot, beet, cassava, turnip, radish, yam, and sweet potato; and beans. The homologous sequences may also be derived from woody species, such as pine, poplar and eucalyptus, or mint or other labiates. In addition, homologous sequences may be derived from plants that are evolutionarily related to crop plants, but which may not have yet been used as crop plants. Examples include deadly nightshade (*Atropa belladona*), related to tomato; jimson weed (Datura strommium), related to peyote; and teosinte (Zea species), related to corn (maize).

Orthologs and Paralogs

Homologous sequences as described above can comprise orthologous or paralogous sequences. Several different methods are known by those of skill in the art for identifying and defining these functionally homologous sequences. Three general methods for defining orthologs and paralogs are described; an ortholog, paralog or homolog may be identified by one or more of the methods described below.

Within a single plant species, gene duplication may cause two copies of a particular gene, giving rise to two or more genes with similar sequence and often similar function known as paralogs. A paralog is therefore a similar gene formed by duplication within the same species. Paralogs typically cluster together or in the same clade (a group of similar genes) when a gene family phylogeny is analyzed using programs such as CLUSTAL (Thompson et al. (1994) Nucleic Acids Res. 22: 4673-4680; Higgins et al. (1996) Methods Enzymol. 266: 383-402). Groups of similar genes can also be identified with pair-wise BLAST analysis (Feng and Doolittle (1987) J. Mol. Evol. 25: 351-360). For examples, a clade of very similar MADS domain transcription factors from *Arabidopsis* all share a common function in flowering time (Ratcliffe et al. (2001) Plant Physiol. 126: 122-132), and a group of very similar AP2 domain transcription factors from *Arabidopsis* are involved in tolerance of plants to freezing (Gilmour et al. (1998) Plant J. 16: 433-442). Analysis of groups of similar genes with similar function that fall within one clade can yield sub-sequences that are particular to the clade. These sub-sequences, known as consensus sequences, can not only be used to define the sequences within each clade, but define the functions of these genes; genes within a clade may contain paralogous sequences, or orthologous sequences that share the same function (for example, in Mount (2001), in Bioinformatics: Sequence and Genome Analysis Cold Spring Harbor Laboratory Press, Cold Spring Harbor, New York, page 543).

Speciation, the production of new species from a parental species, can also give rise to two or more genes with similar sequence and similar function. These genes, termed orthologs, often have an identical function within their host plants and are often interchangeable between species without losing function. Because plants have common ancestors, many genes in any plant species will have a corresponding orthologous gene in another plant species. Once a phylogenic tree for a gene family of one species has been constructed using a program such as CLUSTAL (Thompson et al. (1994) Nucleic Acids Res. 22: 46734680; Higgins et al. (1996) *supra*) potential orthologous sequences can be placed into the phylogenetic tree and their relationship to genes from the species of interest can be determined. Orthologous sequences can also be identified by a reciprocal BLAST strategy. Once an orthologous sequence has been identified, the function of the ortholog can be deduced from the identified function of the reference sequence.

Transcription factor gene sequences are conserved across diverse eukaryotic species lines (Goodrich et al. (1993) Cell 75: 519-530; Lin et al. (1991) Nature 353: 569-571; Sadowski et al. (1988) Nature 335: 563-564). Plants are no exception to this observation; diverse plant species possess transcription factors that have similar sequences and functions.

Orthologous genes from different organisms have highly conserved functions, and very often essentially identical functions (Lee et al. (2002) Genome Res. 12: 493-502; Remm et al. (2001) J. Mol. Biol. 314: 1041-1052). Paralogous genes, which have diverged through gene duplication, may retain similar functions of the encoded proteins. In such cases, paralogs can be used interchangeably with respect to certain embodiments of the instant invention (for example, transgenic expression of a coding sequence). An example of such highly related paralogs is the CBF family, with four well-defined members in *Arabidopsis* (CBF 1, GBF2, CBF3 and CBF4) and at least one ortholog in *Brassica napus,* all of which control pathways involved in both freezing and drought stress (Gilmour et al. (1998) Plant J. 16: 433-442; Jaglo et al. (1998) Plant Physiol. 127: 910-917).

The following references represent a small sampling of the many studies that demonstrate that conserved transcription factor genes from diverse species are likely to function similarly (i.e., regulate similar target sequences and control the same traits), and that transcription factors may be transformed into diverse species to confer or improve traits.

(1) Distinct *Arabidopsis* transcription factors, including G28 (US Patent 6,664,446), G432 (US Patent Application 20040045049; SEQ ID NO: 290 in the present Sequence Listing), G867 (US Patent Application 20040098764; SEQ ID NO: 630 in the present Sequence Listing), and G1073 (US Patent Application 20040128712; SEQ ID NO: 300 in the present Sequence Listing), have been shown to confer abiotic stress tolerance when the sequences are overexpressed. The polypeptides sequences belong to distinct clades of transcription factor polypeptides that include members from diverse species. In each case, a significant number of sequences derived from both dicots and monocots have been shown to confer tolerance to various abiotic stresses when the sequences were overexpressed.

(2) The *Arabidopsis* NPR1 gene regulates systemic acquired resistance (SAR) (Cao et al. (1997) Cell 88: 57-63); over-expression of NPR1 leads to enhanced resistance in *Arabidopsis.* When either *Arabidopsis* NPR1 or the rice NPR1 ortholog was overexpressed in rice (which, as a monocot, is diverse from *Arabidopsis*), challenge with the rice bacterial blight pathogen *Xanthomonas oryzae* pv. Oryzae, the transgenic plants displayed enhanced resistance (Chern et al. (2001) Plant J. 27: 101-113). NPR1 acts through activation of expression of transcription factor genes, such as TGA2 (Fan and Dong (2002) Plant Cell 14: 1377-1389).

(3) E2F genes are involved in transcription of plant genes for proliferating cell nuclear antigen (PCNA). Plant E2Fs share a high degree of similarity in amino acid sequence between monocots and dicots, and are even similar to the conserved domains of the animal E2Fs. Such conservation indicates a functional similarity between plant and animal E2Fs. E2F transcription factors that regulate meristem development act through common cis-elements, and regulate related (PCNA) genes (Kosugi and Ohashi, (2002) Plant J. 29: 45-59).

(4) The ABI5 gene (ABA insensitive 5) encodes a basic leucine zipper factor required for ABA response in the seed and vegetative tissues. Co-transformation experiments with ABI5 cDNA constructs in rice protoplasts resulted in specific transactivation of the ABA-inducible wheat, *Arabidopsis,* bean, and barley promoters. These results demonstrate that sequentially similar ABI5 transcription factors are key targets of a conserved ABA signaling pathway in diverse plants. (Gampala et al. (2001) J. Biol. Chem. 277: 1689-1694).

(5) Sequences of three *Arabidopsis* GAMYB-like genes were obtained on the basis of sequence similarity to GAMYB genes from barley, rice, and *L. temulentum.* These three *Arabidopsis* genes were determined to encode transcription factors (AtMYB33, AtMYB65, and AtMYB101) and could substitute for a barley GAMYB and control $\alpha$-amylase expression (Gocal et al. (2001) Plant Physiol. 127: 1682-1693).

(6) The floral control gene LEAFY from *Arabidopsis* can dramatically accelerate flowering in numerous dictoyledonous plants. Constitutive expression of *Arabidopsis* LEAFY also caused early flowering in transgenic rice (a monocot), with a heading date that was 26-34 days earlier than that of wild-type plants. These observations indicate that floral regulatory genes from *Arabidopsis* are useful tools for heading date improvement in cereal crops (He et al. (2000) Transgenic Res. 9: 223-227).

(7) Bioactive gibberellins (GAs) are essential endogenous regulators of plant growth. GA signaling tends to be conserved across the plant kingdom. GA signaling is mediated via GAI, a nuclear member of the GRAS family of plant transcription factors. *Arabidopsis* GAI has been shown to function in rice to inhibit gibberellin response pathways (Fu et al. (2001) Plant Cell 13: 1791-1802).

(8) The *Arabidopsis* gene SUPERMAN (SUP), encodes a putative transcription factor that maintains the boundary between stamens and carpels. By over-expressing *Arabidopsis* SUP in rice, the effect of the gene's presence on whorl boundaries was shown to be conserved. This demonstrated that SUP is a conserved regulator of floral whorl boundaries and affects cell proliferation (Nandi et al. (2000) Curr. Biol. 10: 215-218).

(9) Maize, petunia and *Arabidopsis* myb transcription factors that regulate flavonoid biosynthesis are genetically similar and affect the same trait in their native species; therefore, sequence and function of these myb transcription factors correlate with each other in these diverse species (Borevitz et al. (2000) Plant Cell 12: 2383-2394).

(10) Wheat reduced height-1 (Rht-B1/Rht-D1) and maize dwarf-8 (d8) genes are orthologs of the *Arabidopsis* gibberellin insensitive (GAI) gene. Both of these genes have been used to produce dwarf grain varieties that have improved grain yield. These genes encode proteins that resemble nuclear transcription factors and contain an SH2-like domain, indicating that phosphotyrosine may participate in gibberellin signaling. Transgenic rice plants containing a mutant GAI allele from *Arabidopsis* have been shown to produce reduced responses to gibberellin and are dwarfed, indicating that mutant GAI orthologs could be used to increase yield in a wide range of crop species (Peng et al. (1999) Nature 400: 256-261).

Transcription factors that are homologous to the listed sequences will typically share at least about 70% amino acid sequence identity in their conserved domain. More closely related transcription factors can share at least about 79% or about 90% or about 95% or about 98% or more sequence identity with the listed sequences, or with the listed sequences but excluding or outside a known consensus sequence or consensus DNA-binding site, or with the listed sequences excluding one or all conserved domains. Factors that are most closely related to the listed sequences share, for example, at least about 85%, about 90% or about 95% or more % sequence identity to the listed sequences, or to the listed sequences but excluding or outside a known consensus sequence or consensus DNA-binding site or outside one or all conserved domain. At the nucleotide level, the sequences will typically share at least about 40% nucleotide sequence identity, preferably at least about 50%, about 60%, about 70% or about 80% sequence identity, and more preferably about 85%, about 90%, about 95% or about 97% or more sequence identity to one or more of the listed sequences, or to a listed sequence but excluding or outside a known consensus sequence or consensus DNA-binding site, or outside one or all conserved domain. The degeneracy of the genetic code enables major variations in the nucleotide sequence of a polynucleotide while maintaining the amino acid sequence of the encoded protein. AP2 domains within the AP2 transcription factor family may exhibit a higher degree of sequence homology, such as at least 70% amino acid sequence identity including conservative substitutions, and preferably at least 80% sequence identity, and more preferably at least 85%, or at least about 86%, or at least about 87%, or at least about 88%, or at least about 90%, or at least about 95%, or at least about 98% sequence identity. Transcription factors that are homologous to the listed sequences should share at least 30%, or at least about 60%, or at least about 75%, or at least about 80%, or at least about 90%, or at least about 95% amino acid sequence identity over the entire length of the polypeptide or the homolog.

Percent identity can be determined electronically, for example, by using the MEGALIGN program (DNASTAR, Inc. Madison, Wis.). The MEGALIGN program can create alignments between two or more sequences according to different methods, for example, the clustal method (for example, in Higgins and Sharp (1988) Gene 73: 237-244). The clustal algorithm groups sequences into clusters by examining the distances between all pairs. The clusters are aligned pairwise and then in groups. Other alignment algorithms or programs may be used, including FASTA, BLAST, or ENTREZ, FASTA and BLAST, and which may be used to calculate percent similarity. These are available as a part of the GCG sequence analysis package (University of Wisconsin, Madison, Wis.), and can be used with or without default settings. ENTREZ is available through the National Center for Biotechnology Information. In one embodiment, the percent identity of two sequences can be determined by the GCG program with a gap weight of 1, for example, each amino acid gap is weighted as if it were a single amino acid or nucleotide mismatch between the two sequences (USPN 6,262,333).

Other techniques for alignment are described in Methods in Enzymology, vol. 266, Computer Methods for Macromolecular Sequence Analysis (1996), ed. Doolittle, Academic Press, Inc., San Diego, Calif., USA. Preferably, an alignment program that permits gaps in the sequence is utilized to align the sequences. The Smith-Waterman is one type of algorithm that permits gaps in sequence alignments (Shpaer (1997) Methods Mol. Biol. 70: 173-187). Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer. This approach improves ability to pick up distantly related matches, and is especially tolerant of small gaps and nucleotide sequence errors. Nucleic acid-encoded amino acid sequences can be used to search both protein and DNA databases.

The percentage similarity between two polypeptide sequences, for example, sequence A and sequence B, is calculated by dividing the length of sequence A, minus the number of gap residues in sequence A, minus the number of gap residues in sequence B, into the sum of the residue matches between sequence A and sequence B, times one hundred. Gaps of low or of no similarity between the two amino acid sequences are not included in determining percentage similarity. Percent identity between polynucleotide sequences can also be counted or calculated by other methods known in the art, for example, the Jotun Hein method (for example, in Hein (1990) Methods Enzymol. 183: 626-645). Identity between sequences can also be determined by other methods known in the art, for example, by varying hybridization conditions (US Patent Application No. 20010010913).

Thus, the invention provides methods for identifying a sequence similar or paralogous or orthologous or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an internet or intranet) and a query is made against the sequence database using the relevant sequences herein and associated plant phenotypes or gene functions.

In addition, one or more polynucleotide sequences or one or more polypeptides encoded by the polynucleotide sequences may be used to search against a BLOCKS (Bairoch et al. (1997) Nucleic Acids Res. 25: 217-221), PFAM, and other databases which contain previously identified and annotated motifs, sequences and gene functions. Methods that search for primary sequence patterns with secondary structure gap penalties (Smith et al. (1992) Protein Engineering 5: 35-51) as well as algorithms such as Basic Local Alignment Search Tool (BLAST; Altschul (1993) J. Mol. Evol. 36: 290-300; Altschul et al. (1990) J. Mol. Biol. 215: 403-410), BLOCKS (Henikoff and Henikoff (1991) Nucleic Acids Res. 19: 6565-6572), Hidden Markov Models (HMM; Eddy (1996) Curr. Opin. Str. Biol. 6: 361-365; Sonnhammer et al. (1997) Proteins 28: 405-420), and the like, can be used to manipulate and analyze polynucleotide and polypeptide sequences encoded by polynucleotides. These databases, algorithms and other methods are well known in the art and are described in Ausubel et al. (1997) Short Protocols in Molecular Biology, John Wiley & Sons, New York, NY, unit 7.7) and in Meyers (1995) Molecular Biology and Biotechnology, Wiley VCH, New York, NY, p 856-853).

A further method for identifying or confirming that specific homologous sequences control the same function is by comparison of the transcript profile(s) obtained upon overexpression or knockout of two or more related transcription factors. Since transcript profiles are diagnostic for specific cellular states, one skilled in the art will appreciate that genes that have a highly similar transcript profile (for example, with greater than 50% regulated transcripts in common, more preferably with greater than 70% regulated transcripts in common, most preferably with greater than 90% regulated transcripts in common) will have highly similar functions. Fowler et al. (2002) Plant Cell 14: 1675-1679) have shown that three paralogous AP2 family genes (CBF1, CBF2 and CBF3), each of which is induced upon cold treatment, and each of which can condition improved freezing tolerance, have highly similar transcript profiles. Once a transcription factor has been shown to provide a specific function, its transcript profile becomes a diagnostic tool to determine whether putative paralogs or orthologs have the same function.

Furthermore, methods using manual alignment of sequences similar or homologous to one or more polynucleotide sequences or one or more polypeptides encoded by the polynucleotide sequences may be used to identify regions of similarity and AP2 binding domains. Such manual methods are well-known of those of skill in the art and can include, for example, comparisons of tertiary structure between a polypeptide sequence encoded by a polynucleotide that comprises a known function with a polypeptide sequence encoded by a polynucleotide sequence which has a function not yet determined. Such examples of tertiary structure may comprise predicted α-helices, β-sheets, amphipathic helices, leucine zipper motifs, zinc finger motifs, proline-rich regions, cysteine repeat motifs, and the like.

Orthologs and paralogs of presently disclosed transcription factors may be cloned using compositions provided by the present invention according to methods well known in the art. cDNAs can be cloned using mRNA from a plant cell or tissue that expresses one of the present transcription factors. Appropriate mRNA sources may be identified by interrogating Northern blots with probes designed from the present transcription factor sequences, after which a library is prepared from the mRNA obtained from a positive cell or tissue. Transcription factor-encoding cDNA is then isolated using, for example, PCR, using primers designed from a presently disclosed transcription factor gene sequence, or by probing with a partial or complete cDNA or with one or more sets of degenerate probes based on the disclosed sequences. The cDNA library may be used to transform plant cells. Expression of the cDNAs of interest is detected using, for example, methods disclosed herein such as microarrays, Northern blots, quantitative PCR, or any other technique for monitoring changes in expression. Genomic clones may be isolated using similar techniques to those.

Identifying Polynucleotides or Nucleic Acids by Hybridization

Polynucleotides homologous to the sequences illustrated in the Sequence Listing and tables can be identified, for example, by hybridization to each other under stringent or under highly stringent conditions. Single-stranded polynucleotides hybridize when they associate based on a variety of well characterized physical-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number thereof), as described in more detail in the references cited above.

Stability of DNA duplexes is affected by such factors as base composition, length, and degree of base pair mismatch. Hybridization conditions may be adjusted to allow DNAs of different sequence relatedness to hybridize. The melting temperature ($T_m$) is defined as the temperature when 50% of the duplex molecules have dissociated into their constituent single strands. The melting temperature of a perfectly matched duplex, where the hybridization buffer contains formamide as a denaturing agent, may be estimated by the following equations:

(I) DNA-DNA:

$$T_m(^\circ C)=81.5+16.6(\log [Na+])+0.41(\% G+C)-0.62(\% formamide)-500/L$$

(II) DNA-RNA:

$$T_m(^\circ C)=79.8+18.5(\log [Na+])+0.58(\% G+C)+0.12(\%G+C)^2-0.5(\% formamide)-820/L$$

(III) RNA-RNA:

$$T_m(^\circ C)=79.8+18.5(\log [Na+])+0.58(\% G+C)+0.12(\%G+C)^2-0.35(\% formamide)-820/L$$

where *L* is the length of the duplex formed, [Na+] is the molar concentration of the sodium ion in the hybridization or washing solution, and % G+C is the percentage of (guanine+cytosine) bases in the hybrid. For imperfectly matched hybrids, approximately 1° C is required to reduce the melting temperature for each 1% mismatch.

Hybridization experiments are generally conducted in a buffer of pH between 6.8 to 7.4, although the rate of hybridization is nearly independent of pH at ionic strengths likely to be used in the hybridization buffer (Anderson and Young (1985) "Quantitative Filter Hybridisation." In: Hames and Higgins, ed., Nucleic Acid Hybridisation, A Practical Approach. Oxford, IRL Press, 73-111). In addition, one or more of the following may be used to reduce non-specific hybridization: sonicated salmon sperm DNA or another non-complementary DNA, bovine serum albumin, sodium pyrophosphate, sodium dodecylsulfate (SDS), polyvinyl-pyrrolidone, ficoll and Denhardt's solution. Dextran sulfate and polyethylene glycol 6000 act to exclude DNA from solution, thus raising the effective probe DNA concentration and the hybridization signal within a given unit of time. In some instances, conditions of even greater stringency may be desirable or required to reduce non-specific and/or background hybridization. These conditions may be created with the use of higher temperature, lower ionic strength and higher concentration of a denaturing agent such as formamide.

Stringency conditions can be adjusted to screen for moderately similar fragments such as homologous sequences from distantly related organisms, or to highly similar fragments such as genes that duplicate functional enzymes from closely related organisms. The stringency can be adjusted either during the hybridization step or in the post-hybridization washes. Salt concentration, formamide concentration, hybridization temperature and probe lengths are variables that can be used to alter stringency (as described by the formula above). As a general guidelines high stringency is typically performed at $T_m$-5° C to $T_m$-20° C, moderate stringency at $T_m$-20° C to $T_m$-35° C and low stringency at $T_m$-35° C to $T_m$-50° C for duplex >150 base pairs. Hybridization may be performed at low to moderate stringency (25-50° C below $T_m$), followed by post-hybridization washes at increasing stringencies. Maximum rates of hybridization in solution are determined empirically to occur at $T_m$-25° C for DNA-DNA duplex and $T_m$-15° C for RNA-DNA duplex. Optionally, the degree of dissociation may be assessed after each wash step to determine the need for subsequent, higher stringency wash steps.

High stringency conditions may be used to select for nucleic acid sequences with high degrees of identity to the disclosed sequences. An example of stringent hybridization conditions obtained in a filter-based method such as a Southern or Northern blot for hybridization of complementary nucleic acids that have more than 100 complementary residues is about 5°C to 20°C lower than the thermal melting point ($T_m$) for the specific sequence at a defined ionic strength and pH. Conditions used for hybridization may include about 0.02 M to about 0.15 M sodium chloride, about 0.5% to about 5% casein, about 0.02% SDS or about 0.1% N-laurylsarcosine, about 0.001 M to about 0.03 M sodium citrate, at hybridization temperatures between about 50° C and about 70° C. More preferably, high stringency conditions are about 0.02 M sodium chloride, about 0.5% casein, about 0.02% SDS, about 0.001 M sodium citrate, at a temperature of about 50° C. Nucleic acid molecules that hybridize under stringent conditions will typically hybridize to a probe based on either the entire DNA molecule or selected portions, for example, to a unique subsequence, of the DNA.

Stringent salt concentration will ordinarily be less than about 750 mM NaCl and 75 mM trisodium citrate. Increasingly stringent conditions may be obtained with less than about 500 mM NaCl and 50 mM trisodium citrate, to even greater stringency with less than about 250 mM NaCl and 25 mM trisodium citrate. Low stringency hybridization can be obtained in the absence of organic solvent, for example, formamide, whereas high stringency hybridization may be obtained in the presence of at least about 35% formamide, and more preferably at least about 50% formamide. Stringent temperature conditions will ordinarily include temperatures of at least about 30° C, more preferably of at least about 37° C, and most preferably of at least about 42° C with formamide present. Varying additional parameters, such as hybridization time, the concentration of detergent, for example, sodium dodecyl sulfate (SDS) and ionic strength, are well known to those skilled in the art. Various levels of stringency are accomplished by combining these various conditions as needed.

The washing steps that follow hybridization may also vary in stringency; the post-hybridization wash steps primarily determine hybridization specificity, with the most critical factors being temperature and the ionic strength of the final wash solution. Wash stringency can be increased by decreasing salt concentration or by increasing temperature. Stringent salt concentration for the wash steps will preferably be less than about 30 mM NaCl and 3 mM trisodium citrate, and most preferably less than about 15 mM NaCl and 1.5 mM trisodium citrate.

Thus, hybridization and wash conditions that may be used to bind and remove polynucleotides with less than the desired homology to the nucleic acid sequences or their complements that encode the present transcription factors include, for example:

6X SSC at 65° C;
50% formamide, 4X SSC at 42° C; or
0.5X SSC, 0.1 % SDS at 65° C;
with, for example, two wash steps of 10 - 30 minutes each. Useful variations on these conditions will be readily apparent to those skilled in the art.

A person of skill in the art would not expect substantial variation among polynucleotide species encompassed within the scope of the present invention because the highly stringent conditions set forth in the above formulae yield structurally similar polynucleotides.

If desired, one may employ wash steps of even greater stringency, including about 0.2X SSC, 0.1% SDS at 65° C and washing twice, each wash step being about 30 min, or about 0.1 X SSC, 0.1% SDS at 65° C and washing twice for 30 min. The temperature for the wash solutions will ordinarily be at least about 25° C, and for greater stringency at least about 42° C. Hybridization stringency may be increased further by using the same conditions as in the hybridization steps, with the wash temperature raised about 3° C to about 5° C, and stringency may be increased even further by using the same conditions except the wash temperature is raised about 6° C to about 9° C. For identification of less closely related homologs, wash steps may be performed at a lower temperature, for example, 50° C.

An example of a low stringency wash step employs a solution and conditions of at least 25° C in 30 mM NaCl, 3 mM trisodium citrate, and 0.1 % SDS over 30 min. Greater stringency may be obtained at 42° C in 15 mM NaCl, with 1.5 mM trisodium citrate, and 0.1% SDS over 30 min. Even higher stringency wash conditions are obtained at 65° C -68° C in a solution of 15 mM NaCl, 1.5 mM trisodium citrate, and 0.1 % SDS. Wash procedures will generally employ at least two final wash steps. Additional variations on these conditions will be readily apparent to those skilled in the art (for example, in US Patent Application No. 20010010913).

Stringency conditions can be selected such that an oligonucleotide that is perfectly complementary to the coding oligonucleotide hybridizes to the coding oligonucleotide with at least about a 5-10x higher signal to noise ratio than the ratio for hybridization of the perfectly complementary oligonucleotide to a nucleic acid encoding a transcription factor known as of the filing date of the application. It may be desirable to select conditions for a particular assay such that a higher signal to noise ratio, that is, about 15x or more, is obtained. Accordingly, a subject nucleic acid will hybridize to a unique coding oligonucleotide with at least a 2x or greater signal to noise ratio as compared to hybridization of the coding oligonucleotide to a nucleic acid encoding known polypeptide. The particular signal will depend on the label used in the relevant assay, for example, a fluorescent label, a colorimetric label, a radioactive label, or the like. Labeled hybridization or PCR probes for detecting related polynucleotide sequences may be produced by oligolabeling, nick translation, end-labeling, or PCR amplification using a labeled nucleotide.

Encompassed by the invention are polynucleotide sequences that are capable of hybridizing to the polynucleotide sequences of the Sequence Listing, and fragments thereof under various conditions of stringency (for example, in Wahl and Berger (1987) Methods Enzymol. 152: 399-407, and Kimmel (1987) Methods Enzymol. 152: 507-511). Estimates of homology are provided by either DNA-DNA or DNA-RNA hybridization under conditions of stringency as is well understood by those skilled in the art (Hames and Higgins, Eds. (1985) Nucleic Acid Hybridisation: A Practical Approach, IRL Press, Oxford, U.K.). Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

Identifying-Polynucleotides or Nucleic Acids with Expression Libraries

In addition to hybridization methods, transcription factor homolog polypeptides can be obtained by screening an expression library using antibodies specific for one or more transcription factors. With the provision herein of the disclosed transcription factor, and transcription factor homolog nucleic acid sequences, the encoded polypeptide(s) can be expressed and purified in a heterologous expression system (for example, *E. coli*) and used to raise antibodies (monoclonal or polyclonal) specific for the polypeptide(s) in question. Antibodies can also be raised against synthetic peptides derived from the amino acid sequences or subsequences of a transcription factor or transcription factor homolog. Methods of raising antibodies are well known in the art and are described in Harlow and Lane (1988), Antibodies: A Laboratory Manual, Cold Spring Harbor Laboratory, New York. Such antibodies can then be used to screen an expression library produced from the plant from which it is desired to clone additional transcription factor homologs, using the methods described above. The selected cDNAs can be confirmed by sequencing and enzymatic activity.

Sequence Variations

It will readily be appreciated by those of skill in the art, that any of a variety of polynucleotide sequences are capable of encoding the transcription factors and transcription factor homolog polypeptides of the invention. Due to the degeneracy of the genetic code, many different polynucleotides can encode identical and/or substantially similar polypeptides in addition to those sequences illustrated in the Sequence Listing. Nucleic acids having a sequence that differs from the sequences shown in the Sequence Listing, or complementary sequences, that encode functionally equivalent peptides (i.e., peptides having some degree of equivalent or similar biological activity) but differ in sequence from the sequence shown in the Sequence Listing due to degeneracy in the genetic code, are also within the scope of the invention.

Altered polynucleotide sequences encoding polypeptides include those sequences with deletions, insertions, or substitutions of different nucleotides, resulting in a polynucleotide encoding a polypeptide with at least one functional characteristic of the instant polypeptides. Included within this definition are polymorphisms which may or may not be readily detectable using a particular oligonucleotide probe of the polynucleotide encoding the instant polypeptides, and improper or unexpected hybridization to allelic variants, with a locus other than the normal chromosomal locus for the polynucleotide sequence encoding the instant polypeptides.

Allelic variant refers to any of two or more alternative forms of a gene occupying the same chromosomal locus. Allelic variation arises naturally through mutation, and may result in phenotypic polymorphism within populations. Gene mutations can be silent (i.e., no change in the encoded polypeptide) or may encode polypeptides having altered amino acid sequence. The term allelic variant is also used herein to denote a protein encoded by an allelic variant of a gene. Splice variant refers to alternative forms ofRNA transcribed from a gene. Splice variation arises naturally through use of alternative splicing sites within a transcribed RNA molecule, or less commonly between separately transcribed RNA molecules, and may result in several mRNAs transcribed from the same gene. Splice variants may encode polypeptides having altered amino acid sequence. The term splice variant is also used herein to denote a protein encoded by a splice variant of an mRNA transcribed from a gene.

Those skilled in the art would recognize that, for example, G2133, SEQ ID NO: 12, represents a single transcription factor, allelic variation and alternative splicing may be expected to occur. Allelic variants of SEQ ID NO: 11 can be cloned by probing cDNA or genomic libraries from different individual organisms according to standard procedures. Allelic variants of the DNA sequence shown in SEQ ID NO: 11, including those containing silent mutations and those in which mutations result in amino acid sequence changes, are within the scope of the present invention, as are proteins which are allelic variants of SEQ ID NO: 12. cDNAs generated from alternatively spliced mRNAs, which retain the properties of the transcription factor are included within the scope of the present invention, as are polypeptides encoded by such cDNAs and mRNAs. Allelic variants and splice variants of these sequences can be cloned by probing cDNA or genomic libraries from different individual organisms or tissues according to standard procedures known in the art (USPN 6,388,064).

Thus, in addition to the sequences set forth in the Sequence Listing, the invention also encompasses related nucleic acid molecules that include allelic or splice variants of the sequences of the Sequence Listing, and include sequences that are complementary to any of the above nucleotide sequences. Related nucleic acid molecules also include nucleotide sequences encoding a polypeptide comprising a substitution, modification, addition and/or deletion of one or more amino acid residues compared to the polypeptide sequences of the Sequence Listing and equivalogs. Such related polypeptides may comprise, for example, additions and/or deletions of one or more N-linked or O-linked glycosylation sites, or an addition and/or a deletion of one or more cysteine residues.

For example. Table 3 illustrates, for example, that the codons AGC, AGT, TCA, TCC, TCG, and TCT all encode the same amino acid: serine. Accordingly, at each position in the sequence where there is a codon encoding serine, any of the above trinucleotide sequences can be used without altering the encoded polypeptide.

Table 3

| Amino acid | | | Possible Codons | | | |
|---|---|---|---|---|---|---|
| Alanine | Ala | A | GCA | GCC | GCG | GCT |
| Cysteine | Cys | C | TGC | TGT | | |

(continued)

| Amino acid | | | Possible Codons | | | | | |
|---|---|---|---|---|---|---|---|---|
| Aspartic acid | Asp | D | GAC | GAT | | | | |
| Glutamic acid | Glu | E | GAA | GAG | | | | |
| Phenylalanine | Phe | F | TTC | TTT | | | | |
| Glycine | Gly | G | GGA | GGC | GGG | GGT | | |
| Histidine | His | H | CAC | CAT | | | | |
| Isoleucine | Ile | I | ATA | ATC | ATT | | | |
| Lysine | Lys | K | AAA | AAG | | | | |
| Leucine | Leu | L | TTA | TTG | CTA | CTC | CTG | CTT |
| Methionine | Met | M | ATG | | | | | |
| Asparagine | Asn | N | AAC | AAT | | | | |
| Proline | Pro | P | CCA | CCC | CCG | CCT | | |
| Glutamine | Gln | Q | CAA | CAG | | | | |
| Arginine | Arg | R | AGA | AGG | CGA | CGC | CGG | CGT |
| Serine | Ser | S | AGC | AGT | TCA | TCC | TCG | TCT |
| Threonine | Thr | T | ACA | ACC | ACG | ACT | | |
| Valine | Val | V | GTA | GTC | GTG | GTT | | |
| Tryptophan | Trp | W | TGG | | | | | |
| Tyrosine | Tyr | Y | TAC | TAT | | | | |

Sequence alterations that do not change the amino acid sequence encoded by the polynucleotide are termed "silent" variations. With the exception of the codons ATG and TGG, encoding methionine and tryptophan, respectively, any of the possible codons for the same amino acid can be substituted by a variety of techniques, for example, site-directed mutagenesis, available in the art. Accordingly, any and all such variations of a sequence selected from the above table are a feature of the invention.

In addition to silent variations, other conservative variations that alter one, or a few amino acid residues in the encoded polypeptide, can be made without altering the function of the polypeptide, these conservative variants are, likewise, a feature of the invention.

For example, substitutions, deletions and insertions introduced into the sequences provided in the Sequence Listing, are also envisioned by the invention. Such sequence modifications can be engineered into a sequence by site-directed mutagenesis (Wu (ed.) Methods Enzymol. (1993) vol. 217, Academic Press) or the other methods noted below. Amino acid substitutions are typically of single residues; insertions usually will be on the order of about from 1 to 10 amino acid residues; and deletions will range about from 1 to 30 residues. In one embodiment, deletions or insertions are made in adjacent pairs, for example, a deletion of two residues or insertion of two residues. Substitutions, deletions, insertions or any combination thereof can be combined to arrive at a sequence. The mutations that are made in the polynucleotide encoding the transcription factor should not place the sequence out of reading frame and should not create complementary regions that could produce secondary mRNA structure. Preferably, the polypeptide encoded by the DNA performs the desired function.

Conservative substitutions are those in which at least one residue in the amino acid sequence has been removed and a different residue inserted in its place. Such substitutions generally are made in accordance with the Table 4 when it is desired to maintain the activity of the protein. Table 4 shows amino acids which can be substituted for an amino acid in a protein and which are typically regarded as conservative substitutions.

**Table 4**

| Residue | Conservative Substitutions |
|---|---|
| Ala | Ser |
| Arg | Lys |
| Asn | Gln; His |

(continued)

| Residue | Conservative Substitutions |
|---|---|
| Asp | Glu |
| Gln | Asn |
| Cys | Ser |
| Glu | Asp |
| Gly | Pro |
| His | Asn; Gln |
| Ile | Leu, Val |
| Leu | Ile; Val |
| Lys | Arg; Gln |
| Met | Leu; Ile |
| Phe | Met; Leu; Tyr |
| Ser | Thr; Gly |
| Thr | Ser; Val |
| Trp | Tyr |
| Tyr | Trp; Phe |
| Val | Ile; Leu |

Similar substitutions are those in which at least one residue in the amino acid sequence has been removed and a different residue inserted in its place. Such substitutions generally are made in accordance with the Table 5 when it is desired to maintain the activity of the protein. Table 5 shows amino acids which can be substituted for an amino acid in a protein and which are typically regarded as structural and functional substitutions. For example, a residue in column 1 of Table 5 may be substituted with a residue in column 2; in addition, a residue in column 2 of Table 5 may be substituted with the residue of column 1.

**Table 5**

| Residue | Similar Substitutions |
|---|---|
| Ala | Ser, Thr, Gly; Val; Leu; Ile |
| Arg | Lys; His; Gly |
| Asn | Gln; His; Gly; Ser; Thr |
| Asp | Glu, Ser; Thr |
| Gln | Asn; Ala |
| Cys | Ser; Gly |
| Gln | Asp |
| Gly | Pro; Arg |
| His | Asn; Gln; Tyr; Phe; Lys; Arg |
| Ile | Ala; Leu; Val; Gly; Met |
| Leu | Ala; Ile; Val; Gly; Met |
| Lys | Arg; His; Gln; Gly; Pro |
| Met | Leu; Ile; Phe |
| Phe | Met; Leu; Tyr; Trp; His; Val; Ala |

(continued)

| Residue | Similar Substitutions |
|---|---|
| Ser | Thr; Gly, Asp; Ala; Val; Ile; His |
| Thr | Ser; Val; Ala; Gly |
| Trp | Tyr, Phe; His |
| Tyr | Trp; Phe; His |
| Val | Ala; Ile; Leu; Gly; Thr; Ser; Glu |

Substitutions that are less conservative than those in Table 5 can be selected by picking residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, for example, seryl or threonyl, is substituted for (or by) a hydrophobic residue, for example, leucyl, isoleucyl, phenylalanyl, valyl or alanyl; (b) a cysteine or proline is substituted for (or by) any other residue; (c) a residue having an electropositive side chain, for example, lysyl, arginyl, or histidyl, is substituted for (or by) an electronegative residue, for example, glutamyl or aspartyl; or (d) a residue having a bulky side chain, for example, phenylalanine, is substituted for (or by) one not having a side chain, for example, glycine.

Further Modifying Sequences of the Invention - Mutation/Forced Evolution

In addition to generating silent or conservative substitutions as noted, above, the present invention optionally includes methods of modifying the sequences of the Sequence Listing. In the methods, nucleic acid or protein modification methods are used to alter the given sequences to produce new sequences and/or to chemically or enzymatically modify given sequences to change the properties of the nucleic acids or proteins.

Thus, in one embodiment, given nucleic acid sequences are modified, for example, according to standard mutagenesis or artificial evolution methods to produce modified sequences. The modified sequences may be created using purified natural polynucleotides isolated from any organism or may be synthesized from purified compositions and chemicals using chemical means well known to those of skill in the art. For example, Ausubel, supra, provides additional details on mutagenesis methods. Artificial forced evolution methods are described, for example, by Stemmer (1994) Nature 370: 389-391, Stemmer (1994) Proc. Natl. Acad. Sci. 91: 10747-10751, and US Patents 5,811,238, 5,837,500, and 6,242,568. Methods for engineering synthetic transcription factors and other polypeptides are described, for example, by Zhang et al. (2000) J. Biol. Chem. 275: 33850-33860, Liu et al. (2001) J. Biol. Chem. 276: 11323-11334, and Isalan et al. (2001) Nature Biotechnol.19: 656-660. Many other mutation and evolution methods are also available and expected to be within the skill of the practitioner.

Similarly, chemical or enzymatic alteration of expressed nucleic acids and polypeptides can be performed by standard methods. For example, sequence can be modified by addition of lipids, sugars, peptides, organic or inorganic compound, by the inclusion of modified nucleotides or amino acids, or the like. For example, protein modification techniques are illustrated in Ausubel, *supra.* Further details on chemical and enzymatic modifications can be found herein. These modification methods can be used to modify any given sequence, or to modify any sequence produced by the various mutation and artificial evolution modification methods noted herein.

Accordingly, the invention provides for modification of any given nucleic acid by mutation, evolution, chemical or enzymatic modification, or other available methods, as well as for the products produced by practicing such methods, for example, using the sequences herein as a starting substrate for the various modification approaches.

For example, optimized coding sequence containing codons preferred by a particular prokaryotic or eukaryotic host can be used for example, to increase the rate of translation or to produce recombinant RNA transcripts having desirable properties, such as a longer half life, as compared with transcripts produced using a non-optimized sequence. Translation stop codons can also be modified to reflect host preference. For example, preferred stop codons for *Saccharomyces cerevisiae* and mammals are TAA and TGA, respectively. The preferred stop codon for monocotyledonous plants is TGA, whereas insects and *E. coli* prefer to use TAA as the stop codon.

The polynucleotide sequences of the present invention can also be engineered in order to alter a coding sequence for a variety of reasons, including but not limited to, alterations which modify the sequence to facilitate cloning, processing and/or expression of the gene product. For example, alterations are optionally introduced using techniques which are well known in the art, for example, site-directed mutagenesis, to insert new restriction sites, to alter glycosylation patterns, to change codon preference, to introduce splice sites, etc.

Furthermore, a fragment or domain derived from any of the polypeptides of the invention can be combined with domains derived from other transcription factors or synthetic domains to modify the biological activity of a transcription factor. For instance, a DNA-binding domain derived from a transcription factor of the invention can be combined with the activation domain of another transcription factor or with a synthetic activation domain. A transcription activation domain assists in initiating transcription from a DNA-binding site. Examples include the transcription activation region of VP16 or GAL4 (Moore et al. (1998) Proc. Natl. Acad. Sci. 95: 376-381; Aoyama et al. (1995) Plant Cell 7: 1773-1785), peptides derived from bacterial sequences (Ma and Ptashne (1987) Cell 51: 113-119) and synthetic peptides (Giniger and Ptashne (1987) Nature 330: 670-672).

Expression and Modification of Polypeptides

Typically, polynucleotide sequences of the invention are incorporated into recombinant DNA (or RNA) molecules that direct expression of polypeptides of the invention in appropriate host cells, transgenic plants, in vitro translation systems, or the like. Due to the inherent degeneracy of the genetic code, nucleic acid sequences which encode substantially the same or a functionally equivalent amino acid sequence can be substituted for any listed sequence to provide for cloning and expressing the relevant homolog.

The transgenic plants of the present invention comprising recombinant polynucleotide sequences are generally derived from parental plants, which may themselves be non-transformed (or non-transgenic) plants. These transgenic plants may either have a transcription factor gene "knocked out" (for example, with a genomic insertion by homologous recombination, an antisense or ribozyme construct) or expressed to a normal or wild-type extent. However, overexpressing transgenic "progeny" plants will exhibit greater mRNA levels, wherein the mRNA encodes a transcription factor, that is, a DNA-binding protein that is capable of binding to a DNA regulatory sequence and inducing transcription, and preferably, expression of a plant trait gene. Preferably, the mRNA expression level will be at least three-fold greater than that of the parental plant, or more preferably at least ten-fold greater mRNA levels compared to said parental plant, and most preferably at least fifty-fold greater compared to said parental plant.

Vectors. Promoters, and Expression System

The present invention includes recombinant constructs comprising one or more of the nucleic acid sequences herein. The constructs typically comprise a vector, such as a plasmid, a cosmid, a phage, a virus (for example, a plant virus), a bacterial artificial chromosome (BAC), a yeast artificial chromosome (YAC), or the like, into which a nucleic acid sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available.

General texts that describe molecular biological techniques useful herein, including the use and production of vectors, promoters and many other relevant topics, include Berger, Sambrook, *supra* and Ausubel, *supra.* Any of the identified sequences can be incorporated into a cassette or vector, for example, for expression in plants. A number of expression vectors suitable for stable transformation of plant cells or for the establishment of transgenic plants have been described including those described in Weissbach and Weissbach (1989) Methods for Plant Molecular Biology Academic Press, and Gelvin et al. (1990) Plant Molecular Biology Manual, Kluwer Academic Publishers. Specific examples include those derived from a Ti plasmid of *Agrobacterium tumefaciens,* as well as those disclosed by Herrera-Estrella et al. (1983) Nature 303: 209, Bevan (1984) Nucleic Acids Res. 12: 8711-8721, Klee (1985) Bio/Technology 3: 637-642, for dicotyledonous plants.

Alternatively, non-Ti vectors can be used to transfer the DNA into monocotyledonous plants and cells by using free DNA delivery techniques. Such methods can involve, for example, the use of liposomes, electroporation, microprojectile bombardment, silicon carbide whiskers, and viruses. By using these methods transgenic plants such as wheat, rice (Christou (1991) Bio/Technology 9: 957-962) and corn (Gordon-Kamm (1990) Plant Cell 2: 603-618) can be produced. An immature embryo can also be a good target tissue for monocots for direct DNA delivery techniques by using the particle gun (Weeks et al. (1993) Plant Physiol. 102: 1077-1084; Vasil (1993) Bio/Technology 10: 667-674; Wan and Lemeaux (1994) Plant Physiol. 104: 37-48, and for *Agrobacterium-mediated* DNA transfer (Ishida et al. (1996) Nature Biotechnol. 14: 745-750).

Typically, plant transformation vectors include one or more cloned plant coding sequence (genomic or cDNA) under the transcriptional control of 5' and 3' regulatory sequences and a dominant selectable marker. Such plant transformation vectors typically also contain a promoter (for example, a regulatory region controlling inducible or constitutive, environmentally-or developmentally-regulated, or cell- or tissue-specific expression), a transcription initiation start site, an RNA processing signal (such as intron splice sites), a transcription termination site, and/or a polyadenylation signal.

A potential utility for the transcription factor polynucleotides disclosed herein is the isolation of promoter elements from these genes that can be used to program expression in plants of any genes. Each transcription factor gene disclosed herein is expressed in a unique fashion, as determined by promoter elements located upstream of the start of translation, and additionally within an intron of the transcription factor gene or downstream of the termination codon of the gene. As is well known in the art, for a significant portion of genes, the promoter sequences are located entirely in the region directly upstream of the start of translation. In such cases, typically the promoter sequences are located within 2.0 kb of the start of translation, or within 1.5 kb of the start of translation, frequently within 1.0 kb of the start of translation, and sometimes within 0.5 kb of the start of translation.

The promoter sequences can be isolated according to methods known to one skilled in the art.

Examples of constitutive plant promoters which can be useful for expressing the TF sequence include: the cauliflower mosaic virus (CaMV) 35S promoter, which confers constitutive, high-level expression in most plant tissues (for example, in Odell et al. (1985) Nature 313: 810-812); the nopaline synthase promoter (An et al. (1988) Plant Physiol. 88: 547-552); and the octopine synthase promoter (Fromm et al. (1989) Plant Cell 1: 977-984).

The transcription factors of the invention may be operably linked with a specific promoter that causes the transcription factor to be expressed in response to environmental, tissue-specific or temporal signals. A variety of plant gene promoters that regulate gene expression in response to environmental, hormonal, chemical, developmental signals, and in a tissue-active manner can be used for expression of a TF sequence in plants. Choice of a promoter is based largely on the phenotype of interest and is determined by such factors as tissue (for example, seed, fruit, root, pollen, vascular tissue, flower, carpel, etc.), inducibility (for example, in response to wounding, heat, cold, drought, light, pathogens, etc.), timing, developmental stage, and the like. Numerous known promoters have been characterized and can favorably be employed to promote expression of a polynucleotide of the invention in a transgenic plant or cell of interest. For example, tissue-specific promoters include: seed-specific promoters (such as the napin, phaseolin or DC3 promoter described in US Pat. No. 5,773,697), fruit-specific promoters that are active during fruit ripening (such as the dru 1 promoter (US Pat. No. 5,783,393), or the 2A11 promoter (US Pat. No. 4,943,674) and the tomato polygalacturonase promoter (Bird et al. (1988) Plant Mol. Biol. 11: 651-662), root-specific promoters, such as those disclosed in US Patent Nos. 5,618,988, 5,837,848 and 5,905,186, pollen-active promoters such as PTA29, PTA26 and PTA13 (US Pat. No. 5,792,929), promoters active in vascular tissue (Ringli and Keller (1998) Plant Mol. Biol. 37: 977-988), flower-specific (Kaiser et al. (1995) Plant Mol. Biol. 28: 231-243), pollen (Baerson et al. (1994) Plant Mol. Biol. 26: 1947-1959), carpels (Ohl et al. (1990) Plant Cell 2: 837-848), pollen and ovules (Baerson et al. (1993) Plant Mol. Biol. 22: 255-267), auxin-inducible promoters (such as that described in van der Kop et al. (1999) Plant Mol. Biol. 39: 979-990 or Baumann et al., (1999) Plant Cell 11: 323-334), cytokinin-inducible promoter (Guevara-Garcia (1998) Plant Mol. Biol. 38: 743-753), promoters responsive to gibberellin (Shi et al. (1998) Plant Mol. Biol. 38: 1053-1060, Willmott et al. (1998) Plant Molec. Biol. 38: 817-825) and the like. Additional promoters are those that elicit expression in response to heat (Ainley et al. (1993) Plant Mol. Biol. 22: 13-23), light (for example, the pea rbcS-3A promoter, Kuhlemeier et al. (1989) Plant Cell 1: 471-478), and the maize rbcS promoter, Schaffner and Sheen (1991) Plant Cell 3: 997-1012); wounding (for example, *wun*I, Siebertz et al. (1989) Plant Cell 1: 961-968); pathogens (such as the PR-1 promoter described in Buchel et al. (1999) Plant Mol. Biol. 40: 387-396, and the PDFI.2 promoter described in Manners et al. (1998) Plant Mol. Biol. 38: 1071-1080), and chemicals such as methyl jasmonate or salicylic acid (Gatz (1997) Annu. Rev. Plant Physiol. Plant Mol. Biol. 48: 89-108). In addition, the timing of the expression can be controlled by using promoters such as those acting at senescence (Gan and Amasino (1995) Science 270: 1986-1988); or late seed development (Odell et al. (1994) Plant Physiol. 106: 447-458).

Plant expression vectors can also include RNA processing signals that can be positioned within, upstream or downstream of the coding sequence. In addition, the expression vectors can include additional regulatory sequences from the 3'-untranslated region of plant genes, for example, a 3' terminator region to increase mRNA stability of the mRNA, such as the PI-II terminator region of potato or the octopine or nopaline synthase 3' terminator regions.

Additional Expression Elements

Specific initiation signals can aid in efficient translation of coding sequences. These signals can include, for example, the ATG initiation codon and adjacent sequences. No additional translational control signals may be needed where a coding sequence, its initiation codon and upstream sequences are inserted into the appropriate expression vector. However, in cases where only coding sequence (for example, a mature protein coding sequence) or a portion thereof is inserted, exogenous transcriptional control signals including the ATG initiation codon can be separately provided. The initiation codon is provided in the correct reading frame to facilitate transcription. Exogenous transcriptional elements and initiation codons can be of various origins, both natural and synthetic. The efficiency of expression can be enhanced by the inclusion of enhancers appropriate to the cell system in use.

## Expression Hosts

The present invention also relates to host cells which are transduced with vectors of the invention, and the production of polypeptides of the invention (including fragments thereof) by recombinant techniques. Host cells are genetically engineered (i.e., nucleic acids are introduced, for example, transduced, transformed or transfected) with the vectors of this invention, which may be, for example, a cloning vector or an expression vector comprising the relevant nucleic acids herein. The vector is optionally a plasmid, a viral particle, a phage, a naked nucleic acid, etc. The engineered host cells can be cultured in conventional nutrient media modified as appropriate for activating promoters, selecting transformants, or amplifying the relevant gene. The culture conditions, such as temperature, pH and the like, are those previously used with the host cell selected for expression, and will be apparent to those skilled in the art and in the references cited herein, including, Sambrook, *supra* and Ausubel, *supra.*

The host cell can be a eukaryotic cell, such as a yeast cell, or a plant cell, or the host cell can be a prokaryotic cell, such as a bacterial cell. Plant protoplasts are also suitable for some applications. For example, the DNA fragments are introduced into plant tissues, cultured plant cells or plant protoplasts by standard methods including electroporation (Fromm et al. (1985) Proc. Natl. Acad. Sci. 82: 5824-5828), infection by viral vectors such as cauliflower mosaic virus (CaMV) (Hohn et aL (1982) Molecular Biology of Plant Tumors Academic Press, New York, NY, pp. 549-560; US 4,407,956), high velocity ballistic penetration by small particles with the nucleic acid either within the matrix of small beads or particles, or on the surface (Klein et al. (1987) Nature 327: 70-73), use of pollen as vector (WO 85/01856), or use of *Agrobacterium tumefaciens* or *A. rhizogenes* carrying a T-DNA plasmid in which DNA fragments are cloned. The T-DNA plasmid is transmitted to plant cells upon infection by *Agrobacterium tumefaciens,* and a portion is stably integrated into the plant genome (Horsch et al. (1984) Science 233: 496-498; Fraley et al. (1983) Proc. Natl. Acad. Sci. 80: 4803-4807).

The cell can include a nucleic acid of the invention that encodes a polypeptide, wherein the cell expresses a polypeptide of the invention. The cell can also include vector sequences, or the like. Furthermore, cells and transgenic plants that include any polypeptide or nucleic acid above or throughout this specification, for example, produced by transduction of a vector of the invention, are an additional feature of the invention.

For long-term, high-yield production of recombinant proteins, stable expression can be used. Host cells transformed with a nucleotide sequence encoding a polypeptide of the invention are optionally cultured under conditions suitable for the expression and recovery of the encoded protein from cell culture. The protein or fragment thereof produced by a recombinant cell may be secreted, membrane-bound, or contained intracellularly, depending on the sequence and/or the vector used. As will be understood by those of skill in the art, expression vectors containing polynucleotides encoding mature proteins of the invention can be designed with signal sequences which direct secretion of the mature polypeptides through a prokaryotic or eukaryotic cell membrane.

## Modified Amino Acid Residues

Polypeptides of the invention may contain one or more modified amino acid residues. The presence of modified amino acids may be advantageous in, for example, increasing polypeptide half life, reducing polypeptide antigenicity or toxicity, increasing polypeptide storage stability, or the like. Amino acid residue(s) are modified, for example, co-translationally or post-translationally during recombinant production or modified by synthetic or chemical means.

Non-limiting examples of a modified amino acid residue include incorporation or other use of acetylated amino acids, glycosylated amino acids, sulfated amino acids, prenylated (for example, farnesylated, geranylgeranylated) amino acids, PEG modified (for example, "PEGylated") amino acids, biotinylated amino acids, carboxylated amino acids, phosphorylated amino acids, etc. References adequate to guide one of skill in the modification of amino acid residues are replete throughout the literature.

The modified amino acid residues may prevent or increase affinity of the polypeptide for another molecule, including, but not limited to, polynucleotide, proteins, carbohydrates, lipids and lipid derivatives, and other organic or synthetic compounds.

## Identification of Additional Protein Factors

A transcription factor provided by the present invention can also be used to identify additional endogenous or exogenous molecules that can affect a phenotype or trait of interest. Such molecules include endogenous molecules that are acted upon either at a transcriptional level by a transcription factor of the invention to modify a phenotype as desired. For example, the transcription factors can be employed to identify one or more downstream genes that are subject to a regulatory effect of the transcription factor. In one approach, a transcription factor or transcription factor homolog of the invention is expressed in a host cell, for example, a transgenic plant cell, tissue or explant, and expression products, either RNA or protein, of likely or random targets are monitored, for example, by hybridization to a microarray of nucleic acid probes corresponding to genes expressed in a tissue or cell type of interest, by two-dimensional gel electrophoresis of protein products, or by any other method known in the art for assessing expression of gene products at the level of RNA or protein. Alternatively, a transcription factor of the invention can be used to identify promoter sequences (such as binding sites on DNA sequences) involved in the regulation of a downstream target. After identifying a promoter sequence, interactions between the transcription factor and the promoter sequence can be modified by changing specific nucleotides in the promoter sequence or specific amino acids in the transcription factor that interact with the promoter sequence to alter a plant trait. Typically, transcription factor DNA-binding sites are identified by gel shift assays. After identifying the promoter regions, the promoter region sequences can be employed in double-stranded DNA arrays to identify molecules that affect the interactions of the transcription factors with their promoters (Bulyk et al. (1999) Nature Biotechnol. 17: 573-577).

The identified transcription factors are also useful to identify proteins that modify the activity of the transcription factor. Such modification can occur by covalent modification, such as by phosphorylation, or by protein-protein (homo or-heteropolymer) interactions. Any method suitable for detecting protein-protein interactions can be employed. Among the methods that can be employed are co-immunoprecipitation, cross-linking and co-purification through gradients or chromatographic columns, and the two-hybrid yeast system.

The two-hybrid system detects protein interactions in vivo and is described in Chien et al. ((1991) Proc. Natl. Acad. Sci. 88: 9578-9582) and is commercially available from Clontech (Palo Alto, Calif.). In such a system, plasmids are constructed that encode two hybrid proteins: one consists of the DNA-binding domain of a transcription activator protein fused to the TF polypeptide and the other consists of the transcription activator protein's activation domain fused to an unknown protein that is encoded by a cDNA that has been recombined into the plasmid as part of a cDNA library. The DNA-binding domain fusion plasmid and the cDNA library are transformed into a strain of the yeast *Saccharomyces cerevisiae* that contains a reporter gene (for example, lacZ) whose regulatory region contains the transcription activator's binding site. Either hybrid protein alone cannot activate transcription of the reporter gene. Interaction of the two hybrid proteins reconstitutes the functional activator protein and results in expression of the reporter gene, which is detected by an assay for the reporter gene product. Then, the library plasmids responsible for reporter gene expression are isolated and sequenced to identify the proteins encoded by the library plasmids. After identifying proteins that interact with the transcription factors, assays for compounds that interfere with the TF protein-protein interactions can be performed.

## Subsequences

Also contemplated are uses of polynucleotides, also referred to herein as oligonucleotides, typically having at least 12 or more bases that h hybridize under stringent or highly stringent conditions to a polynucleotide sequence described above. The polynucleotides may be used as probes, primers, sense and antisense agents, and the like, according to methods as noted above.

Subsequences of the polynucleotides of the invention, including polynucleotide fragments and oligonucleotides are useful as nucleic acid probes and primers. An oligonucleotide suitable for use as a probe or primer is at least about 15 nucleotides in length, more often at least about 18 nucleotides, often at least about 21 nucleotides, frequently at least about 30 nucleotides, or about 40 nucleotides, or more in length. A nucleic acid probe is useful in hybridization protocols, for example, to identify additional polypeptide homologs of the invention, including protocols for microarray experiments. Primers can be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, for example, by the polymerase chain reaction (PCR) or other nucleic-acid amplification methods (Sambrook, *supra,* and Ausubel, *supra).*

In addition, the invention includes an isolated or recombinant polypeptide including a subsequence of at least about 15 contiguous amino acids encoded by the recombinant or isolated polynucleotides of the invention. For example, such polypeptides, or domains or fragments thereof, can be used as immunogens, for example, to produce antibodies specific for the polypeptide sequence, or as probes for detecting a sequence of interest. A subsequence can range in size from about 15 amino acids in length up to and including the full length of the polypeptide.

To be encompassed by the present invention, an expressed polypeptide which comprises such a polypeptide subsequence performs at least one biological function of the intact polypeptide in substantially the same manner, or to a similar extent, as does the intact polypeptide. For example, a polypeptide fragment can comprise a recognizable structural motif or functional domain such as a DNA binding domain that activates transcription, for example, by binding to a specific DNA promoter region an activation domain, or a domain for protein-protein interactions.

Production of Transgenic Plants

Modification of Traits

The polynucleotides of the invention are favorably employed to produce transgenic plants with various traits or characteristics that have been modified in a desirable manner, for example, to improve the seed characteristics of a plant. For example, alteration of expression levels or patterns (for example, spatial or temporal expression patterns) of one or more of the transcription factors (or transcription factor homologs) of the invention, as compared with the levels of the same protein found in a wild-type plant, can be used to modify a plant's traits. An illustrative example of trait modification, improved characteristics, by altering expression levels of a particular transcription factor is described further in the Examples and the Sequence Listing.

*Arabidopsis as a model system*

*Arabidopsis thaliana* is the object of rapidly growling attention as a model for genetics and metabolism in plants. *Arabidopsis* has a small genome, and well-documented studies are available. It is easy to grow in large numbers and mutants defining important genetically controlled mechanisms are either available, or can readily be obtained. Various methods to introduce and express isolated homologous genes are available (Koncz et al., eds., Methods in Arabidopsis Research (1992) World Scientific, New Jersey, NJ, in "Preface"). Because of its small size, short life cycle, obligate autogamy and high fertility, *Arabidopsis* is also a choice organism for the isolation of mutants and studies in morphogenetic and development pathways, and control of these pathways by transcription factors (Koncz (1992) *supra,* p. 72). A number of studies introducing transcription factors into *A. thaliana* have demonstrated the utility of this plant for understanding the mechanisms of gene regulation and trait alteration in plants (for example, in Koncz (1992) *supra,* and in US Patent Number 6,417,428).

*Arabidopsis genes* in transgenic plants.

Expression of genes which encode transcription factors modify expression of endogenous genes, polynucleotides, and proteins are well know in the art. In addition, transgenic plants comprising isolated polynucleotides encoding transcription factors may also modify expression of endogenous genes, polynucleotides, and proteins. Examples include Peng et al. (1997) Genes and Development 11: 3194-3205 and Peng et al. (1999) Nature 400: 256-261. In addition, many others have demonstrated that an *Arabidopsis* transcription factor expressed in an exogenous plant species elicits the same or very similar phenotypic response (Fu et al. (2001) Plant Cell 13: 1791-1802; Nandi et al. (2000) Curr. Biol. 10: 215-218; Coupland (1995) Nature 377: 482-483; and Weigel and Nilsson (1995) Nature 377: 482-500.

Homologous genes introduced into transgenic plants.

Homologous genes that may be derived from any plant, or from any source whether natural, synthetic, semi-synthetic or recombinant, and that share significant sequence identity or similarity to those provided by the present invention, may be introduced into plants, for example, crop plants, to confer desirable or improved traits. Consequently, transgenic plants may be produced that comprise a recombinant expression vector or cassette with a promoter operably linked to one or more sequences homologous to presently disclosed sequences. The promoter may be, for example, a plant or viral promoter.

The invention thus provides for methods for preparing transgenic plants, and for modifying plant traits. These methods include introducing into a plant a recombinant expression vector or cassette comprising a functional promoter operably linked to one or more sequences homologous to presently disclosed sequences. Plants and kits for producing these plants that result from the application of these methods are also encompassed by the present invention.

Transcription factors of interest for the modification of plant traits

Currently, the existence of a series of maturity groups for different latitudes represents a major barrier to the introduction of new valuable traits. Any trait (for example disease resistance) has to be bred into each of the different maturity groups separately, a laborious and costly exercise. The availability of single strain, which could be grown at any latitude, would therefore greatly increase the potential for introducing new traits to crop species such as soybean and cotton.

For the specific effects, traits and utilities conferred to plants, one or more transcription factor genes of the present invention may be used to increase or decrease, or improve or prove deleterious to a given trait. For example, knocking out a transcription factor gene that naturally occurs in a plant, or suppressing the gene (with, for example, antisense suppression), may cause decreased tolerance to shade or a drought stress relative to non-transformed or wild-type plants. By overexpressing this gene, the plant may experience increased tolerance to the same stress. More than one transcription factor gene may be introduced into a plant, either by transforming the plant with one or more vectors comprising two or more transcription factors, or by selective breeding of plants to yield hybrid crosses that comprise more than one introduced transcription factor.

Genes, traits and utilities that affect plant characteristics

Plant transcription factors can modulate gene expression, and, in turn, be modulated by the environmental experience of a plant. Significant alterations in a plant's environment invariably result in a change in the plant's transcription factor gene expression pattern. Altered transcription factor expression patterns generally result in phenotypic changes in the plant. Transcription factor gene product(s) in transgenic plants then differ(s) in amounts or proportions from that found in wild-type or non-transformed plants, and those transcription factors likely represent polypeptides that are used to alter the response to the environmental change. By way of example, it is well accepted in the art that analytical methods based on altered expression patterns may be used to screen for phenotypic changes in a plant far more effectively than can be achieved using traditional methods.

Potential Applications of Presently Disclosed Sequences that Regulate Abiotic Stress Tolerance

Sugar sensing. In addition to their important role as an energy source and structural component of the plant cell, sugars are central regulatory molecules that control several aspects of plant physiology, metabolism and development (Hsieh et al. (1998) Proc. Natl. Acad. Sci. 95: 13965-13970). It is thought that this control is achieved by regulating gene expression and, in higher plants, sugars have been shown to repress or activate plant genes involved in many essential processes such as photosynthesis, glyoxylate metabolism, respiration, starch and sucrose synthesis and degradation, pathogen response, wounding response, cell cycle regulation, pigmentation, flowering and senescence. The mechanisms by which sugars control gene expression are not understood.

Several sugar sensing mutants have turned out to be allelic to ABA and ethylene mutants. ABA is found in all photosynthetic organisms and acts as a key regulator of transpiration, stress responses, embryogenesis, and seed germination. Most ABA effects are related to the compound acting as a signal of decreased water availability, whereby it triggers a reduction in water loss, slows growth, and mediates adaptive responses. However, ABA also influences plant growth and development via interactions with other phytohormones. Physiological and molecular studies indicate that maize and *Arabidopsis* have almost identical pathways with regard to ABA biosynthesis and signal transduction (for example, in Finkelstein and Rock (2002) "Abscisic acid biosynthesis and response", in The Arabidopsis Book, Somerville and Meyerowitz, editors (American Society of Plant Biologists, Rockville, MD).

This potentially implicates the sequences of the invention that, when overexpressed, confer a sugar sensing or hormone signaling phenotype in plants. On the other hand, the sucrose treatment used in these experiments (9.4% w/v) could also be an osmotic stress. Therefore, one could interpret these data as an indication that these transgenic lines are more tolerant to osmotic stress. However, it is well known that plant responses to ABA, osmotic and other stress may be linked, and these different treatments may even act in a synergistic manner to increase the degree of a response. For example, Xiong, Ishitani, and Zhu ((1999) Plant Physiol. 119: 205-212) have shown that genetic and molecular studies may be used to show extensive interaction between osmotic stress, temperature stress, and ABA responses in plants. These investigators analyzed the expression of *RD29A-LUC* in response to various treatment regimes *in Arabidopsis.* The RD29A promoter contains both the ABA-responsive and the dehydration-responsive element - also termed the C-repeat - and can be activated by osmotic stress, low temperature, or ABA treatment; transcription of the RD29A gene in response to osmotic and cold stresses is mediated by both ABA-dependent and ABA-independent pathways (Xiong, Ishitani, and Zhu (1999) *supra).* LUC refers to the firefly luciferase coding sequence, which, in this case, was driven by the stress responsive RD29A promoter. The results revealed both positive and negative interactions, depending on the nature and duration of the treatments. Low temperature stress was found to impair osmotic signaling but moderate heat stress strongly enhanced osmotic stress induction, thus acting synergistically with osmotic signaling pathways. In this study, the authors reported that osmotic stress and ABA can act synergistically by showing that the treatments simultaneously induced transgene and endogenous gene expression. Similar results were reported by Bostock and Quatrano ((1992) Plant Physiol. 98: 1356-1363), who found that osmotic stress and ABA act synergistically and induce maize *Em* gene expression. Ishitani et al (1997) Plant Cell 9: 1935-1949) isolated a group of *Arabidopsis* single-gene mutations that confer enhanced responses to both osmotic stress and ABA. The nature of the recovery of these mutants from osmotic stress and ABA treatment suggested that although separate signaling pathways exist for osmotic stress and ABA, the pathways share a number of components; these common components may mediate synergistic interactions between osmotic stress and ABA. Thus, contrary to the previously-held belief that ABA-dependent and ABA-independent stress signaling pathways act in a parallel manner, our data reveal that these pathways cross-talk and converge to activate stress gene expression.

Because sugars are important signaling molecules, the ability to control either the concentration of a signaling sugar or how the plant perceives or responds to a signaling sugar could be used to control plant development, physiology or metabolism. For example, the flux of sucrose (a disaccharide sugar used for systemically transporting carbon and energy in most plants) has been shown to affect gene expression and alter storage compound accumulation in seeds. Manipulation of the sucrose signaling pathway in seeds may therefore cause seeds to have more protein, oil or carbohydrate, depending on the type of manipulation. Similarly, in tubers, sucrose is converted to starch which is used as an energy store. It is thought that sugar signaling pathways may partially determine the levels of starch synthesized in the tubers. The manipulation of sugar signaling in tubers could lead to tubers with a higher starch content.

Thus, altering the expression of the presently disclosed transcription factor genes that manipulate the sugar signal transduction pathway, including, for example, G175, G303, G354, G481, G916, G922, G1069, G1073, G1820, G2053, G2701, G2789, G2839, G2854, along with their equivalogs, or that exhibit an osmotic stress phenotype, including, for example, G47, G482, G489 or G1069, G1073, as evidenced by their tolerance to, for example, high mannitol, salt or PEG, may be used to produce plants with desirable traits, including increased drought tolerance. In particular, manipulation of sugar signal transduction pathways could be used to alter source-sink relationships in seeds, tubers, roots and other storage organs leading to increase in yield.

Abiotic stress: drought and low humidity tolerance. Exposure to dehydration invokes similar survival strategies in plants as does freezing stress (for example, in Yelenosky (1989) Plant Physiol 89: 444-451) and drought stress induces freezing tolerance (for example, in Siminovitch et al. (1982) Plant Physiol 69: 250-255; and Guy et al. (1992) Planta 188: 265-270). In addition to the induction of cold-acclimation proteins, strategies that allow plants to survive in low water conditions may include, for example, reduced surface area, or surface oil or wax production. Modifying the expression of the presently disclosed transcription factor genes, including G2133, G1274, G922, G2999, G3086, G354, G1792, G2053, G975, G1069, G916, G1820, G2701, G47, G2854, G2789, G634, G175, G2839, G1452, G3083, G489, G303, G2992, and G682, and their equivalogs, may be used to increase a plant's tolerance to low water conditions and provide the benefits of improved survival, increased yield and an extended geographic and temporal planting range.

Osmotic stress. Modification of the expression of a number of presently disclosed transcription factor genes, for example, G47, G482, G489 or G1069, G2053 and their equivalogs, may be used to increase germination rate or growth under adverse osmotic conditions, which could impact survival and yield of seeds and plants. Osmotic stresses may be regulated by specific molecular control mechanisms that include genes controlling water and ion movements, functional and structural stress-induced proteins, signal perception and transduction, and free radical scavenging, and many others (Wang et al. (2001) Acta Hort. (ISHS) 560: 285-292). Instigators of osmotic stress include freezing, drought and high salinity, each of which are discussed in more detail below.

In many ways, freezing, high salt and drought have similar effects on plants, not the least of which is the induction of common polypeptides that respond to these different stresses. For example, freezing is similar to water deficit in that freezing reduces the amount of water available to a plant. Exposure to freezing temperatures may lead to cellular dehydration as water leaves cells and forms ice crystals in intercellular spaces (Buchanan et al. (2000) in Biochemistry and Molecular Biology of Plants. American Society of Plant Physiologists, Rockville, MD). As with high salt concentration and freezing, the problems for plants caused by low water availability include mechanical stresses caused by the withdrawal of cellular water. Thus, the incorporation of transcription factors that modify a plant's response to osmotic stress into, for example, a crop or ornamental plant, may be useful in reducing damage or loss. Specific effects caused by freezing, high salt and drought are addressed below.

The relationship between salt, drought and freezing tolerance. Plants are subject to a range of environmental challenges. Several of these, including drought stress, have the ability to impact whole plant and cellular water availability. Not surprisingly, then, plant responses to this collection of stresses are related. In a recent review, Zhu notes that "most studies on water stress signaling have focused on salt stress primarily because plant responses to salt and drought are closely related and the mechanisms overlap" (Zhu (2002) Ann. Rev. Plant Biol. 53: 247-273). Many examples of similar responses and pathways to this set of stresses have been documented. For example, the CBF transcription factors have been shown to condition resistance to salt, freezing and drought (Kasuga et al. (1999) Nature Biotech. 17: 287-291). The *Arabidopsis rd29B* gene is induced in response to both salt and dehydration stress, a process that is mediated largely through an ABA signal transduction process (Uno et al. (2000) Proc. Natl. Acad. Sci. USA 97:11632-11637), resulting in altered activity of transcription factors that bind to an upstream element within the *rd29B* promoter. In *Mesembryanthemum crystallinum* (ice plant), Patharker and Cushman have shown that a calcium-dependent protein kinase (McCDPKl) is induced by exposure to both drought and salt stresses (Patharker and Cushman (2000) Plant J. 24: 679-691). The stress-induced kinase was also shown to phosphorylate a transcription factor, presumably altering its activity, although transcript levels of the target transcription factor are not altered in response to salt or drought stress. Similarly, Saijo et al. demonstrated that a rice salt/drought-induced calmodulin-dependent protein kinase (OsCDPR7) conferred increased salt and drought tolerance to rice when overexpressed (Saijo et al. (2000) Plant J. 23: 319-327).

Exposure to dehydration invokes similar survival strategies in plants as does freezing stress (for example, in Yelenosky (1989) Plant Physiol 89: 444-451) and drought stress induces freezing tolerance (for example, in Siminovitch et al. (1982) Plant Physiol 69: 250-255; and Guy et al. (1992) Planta 188: 265-270). In addition to the induction of cold-acclimation proteins, strategies that allow plants to survive in low water conditions may include, for example, reduced surface area, or surface oil or wax production.

Consequently, one skilled in the art would expect that some pathways involved in resistance to one of these stresses, and hence regulated by an individual transcription factor, will also be involved in resistance to another of these stresses, regulated by the same or homologous transcription factors. Of course, the overall resistance pathways are related, not identical, and therefore not all transcription factors controlling resistance to one stress will control resistance to the other stresses. Nonetheless, if a transcription factor conditions resistance to one of these stresses, it would be apparent to one skilled in the art to test for resistance to these related stresses.

Thus, the genes of the sequence listing, including, for example, G175, G922, G1452, G1820, G2701, G2999, G3086 and their equivalogs that provide tolerance to salt may be used to engineer salt tolerant crops and trees that can flourish in soils with high saline content or under drought conditions. In particular, increased salt tolerance during the germination stage of a plant enhances survival and yield. Presently disclosed transcription factor genes that provide increased salt tolerance during germination, the seedling stage, and throughout a plant's life cycle, would find particular value for imparting survival and yield in areas where a particular crop would not normally prosper.

Summary of altered drouabt-related plant characteristics. The clades of structurally and functionally related sequences that derive from a wide range of plants, including polynucleotides of the Sequence Listing and their encoded polypeptides, fragments thereof, paralogs, orthologs, equivalogs, and fragments thereof, is provided. These sequences have been shown in laboratory and field experiments to confer altered size and abiotic stress tolerance phenotypes in plants. The invention also provides the polypeptides of the Sequence Listing, and fragments thereof, conserved domains thereof, paralogs, orthologs, equivalogs, and fragments thereof. Plants that overexpress these sequences have been observed to exhibit a sugar sensing phenotype and/or be more tolerant to a wide variety of abiotic stresses, including drought and high salt stress. Many of the orthologs of these sequences are listed in the Sequence Listing, and due to the high degree of structural similarity to the sequences of the invention, it is expected that these sequences will also function to increase drought stress tolerance. The invention also encompasses the complements of the polynucleotides. The polynucleotides are useful for screening libraries of molecules or compounds for specific binding and for creating transgenic plants having increased drought stress tolerance.

## Antisense and Co-suppression

In addition to expression of the nucleic acids of the invention as gene replacement or plant phenotype modification nucleic acids, the nucleic acids are also useful for sense and anti-sense suppression of expression, for example, to down-regulate expression of a nucleic acid of the invention. That is, the nucleic acids of the invention, or subsequences or anti-sense sequences thereof, can be used to block expression of naturally occurring homologous nucleic acids. A variety of sense and anti-sense technologies are known in the art, for example, as set forth in Lichtenstein and Nellen (1997) Antisense Technology: A Practical Approach, IRL Press at Oxford University Press, Oxford, U.K. Antisense regulation is also described in Crowley et al. (1985) Cell 43: 633-641; Rosenberg et al. (1985) Nature 313: 703-706; Preiss et al. (1985) Nature 313: 27-32; Melton (1985) Proc. Natl. Acad. Sci. 82:144-148; Izant and Weintraub (1985) Science 229: 345-352; and Kim and Wold (1985) Cell 42:129-138. Additional methods for antisense regulation are known in the art. Antisense regulation has been used to reduce or inhibit expression of plant genes in, for example in European Patent Publication No. 271988. Antisense RNA may be used to reduce gene expression to produce a visible or biochemical phenotypic change in a plant (Smith et al. (1988) Nature, 334: 724-726; Smith et al. (1990) Plant Mol. Biol. 14: 369-379). In general, sense or anti-sense sequences are introduced into a cell, where they are optionally amplified, for example, by transcription. Such sequences include both simple oligonucleotide sequences and catalytic sequences such as ribozymes.

For example, a reduction or elimination of expression (i.e., a "knock-out") of a transcription factor or transcription factor homolog polypeptide in a transgenic plant, for example, to modify a plant trait, can be obtained by introducing an antisense construct corresponding to the polypeptide of interest as a cDNA For antisense suppression, the transcription factor or homolog cDNA is arranged in reverse orientation (with respect to the coding sequence) relative to the promoter sequence in the expression vector. The introduced sequence need not be the full length cDNA or gene, and need not be identical to the cDNA or gene found in the plant type to be transformed. Typically, the antisense sequence need only be capable of hybridizing to the target gene or RNA of interest. Thus, where the introduced sequence is of shorter length, a higher degree of homology to the endogenous transcription factor sequence will be needed for effective antisense suppression. While antisense sequences of various lengths can be utilized, preferably, the introduced antisense sequence in the vector will be at least 30 nucleotides in length, and improved antisense suppression will typically be observed as the length of the antisense sequence increases. Preferably, the length of the antisense sequence in the vector will be greater than 100 nucleotides. Transcription of an antisense construct as described results in the production of RNA molecules that are the reverse complement of mRNA molecules transcribed from the endogenous transcription factor gene in the plant cell.

Suppression of endogenous transcription factor gene expression can also be achieved using RNA interference, or RNAi. RNAi is a post-transcriptional, targeted gene-silencing technique that uses double-stranded RNA (dsRNA) to incite degradation of messenger RNA (mRNA) containing the same sequence as the dsRNA (Constans, (2002) The Scientist 16:36). Small interfering RNAs, or siRNAs are produced in at least two steps: an endogenous ribonuclease cleaves longer dsRNA into shorter, 21-23 nucleotide-long RNAs. The siRNA segments then mediate the degradation of the target mRNA (Zamore, (2001) Nature Struct. Biol., 8:746-50). RNAi has been used for gene function determination in a manner similar to antisense oligonucleotides (Constans, (2002) The Scientist 16:36). Expression vectors that continually express siRNAs in transiently and stably transfected cells have been engineered to express small hairpin RNAs (shRNAs), which get processed in vivo into siRNAs-like molecules capable of carrying out gene-specific silencing (Brummelkamp et al., (2002) Science 296:550-553, and Paddison, et al. (2002) Genes & Dev. 16:948-958). Post-transcriptional gene silencing by double-stranded RNA is discussed in further detail by Hammond et al. (2001) Nature Rev Gen 2: 110-119, Fire et, al. (1998) Nature 391: 806-811 and Timmons and Fire (1998) Nature 395: 854. Vectors in which RNA encoded by a transcription factor or transcription factor homolog cDNA is over-expressed can also be used to obtain co-suppression of a corresponding endogenous gene, for example, in the manner described in US Patent No. 5,231,020 by Jorgensen. Such co-suppression (also termed sense suppression) does not require that the entire transcription factor cDNA be introduced into the plant cells, nor does it require that the introduced sequence be exactly identical to the endogenous transcription factor gene of interest. However, as with antisense suppression, the suppressive efficiency will be enhanced as specificity of hybridization is increased, for example, as the introduced sequence is lengthened, and/or as the sequence similarity between the introduced sequence and the endogenous transcription factor gene is increased.

Vectors expressing an untranslatable form of the transcription factor mRNA (for example, sequences comprising one or more stop codon or nonsense mutation) can also be used to suppress expression of an endogenous transcription factor, thereby reducing or eliminating its activity and modifying one or more traits. Methods for producing such constructs are described in US Patent No. 5,583,021. Preferably, such constructs are made by introducing a premature stop codon into the transcription factor gene. Alternatively, a plant trait can be modified by gene silencing using double-strand RNA (Sharp (1999) Genes and Development 13: 139-141). Another method for abolishing the expression of a gene is by insertion mutagenesis using the T-DNA of *Agrobacterium tumefaciens*. After generating the insertion mutants, the mutants can be screened to identify those containing the insertion in a transcription factor or transcription factor homolog gene. Plants containing a single transgene insertion event at the desired gene can be crossed to generate homozygous plants for the mutation. Such methods are well known to those of skill in the art (for example, in Koncz et al. (1992) Methods in Arabidopsis Research. World Scientific Publishing Co. Pte. Ltd., River Edge, NJ).

Alternatively, a plant phenotype can be altered by eliminating an endogenous gene, such as a transcription factor or transcription factor homolog, for example, by homologous recombination (Kempin et al. (1997) Nature 389: 802-803).

A plant trait can also be modified by using the Cre-lox system (for example, as described in US Pat. No. 5,658,772). A plant genome can be modified to include first and second lox sites that are then contacted with a Cre recombinase. If the lox sites are in the same orientation, the intervening DNA sequence between the two sites is excised. If the lox sites are in the opposite orientation, the intervening sequence is inverted.

The polynucleotides and polypeptides of this invention can also be expressed in a plant in the absence of an expression cassette by manipulating the activity or expression level of the endogenous gene by other means, such as, for example, by ectopically expressing a gene by T-DNA activation tagging (Ichikawa et al. (1997) Nature 390 698-701; Kakimoto et al. (1996) Science 274: 982-985). This method entails transforming a plant with a gene tag containing multiple transcriptional enhancers and once the tag has inserted into the genome, expression of a flanking gene coding sequence becomes deregulated. In another example, the transcriptional machinery in a plant can be modified so as to increase transcription levels of a polynucleotide of the invention (for example, in PCT Publications WO 96/06166 and WO 98/53057, which describe the modification of the DNA-binding specificity of zinc finger proteins by changing particular amino acids in the DNA-binding motif).

The transgenic plant can also include the machinery necessary for expressing or altering the activity of a polypeptide encoded by an endogenous gene, for example, by altering the phosphorylation state of the polypeptide to maintain it in an activated state.

Transgenic plants (or plant cells, or plant explants, or plant tissues) incorporating the polynucleotides of the invention and/or expressing the polypeptides of the invention can be produced by a variety of well established techniques as described above. Following construction of a vector, most typically an expression cassette, including a polynucleotide, for example, encoding a transcription factor or transcription factor homolog, of the invention, standard techniques can be used to introduce the polynucleotide into a plant, a plant cell, a plant explant or a plant tissue of interest Optionally, the plant cell, explant or tissue can be regenerated to produce a transgenic plant.

The plant can be any higher plant, including gymnosperms, monocotyledonous and dicotyledonous plants. Suitable protocols are available for *Leguminosae* (alfalfa, soybean, clover, etc.), *Umbelliferae* (carrot, celery, parsnip), *Cruciferae* (cabbage, radish, rapeseed, broccoli, etc.), *Curcurbitaceae* (melons and cucumber), *Gramineae* (wheat, corn, rice, barley, millet, etc.), *Solanaceae* (potato, tomato, tobacco, peppers, etc.), and various other crops (for example, in protocols described in Ammirato et al., eds., (1984) Handbook of Plant Cell Culture-Crop Species, Macmillan Publ. Co., New York, NY; Shimamoto et al. (1989) Nature 338: 274-276; Fromm et al. (1990) Bio/Technol. 8: 833-839; and Vasil et al. (1990) Bio/Technol. 8: 429-434.

Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and *Agrobacterium tumefaciens* mediated transformation. Transformation means introducing a nucleotide sequence into a plant in a manner to cause stable or transient expression of the sequence.

Successful examples of the modification of plant characteristics by transformation with cloned sequences which serve to illustrate the current knowledge in this field of technology, and which are herein incorporated by reference, include: US Patent Nos. 5,571,706; 5,677,175; 5,510,471; 5,750,386; 5,597,945; 5,589,615; 5,750,871; 5,268,526; 5,780,708; 5,538,880; 5,773,269; 5,736,369 and 5,610,042.

Following transformation, plants are preferably selected using a dominant selectable marker incorporated into the transformation vector. Typically, such a marker will confer antibiotic or herbicide resistance on the transformed plants, and selection of transformants can be accomplished by exposing the plants to appropriate concentrations of the antibiotic or herbicide.

After transformed plants are selected and grown to maturity, those plants showing a modified trait are identified. The modified trait can be any of those traits described above. Additionally, to confirm that the modified trait is due to changes in expression levels or activity of the polypeptide or polynucleotide of the invention can be determined by analyzing mRNA expression using Northern blots, RT-PCR or microarrays, or protein expression using immunoblots or Western blots or gel shift assays.

## Integrated Systems - Sequence Identity

Additionally, the present invention may be an integrated system, computer or computer readable medium that comprises an instruction set for determining the identity of one or more sequences in a database. In addition, the instruction set can be used to generate or identify sequences that meet any specified criteria. Furthermore, the instruction set may be used to associate or link certain functional benefits, such improved characteristics, with one or more identified sequence.

For example, the instruction set can include, for example, a sequence comparison or other alignment program, for example, an available program such as, for example, the Wisconsin Package Version 10.0, such as BLAST, FASTA, PILEUP, FINDPATTERNS or the like (GCG, Madison, WI). Public sequence databases such as GenBank, EMBL, Swiss-Prot and PIR or private sequence databases such as PHYTOSEQ sequence database (Incyte Genomics, Palo Alto, CA) can be searched.

Alignment of sequences for comparison can be conducted by the local homology algorithm of Smith and Waterman (1981) Adv. AppL Math. 2: 482-489, by the homology alignment algorithm of Needleman and Wunsch (1970) J. Mol. Biol. 48: 443-453, by the search for similarity method of Pearson and Lipman (1988) Proc. Natl. Acad. Sci. 85: 2444-2448, by computerized implementations of these algorithms. After alignment, sequence comparisons between two (or more) polynucleotides or polypeptides are typically performed by comparing sequences of the two sequences over a comparison window to identify and compare local regions of sequence similarity. The comparison window can be a segment of at least about 20 contiguous positions, usually about 50 to about 200, more usually about 100 to about 150 contiguous positions. A description of the method is provided in Ausubel et al. *supra.*

A variety of methods for determining sequence relationships can be used, including manual alignment and computer assisted sequence alignment and analysis. This later approach is a preferred approach in the present invention, due to the increased throughput afforded by computer assisted methods. As noted above, a variety of computer programs for performing sequence alignment are available, or can be produced by one of skill.

One example algorithm that is suitable for determining percent sequence identity and sequence similarity is the BLAST algorithm, which is described in Altschul et aL (1990) J. Mol. Biol. 215: 403-410. Software for performing BLAST analyses is publicly available, for example, through the National Library of Medicine's National Center for Biotechnology Information (ncbi.nlm.nih; world wide web (www) National Institutes of Health US government (gov) website). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al. *supra).*

These initial neighborhood word hits act as seeds for initiating searches to find longer HSPs containing them: The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=S, N=-4, and a comparison of both strands. For amino acid sequences, the BLASTP program uses as defaults a wordlength (W) of 3, an expectation (E) of 10, and the BLOSUM62 scoring matrix (Henikoff and Henikoff (1992) Proc. Natl. Acad. Sci. 89: 10915-10919). Unless otherwise indicated, "sequence identity" here refers to the % sequence identity generated from a tblastx using the NCBI version of the algorithm at the default settings using gapped alignments with the filter "off (for example, in the NIH NLM NCBI website at ncbi.nlm.nih, *supra*).

In addition to calculating percent sequence identity, the BLAST algorithm also performs a statistical analysis of the similarity between two sequences (for example, in Karlin and Altschul (1993) Proc. Natl. Acad. Sci. 90: 5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0.01, and or even less than about 0.001. An additional example of a useful sequence alignment algorithm is PILEUP. PILEUP creates a multiple sequence alignment from a group of related sequences using progressive, pairwise alignments. The program can align, for example, up to 300 sequences of a maximum length of 5,000 letters,

The integrated system, or computer typically includes a user input interface allowing a user to selectively view one or more sequence records corresponding to the one or more character strings, as well as an instruction set which aligns the one or more character strings with each other or with an additional character string to identify one or more region of sequence similarity. The system may include a link of one or more character strings with a particular phenotype or gene function. Typically, the system includes a user readable output element that displays an alignment produced by the alignment instruction set.

The methods of this invention can be implemented in a localized or distributed computing environment. In a distributed environment, the methods may be implemented on a single computer comprising multiple processors or on a multiplicity of computers. The computers can be linked, for example, through a common bus, but more preferably the computer(s) are nodes on a network. The network can be a generalized or a dedicated local or wide-area network and, in certain preferred embodiments, the computers may be components of an intra-net or an internet.

Thus, the invention provides methods for identifying a sequence similar or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an inter or intra net) and a query is made against the sequence database using the relevant sequences herein and associated plant phenotypes or gene functions.

Any sequence herein can be entered into the database, before or after querying the database. This provides for both expansion of the database and, if done before the querying step, for insertion of control sequences into the database. The control sequences can be detected by the query to ensure the general integrity of both the database and the query. As noted, the query can be performed using a web browser based interface. For example, the database can be a centralized public database such as those noted herein, and the querying can be done from a remote terminal or computer across an internet or intranet.

Any sequence herein can be used to identify a similar, homologous, paralogous, or orthologous sequence in another plant. This provides means for identifying endogenous sequences in other plants that may be useful to alter a trait of progeny plants, which results from crossing two plants of different strain. For example, sequences that encode an ortholog of any of the sequences herein that naturally occur in a plant with a desired trait can be identified using the sequences disclosed herein. The plant is then crossed with a second plant of the same species but which does not have the desired trait to produce progeny which can then be used in further crossing experiments to produce the desired trait in the second plant. Therefore the resulting progeny plant contains no transgenes; expression of the endogenous sequence may also be regulated by treatment with a particular chemical or other means, such as EMR. Some examples of such compounds well known in the art include: ethylene; cytokinins; phenolic compounds; which stimulate the transcription of the genes needed for infection; specific monosaccharides and acidic environments which potentiate vir gene induction; acidic polysaccharides which induce one or more chromosomal genes; and opines; other mechanisms include light or dark treatment (for example, in Winans (1992) Microbiol. Rev. 56: 12-31; Eyal et al. (1992) Plant. Mol. Biol. 19: 589-599; Chrispeels et al. (2000) Plant Mol. Biol. 42: 279-290; Piazza et al. (2002) Plant Physiol. 128:1077-1086).

Table 6 lists the gene identification number (GID) and homologous relationships found using analyses according to the Examples for the sequences of the Sequence Listing. Those sequences listed as "reference sequences" were originally determined by experimentation to confer drought tolerance when their expression was altered. Generally, each reference sequence was used to identify the clade in which functionally related homologous sequences may be found.

Table 6. Homologs and Other Related Genes of Representative *Arabidopsis* Transcription Factor Genes Identified using BLAST

| SEQ ID NO | GID No | Polynucleotide (DNA) or polypeptide (PRT) | Species from Which Homologous Sequence is Derived | Relationship SEQ ID NO to OtherGenes |
|---|---|---|---|---|
| 1 | G47 | DNA | *Arabidopsis thaliana* | Reference sequence; predicted polypeptide sequence is paralogous to G2133 |
| 2 | G47 | PRT | *Arabidopsis thaliana* | Reference sequence; paralogous to G2133 |
| 11 | G2133 | DNA | *Arabidopsis thaliana* | Reference sequence; predicted polypeptide sequence is paralogous to G47 |
| 12 | G213 | PRT | *Arabidopsis thaliana* | Reference sequence; paralogous to G47 |

Molecular Modeling

Another means that may be used to confirm the utility and function of transcription factor sequences that are orthologous or paralogous to presently disclosed transcription factors is through the use of molecular modeling software. Molecular modeling is routinely used to predict polypeptide structure, and a variety of protein structure modeling programs, such as "Insight II" (Accelrys, Inc.) are commercially available for this purpose. Modeling can thus be used to predict which residues of a polypeptide can be changed without altering function (Crameri et al. (2003) U.S. Patent No. 6,521, 453). Thus, polypeptides that are sequentially similar can be shown to have a high likelihood of Similar function by their structural similarity, which may, for example, be established by comparison of regions of superstructure. The relative tendencies of amino acids to form regions of superstructure (for example, helixes and β-sheets) are well established. For example, O'Neil et al. ((1990) Science 250: 646-651) have discussed in detail the helix forming tendencies of amino acids. Tables of relative structure forming activity for amino acids can be used as substitution tables to predict which residues can be functionally substituted in a given region, for example, in DNA-binding domains of known transcription factors and equivalogs. Homologs that are likely to be functionally similar can then be identified.

Of particular interest is the structure of a transcription factor in the region of its conserved domains, such as those identified in Table 1 and Table 3. Structural analyses may be performed by comparing the structure of the known transcription factor around its conserved domain with those of orthologs and paralogs. Analysis of a number of polypeptides within a transcription factor group or clade, including the functionally or sequentially similar polypeptides provided in the Sequence Listing, may also provide an understanding of structural elements required to regulate transcription within a given family.

**EXAMPLES**

The invention, now being generally described, will be more readily understood by reference to the following examples, which are included merely for purposes of illustration of certain aspects and embodiments of the present invention and are not intended to limit the invention. It will be recognized by one of skill in the art that a transcription factor that is associated with a particular first trait may also be associated with at least one other, unrelated and inherent second trait which was not predicted by the first trait.

The complete descriptions of the traits associated with each polynucleotide of the invention are fully disclosed in Examples VIII, IX and X.

**Example I: Fall Length Gene Identification and Cloning**

Putative transcription factor sequences (genomic or ESTs) related to known transcription factors were identified in the *Arabidopsis thaliana* GenBank database using the tblastn sequence analysis program using default parameters and a P-value cutoff threshold of -4 or -5 or lower, depending on the length of the query sequence. Putative transcription factor sequence hits were then screened to identify those containing particular sequence strings. If the sequence hits contained such sequence strings, the sequences were confirmed as transcription factors.

Alternatively, *Arabidopsis thaliana* cDNA libraries derived from different tissues or treatments, or genomic libraries were screened to identify novel members of a transcription family using a low stringency hybridization approach. Probes were synthesized using gene specific primers in a standard PCR reaction (annealing temperature 60° C) and labeled with $^{32}P$ dCTP using the High Prime DNA Labeling Kit (Roche Diagnostics Corp., Indianapolis, IN). Purified radiolabelled probes were added to filters immersed in Church hybridization medium (0.5 M $NaPO_4$ pH 7.0, 7% SDS, 1% w/v bovine serum albumin) and hybridized overnight at 60° C with shaking. Filters were washed two times for 45 to 60 minutes with 1 x SCC, 1 % SDS at 60° C.

To identify additional sequence 5' or 3' of a partial cDNA sequence in a cDNA library, 5' and 3' rapid amplification of cDNA ends (RACE) was performed using the MARATHON cDNA amplification kit (Clontech, Palo Alto, CA). Generally, the method entailed first isolating poly(A) mRNA, performing first and second strand cDNA synthesis to generate double-stranded cDNA, blunting cDNA ends, followed by ligation of the MARATHON Adaptor to the cDNA to form a library of adaptor-ligated ds cDNA.

Gene-specific primers were designed to be used along with adaptor specific primers for both 5' and 3' RACE reactions. Nested primers, rather than single primers, were used to increase PCR specificity. Using 5' and 3' RACE reactions, 5' and 3' RACE fragments were obtained, sequenced and cloned. The process can be repeated until 5' and 3' ends of the full-length gene were identified. Then the full-length cDNA was generated by PCR using primers specific to 5' and 3' ends of the gene by end-to-end PCR.

**Example II: Construction of Expression Vectors**

The sequence was amplified from a genomic or cDNA library using primers specific to sequences upstream and downstream of the coding region. The expression vector was pMEN20 or pMEN65, which are both derived from pMON316 (Sanders et al. (1987) Nucleic Acids Res. 15:1543-1558) and contain the CaMV 35S promoter to express transgenes. To clone the sequence into the vector, both pMEN20 and the amplified DNA fragment were digested separately with SalI and NotI restriction enzymes at 37° C for 2 hours. The digestion products were subject to electrophoresis in a 0.8% agarose gel and visualized by ethidium bromide staining. The DNA fragments containing the sequence and the linearized plasmid were excised and purified by using a QIAQUICK gel extraction kit (Qiagen, Valencia CA). The fragments of interest were ligated at a ratio of 3:1 (vector to insert). Ligation reactions using T4 DNA ligase (New England Biolabs, Beverly MA) were carried out at 16° C for 16 hours. The ligated DNAs were transformed into competent cells of the *E. coli* strain DH5$\alpha$ by using the heat shock method. The transformations were plated on LB plates containing 50 mg/l kanamycin (Sigma Chemical Co. St. Louis MO). Individual colonies were grown overnight in five milliliters of LB broth containing 50 mg/l kanamycin at 37° C. Plasmid DNA was purified by using Qiaquick Mini Prep kits (Qiagen).

**Example III: Transformation of *Agrobacterium* with the Expression Vector**

After the plasmid vector containing the gene was constructed, the vector was used to transform *Agrobacterium tumefaciens* cells expressing the gene products. The stock of *Agrobacterium tumefaciens* cells for transformation was made as described by Nagel et al. (1990) FEMS Microbiol Letts. 67: 325-328. *Agrobacterium* strain ABI was grown in 250 ml LB medium (Sigma) overnight at 28°C with shaking until an absorbance over 1 cm at 600 nm ($A_{600}$) of 0.5 -1.0 was reached. Cells were harvested by centrifugation at 4,000 x g for 15 min at 4°C. Cells were then resuspended in 250 $\mu$l chilled buffer (1 mM HEPES, pH adjusted to 7.0 with KOH). Cells were centrifuged again as described above and resuspended in 125 $\mu$l chilled buffer. Cells were then centrifuged and resuspended two more times in the same HEPES buffer as described above at a volume of 100 $\mu$l and 750 $\mu$l, respectively. Resuspended cells were then distributed into 40 $\mu$l aliquots, quickly frozen in liquid nitrogen, and stored at -80° C.

*Agrobacterium* cells were transformed with plasmids prepared as described above following the protocol described by Nagel et al. (1990) *supra.* For each DNA construct to be transformed, 50 - 100 ng DNA (generally resuspended in 10 mM Tris-HCl, 1 mM EDTA, pH 8.0) was mixed with 40 $\mu$l of *Agrobacterium* cells. The DNA/cell mixture was then transferred to a chilled cuvette with a 2 mm electrode gap and subject to a 2.5 kV charge dissipated at 25 $\mu$F and 200 $\mu$F using a Gene Pulser II apparatus (Bio-Rad, Hercules, CA). After electroporation, cells were immediately resuspended in 1.0 ml LB and allowed to recover without antibiotic selection for 2 - 4 hours at 28° C in a shaking incubator. After recovery, cells were plated onto selective medium of LB broth containing 100 $\mu$g/ml spectinomycin (Sigma) and incubated for 24-48 hours at 28˚C. Single colonies were then picked and inoculated in fresh medium. The presence of the plasmid construct was verified by PCR amplification and sequence analysis.

**Example IV: Transformation *of Arabidopsis* Plants with *Agrobacterium tumefaciens* with Expression Vector**

After transformation of *Agrobacterium tumefaciens* with plasmid vectors containing the gene, single *Agrobacterium* colonies were identified, propagated, and used to transform *Arabidopsis* plants. Briefly, 500 ml cultures of LB medium containing 50 mg/l kanamycin were inoculated with the colonies and grown at 28˚ C with shaking for 2 days until an optical absorbance at 600 nm wavelength over 1 cm ($A_{600}$) of > 2.0 is reached. Cells were then harvested by centrifugation at 4,000 x g for 10 min, and resuspended in infiltration medium (1/2 X Murashige and Skoog salts (Sigma), 1 X Gamborg's B-5 vitamins (Sigma), 5.0% (w/v) sucrose (Sigma), 0.044 μM benzylamino purine (Sigma), 200 μl/l Silwet L-77 (Lehle Seeds)) until an $A_{600}$ of 0.8 was reached.

Prior to transformation, *Arabidopsis thaliana* seeds (ecotype Columbia) were sown at a density of ~10 plants per 4" pot onto Pro-Mix BX potting medium (Hummert International) covered with fiberglass mesh (18 mm X 16 mm). Plants were grown under continuous illumination (50-75 $\mu E/m^2/sec$) at 22-23˚ C with 65-70% relative humidity. After about 4 weeks, primary inflorescence stems (bolts) are cut off to encourage growth of multiple secondary bolts. After flowering of the mature secondary bolts, plants were prepared for transformation by removal of all siliques and opened flowers.

The pots were then immersed upside down in the mixture of *Agrobacterium* infiltration medium as described above for 30 sec, and placed on their sides to allow draining into a 1' x 2' flat surface covered with plastic wrap. After 24 h, the plastic wrap was removed and pots are turned upright. The immersion procedure was repeated one week later, for a total of two immersions per pot. Seeds were then collected from each transformation pot and analyzed following the protocol described below.

**Example V: Identification of *Arabidopsis* Primary Transformants**

Seeds collected from the transformation pots were sterilized essentially as follows. Seeds were dispersed into in a solution containing 0.1 % (v/v) Triton X-100 (Sigma) and sterile water and washed by shaking the suspension for 20 min. The wash solution was then drained and replaced with fresh wash solution to wash the seeds for 20 min with shaking. After removal of the ethanol/detergent solution, a solution containing 0.1 % (v/v) Triton X-100 and 30% (v/v) bleach (CLOROX; Clorox Corp. Oakland CA) was added to the seeds, and the suspension was shaken for 10 min. After removal of the bleach/detergent solution, seeds were then washed five times in sterile distilled water. The seeds were stored in the last wash water at 4˚ C for 2 days in the dark before being plated onto antibiotic selection medium (1 X Murashige and Skoog salts (pH adjusted to 5.7 with 1M KOH), 1 X Gamborg's B-5 vitamins, 0.9% phytagar (Life Technologies), and 50 mg/l kanamycin). Seeds were germinated under continuous illumination (50-75 $\mu E/m^2/sec$) at 22-23˚ C. After 7-10 days of growth under these conditions, kanamycin resistant primary transformants (T1 generation) were visible and obtained. These seedlings were transferred first to fresh selection plates where the seedlings continued to grow for 3-5 more days, and then to soil (Pro-Mix BX potting medium).

Primary transformants were crossed and progeny seeds ($T_2$) collected; kanamycin resistant seedlings were selected and analyzed. The expression levels of the recombinant polynucleotides in the transformants varies from about a 5% expression level increase to a least a 100% expression level increase. Similar observations are made with respect to polypeptide level expression.

**Example VI: Identification *of Arabidopsis* Plants with Transcription Factor Gene Knockouts**

The screening of insertion mutagenized-*Arabidopsis* collections for null mutants in a known target gene was essentially as described in Krysan et al. (1999) Plant Cell 11: 2283-2290. Briefly, gene-specific primers, nested by 5-250 base pairs to each other, were designed from the 5' and 3' regions of a known target gene. Similarly, nested sets of primers were also created specific to each of the T-DNA or transposon ends (the "right" and "left" borders). All possible combinations of gene specific and T-DNA/transposon primers were used to detect by PCR an insertion event within or close to the target gene. The amplified DNA fragments were then sequenced which allows the precise determination of the T-DNA/transposon insertion point relative to the target gene. Insertion events within the coding or intervening sequence of the genes were deconvoluted from a pool comprising a plurality of insertion events to a single unique mutant plant for functional characterization. The method is described in more detail in Yu and Adam, US Application Serial No. 09/177,733 filed October 23,1998.

**Example VII: Identification of Modified Phenotypes in Overexpression or Gene Knockout Plants.**

*Arabidopsis thaliana* ecotype Columbia (Col-0) was used to create all overexpressing lines. The control plants for the assay were Col-0 plants transformed with an empty transformation vector (pMEN65).

Microarray experiments

In some instances, expression patterns of the stress-induced genes may be monitored by microarray experiments. In these experiments, cDNAs are generated by PCR and resuspended at a final concentration of ~ 100 ng/ μl in 3x SSC or 150 mM Na-phosphate (Eisen and Brown (1999) Methods Enzymol. 303: 179-205). The cDNAs are spotted on microscope glass slides coated with polylysine. The prepared cDNAs are aliquoted into 384 well plates and spotted on the slides using, for example, an x-y-z gantry (OmniGrid) which may be purchased from GeneMachines (Menlo Park, CA) outfitted with quill type pins which may be purchased from Telechem International (Sunnyvale, CA). After spotting, the arrays are cured for a minimum of one week at room temperature, rehydrated and blocked following the protocol recommended by Eisen and Brown (1999) *supra.*

Sample total RNA (10 μg) samples are labeled using fluorescent Cy3 and Cy5 dyes. Labeled samples are resuspended in 4X SSC/0.03% SDS/4 μg salmon sperm DNA/2 μg tRNA/ 50mM Na-pyrophosphate, heated for 95° C for 2.5 minutes, spun down and placed on the array. The array is then covered with a glass coverslip and placed in a sealed chamber. The chamber is then kept in a water bath at 62° C overnight. The arrays are washed as described in Eisen and Brown (1999) *supra*) and scanned on a General Scanning 3000 laser scanner. The resulting files are subsequently quantified using IMAGENE, software (BioDiscovery, Los Angeles CA).

RT-PCR experiments may be performed to identify those genes induced after exposure to abiotic stresses. Generally, the gene expression patterns from ground plant leaf tissue is examined.

Reverse transcriptase PCR was conducted using gene specific primers within the coding region for each sequence identified. The primers were designed near the 3' region of each DNA binding sequence initially identified.

Total RNA from these ground leaf tissues was isolated using the CTAB extraction protocols Once extracted total RNA was normalized in concentration across all the tissue types to ensure that the PCR reaction for each tissue received the same amount of cDNA template using the 28S band as reference. Poly(A+) RNA was purified using a modified protocol from the Qiagen OLIGOTEX purification kit batch protocol. cDNA was synthesized using standard protocols. After the first strand cDNA synthesis, primers for Actin 2 were used to normalize the concentration of cDNA across the tissue types. Actin 2 is found to be constitutively expressed in fairly equal levels across the tissue types being investigated.

For RT PCR, cDNA template was mixed with corresponding primers and Taq DNA polymerase. Each reaction consisted of 0.2 μl cDNA template, 2 μl 10X Tricine buffer, 2 μl 10X Tricine buffer and 16.8 μl water, 0.05 μl Primer 1, 0.05 μl, Primer 2, 0.3 μl Taq DNA polymerase and 8.6 μl water.

The 96 well plate is covered with microfilm and set in the thermocycler to start the reaction cycle. By way of illustration, the reaction cycle may comprise the following steps:

STEP 1: 93° C for 3 minutes;
STEP 2: 93° C for 30 seconds;
STEP 3: 65° C for 1 minute;
STEP 4: 72° C for 2 minutes;
STEPS 2, 3 and 4 are repeated for 28 cycles;
STEP 5: 72° C for 5 minutes; and
STEP 6 4° C.

To amplify more products, for example, to identify genes that have very low expression, additional steps may be performed: the following method illustrates a method that may be used in this regard the PCR plate is placed back in the thermocycler for 8 more cycles of Steps 2-4.

STEP 2 93° C for 30 seconds;
STEP 3 65° C for 1 minute;
STEP 4 72° C for 2 minutes, repeated for 8 cycles; and
STEP 5 4° C.

Eight microliters of PCR product and 1.5 μl of loading dye are loaded on a 1.2% agarose gel for analysis after 28 cycles and 36 cycles. Expression levels of specific transcripts are considered low if they were only detectable after 36 cycles of PCR Expression levels are considered medium or high depending on the levels of transcript compared with observed transcript levels for an internal control such as actin2. Transcript levels are determined in repeat experiments and compared to transcript levels in control (e.g., non-transformed) plants.

Abiotic stress assays

Modified phenotypes observed for particular overexpressor plants may include increased biomass, and/or increased or decreased abiotic stress tolerance or resistance. For a particular overexpressor that shows a less beneficial characteristic, such as reduced abiotic stress tolerance or resistance, it may be more useful to select a plant with a decreased expression of the particular transcription factor. For a particular knockout that shows a less beneficial characteristic, such as decreased abiotic stress tolerance, it may be more useful to select a plant with an increased expression of the particular transcription factor.

The germination assays in this example followed modifications of the same basic protocol. Sterile seeds were sown on the conditional media listed below. Plates were incubated at 22° C under 24-hour light (120-130 $\mu$Ein/$m^2$/s) in a growth chamber. Evaluation of germination and seedling vigor was conducted 3 to 15 days after planting. The basal media was 80% Murashige-Skoog medium (MS) + vitamins.

For stress experiments conducted with more mature plants, seeds were germinated and grown for seven days on MS + vitamins + 1% sucrose at 22 °C and then transferred to cold and heat stress conditions. The plants were either exposed to cold stress (6 hour exposure to 4-8° C), or heat stress (32° C was applied for five days, after which the plants were transferred back 22 °C for recovery and evaluated after 5 days relative to controls not exposed to the depressed or elevated temperature).

The salt stress assays were intended to find genes that confer better germination, seedling vigor or growth in high salt. Evaporation from the soil surface causes upward water movement and salt accumulation in the upper soil layer where the seeds are placed. Thus, germination normally takes place at a salt concentration much higher than the mean salt concentration of in the whole soil profile. Plants differ in their tolerance to NaCl depending on their stage of development, therefore seed germination, seedling vigor, and plant growth responses were evaluated.

Osmotic stress assays (including NaCl and mannitol assays) were conducted to determine if an osmotic stress phenotype was NaCl-specific or if it was a general osmotic stress related phenotype. Plants tolerant to osmotic stress could also have more tolerance to drought and/or freezing.

For salt and osmotic stress germination experiments, the medium was supplemented with 150 mM NaCl or 300 mM mannitol. Growth regulator sensitivity assays were performed in MS media, vitamins, and either 0.3 $\mu$M ABA, 9.4% sucrose, or 5% glucose.

Experiments were performed to identify those transformants that exhibited modified sugar-sensing. For such studies, seeds from transformants were germinated on high sugar-containing media (5% glucose, 9.4% sucrose) that normally partially restrict hypocotyl elongation. Plants with altered sugar sensing may have either longer or shorter hypocotyls than normal plants when grown on this media. Additionally, other plant traits may be varied such as root mass. Sugar sensing assays were intended to find genes involved in sugar sensing by germinating seeds on high concentrations of sucrose and glucose and looking for degrees of hypocotyl elongation. The germination assay on mannitol controlled for responses related to osmotic stress. Sugars are key regulatory molecules that affect diverse processes in higher plants including germination, growth, flowering, senescence, sugar metabolism and photosynthesis. Sucrose is the major transport form of photosynthate and its flux through cells has been shown to affect gene expression and alter storage compound accumulation in seeds (source-sink relationships). Glucose-specific hexose-sensing has also been described in plants and is implicated in cell division and repression of "famine" genes (photosynthetic or glyoxylate cycles).

Temperature stress assays were carried out to find genes that confer better germination, seedling vigor or plant growth under temperature stress (cold, freezing and heat). Temperature stress cold germination experiments were carried out at 8°C. Heat stress germination experiments were conducted at 32 °C to 37° C for 6 hours of exposure.

Soil-based drought screens were performed with *Arabidopsis* plants overexpressing the transcription factors listed in the Sequence Listing. Seeds from wild-type *Arabidopsis* plants, or plants overexpressing a polypeptide of the invention, were stratified for three days at 4° C in 0.1 % agarose. Fourteen seeds of each overexpressor or wild-type were then sown in three inch clay pots containing a 50:50 mix of vermiculite:perlite topped with a small layer of MetroMix 200 and grown for fifteen days under 24 hr light. Pots containing wild-type and overexpressing seedlings were placed in flats in random order. Drought stress was initiated by placing pots on absorbent paper for seven to eight days. The seedlings were considered to be sufficiently stressed when the majority of the pots containing wild-type seedlings within a flat had become severely wilted. Pots were then re-watered and survival was scored four to seven days later. Plants were ranked against wild-type controls for each of two criteria: tolerance to the drought conditions and recovery (survival) following re-watering

At the end of the initial drought period, each pot was assigned a numeric value score depending on the above criteria. Scores of 0-6 were assigned (Table 11), with a low value of "0" assigned to plants with an extremely poor appearance (i.e., the plants were uniformly brown) and a value of "6" given to plants that were rated very healthy in appearance (i.e., the plants were all green). After the plants were rewatered and incubated an additional four to seven days, the plants were reevaluated to indicate the degree of recovery from the water deprivation treatment.

An analysis was then conducted to determine which plants best survived water deprivation, identifying the transgenes that consistently conferred drought-tolerant phenotypes and their ability to recover from this treatment. The analysis was performed by comparing overall and within-flat tabulations with a set of statistical models to account for variations between batches. Several measures of survival were tabulated, including: (a) the average proportion of plants surviving relative to wild-type survival within the same flat; (b) the median proportion surviving relative to wild-type survival within the same flat; (c) the overall average survival (taken over all batches, flats, and pots); (d) the overall average survival relative to the overall wild-type survival; and (e) the average visual score of plant health before rewatering.

Examples of genes and homologs that confer significant improvements to knockout or overexpressing plants are noted below. Experimental observations made by us with regard to specific genes whose expression has been modified in overexpressing or knock-out plants, and potential applications based on these observations, are also presented.

**Example VIII: Results of Drought Stress Analyses**

This example provides experimental evidence for increased abiotic stress tolerance controlled by transcription factor polypeptides and polypeptides of the invention.

**Results:**

As noted below, overexpression of G2133, was shown to increase drought stress tolerance in plants. A number of orthologs of some of these sequences were also able to increase abiotic stress tolerance, as noted below.

**The G47 Clade of Transcription Factor Polypeptides**

**G47 (SEQ ID NO: 1 and 2)**

Published Information. G47 corresponds to gene T22J18.2 (AAC25505). No information is available about the function(s) of G47.

Experimental Observations. The function of G47 was studied using transgenic *Arabidopsis* plants in which the gene was expressed under the control of the 35S promoter. Overexpression of G47 resulted in a variety of morphological and physiological phenotypic alterations.

35S::G47 plants showed enhanced tolerance to osmotic stress; osmotic stress assays were conducted using growth medium containing polyethylene glycol (PEG). After germination, the seedlings of 35S::G47 overexpressing lines generally appeared larger and had more root growth than wild-type control seedlings.

As would be predicted by these osmotic stress assays, G47 plants also showed enhanced survival and drought tolerance in a soil-based drought assay.

Overexpression of G47 also produced a substantial delay in flowering time and caused a marked change in shoot architecture. 35S::G47 transformants were small at early stages and switched to flowering more than a week later than wild-type controls (continuous light conditions). Interestingly, the inflorescences from these plants appeared thick and fleshy, had reduced apical dominance, and exhibited reduced internode elongation leading to a short compact stature. The branching pattern of the stems also appeared abnormal, with the primary shoot becoming 'kinked' at each coflorescence node. Additionally, the plants showed slightly reduced fertility and formed rather small siliques that were borne on short pedicels and held vertically, close against the stem.

Additional alterations were detected in the inflorescence stems of 35S::G47 plants. Stem sections from T2-21 and T2-24 plants were of wider diameter, and had large irregular vascular bundles containing a much greater number of xylem vessels than wild type. Furthermore some of the xylem vessels within the bundles appeared narrow and were possibly more lignified than were those of controls.

G47 was expressed at higher levels in rosette leaves, and transcripts can be detected in other tissues (flower, embryo, silique, and germinating seedling), but apparently not in roots.

Utilities. G47 or its equivalogs can be used to increase the tolerance of plants to drought and to other osmotic stresses. G47 or its equivalogs could also be used to manipulate flowering time, to modify plant architecture and stem structure, including development of vascular tissues and lignin content. The use of G47 or its equivalogs from tree species could offer the potential for modulating lignin content. This might allow the quality of wood used for furniture or construction to be improved. G47 equivalogs include, for example, *Arabidopsis thaliana* SEQ ID NO: 12 (G2133); *Oryza sativa* (*japonica* cultivar-group) SEQ ID NOs: 98 (G3649), SEQ ID NO: 100 (G3651), and SEQ ID NO: 90 (G3644);

*Glycine max* SEQ ID NO: 88 (G3643); *Zinnia elegans* SEQ ID NO: 96 (G3647); *Brassica rapa* subsp. Pekinensis SEQ ID NO: 92 (G3645); and *Brassica oleracea* SEQ ID NO: 94 (G3646).

**G2133 (SEQ ID NO: 11 and 12)**

Published Information. G2133 is a paralog of G47. G2133 corresponds to gene F26A9.11 (AAF23336). No information is available about the function(s) of G2133.

Experimental Observations. The function of G2133 was studied using transgenic *Arabidopsis* plants in which the gene was expressed under the control of the 35S promoter.

G2133 expression was detected in a variety of tissues: flower, leaf, embryo, and silique samples. Its expression might be altered by several conditions, including auxin treatment, osmotic stress, and *Fusarium* infection. Overexpression of G2133 caused a variety of alterations in plant growth and development: delayed flowering, altered inflorescence architecture, and a decrease in overall size and fertility.

At early stages, 35S::G2133 transformants were markedly smaller than controls and displayed curled, dark-green leaves. Most of these plants remained in a vegetative phase of development substantially longer than controls, and produced an increased number of leaves before bolting. In the most severely affected plants, bolting occurred more than a month later than in wild type (24-hour light). In addition, the plants displayed a reduction in apical dominance and formed large numbers of shoots simultaneously, from the axils of rosette leaves. These inflorescence stems had short internodes, and carried increased numbers of cauline leaf nodes, giving them a very leafy appearance. The fertility of 35S::G2133 plants was generally very low. In addition, G2133 overexpressing lines were found to be more resistant to the herbicide glyphosate in initial and repeat experiments.

No alterations were detected in 35S::G2133 plants in the biochemical analyses that were performed.

G2133 is a paralog of G47, the latter having been known from earlier studies to confer a drought tolerance phenotype when overexpressed. It was thus not surprising when G2133 was also shown to induce drought tolerance in a number of 35S::G2133 lines challenged in soil-based drought assays (Tables 11 and 12). Experiments comparing the recovery of wild-type controls and two lines of *Arabidopsis* plants overexpressing G2133 (a paralog of G47) from a drought treatment were conducted under constant light. The 35S::G2133 and control lines were grown in pots with each pot containing several plants. All were deprived of water for eight days, and then re-watered. After re-watering, all of the plants of both G2133 overexpressor lines became reinvigorated, and all of the control plants died or were severely affected by the drought treatment (Table 12).

Utilities. G2133 and its equivalogs can be used to increase the tolerance of plants to drought and to other osmotic stresses. G2133 could also be used for the generation of glyphosate resistant plants, and to increase plant resistance to oxidative stress. G2133 equivalogs include, for example, *Arabidopsis thaliana* SEQ ID NO: 2 (G47); *Oryza sativa* (*japonica* cultivar-group) SEQ ID NO: 98 (G3649), SEQ ID NO: 100 (G3651), and SEQ ID NO: 90 (G3644); *Glycine max* SEQ ID NO: 88 (G3643); *Zinnia elegans* SEQ ID NO: 96 (G3647); *Brassica rapa* subsp. Pekinensis SEQ ID NO: 92 (G3645); and *Brassica oleracea* SEQ ID NO: 94 (G3646).

**G3643 (SEQ ID NO: 87 and 88)**

G3643 is a soy ortholog of G47 and G2133.

Experimental Observations. The function of G3 643 was studied using transgenic *Arabidopsis* plants in which the gene was expressed under the control of the 35S promoter.

G3643-overexpressing *Arabidopsis* plants were more tolerant to cold than wild-type control plants grown under similar conditions in plate-based germination assays. One of these lines was also more tolerant to desiccation and growth in cold conditions in plate-based assays.

Utilities. G3643 or its equivalogs can be used to increase the tolerance of plants to cold conditions and low water conditions, including drought.

**G3644 (SEQ ID NO: 89 and 90)**

G3644 is a rice ortholog of G47 and G2133.

Experimental Observations. The function of G3644 was studied using transgenic *Arabidopsis* plants in which the gene was expressed under the control of the 35S promoter.

Several G3644-overexpressing *Arabidopsis* plants were found to be more tolerant to desiccation than wild-type control plants grown under similar conditions in plate based-assays. Two lines were shown to be more salt tolerant than wild type.

Utilities. G3644 or its equivalogs can be used to increase the tolerance of plants to high salt and low water conditions, including drought.

**G3649 (SEQ ID NO: 97 and 98)**

G3649 is a rice ortholog of G47 and G2133.

Experimental Observations. The function of G3649 was studied using transgenic *Arabidopsis* plants in which the gene was expressed under the control of the 35S promoter.

Several G3649-overexpressing *Arabidopsis* plants were more tolerant to cold than wild-type control plants grown under similar conditions in plate-based germination assays. Two overexpressing lines were more heat tolerant than wild-type plants, and one 35S::G3649 line was found to be more desiccation tolerant than wild type.

Utilities. G3649 or its equivalogs can be used to increase the tolerance of plants to cold conditions and low water conditions, including drought.

**Summary of drought assay results**

Table 7 presents the results obtained in an assay in which *Arabidopsis* plants were subjected to water deprivation for seven to eight days. At the end of this dry-down period, each pot was assigned a numeric score depending on the health of its plants. A score of 0 to 6 was assigned based on a plant's color and general appearance, with plants that were all brown receiving a "0" and, at the other end of the spectrum, plants that bad an excellent appearance (all green) receiving a "6". The mean of the recorded numeric score of all pots of a given genotype per line of all flats tested is presented in order of decreasing health.

Table 7. Comparison of recorded numeric score plants subjected to drought treatment.

| GID | Mean score |
|---|---|
| G2133 | 5.875 |
| G634 | 4.778 |
| G922 | 4.667 |
| G916 | 4.6 |
| G1274 | 4.273 |
| G864 | 3.733 |
| G2999 | 3.7 |
| G2992 | 3.7 |
| G353 | 3.6 |
| G47 | 3.459 |
| G2053 | 3.404 |
| G975 | 3.393 |
| G489 | 3.364 |
| G1792 | 3.281 |
| G1820 | 3.2 |
| G24S3 | 3.2 |
| G2140 | 3.139 |
| G2701 | 3.108 |
| G3086 | 3.056 |
| G611 | 3.048 |
| G1452 | 3.042 |
| G481 | 3.041 |
| G624 | 3.000 |
| G2854 | 2.829 |

(continued)

| GID | Mean score |
|---|---|
| G303 | 2.812 |
| G2839 | 2.783 |
| G2789 | 2708 |
| G188 | 2692 |
| G325 | 2.556 |
| G2776 | 2.513 |
| G175 | 2.467 |
| G2110 | 2.432 |
| G1206 | 2.412 |
| G682 | 2.381 |
| G1730 | 2.341 |
| G2969 | 2.333 |
| G2998 | 2333 |
| G1069 | 2.316 |
| Wild-type | 2.284 |

Table 8 compares the survival ratings *of Arabidopsis* plants overexpressing various polypeptides, evaluated after seven to eight days of drought treatment, rewatering, and two to three days of a recovery period Values indicate the median odds of survival within a given flat (the 50th percentile of survival within each pot of a given genotype per line divided by the average wild-type survival in the flat).

Table 8. Survival ratings of *Arabidopsis* plants after drought and rewatering treatment

| GID | Median per flat |
|---|---|
| G2133 | 3.365 |
| G1274 | 2.059 |
| G922 | 1.406 |
| G2999 | 1.255 |
| G3086 | 1.179 |
| G354 | 1.167 |
| G1792 | 1.161 |
| G2053 | 1.091 |
| G975 | 1:090 |
| G1069 | 1.037 |
| G916 | 1.023 |
| G2T01 | 1.000 |
| G1820 | 1.000 |
| G47 | 0.921 |
| G2854 | 0.889 |
| G2789 | 0.845 |
| G481 | 0.843 |

(continued)

| GID | Median per flat |
|---|---|
| G634 | 0.834 |
| G175 | 0.814 |
| G2839 | 0.805 |
| G1452 | 0.803 |
| Wild-type | 0.800 |

**Example IX: Identification of Homologous Sequences**

This example describes identification of genes that are orthologous to *Arabidopsis thaliana* transcription factors from a computer homology search.

Homologous sequences, including those of paralogs and orthologs from *Arabidopsis* and other plant species, were identified using database sequence search tools, such as the Basic Local Alignment Search Tool (BLAST) (Altschul et al. (1990) *supra;* and Altschul et al. (1997) Nucleic Acid Res. 25: 3389-3402). The tblastx sequence analysis programs were employed using the BLOSUM-62 scoring matrix (Henikoff and Henikoff (1992) Proc Natl. Acad Sci. 89: 10915-10919). The entire NCBI GenBank database was filtered for sequences from all plants except *Arabidopsis thaliana* by selecting all entries in the NCBI GenBank database associated with NCBI taxonomic ID 33090 (Viridiplantae; all plants) and excluding entries associated with taxonomic ID 3701 (*Arabidopsis thaliana*).

These sequences are compared to sequences representing genes of the invention, for example, polynucleotides found in the Sequence Listing, using the Washington University TBLASTX algorithm (version 2.0a19MP) at the default settings using gapped alignments with the filter "off". For each polynucleotide sequence found in the Sequence Listing individual comparisons-were ordered by probability score (P-value), where the score reflects the probability that a particular alignment occurred by chance. For example, a score of 3.6E-40 is 3.6 x 10-40. In addition to P-values, comparisons were also scored by percentage identity. Percentage identity reflects the degree to which two segments of DNA or protein are identical over a particular length. Examples of sequences so identified are presented in Table 6. The percent sequence identity among these sequences can be as low as 47%, or even lower sequence identity.

Candidate paralogous sequences were identified among *Arabidopsis* transcription factors through alignment, identity, and phylogenic relationships. Candidate orthologous sequences were identified from proprietary unigene sets of plant gene sequences in *Zea mays, Glycine* max and *Oryza* sativa based on significant homology to *Arabidopsis* transcription factors. These candidates were reciprocally compared to the set of *Arabidopsis* transcription factors. If the candidate showed maximal similarity in the protein domain to the eliciting transcription factor or to a paralog of the eliciting transcription factor, then it was considered to be an ortholog. Identified non-*Arabidopsis* sequence that were shown in this manner to be orthologous to the *Arabidopsis* sequences are provided in Table 6

**Example 7 Screen of Plant cDNA library for Sequence Encoding a Transcription Factor DNA Binding Domain That Binds To a Transcription Factor Binding Promoter Element and Demonstration of Protein Transcription Regulation Activity.**

The "one-hybrid" strategy (Li and Herskowitz (1993) Science 262:1870-1874) is used to screen for plant cDNA clones encoding a polypeptide comprising a transcription factor DNA binding domain, a conserved domain. In brief, yeast strains are constructed that contain a lacZ reporter gene with either wild-type or mutant transcription factor binding promoter element sequences in place of the normal UAS (upstream activator sequence) of the GALA promoter. Yeast reporter strains are constructed that carry transcription factor binding promoter element sequences as UAS elements are operably plinked upstream (5') of a lacZ reporter gene with a minimal GAL4 promoter. The strains are transformed with a plant expression library that contains random cDNA inserts fused to the GALA activation domain (GAL4-ACT) and screened for blue colony formation on X-gal-treated filters (X-gal: 5-bromo-4-chloro-3-indolyl-β-D-galactoside; Invitrogen Corporation. Carlsbad CA). Alternatively, the strains are transformed with a cDNA polynucleotide encoding a known transcription factor DNA binding domain polypeptide sequence.

Yeast strains carrying these reporter constructs produce low levels of β-galactosidase and form white colonies on filters containing X-gaL The reporter strains carrying wild-type transcription factor binding promoter element sequences are transformed with a polynucleotide that encodes a polypeptide comprising a plant transcription factors DNA binding domain operably linked to the acidic activator domain of the yeast GALA transcription factor, "GAL4-ACT". The clones that contain a polynucleotide encoding a Transcription factor DNA binding domain operably linked to GALA-ACT can bind upstream of the lacZ reporter genes carrying the wild-type transcription factor binding promoter element sequence, activate transcription of the lacZ gene and result in yeast forming blue colonies on X-gal-treated filters.

Upon screening about 2 x $10^6$ yeast transformants, positive cDNA clones are isolated; i.e., clones that cause yeast strains carrying lacZ reporters operably linked to wild-type transcription factor binding promoter elements to form blue colonies on X-gal-treated filters. The cDNA clones do not cause a yeast strains carrying a mutant type transcription factor binding promoter elements fused to LacZ to turn blue. Thus, a polynucleotide encoding transcription factor DNA binding domain, a conserved domain, is shown to activate transcription of a gene.

**Example XI: Gel Shift Assays.**

The presence of a transcription factor comprising a DNA binding domain which binds to a DNA transcription factor binding element is evaluated using the following gel shift assay. The transcription factor is recombinantly expressed and isolated from *E. coli* or isolated from plant material. Total soluble protein, including transcription factor, (40 ng) is incubated at room temperature in 10 μl of 1 x binding buffer (15 mM HEPES (pH 7.9), 1 mM EDTA, 30 mM KCl, 5% glycerol, 5% bovine serum albumin, 1 mM DTT) plus 50 ng poly(dl-dC):poly(dl-dC) (Pharmacia, Piscataway NJ) with or without 100 ng competitor DNA. After 10 minutes incubation, probe DNA comprising a DNA transcription factor binding element (1 ng) that has been $^{32}$P-labeled by end-filling (Sambrook et al. *supra*) is added and the mixture incubated for an additional 10 minutes. Samples are loaded onto polyacrylamide gels (4% w/v) and fractionated by electrophoresis at 150V for 2h (Sambrook et al. *supra*). The degree of transcription factor-probe DNA binding is visualized using autoradiography. Probes and competitor DNAs are prepared from oligonucleotide inserts ligated into the BamHI site of pUC118 (Vieira et al. (1987) Methods Enzymol. 153: 3-11). Orientation and concatenation number of the inserts are determined by dideoxy DNA sequence analysis (Sambrook et al. supra). Inserts are recovered after restriction digestion with EcoRI and HindIII and fractionation on polyacrylamide gels (12% w/v) (Sambrook et al. *supra*).

**Example XII. Transformation of dicots.**

Crop species overexpressing members of the G1792 clade of transcription factor polypeptides have been shown experimentally to produce plants with increased tolerance to disease. This observation indicates that these genes, when overexpresed, will result in larger yields of various plant species, particularly during conditions of biotic stress.

Thus, transcription factor sequences listed in the Sequence Listing recombined into pMEN20 or pMEN65 expression vectors may be transformed into a plant for the purpose of modifying plant traits. The cloning vector may be introduced into a variety of cereal plants by means well known in the art such as, for example, direct DNA transfer or *Agrobacterium tumefaciens*-mediated transformation. It is now routine to produce transgenic plants using most dicot plants (see Weissbach and Weissbach, (1989) *supra*; Gelvin et al. (1990) *supra*; Herrera-Estrella et al. (1983) *supra;* Bevan (1984) *supra*; and Klee (1985) *supra*). Methods for analysis of traits are routine in the art and examples are disclosed above.

Methods for transforming cotton may be found in U.S. Pat. Nos. 5,004,863, 5,159,135 and 5,518,908; for transforming brassica species may be found in U.S. Pat. No. 5,463,174; for transforming peanut plants may be found in Cheng et al. (1996) Plant Cell Rep.15: 653-657, and McKently et al. (1995) Plant Cell Rep. 14: 699-703; and for transforming pea may be found in Grant et al. (1995) Plant Cell Rep. 15: 254-258.

Numerous protocols for the transformation of tomato and soy plants have been previously described, and are well known in the art. Gruber et al. ((1993) in Methods in Plant Molecular Biology and Biotechnology, p. 89-119, Glick and Thompson, eds., CRC Press, Inc., Boca Raton) describe several expression vectors and culture methods that may be used for cell or tissue transformation and subsequent regeneration. For soybean transformation, methods are described by Miki et al. (1993) in Methods in Plant Molecular Biology and Biotechnology, p. 67-88, Glick and Thompson, eds., CRC Press, Inc., Boca Raton; and U.S. Pat. No. 5,563,055, (Townsend and Thomas), issued Oct. 8, 1996.

There are a substantial number of alternatives to *Agrobacterium*-mediated transformation protocols, other methods for the purpose of transferring exogenous genes into soybeans or tomatoes. One such method is microprojectile-mediated transformation, in which DNA on the surface of microprojectile particles is driven into plant tissues with a biolistic device (see, for example, Sanford et al., (1987) Part. Sci. Technol. 5:27-37; Christou et al. (1992) Plant. J. 2: 275-281; Sanford (1993) Methods Enzymol. 217: 483-509; Klein et al. (1987) Nature 327: 70-73; U.S. Pat. No. 5,015,580 (Christou et al), issued May 14,1991; and U.S. Pat. No. 5,322,783 (Tomes et al.), issued Jun. 21,1994.

Alternatively, sonication methods (see, for example, Zhang et al. (1991)Bio/Technology 9: 996-997); direct uptake of DNA into protoplasts using CaCl2 precipitation, polyvinyl alcohol or poly-L-ornithine (see, for example, Hain et al. (1985) Mol. Gen. Genet. 199: 161-168; Draper et al., Plant Cell Physiol. 23: 451-458 (1982)); liposome or spheroplast fusion (see, for example, Deshayes et al. (1985) EMBO J., 4: 2731-2737; Christou et al. (1987) Proc. Natl. Acad. Sci. USA 84: 3962-3966); and electroporation of protoplasts and whole cells and tissues (see, for example, Donn et al.(1990) in Abstracts of VIIth International Congress on Plant Cell and Tissue Culture IAPTC, A2-38: 53; D'Halluin et al. (1992) Plant Cell 4: 1495-1505; and Spencer et al. (1994) Plant Mol. Biol. 24: 51-61) have been used to introduce foreign DNA and expression vectors into plants.

After a plant or plant cell is transformed (and the latter regenerated into a plant), the transformed plant may be crossed with itself or a plant from the same line, a non-transformed or wild-type plant, or another transformed plant from a different transgenic line of plants. Crossing provides the advantages of producing new and often stable transgenic varieties. Genes and the traits they confer that have been introduced into a tomato or soybean line may be moved into distinct line of plants using traditional backcrossing techniques well known in the art. Transformation of tomato plants may be conducted using the protocols of Koornneef et al (1986) In Tomato Biotechnology: Alan R Liss, Inc., 169-178, and in U.S. Patent 6,613,962, the latter method described in brief here. Eight day old cotyledon explants are precultured for 24 hours in Petri dishes containing a feeder layer of Petunia hybrida suspension cells plated on MS medium with 2% (w/v) sucrose and 0.8% agar supplemented with 10 μM α-naphthalene acetic acid and 4.4 μM 6-benzylaminopurine. The explants are then infected with a diluted overnight culture of *Agrobacterium tumefaciens* containing an expression vector comprising a polynucleotide of the invention for 5-10 minutes, blotted dry on sterile filter paper and cocultured for 48 hours on the original feeder layer plates. Culture conditions are as described above. Overnight cultures of *Agrobacterium tumefaciens* are diluted in liquid MS medium with 2% (w/v/) sucrose, pH 5.7) to an $OD_{600}$ of 0.8.

Following cocultivation, the cotyledon explants are transferred to Petri dishes with selective medium comprising MS medium with 4.56 μM zeatin, 67.3 μM vancomycin, 418.9 μM cefotaxime and 171.6 μM kanamycin sulfate, and cultured under the culture conditions described above. The explants are subcultured every three weeks onto fresh medium. Emerging shoots are dissected from the underlying callus and transferred to glass jars with selective medium without zeatin to form roots. The formation of roots in a kanamycin sulfate-containing medium is a positive indication of a successful transformation.

Transformation of soybean plants may be conducted using the methods found in, for example, U.S. Patent 5,563,055. In this method, soybean seed is surface sterilized by exposure to chlorine gas evolved in a glass bell jar. Seeds are germinated by plating on 1/10 strength agar solidified medium without plant growth regulators and culturing at 28° C with a 16 hour day length. After three or four days, seed may be prepared for cocultivation. The seedcoat is removed and the elongating radicle removed 3-4 mm below the cotyledons.

Overnight cultures *of Agrobacterium tumefaciens* harboring the expression vector comprising a polynucleotide of the invention are grown to log phase, pooled, and concentrated by centrifugation. Inoculations are conducted in batches such that each plate of seed was treated with a newly resuspended pellet of *Agrobacterium.* The pellets are resuspended in 20 ml inoculation medium. The inoculum is poured into a Petri dish containing prepared seed and the cotyledonary nodes are macerated with a surgical blade. After 30 minutes the explants are transferred to plates of the same medium that has been solidified. Explants are embedded with the adaxial side up and level with the surface of the medium and cultured at 22° C for three days under white fluorescent light. These plants may then be regenerated according to methods well established in the art, such as by moving the explants after three days to a liquid counter-selection medium (see U.S. Patent 5,563,055).

The explant may then be picked, embedded and cultured in solidified selection medium. After one month on selective media transformed tissue becomes visible as green sectors of regenerating tissue against a background of bleached, less healthy tissue. Explants with green sectors are transferred to an elongation medium. Culture is continued on this medium with transfers to fresh plates every two weeks. When shoots are 0.5 cm in length they may be excised at the base and placed in a rooting medium.

**Example XIII: Altered abiotic stress tolerance in monocots**

Cereal plants such as, but not limited to, corn, wheat, rice, sorghum, or barley, may be transformed with the present polynucleotide sequences, including monocot or dicot-derived sequences such as those presented in Tables 1, 3, or 6, cloned into a vector such as pGA643 and containing a kanamycin-resistance marker, and expressed constitutively under, for example, the CaMV 35S or COR15 promoters. pMEN20 or pMEN65 and other expression vectors may also be used for the purpose of modifying plant traits. For example, pMEN020 may be modified to replace the NptII coding region with the BAR gene of *Streptomyces hygroscopicus* that confers resistance to phosphinothricin. The KpnI and BglII sites of the Bar gene are removed by site-directed mutagenesis with silent codon changes.

The cloning vector may be introduced into a variety of cereal plants by means well known in the art including direct DNA transfer or *Agrobacterium tunefaciens*-mediated transformation. The latter approach may be accomplished by a variety of means, including, for example, that of U.S. Patent No. 5,591,616, in which monocotyledon callus is transformed by contacting dedifferentiating tissue with the *Agrobacterium* containing the cloning vector.

The sample tissues are immersed in a suspension of $3x10^{-9}$ cells of *Agrobacterium* containing the cloning vector for 3-10 minutes. The callus material is cultured on solid medium at 23° C in the dark for several days. The calli grown on this medium are transferred to Regeneration medium. Transfers are continued every 2-3 weeks (2 or 3 times) until shoots develop. Shoots are then transferred to Shoot-Elongation medium every 2-3 weeks. Healthy looking shoots are transferred to rooting medium and after roots have developed, the plants are placed into moist potting soil.

The transformed plants are then analyzed for the presence of the NPTII gene/ kanamycin resistance by ELISA, using the ELISA NPTII kit from 5Prime-3Prime Inc. (Boulder, CO).

It is also routine to use other methods to produce transgenic plants of most cereal crops (Vasil (1994) Plant Mol. Biol. 25: 925-937) such as corn, wheat, rice, sorghum (Cassas et al. (1993) Proc. Natl. Acad. Sci. USA 90: 11212-11216, and barley (Wan and Lemeaux (1994) Plant Physiol. 104:37-48). DNA transfer methods such as the microprojectile method can be used for corn (Fromm et al. (1990) Bio/Technol. 8: 833-839); Gordon-Kamm et al. (1990) Plant Cell 2: 603-618; Ishida (1990) Nature Biotechnol. 14:745-750), wheat (Vasil et al. (1992) Bio/Technol. 10:667-674; Vasil et al. (1993). Bio/Technol. 11:1553-1558; Weeks et al. (1993) Plant Physlol. 102:1077-1084), and rice (Christou (1991) Bio/Technol. 9:957-962; Hiei et al. (1994) Plant J. 6:271-282; Aldemita and Hodges (1996) Planta 199:612-617; and Hiei et al. (1997) Plant Mol. Biol. 35:205-218). For most cereal plants, embryogenic cells deprived from immature scutellum tissues are the preferred cellular targets for transformation (Hiei et aL (1997) Plant Mol. Biol. 35:205-218; Vasil (1994) Plant Mol. Biol. 25: 925-937). For transforming corn embryogenic cells derived from immature scutellar tissue using microprojectile bombardment, the A188XB73 genotype is the preferred genotype (Fromm et al. (1990) Bio/ Technol. 8: 833-839; Gordon-Kamm et al. (1990) Plant Cell 2: 603-618). After microprojectile bombardment the tissues are selected on phosphinothricin to identify the transgenic embryogenic cells (Gordon-Kamm et al. (1990) Plant Cell 2: 603-618). Transgenic plants are regenerated by standard corn regeneration techniques (Fromm et al. (1990) Biol/ Technol. 8: 833-839; Gordon-Kamm et al. (1990) Plant Cell 2: 603-618).

Northern blot analysis, RT-PCR or microarray analysis of the regenerated, transformed plants may be used to show expression of G1792 and related genes that are capable of conferring tolerance to biotic or abiotic stress.

To verify the ability to confer abiotic stress tolerance, mature plants overexpressing a transcription factor of the invention, or alternatively, seedling progeny of these plants, may be challenged in an abiotic stress assay, such as a drought, heat, high salt, or freezing assay, in an osmotic stress conditions that may also measure altered sugar sensing, such as a high sugar condition, in a shade tolerance assay, or in a C/N sensing assay to identify plants with altered stress or shade tolerance of altered C/N sensing. By comparing wild type and transgenic plants similarly treated, the transgenic plants may be shown to have greater tolerance to abiotic stress.

After a monocot plant or plant cell has been transformed (and the latter regenerated into a plant) and shown to have greater tolerance to biotic or abiotic stress, or produce greater yield relative to a control plant under the stress conditions, the transformed monocot plant may be crossed with itself or a plant from the same line, a non-traasfonned or wild-type monocot plant, or another transformed monocot plant from a different transgenic line of plants.

**Example XIV Genes that Confer Significant Improvements to *non-Arabidopsis* species**

The function of specific orthologs of transcription factors of the invention has been analyzed and may be further characterized by incorporation into crop plants. The function of specific orthologs of the sequences in the Sequence Listing may be analyzed through their altered expression (e.g., ectopic overexpression, or knocking out) in plants, using constitutive, inducible, or tissue specific regulatory elements, as disclosed above. These sequences include polynucleotide sequences found in the Sequence Listing such as, for example; SED ID NO:12 (G2133).

The polynucleotide and polypeptide sequences derived from monocots may be used to random both monocot and dicot plants, and those derived from dicots may be used to transform either group, although some of these sequences will function best if the gene is transformed into a plant from the same group as that from which the sequence is derived.

Transformation procedures are provided in these Examples, and may employ the use of an expression vector. After the vector is introduced into a plant cell, a plant may be regenerated from the cell, alter which the plant is allowed to overexpress one of the polypeptides of the invention that have the property of increasing abiotic stress tolerance, shade tolerance, or altered C/N sensing in the transgenic plant. Plants with these altered traits may be identified by comparison with wild-type or non-transformed plants that do not overexpress the polypeptide, after which one or more plant with a ' desirable degree of one or more improved traits may be selected. In this manner, plants with enhanced shade tolerance, increased abiotic stress tolerance, altered C/N sensing, or more than one of these altered traits may be selected.

For drought tolerance-related analysis, seeds of these transgenic plants are subjected to germination assays to measure sucrose sensing. Sterile monocot seeds, including, but not limited to, corn, rice, wheat, rye and sorghum, as well as dicots including, but not limited to soybean and alfalfa, are sown on 80% MS medium plus vitamin with 9.4% sucrose; control media lack sucrose. All assay plates are then incubated at 22° C under 24-hour light, 120-130 $\mu$Ein/$m^2$/s, in a growth chamber. Evaluation of germination, and seedling vigor is then conducts three days after planting. Overexpressors of these sequences may be found to be more tolerant to high sucrose by having better germination, longer radicles, and more cotyledon expansion. These results would indicate that overexpressors of the orthologs in the Sequence Listing are involved in sucrose-specific sugar sensing.

Plants overexpressing these orthologs may also be subjected to soil-based drought assays to identify those lines that are more tolerant to water deprivation than wild-type control plants. Generally, ortholog overexpressing plants will appear significantly larger and greener, with less wilting or desiccation, than wild-type controls plants, particularly after a period of water deprivation is followed by rewatering and a subsequent incubation period.

Plants overexpressing these orthologs may also be subjected to abiotic stress assays to identify those lines that are more tolerant to abiotic stresses than wild-type control plants in these conditions. Generally, ortholog overexpressing plants will show morphological features that are associated with a shade avoidance phenotype (e.g., altered cotyledon, altered hypocotyl, altered leaf orientation, altered petiole, and/or constitutive photomorphogenesis), and may also appear larger, greener, and healthier than wild-type controls plants.

**Example XV Identification of Orthologous and Paralogous Sequences**

Orthologs to Ambidopsis genes may identified by several methods, including hybridization amplification, or bioinformatically. This example describes how one may identify homologs to the Arabidopsis AP2 family transcription factor CBF1, which confers tolerance to abiotic stresses (Thomashow et al. (2002) US Patent No. 6,417,428), and an example to confirm the function of homologous sequences. In this example, orthologs to CBF1 were found in canola (*Brassica napus*) using polymerase chain reaction (PCR).

Degenerate primer were designed for regions of AP2 binding domain and outside of the AP2 (carboxyl terminal domain; US Patent No. 6,417,428):

Mol 368 (reverse) 5'- CAY CCN ATH TAY MGN GGN GT -3'

Mol 378 (forward) 5'- GGN ARN ARC ATN CCY TCN GCC -3'

(Y: C/T, N: A/C/G/T, H: A/C/T, M: A/C, R: A/G)

Primer Mol 368 is in the AP2 binding domain of CBF1 (amino acid sequence: His-Pro-Ile-Tyr-Arg-Gly-Val) while primer Mol 378 is outside the AP2 domain. (carboxyl terminal domain) (amino acid sequence: Met-Ala-Glu-Gly-Met-Leu-Pro).

The genomic DNA isolated from *B. napus* was PCR-amplified by using these primers following these conditions: an initial denaturation step of 2 min at 93° C; 35 cycles of 93° C for 1 min, 55° C for min, and 72°C for 1 min ; and a final incubation of 7 min at 72° C at the end of cycling.

The PCR products were separated by electrophoresis on a 1.2% agarose gel and transferred to nylon membrane and hybridized with the AT CBF1 probe prepared from *Arabidopsis* genomic DNA by PCR amplification. The hybridized products were visualized by colorimetric detection system (Boehringer Mannheim) and the corresponding bands from a similar agarose gel were isolated using the Qiagen Extraction Kit (Qiagen). The DNA fragments were ligated into the TA clone vector from TOPO TA Cloning Kit (Invitrogen) and transformed into *E. coli* strain TOP10 (Invitrogen).

Seven colonies were picked and the inserts were sequenced on an ABI 377 machine from both strands of sense and antisense after plasmids DNA isolation. The DNA sequence was edited by sequencer and aligned with the AtCBF1 by GCG software and NCBI blast searching.

The nucleic acid sequence and amino acid sequences of one canola ortholog found in this manner (bnCBF1; US Patent No: 6,417,428) identified by this process is shown in the Sequence Listing.

The aligned amino acid sequences show that the bnCBF1 gene has 88% identity with the *Arabidopsis* sequence in the AP2 domain region and 85% identity with the *Arabidopsis* sequence outside the AP2 domain when aligned for two insertion sequences that are outside the AP2 domain.

Similarly, paralogous sequences to *Arabidopsis* genes, such as *CBF1,* may also be identified.

Two paralogs of CBF1 from *Arabidopsis thaliana*: *CBF2* and *CBF3. CBF2* and *CBF3* have been cloned and sequenced as described below. The sequences of the DNA and encoded proteins are set forth in US Patent No. 6,417,428.

A lambda cDNA library prepared from RNA isolated from *Arabidopsis thaliana* ecotype Columbia (Lin and Thomashow (1992) Plant Physiol. 99: 519-525) was screened for recombinant clones that carried inserts related to the *CBF1* gene (Stockinger et al. (1997) Proc. Natl. Acad. Sci. 94: 1035-1040). CBF1 was $^{32}$P-radiolabeled by random priming (Sambrook et al. *supra*) and used to screen the library by the plaque-lift technique using standard stringent hybridization and wash conditions (Hajela et al. (1990) Plant Physiol. 93:1246-1252; Sambrook et al. *supra)* 6 X SSPE buffer, 60° C for hybridization and 0.1 X SSPE buffer and 60° C for washes). Twelve positively hybridizing clones were obtained and the DNA sequences of the cDNA inserts were determined. The results indicated that the clones fell into three classes. One class carried inserts corresponding to *CBF1.* The two other classes carried sequences corresponding to two different homologs of *CBF1,* designated *CBF2* and *CBF3.* The nucleic acid sequences and predicted protein coding sequences for *Arabidopsis CBF1, CBF2, CBF3,* and the *Brassica napus CBF* ortholog are set forth in US Patent No. 6,417,428.

A comparison of the nucleic acid sequences of *arabidopsis CBF1, CBF2* and *CBF3* indicate that they are 83 to 85% identical as shown in Table 9.

TABLE 9

| | Percent identity[a] | |
|---|---|---|
| | DNA[b] | Polypeptide |
| cbf1/cbf2 | 85 | 86 |
| cbf1/cbf2 | 83 | 84 |
| cbf2/cbf3 | 84 | 85 |

[a]Percent identity was determined using the *Clustal* algorithm from the MEGALIGN program (DNASTAR, Inc.).
[b]Comparisons of the nucleic acid sequences of the open reading frames are shown.

Similarly, the amino acid sequences of the three CBP polypeptides range from 84 to 86% identity. An alignment of the three amino acid sequences reveals that most of the differences in amino acid sequence occur in the acidic C-terminal half of the polypeptide. This region of CBF1 serves as an activation domain in both yeast and *Arabidopsis* (not shown).

Residues 47 to 106 of CBF1 correspond to the AP2 domain of the protein, a DNA binding motif that to date, has only been found in plant proteins. A comparison of the AP2 domains of CBF1, CBF2 and CBF3 indicates that there are a few differences in amino acid sequence. These differences in amino acid sequence might have an effect on DNA binding specificity.

**Example XVI Transformation of Canola with a Plasmid Containing CBF1, CBF2, or CBF3**

After identifying homologous genes to CHF1, canola was transformed with a plasmid containing the *Arabidopsis* CBF1, CBF2, or CBF3 genes cloned into the vector pGA643 (An (1987) Methods Enzymol. 253: 292). In these constructs the CBF genes were expressed constitutively under the CaMV 35S promoter in addition, the CBF1 gene was cloned under the controls of the *Arabidopsis* COR15 promoter in the same vector pGA643. Each construct was transformed into *Agrobacterium* strain GV3101. Transformed Agrobacteria were grown for 2 days in minimal AB medium containing appropriate antibiotics.

Spring canola (B. *napus* cv. Westar) was transformed using the protocol of Moloney et al. ((1989) Plant Cell Reports 8: 238) with some modifications as described. Briefly, seeds were sterilized and plated on half strength MS medium, containing 1% sucrose. Plates were incubated at 24° C under 60-80 $\mu E/m^2 s$ light using a 16 hour light/8 8 hour dark photoperiod. Cotyledons from 4-5 day old seedlings were collected, the petioles cut and dipped into the *Agrobacterium* solution. The dipped cotyledons were placed on co-cultivation medium at a density of 20 cotyledons/plate and incubated as described above for 3 days. Explants were transferred to the same media, but containing 300 mg/l timentin (SmithKline Beecham, PA) and thinned to 10 cotyledons/plate. After 7 days explants were transferred to Selection/Regeneration medium. Transfers were continued every 2-3 weeks (2 or 3 times) until shoots had developed. Shoots were transferred to Shoot-Elongation medium every 2-3 weeks. Healthy looking shoots were transferred to rooting medium. Once good roots had developed, the plants were placed into moist potting soil.

The transformed plants were then analyzed for the presence of the NPTII gene/ kanamycin resistance by ELISA, using the ELISA NPTII kit from 5Prime-3Prime Inc. (Boulder, CO). Approximately 70% of the screened plants were NPTII positive. Only those plants were further analyzed.

From Northern blot analysis of the plants that were transformed with the constitutively expressing constructs, showed expression of the CBF genes and all CBF genes were capable of inducing the *Brassica napus* cold-regulated gene BN115 (homolog of the *Arabidopsis* COR15 gene). Most of the transgenic plants appear to exhibit a normal growth phenotype. As expected, the transgenic plants are more freezing tolerant than the wild-type plants. Using the electrolyte leakage of leaves test, the control showed a 50% leakage at-2 to -3° C. Spring canola transformed with either CBF1 or CBF2 showed a 30% leakage at-6 to -7° C. Spring canola transformed with CBF3 shows a 50% leakage at about -10 to -15° C. Winter canola transformed with CBF3 may show a 50% leakage at about -16 to-20° C. Furthermore, if the spring or winter canola are cold acclimated the transformed plants may exhibit a further increase in freezing tolerance of at least -2˚C.

To test salinity tolerance of the transformed plants, plants were watered with 150 mM NaCl. Plants overexpressing CBF1, CBF2 or CBF3 grew better compared with plants that had not been transformed with CBF1, CBF2 or CBF3.

These results demonstrate that homologs of *Arabidopsis* Transcription factors can be identified and shown to confer similar functions in non-*Arabidopsis* plant species.

**Example XVII Cloning of transcription factor promoters**

Promoters are isolated from transcription factor genes that have gene expression patterns useful for a range of applications, as determined by methods well known in the art (including transcript profile analysis with cDNA or oligonucleotide microarrays, Northern blot analysis, semi-quantitative or quantitative RT-PCR). Interesting gene expression profiles are revealed by determining transcript abundance for a selected transcription factor gene after exposure of plants to a range of different experimental conditions, and in a range of different tissue or organ types, or developmental stages. Experimental conditions to which plants are exposed for this purpose includes cold, heat, drought, osmotic challenge, varied hormone concentrations (ABA, GA, auxin, cytokinin, salicylic acid, brassinosteroid), Pathogen and pest challenge. The tissue types and developmental stages include stern, root, flower, rosette leaves, cauline leaves, siliques, germinating seed, and meristematic tissue. The set of expression levels provides a pattern that is determined by the regulatory element of the gene promoter.

Transcription factor promoters for the genes disclosed herein are obtained by cloning 1.5 kb to 2.0 kb of genomic sequence immediately upstream of the translation start codon for the coding sequence of the encoded transcription factor protein. This region includes the 5'-UTR of the transcription factor gene, which can comprise regulatory elements. The 1.5 kb to 2.0 kb region is cloned through PCR methods, using primers that include one in the 3' direction located at the translation start codon (including appropriate adaptor sequence), and one in the 5' direction located from 1.5 kb to 2.0 kb upstream of the translation start codon (including appropriate adaptor sequence). The desired fragments are PCR-amplified from *Arabidopsis* Col-0 genomic DNA using high-fidelity Taq DNA polymerase to minimize the incorporation of point mutation(s). The cloning primers incorporate two rare restriction sites, such as Notl and Sfi1, found at low frequency throughout the *Arabidopsis* genome. Additional restriction sites are used in the instances where a Not1 or Sfi1 restriction site is present within the promoter.

The 1.5-2.0 kb fragment upstream from the translation start codon, including the 5'-untranslated region of the transcription factor, is cloned in a binary transformation vector immediately upstream of a suitable reporter gene, or a transactivator gene that is capable of programming expression of a reporter gene in a second gene construct. Reporter genes used include green fluorescent protein (and related fluorescent protein color variants), β-glucuronidase, and luciferase. Suitable transactivator genes includes LexA-GAL4, along with a transactivatable reporter in a second binary plasmid (as disclosed in US patent application 09/958,131, incorporated herein by reference). The binary plasmid(s) is transferred into *Agrobacterium* and the structure of the plasmid confirmed by PCR. These strains are introduced into *Arabidopsis* plants as described in other examples, and gene expression patterns determined according to standard methods know to one skilled in the art for monitoring GFP fluorescence, β-glucuronidase activity, or luminescence.

The present invention is not limited by the specific embodiments described herein. The invention now being fully described, it will be apparent to one of ordinary skill in the art that many changes and modification can be made thereto without departing from scope of the appended claims. Modifications that become apparent from the foregoing description and accompanying figures fall within the scope of the claims.

## Claims

1. A method for producing a transgenic plant having greater tolerance to water deprivation than a control plant, the method steps comprising:

   (a) producing an expression vector comprising a nucleotide sequence encoding a polypeptide comprising a conserved domain with at least 70% amino acid sequence identity to amino acid coordinates 10-77 of SEQ ID NO: 12; wherein the polypeptide has a property of SEQ ID NO: 12 of increasing tolerance to water deprivation in a plant relative to the control plant; and
   (b) introducing the expression vector into a target plant to produce a transgenic plant; wherein the polypeptide is overexpressed in the transgenic plant and said overexpression results in the transgenic plant having greater tolerance to water deprivation than the control plant.

2. The method of claim 1, wherein the conserved domain has at least 89% sequence identity to amino acid coordinates 10-77 of SEQ ID NO: 12.

3. The method of claim 1 or 2, wherein the expression vector comprises a constitutive, inducible, or tissue-specific promoter operably linked to the nucleotide sequence.

4. A transgenic seed which comprises an expression vector comprising a nucleotide sequence encoding a polypeptide comprising a conserved domain with at least 70% amino acid sequence identity to amino acid coordinates 10-77 of SEQ ID NO:12 produced by a transgenic plant produced by any of the methods of claims 1 to 3, wherein a progeny plant grown from the transgenic seed has greater tolerance to drought relative to a control plant; and wherein said sequence is operably linked to a promoter which is inducible in response to heat or drought.

5. A seed, fruit, leaf, root, or progeny of a plant comprising an expression vector comprising a nucleotide sequence encoding a polypeptide comprising a conserved domain with at least 70% amino acid sequence identity to amino acid coordinates 10-77 of SEQ ID NO: 12; wherein the polypeptide has a property of SEQ ID NO: 12 of increasing tolerance to water deprivation in a plant relative to the control plant obtainable by the method of any one of claims 1 to 3, wherein said plant, seed, fruit, leaf, root, plant cell or progeny has greater tolerance to water deprivation than the control plant, and wherein said sequence is operably linked to a promoter which is inducible in response to heat or drought stress.

6. Use of an expression vector to produce a transgenic plant having greater tolerance to water deprivation relative to a control plant, wherein the expression vector encodes a polypeptide comprising a conserved domain with at least 70% amino acid sequence identity to amino acid coordinates 10-77 of SEQ ID NO: 12.

## Patentansprüche

1. Verfahren zur Herstellung einer transgenen Pflanze mit höherer Toleranz gegenüber Wasserentzug als eine Kontrollpflanze, wobei das Verfahren die folgenden Schritte umfasst:

(a) das Herstellen eines Expressionsvektors, der eine Nucleotidsequenz umfasst, welche für ein Polypeptid kodiert, das eine konservierte Domäne mit zumindest 70 % Aminosäuresequenzidentität mit den Aminosäurekoordinaten 10 bis 77 aus Seq.-ID Nr. 12 umfasst; worin das Polypeptid die Eigenschaft der Seq.-ID Nr. 12 von im Verhältnis zur Kontrollpflanze höherer Toleranz gegenüber Wasserentzug in einer Pflanze aufweist; und
(b) das Einführen des Expressionsvektors in eine Zielpflanze, um eine transgene Pflanze herzustellen; worin das Polypeptid in der transgenen Pflanze überexprimiert wird und diese Überexpression dazu führt, dass die transgene Pflanze eine höhere Toleranz gegenüber Wasserentzug aufweist als die Kontrollpflanze.

2. Verfahren nach Anspruch 1, worin die konservierte Domäne zumindest 89 % Sequenzidentität mit den Aminosäurekoordinaten 10 bis 77 aus Seq.-ID Nr. 12 aufweist.

3. Verfahren nach Anspruch 1 oder 2, worin der Expressionsvektor einen konstitutiven, induzierbaren oder gewebespezifischen Promotor umfasst, der an die Nucleotidsequenz operabel gebunden ist.

4. Transgene Saat, die einen Expressionsvektor umfasst, welcher eine Nucleotidsequenz umfasst, die für ein Polypeptid kodiert, das eine konservierte Domäne mit zumindest 70 % Aminosäuresequenzidentität mit den Aminosäurekoordinaten 10 bis 77 aus Seq.-ID Nr. 12 umfasst, die von einer durch eines der Verfahren nach den Ansprüchen 1 bis 3 hergestellten transgenen Pflanze produziert wurde, worin eine Abkömmlingspflanze, die aus der transgenen Saat gezüchtet wird, im Verhältnis zu einer Kontrollpflanze eine höhere Toleranz gegenüber Dürre aufweist; und worin die Sequenz an einen Promotor, der in Reaktion auf Hitze- oder Dürrebelastung induzierbar ist, operabel gebunden ist.

5. Saat, Frucht, Blatt, Wurzel oder Abkömmling einer Pflanze, umfassend einen Expressionsvektor, der eine Nucleotidsequenz umfasst, die für ein Polypeptid kodiert, welches eine konservierte Domäne mit zumindest 70 % Aminosäuresequenzidentität mit den Aminosäurekoordinaten 10 bis 77 aus Seq.-ID Nr. 12 umfasst; worin das Polypeptid die Eigenschaft der Seq.-ID Nr. 12 von im Verhältnis zur Kontrollpflanze höherer Toleranz gegenüber Wasserentzug in einer Pflanze aufweist, die durch ein Verfahren nach einem der Ansprüche 1 bis 3 erhältlich ist, worin die Pflanze, die Saat, die Frucht, das Blatt, die Wurzel, eine Pflanzenzelle oder der Abkömmling eine höhere Toleranz gegenüber Wasserentzug aufweist als die Kontrollpflanze und worin die Sequenz an einen Promotor, der in Reaktion auf Hitze- oder Dürrebelastung induzierbar ist, operabel gebunden ist.

6. Verwendung eines Expressionsvektors zur Herstellung einer transgenen Pflanze mit im Verhältnis zu einer Kontrollpflanze höherer Toleranz gegenüber Wasserentzug, worin der Expressionsvektor für ein Polypeptid kodiert, das eine konservierte Domäne mit zumindest 70 % Aminosäuresequenzidentität mit den Aminosäurekoordinaten 10 bis 77 aus Seq.-ID Nr. 12 umfasst.

## Revendications

1. Méthode de production d'une plante transgénique possédant une plus grande tolérance à la privation d'eau qu'une plante de contrôle, les étapes de la méthode comprenant:

   (a) produire un vecteur d'expression comprenant une séquence de nucléotides codant pour un polypeptide comprenant un domaine conservé avec au moins 70% d'identité de séquence d'acides aminés aux coordonnées d'acides aminés 10-77 de la SEQ ID NO: 12; où le polypeptide a une propriété de la SEQ ID NO: 12 à augmenter la tolérance à la privation d'eau dans une plante relativement à la plante de contrôle; et
   (b) introduire le vecteur d'expression dans une plante cible pour produire une plante transgénique; où le polypeptide est surexprimé dans la plante transgénique, et ladite surexpression se traduit par le fait que la plante transgénique a une plus grande tolérance à la privation d'eau de la plante de contrôle.

2. Méthode selon la revendication 1, dans laquelle le domaine conservé a au moins 89% d'identité de séquence avec les coordonnées d'acides aminés 10-77 de la SEQ ID NO: 12.

3. Méthode selon la revendication 1 ou 2, dans laquelle le vecteur d'expression comprend un promoteur constitutif, inductible ou spécifique au tissu fonctionnellement lié à la séquence de nucléotides.

4. Semence transgénique qui comprend un vecteur d'expression comprenant une séquence de nucléotides codant pour un polypeptide comprenant un domaine conservé avec au moins 70% d'identité de la séquence d'acides aminés aux coordonnées d'acides aminés 10-77 de la SEQ ID NO: 12 produite par une plante transgénique produite selon l'une quelconque des méthodes des revendications 1 à 3, où une plante de progéniture qui a poussé à partir de la semence transgénique a une plus grande tolérance à la sécheresse par rapport à une plante de contrôle; et où ladite séquence est fonctionnellement liée à un promoteur qui est inductible en réponse à une contrainte de chaleur ou de sécheresse.

5. Semence, fruit, feuille, racine ou progéniture d'une plante comprenant un vecteur d'expression comprenant une séquence de nucléotides codant pour un polypeptide comprenant un domaine conservé avec au moins 70% d'identité de la séquence des acides aminés aux coordonnées des acides aminés 10-77 de la SEQ ID NO: 12; où le polypeptide a une propriété de SEQ ID NO: 12 à augmenter la tolérance à la privation d'eau dans une plante relativement à la plante de contrôle, pouvant être obtenu par la méthode selon l'une quelconque des revendications 1 à 3, où ladite plante, semence, fruit, feuille, racine, cellule de plante ou progéniture a une plus grande tolérance à la privation d'eau que la plante de contrôle, et où ladite séquence est fonctionnellement liée à un promoteur qui est inductible en réponse à des contraintes de chaleur ou de sécheresse.

6. Utilisation d'un vecteur d'expression pour produire une plante transgénique ayant une plus grande tolérance à la privation d'eau relativement à une plante de contrôle, où le vecteur d'expression code pour un polypeptide comprenant un domaine conservé avec au moins 70% d'identité de la séquence des acides aminés aux coordonnées des acides aminés 10-77 de la SEQ ID NO: 12.

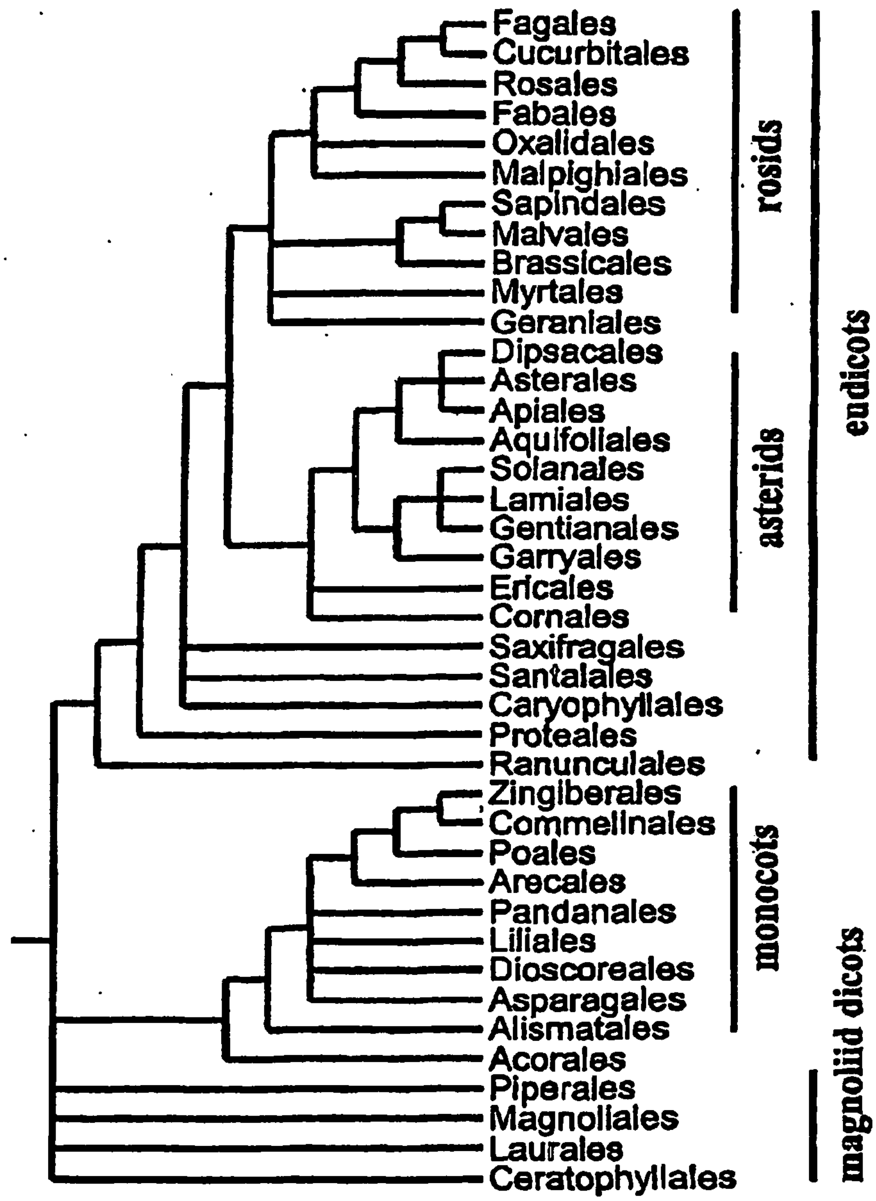
Fagales
Cucurbitales
Rosales
Fabales
Oxalidales
Malpighiales
Sapindales
Malvales
Brassicales
Myrtales
Geraniales
Dipsacales
Asterales
Apiales
Aquifoliales
Solanales
Lamiales
Gentianales
Garryales
Ericales
Cornales
Saxifragales
Santalales
Caryophyllales
Proteales
Ranunculales
Zingiberales
Commelinales
Poales
Arecales
Pandanales
Liliales
Dioscoreales
Asparagales
Alismatales
Acorales
Piperales
Magnoliales
Laurales
Ceratophyllales
rosids
asterids
eudicots
monocots
magnoliid dicots

FIGURE 1

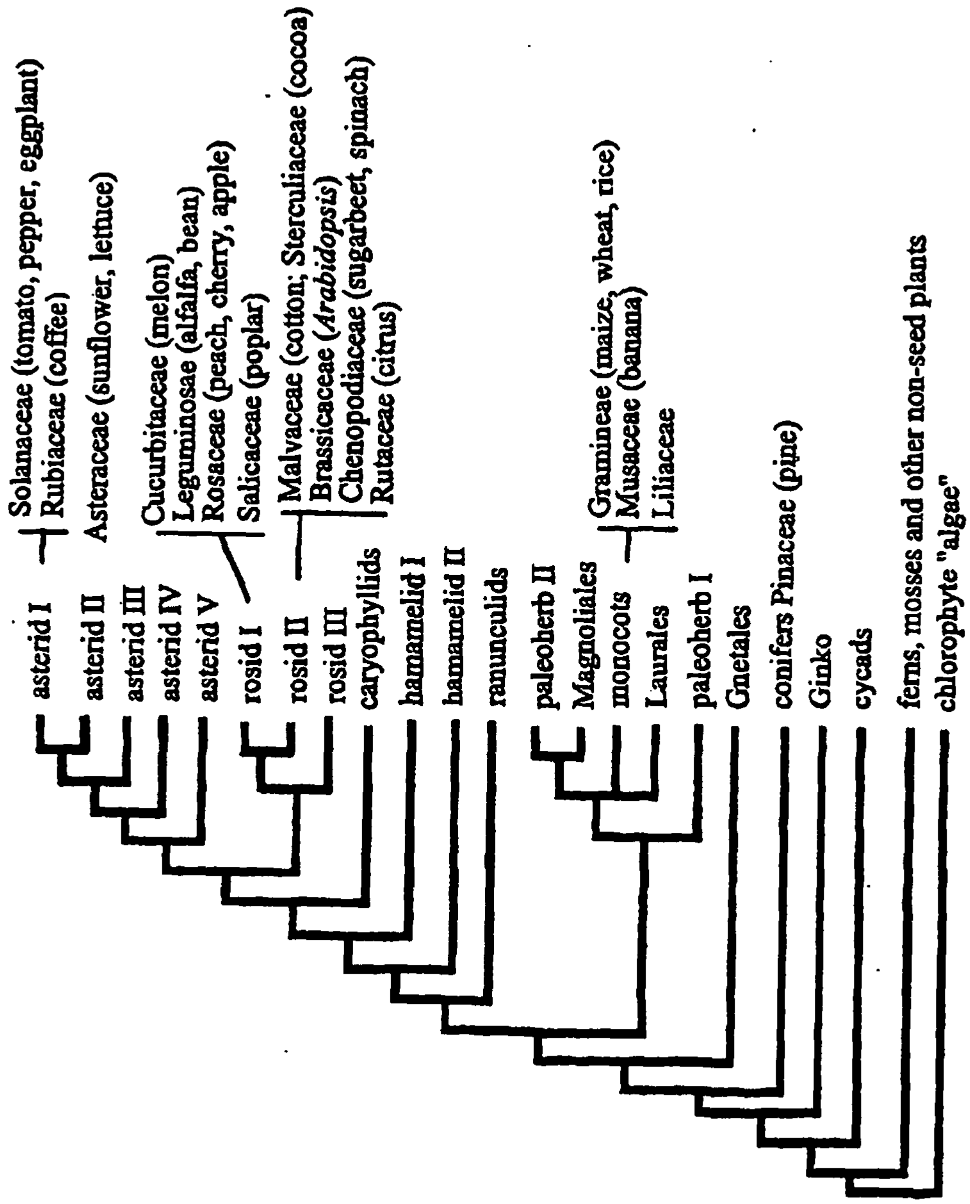
asterid I
asterid II
asterid III
asterid IV
asterid V
rosid I
rosid II
rosid III
caryophyllids
hamamelid I
hamamelid II
ranunculids
paleoherb II
Magnoliales
monocots
Laurales
paleoherb I
Gnetales
conifers Pinaceae (pipe)
Ginko
cycads
ferns, mosses and other non-seed plants
chlorophyte "algae"
Solanaceae (tomato, pepper, eggplant)
Rubiaceae (coffee)
Asteraceae (sunflower, lettuce)
Cucurbitaceae (melon)
Leguminosae (alfalfa, bean)
Rosaceae (peach, cherry, apple)
Salicaceae (poplar)
Malvaceae (cotton; Sterculiaceae (cocoa)
Brassicaceae (*Arabidopsis*)
Chenopodiaceae (sugarbeet, spinach)
Rutaceae (citrus)
Gramineae (maize, wheat, rice)
Musaceae (banana)
Liliaceae

FIGURE 2

| | |
|---|---|
| G2133 | DQSKYKGIRRRKWGKWVSEIRVPGTRQRLWLGSFSTAEGAAVAHDVAFYC |
| G3646 | HQAKYKGIRRRKWGKWVSEIRVPATRERLWLGSFSTAEGAAVAHDVAFYC |
| G3645 | TQSKYKGIRRRKWGKWVSEIRVPGTRDRLWLGSFSTAEGAAVAHDVAFYC |
| G47 | SQSKYKGIRRRKWGKWVSEIRVPGTRDRLWLGSFSTAEGAAVAHDVAFFC |
| G3643 | TNNKLKGVRRRKWGKWVSEIRVPGTQERLWLGTYATPEAAAVAHDVAVYC |
| G3647 | SQKTYKGVRCRRWGKWVSEIRVPGSRERLWLGTYSTPEGAAVAHDVASYC |
| G3648 | SNKKFKGVRRRKWGKWVSEIRVPGTQERLWLGTYATPEAAAVAHDVAFYC |
| G3644 | ERCRYRGVRRRRWGKWVSEIRVPGTRERLWLGSYATPEAAAVAHDTAVYF |
| G3649 | EMMRYRGVRRRRWGKWVSEIRVPGTRERLWLGSYATAEAAAVAHDAAVCL |
| G3650 | RRCRYRGVRRRAWGKWVSEIRVPGTRERLWLGSYAAPEAAAVAHDAAACL |
| G3651 | ERCRYRGVRRRRWGKWVSEIRVPGTRERLWLGSYATPEAAAVAHDTAVYF |
| G3654 | TTTKYRGVRLRKWGKWVSEIRLPNSRERIWLGSYDTPEEAARAFDAAFVC |
| G3652 | KETRYKGVRLRQWGKWVAEIRLPNSRKRIWLGSYYTPEKAARAFDAAFIC |
| G3653 | VERKYRGVRLRQWGKWVAEIRLPNSLKRIWLGSYDSPEKAARAFDAAFIC |
| G3655 | ERRKYKGVRLRQWGKWAAEIRLPSSCERIWLGSYDTPEKAARAFDAAFIC |
| G2576 | MQSKYKGVRKRKWGKWVSEIRLPNSRERIWLGSYDTPEKAARAFDAALYC |
| G872 | MQSKYKGVRKRKWGKWVSEIRLPHSRERIWLGSYDTPEKAARAFDAAQFC |
| G1277 | RERPFKGIRMRKWGKWVAEIREPNKRSRLWLGSYSTPEAAARAYDTAVFY |
| G12 | RDKPYKGIRMRKWGKWVAEIREPNKRSRIWLGSYSTPEAAARAYDTAVFY |
| G24 | DLKPYKGIRMRKWGKWVAEIREPNKRSRIWLGSYATPEAAARAYDTAVFY |
| G867 | PSSKYKGVVPQPNGRWGAQIYEKHQRVWLGTFNEEDEAARAYDVAVHR-- |
| G40 | RHPIYRGVRQRNSGKWVSEVREPNKKTRIWLGTFQTAEMAARAHDVAALA |
| G2294 | RMKKYKGVRMRSWGSWVSEIRAPNQKTRIWLGSYSTAEAAARAYDAALLC |
| G2115 | KIKKYKGVRMRSWGSWVSEIRAPNQKTRIWLGSYSTAEAAARAYDVALLC |

Figure 3

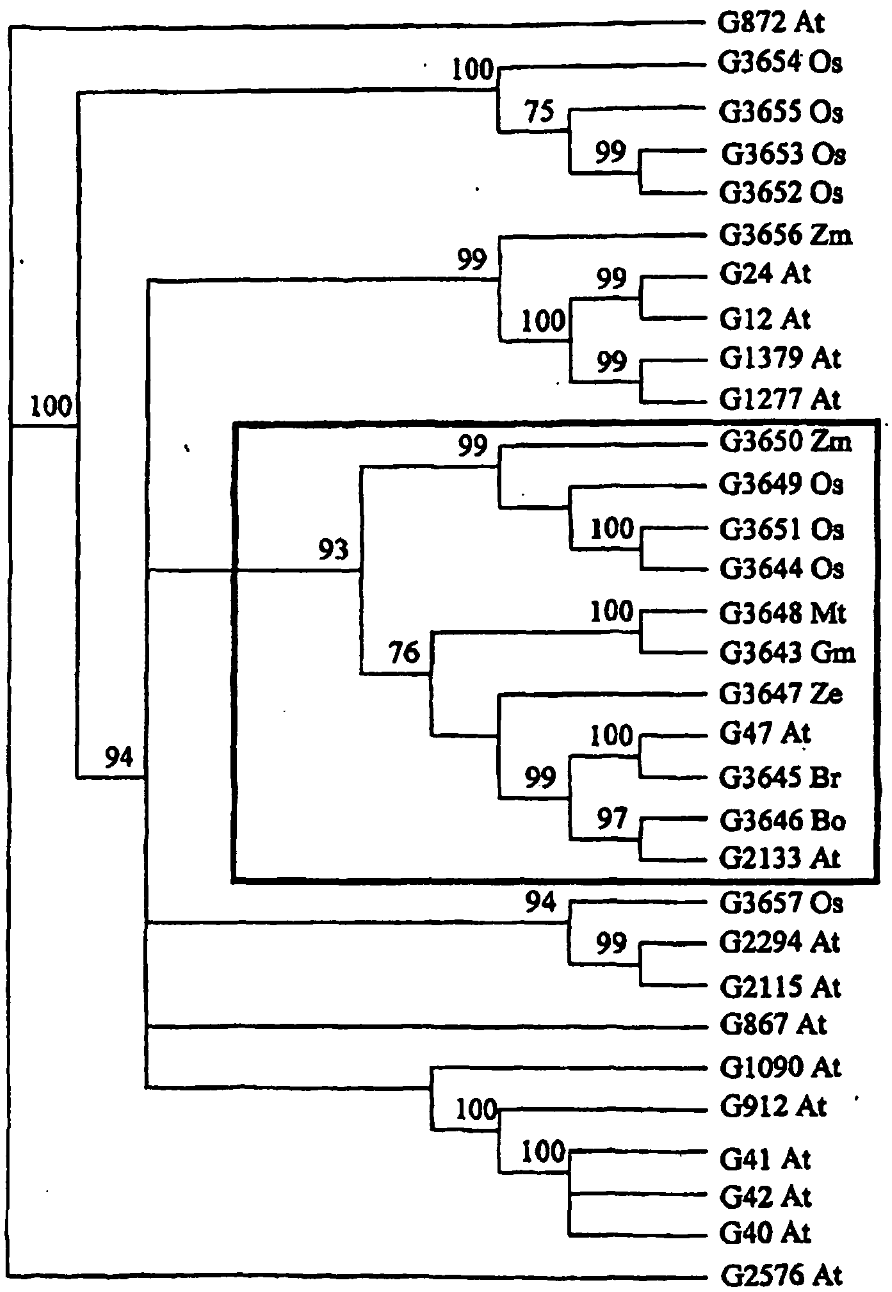
G872 At
100
G3654 Os
75
G3655 Os
99
G3653 Os
G3652 Os
G3656 Zm
99
99
G24 At
100
G12 At
99
G1379 At
100
G1277 At
99
G3650 Zm
G3649 Os
100
G3651 Os
93
G3644 Os
100
G3648 Mt
76
G3643 Gm
G3647 Ze
100
G47 At
94
99
G3645 Br
97
G3646 Bo
G2133 At
94
G3657 Os
99
G2294 At
G2115 At
G867 At
G1090 At
100
G912 At
100
G41 At
G42 At
G40 At
G2576 At

FIGURE 4

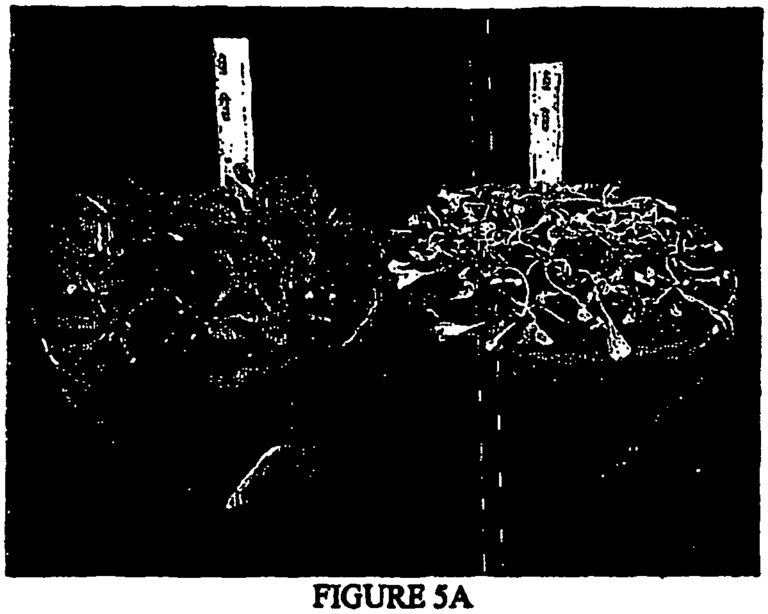

FIGURE 5A

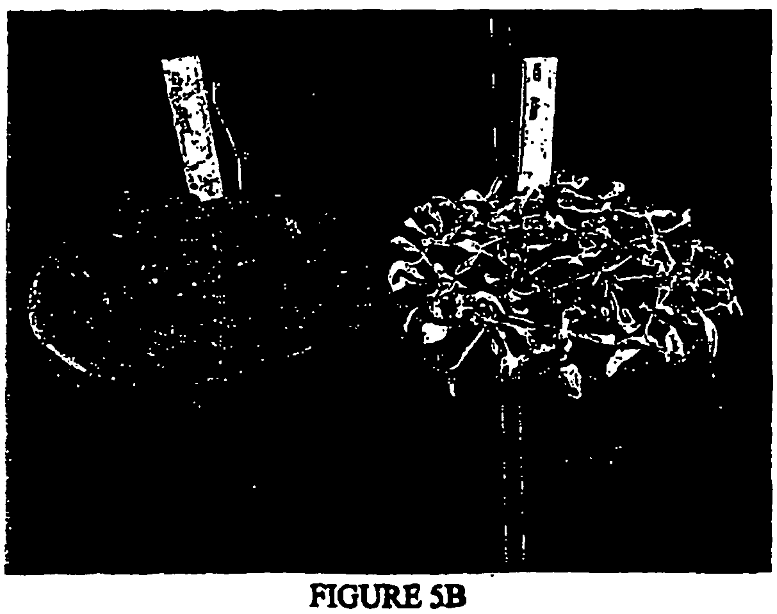

FIGURE 5B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5426039 A, Wallace
- US 6664446 B
- US 20010010913 A
- US 5811238 A
- US 5837500 A
- US 6242568 B
- US 5773697 A
- US 5783393 A
- US 4943674 A
- US 5618988 A
- US 5837848 A
- US 5905186 A
- US 5792929 A
- US 4407956 A
- WO 8501856 A
- US 6417428 B
- EP 271988 A
- US 5231020 A, Jorgensen
- US 5583021 A
- US 5658772 A
- WO 9606166 A
- WO 9853057 A
- US 5571706 A
- US 5677175 A
- US 5510471 A
- US 5750386 A
- US 5597945 A
- US 5589615 A
- US 5750871 A
- US 5268526 A
- US 5780708 A
- US 5538880 A
- US 5773269 A
- US 5736369 A
- US 5610042 A
- US 6521453 B
- US 17773398 A
- US 5004863 A
- US 5159135 A
- US 5518908 A
- US 5463174 A
- US 5563055 A, Townsend and Thomas
- US 5015580 A, Christou
- US 5322783 A, Tomes
- US 6613962 B
- US 5591616 A
- US 09958131 A


### Non-patent literature cited in the description


- The Value of Physiological Knowledge of Water Stress in Plants. Water Stress on Plants. Praeger, 1981, 235-265
- **McCue ; Hanson.** *Trends Biotechnol.,* 1990, vol. 8, 358-362
- **Xiong ; Zhu.** *Plant Cell Environ.,* 2002, vol. 25, 131-139
- **Ohta et al.** *Proc Natl Acad Sci USA,* 2003, vol. 100, 11771-11776
- **Knight.** *Int. Rev. Cytol.,* 2000, vol. 195, 269-324
- **Sanders et al.** *Plant. Cell,* 1999, vol. 11, 691-706
- **Merlot et al.** *Plant J.,* 2001, vol. 25, 295-303
- **Tahtiharju ; Palva.** *Plant J.,* 2001, vol. 26, 461-470
- **Xiong et al.** *Genes Dev.,* 2001, vol. 15, 1971-1984
- **Frank et al.** *Plant Cell,* 2000, vol. 12, 111-124
- **Hasegawa et al.** *Annu. Rev Plant Mol. Plant Physiol.,* 2000, vol. 51, 463-499
- **Saijo et al.** *Plant J.,* 2000, vol. 23, 319-327
- **Gaxiola et al.** *Proc. Natl. Acad. Sci. USA,* 2001, vol. 98, 11444-11449
- Ann. Missouri Bot. Gard. Angiosperm Phylogeny Group, 1998, vol. 84, 1-49
- **Daly et al.** *Plant Physiol,* 2001, vol. 127, 1328-1333
- **Ku et al.** *Proc. Natl. Acad. Sci.,* 2000, vol. 97, 9121-9126
- **Chase et al.** *Ann. Missouri Bot. Gard.,* 1993, vol. 80, 528-580
- **Rieger et al.** Glossary of Genetics and Cytogenetics: Classical and Molecular. Springer Verlag, 1976
- Current Protocols in Molecular Biology. John Wiley & Sons, 1998

- **Kashima et al.** *Nature,* 1985, vol. 313, 402-404
- **Sambrook et al.** Molecular Cloning: A Laboratory Manual. Cold Spring Harbor Laboratory, 1989
- **Hames ; Higgins.** Nucleic Acid Hybridisation: A Practical Approach. IRL Press, 1985
- **Daly et al.** *Plant Physiol.,* 2001, vol. 127, 1328-1333
- **Tudge.** The Variety of Life. Oxford University Press, 2000, 547-606
- **Riechmann et al.** *Science,* 2000, vol. 290, 2105-2110
- **Peng et al.** *Genes Development,* 1997, vol. 11, 3194-3205
- **Peng et al.** *Nature,* 1999, vol. 400, 256-261
- **Fu et al.** *Plant Cell,* 2001, vol. 13, 1791-1802
- **Nandi et al.** *Curr. Biol,* 2000, vol. 10, 215-218
- **Coupland.** *Nature,* 1995, vol. 377, 482483
- **Weigel ; Nilsson.** *Nature,* 1995, vol. 377, 482-500
- **Mandel et al.** *Cell,* 1992, 71-133143
- **Suzuki et al.** *Plant J.,* 2001, vol. 28, 409-418
- **Muller et al.** *Plant J.,* 2001, vol. 28, 169-179
- **Kim et al.** *Plant J.,* 2001, vol. 25, 247-259
- **Kyozuka ; Shimamoto.** *Plant Cell Physiol.,* 2002, vol. 43, 130-135
- **Boss ; Thomas.** *Nature,* 2002, vol. 416, 847-850
- **He et al.** *Transgenic Res.,* 2000, vol. 9, 223-227
- **Robson et al.** *Plant J.,* 2001, vol. 28, 619-631
- **Gilmour et al.** *Plant J.,* 1998, vol. 16, 433-442
- **Jaglo et al.** *Plant Physiol.,* 2001, vol. 127, 910-917
- **Bruce et al.** *Plant Cell,* 2000, vol. 12, 65-79
- **Borevitz et al.** *Plant Cell,* 2000, vol. 12, 2383-93
- **Bhattacharjee et al.** *Proc Natl. Acad. Sci.,* 2001, vol. 98, 13790-13795
- **Xu et al.** *Proc. Natl Acad. Sci.,* 2001, vol. 98, 15089-15094
- **Riechmann ; Meyerowitz.** *Biol. Chem.,* 1998, vol. 379, 633-646
- **Jofuku et al.** *Plant Cell,* 1994, vol. 6, 1211-122
- **Ohme-Takagi et al.** *Plant Cell,* 1995, vol. 7, 173-182
- **Weigel.** *Plant Cell,* 1995, vol. 7, 388-389
- **Okamuro et al.** *Proc Natl. Acad. Sci,* 1997, vol. 94, 7076-7081
- **Kagaya et al.** *Nucleic Acids Res.,* 1999, vol. 27, 470-478
- **Bowman et al.** *Development,* 1991, vol. 112, 1-20
- **Elliott et al.** *Plant Cell,* 1996, vol. 8, 155-168
- **Klucher et al.** *Plant Cell,* 1996, vol. 8, 137-153
- **Moose et al.** *Genes Dev.,* 1996, vol. 10, 3018-3027
- **Zhou et al.** *EMBO J.,* 1997, vol. 16, 3207-3218
- **Stockinger et al.** *Proc. Natl. Acad. Sci. USA,* 1997, vol. 94, 1035-1040
- **Jaglo-Ottosen et al.** *Science,* 1998, vol. 280, 104-106
- **Finkelstein et al.** *Plant Cell,* 1998, vol. 10, 1043-1054
- Guide to Molecular Cloning Techniques. **Berger ; Kimmel.** Methods in Enzymology. Academic Press, Inc, vol. 152
- **Sambrook et al.** Molecula Clonining - A Laboratory Manual. Cold Spring Harbor Laboratory, 1989, vol. 1-3
- Current Protocols in Molecular Biology. Current Protocols, 2000
- **Mullis et al.** PCR Protocols A Guide to Methods and Applications. Academic Press Inc, 1987
- **Cheng et al.** *Nature,* 1994, vol. 369, 684-685
- **Beaucage et al.** *Tetrahedron Letters,* 1981, vol. 22, 1859-1869
- **Matthes et al.** *EMBO J.,* 1984, vol. 3, 801-805
- **Thompson et al.** *Nucleic Acids Res.,* 1994, vol. 22, 4673-4680
- **Higgins et al.** *Methods Enzymol.,* 1996, vol. 266, 383-402
- **Feng ; Doolittle.** *J. Mol. Evol.,* 1987, vol. 25, 351-360
- **Ratcliffe et al.** *Plant Physiol.,* 2001, vol. 126, 122-132
- **Mount.** Bioinformatics: Sequence and Genome Analysis. Cold Spring Harbor Laboratory Press, 2001, 543
- **Thompson et al.** *Nucleic Acids Res.,* 1994, vol. 22, 46734680
- **Goodrich et al.** *Cell,* 1993, vol. 75, 519-530
- **Lin et al.** *Nature,* 1991, vol. 353, 569-571
- **Sadowski et al.** *Nature,* 1988, vol. 335, 563-564
- **Lee et al.** *Genome Res.,* 2002, vol. 12, 493-502
- **Remm et al.** *J. Mol. Biol.,* 2001, vol. 314, 1041-1052
- **Jaglo et al.** *Plant Physiol.,* 1998, vol. 127, 910-917
- **Cao et al.** *Cell,* 1997, vol. 88, 57-63
- **Chern et al.** *Plant J.,* 2001, vol. 27, 101-113
- **Fan ; Dong.** *Plant Cell,* 2002, vol. 14, 1377-1389
- **Kosugi ; Ohashi.** *Plant J.,* 2002, vol. 29, 45-59
- **Gampala et al.** *J. Biol. Chem.,* 2001, vol. 277, 1689-1694

- **Gocal et al.** *Plant Physiol.,* 2001, vol. 127, 1682-1693
- **Nandi et al.** *Curr. Biol.,* 2000, vol. 10, 215-218
- **Borevitz et al.** *Plant Cell,* 2000, vol. 12, 2383-2394
- **Higgins ; Sharp.** *Gene,* 1988, vol. 73, 237-244
- Computer Methods for Macromolecular Sequence Analysis. Methods in Enzymology. Academic Press, Inc, 1996, vol. 266
- **Shpaer.** *Methods Mol. Biol.,* 1997, vol. 70, 173-187
- **Hein.** *Methods Enzymol.,* 1990, vol. 183, 626-645
- **Bairoch et al.** *Nucleic Acids Res,* 1997, vol. 25, 217-221
- **Smith et al.** *Protein Engineering,* 1992, vol. 5, 35-51
- **Altschul.** *J. Mol. Evol.,* 1993, vol. 36, 290-300
- **Altschul et al.** *J. Mol. Biol.,* 1990, vol. 215, 403-410
- **Henikoff ; Henikoff.** *Nucleic Acids Res.,* 1991, vol. 19, 6565-6572
- **Eddy.** *Curr. Opin. Str. Biol.,* 1996, vol. 6, 361-365
- **Sonnhammer et al.** *Proteins,* 1997, vol. 28, 405-420
- **Ausubel et al.** Short Protocols in Molecular Biology. John Wiley & Sons, 1997
- **Meyers.** Molecular Biology and Biotechnology. Wiley VCH, 1995, 856-853
- **Fowler et al.** *Plant Cell,* 2002, vol. 14, 1675-1679
- Quantitative Filter Hybridisation. **Anderson ; Young.** Nucleic Acid Hybridisation, A Practical Approach. IRL Press, 1985, 73-111
- **Wahl ; Berger.** *Methods Enzymol.,* 1987, vol. 152, 399-407
- **Kimmel.** *Methods Enzymol.,* 1987, vol. 152, 507-511
- Nucleic Acid Hybridisation: A Practical Approach. IRL Press, 1985
- **Harlow ; Lane.** Antibodies: A Laboratory Manual. Cold Spring Harbor Laboratory, 1988
- Methods Enzymol. Academic Press, 1993, vol. 217
- **Stemmer.** *Nature,* 1994, vol. 370, 389-391
- **Stemmer.** *Proc. Natl. Acad. Sci.,* 1994, vol. 91, 10747-10751
- **Zhang et al.** *J. Biol. Chem.,* 2000, vol. 275, 33850-33860
- **Liu et al.** *J. Biol. Chem.,* 2001, vol. 276, 11323-11334
- **Isalan et al.** *Nature Biotechnol.,* 2001, vol. 19, 656-660
- **Moore et al.** *Proc. Natl. Acad. Sci.,* 1998, vol. 95, 376-381
- **Aoyama et al.** *Plant Cell,* 1995, vol. 7, 1773-1785
- **Ma ; Ptashne.** *Cell,* 1987, vol. 51, 113-119
- **Giniger ; Ptashne.** *Nature,* 1987, vol. 330, 670-672
- **Weissbach ; Weissbach.** Methods for Plant Molecular Biology. Academic Press, 1989
- **Gelvin et al.** Plant Molecular Biology Manual. Kluwer Academic Publishers, 1990
- **Herrera-Estrella et al.** *Nature,* 1983, vol. 303, 209
- **Bevan.** *Nucleic Acids Res.,* 1984, vol. 12, 8711-8721
- **Klee.** *Bio/Technology,* 1985, vol. 3, 637-642
- **Christou.** *Bio/Technology,* 1991, vol. 9, 957-962
- **Gordon-Kamm.** *Plant Cell,* 1990, vol. 2, 603-618
- **Weeks et al.** *Plant Physiol.,* 1993, vol. 102, 1077-1084
- **Vasil.** *Bio/Technology,* 1993, vol. 10, 667-674
- **Wan ; Lemeaux.** *Plant Physiol.,* 1994, vol. 104, 37-48
- **Ishida et al.** *Nature Biotechnol.,* 1996, vol. 14, 745-750
- **Odell et al.** *Nature,* 1985, vol. 313, 810-812
- **An et al.** *Plant Physiol.,* 1988, vol. 88, 547-552
- **Fromm et al.** *Plant Cell,* 1989, vol. 1, 977-984
- **Bird et al.** *Plant Mol. Biol.,* 1988, vol. 11, 651-662
- **Ringli ; Keller.** *Plant Mol. Biol.,* 1998, vol. 37, 977-988
- **Kaiser et al.** *Plant Mol. Biol.,* 1995, vol. 28, 231-243
- **Baerson et al.** *Plant Mol. Biol.,* 1994, vol. 26, 1947-1959
- **Ohl et al.** *Plant Cell,* 1990, vol. 2, 837-848
- **Baerson et al.** *Plant Mol. Biol.,* 1993, vol. 22, 255-267
- **van der Kop et al.** *Plant Mol. Biol.,* 1999, vol. 39, 979-990
- **Baumann et al.** *Plant Cell,* 1999, vol. 11, 323-334
- **Guevara-Garcia.** *Plant Mol. Biol.,* 1998, vol. 38, 743-753
- **Shi et al.** *Plant Mol. Biol.,* 1998, vol. 38, 1053-1060
- **Willmott et al.** *Plant Molec. Biol.,* 1998, vol. 38, 817-825
- **Ainley et al.** *Plant Mol. Biol.,* 1993, vol. 22, 13-23
- **Kuhlemeier et al.** *Plant Cell,* 1989, vol. 1, 471-478
- **Schaffner ; Sheen.** *Plant Cell,* 1991, vol. 3, 997-1012
- **Siebertz et al.** *Plant Cell,* 1989, vol. 1, 961-968

- **Buchel et al.** *Plant Mol. Biol.,* 1999, vol. 40, 387-396
- **Manners et al.** *Plant Mol. Biol.,* 1998, vol. 38, 1071-1080
- **Gatz.** *Annu. Rev. Plant Physiol. Plant Mol. Biol.,* 1997, vol. 48, 89-108
- **Gan ; Amasino.** *Science,* 1995, vol. 270, 1986-1988
- **Odell et al.** *Plant Physiol.,* 1994, vol. 106, 447-458
- **Fromm et al.** *Proc. Natl. Acad. Sci.,* 1985, vol. 82, 5824-5828
- **Hohn et al.** Molecular Biology of Plant Tumors. Academic Press, 1982, 549-560
- **Klein et al.** *Nature,* 1987, vol. 327, 70-73
- **Horsch et al.** *Science,* 1984, vol. 233, 496-498
- **Fraley et al.** *Proc. Natl. Acad. Sci.,* 1983, vol. 80, 4803-4807
- **Bulyk et al.** *Nature Biotechnol.,* 1999, vol. 17, 573-577
- **Chien et al.** *Proc. Natl. Acad. Sci.,* 1991, vol. 88, 9578-9582
- Methods in Arabidopsis Research. World Scientific, 1992
- **Peng et al.** *Genes and Development,* 1997, vol. 11, 3194-3205
- **Coupland.** *Nature,* 1995, vol. 377, 482-483
- **Hsieh et al.** *Proc. Natl. Acad. Sci.,* 1998, vol. 95, 13965-13970
- Abscisic acid biosynthesis and response. **Finkelstein ; Rock.** The Arabidopsis Book. American Society of Plant Biologists, 2002
- **Xiong ; Ishitani ; Zhu.** *Plant Physiol.,* 1999, vol. 119, 205-212
- **Bostock ; Quatrano.** *Plant Physiol.,* 1992, vol. 98, 1356-1363
- **Ishitani et al.** *Plant Cell,* 1997, vol. 9, 1935-1949
- **Yelenosky.** *Plant Physiol,* 1989, vol. 89, 444-451
- **Siminovitch et al.** *Plant Physiol,* 1982, vol. 69, 250-255
- **Guy et al.** *Planta,* 1992, vol. 188, 265-270
- **Wang et al.** *Acta Hort.,* 2001, vol. 560, 285-292
- **Buchanan et al.** Biochemistry and Molecular Biology of Plants. American Society of Plant Physiologists, 2000
- **Zhu.** *Ann. Rev. Plant Biol.,* 2002, vol. 53, 247-273
- **Kasuga et al.** *Nature Biotech.,* 1999, vol. 17, 287-291
- **Uno et al.** *Proc. Natl. Acad. Sci. USA,* 2000, vol. 97, 11632-11637
- **Patharker ; Cushman.** *Plant J.,* 2000, vol. 24, 679-691
- **Lichtenstein ; Nellen.** Antisense Technology: A Practical Approach. IRL Press at Oxford University Press, 1997
- **Crowley et al.** *Cell,* 1985, vol. 43, 633-641
- **Rosenberg et al.** *Nature,* 1985, vol. 313, 703-706
- **Preiss et al.** *Nature,* 1985, vol. 313, 27-32
- **Melton.** *Proc. Natl. Acad. Sci.,* 1985, vol. 82, 144-148
- **Izant ; Weintraub.** *Science,* 1985, vol. 229, 345-352
- **Kim ; Wold.** *Cell,* 1985, vol. 42, 129-138
- **Smith et al.** *Nature,* 1988, vol. 334, 724-726
- **Smith et al.** *Plant Mol. Biol.,* 1990, vol. 14, 369-379
- **Constans.** *The Scientist,* 2002, vol. 16, 36
- **Zamore.** *Nature Struct. Biol.,* 2001, vol. 8, 746-50
- **Brummelkamp et al.** *Science,* 2002, vol. 296, 550-553
- **Paddison et al.** *Genes & Dev.,* 2002, vol. 16, 948-958
- **Hammond et al.** *Nature Rev Gen,* 2001, vol. 2, 110-119
- **Fire.** *Nature,* 1998, vol. 391, 806-811
- **Timmons ; Fire.** *Nature,* 1998, vol. 395, 854
- **Sharp.** *Genes and Development,* 1999, vol. 13, 139-141
- **Koncz et al.** Methods in Arabidopsis Research. World Scientific Publishing Co. Pte. Ltd, 1992
- **Kempin et al.** *Nature,* 1997, vol. 389, 802-803
- **Ichikawa et al.** *Nature,* 1997, vol. 390, 698-701
- **Kakimoto et al.** *Science,* 1996, vol. 274, 982-985
- Handbook of Plant Cell Culture-Crop Species. Macmillan Publ. Co, 1984
- **Shimamoto et al.** *Nature,* 1989, vol. 338, 274-276
- **Fromm et al.** *Bio/Technol.,* 1990, vol. 8, 833-839
- **Vasil et al.** *Bio/Technol.,* 1990, vol. 8, 429-434
- **Smith ; Waterman.** *Adv. AppL Math.,* 1981, vol. 2, 482-489
- **Needleman ; Wunsch.** *J. Mol. Biol.,* 1970, vol. 48, 443-453
- **Pearson ; Lipman.** *Proc. Natl. Acad. Sci.,* 1988, vol. 85, 2444-2448
- **Henikoff ; Henikoff.** *Proc. Natl. Acad. Sci.,* 1992, vol. 89, 10915-10919
- **Karlin ; Altschul.** *Proc. Natl. Acad. Sci.,* 1993, vol. 90, 5873-5787
- **Winans.** *Microbiol. Rev.,* 1992, vol. 56, 12-31
- **Eyal et al.** *Plant. Mol. Biol.,* 1992, vol. 19, 589-599

- **Chrispeels et al.** *Plant Mol. Biol.,* 2000, vol. 42, 279-290
- **Piazza et al.** *Plant Physiol.,* 2002, vol. 128, 1077-1086
- **O'Neil et al.** *Science,* 1990, vol. 250, 646-651
- **Sanders et al.** *Nucleic Acids Res.,* 1987, vol. 15, 1543-1558
- **Nagel et al.** *FEMS Microbiol Letts,* 1990, vol. 67, 325-328
- **Krysan et al.** *Plant Cell,* 1999, vol. 11, 2283-2290
- **Eisen ; Brown.** *Methods Enzymol.,* 1999, vol. 303, 179-205
- **Altschul et al.** *Nucleic Acid Res.,* 1997, vol. 25, 3389-3402
- **Henikoff ; Henikoff.** *Proc Natl. Acad Sci.,* 1992, vol. 89, 10915-10919
- **Li ; Herskowitz.** *Science,* 1993, vol. 262, 1870-1874
- **Vieira et al.** *Methods Enzymol.,* 1987, vol. 153, 3-11
- **Cheng et al.** *Plant Cell Rep.,* 1996, vol. 15, 653-657
- **McKently et al.** *Plant Cell Rep.,* 1995, vol. 14, 699-703
- **Grant et al.** *Plant Cell Rep.,* 1995, vol. 15, 254-258
- **Gruber et al.** Methods in Plant Molecular Biology and Biotechnology. CRC Press, Inc, 1993, 89-119
- **Miki et al.** Methods in Plant Molecular Biology and Biotechnology. CRC Press, Inc, 1993, 67-88
- **Sanford et al.** *Part. Sci. Technol.,* 1987, vol. 5, 27-37
- **Christou et al.** *Plant. J.,* 1992, vol. 2, 275-281
- **Sanford.** *Methods Enzymol.,* 1993, vol. 217, 483-509
- **Zhang et al.** *Bio/Technology,* 1991, vol. 9, 996-997
- **Hain et al.** *Mol. Gen. Genet.,* 1985, vol. 199, 161-168
- **Draper et al.** *Cell Physiol.,* 1982, vol. 23, 451-458
- **Deshayes et al.** *EMBO J.,* 1985, vol. 4, 2731-2737
- **Christou et al.** *Proc. Natl. Acad. Sci. USA,* 1987, vol. 84, 3962-3966
- **Donn et al.** *Abstracts of VIIth International Congress on Plant Cell and Tissue Culture IAPTC,* 1990, vol. A2-38, 53
- **D'Halluin et al.** *Plant Cell,* 1992, vol. 4, 1495-1505
- **Spencer et al.** *Plant Mol. Biol.,* 1994, vol. 24, 51-61
- **Koornneef et al.** Tomato Biotechnology. Alan R Liss, Inc, 1986, 169-178
- **Vasil.** *Plant Mol. Biol.,* 1994, vol. 25, 925-937
- **Cassas et al.** *Proc. Natl. Acad. Sci. USA,* 1993, vol. 90, 11212-11216
- **Gordon-Kamm et al.** *Plant Cell,* 1990, vol. 2, 603-618
- **Ishida.** *Nature Biotechnol.,* 1990, vol. 14, 745-750
- **Vasil et al.** *Bio/Technol.,* 1992, vol. 10, 667-674
- **Vasil et al.** *Bio/Technol.,* 1993, vol. 11, 1553-1558
- **Weeks et al.** *Physlol.,* 1993, vol. 102, 1077-1084
- **Christou.** *Bio/Technol.,* 1991, vol. 9, 957-962
- **Hiei et al.** *Plant J.,* 1994, vol. 6, 271-282
- **Aldemita ; Hodges.** *Planta,* 1996, vol. 199, 612-617
- **Hiei et al.** *Plant Mol. Biol.,* 1997, vol. 35, 205-218
- **Fromm et al.** *Biol/Technol.,* 1990, vol. 8, 833-839
- **Lin ; Thomashow.** *Plant Physiol.,* 1992, vol. 99, 519-525
- **Stockinger et al.** *Proc. Natl. Acad. Sci.,* 1997, vol. 94, 1035-1040
- **Hajela et al.** *Plant Physiol.,* 1990, vol. 93, 1246-1252
- **An.** *Methods Enzymol.,* 1987, vol. 253, 292
- **Moloney et al.** *Plant Cell Reports,* 1989, vol. 8, 238